# EUROPÄISCHE PATENTSCHRIFT

(11) **EP 2 494 073 B1**
(45) Veröffentlichungstag und Bekanntmachung des Hinweises auf die Patenterteilung: **29.11.2017**
(21) Anmeldenummer: 10784437.5
(22) Anmeldetag: 26.10.2010
(51) Int. Cl.: C12Q 1/68

(54) **KONJUGATE VON NUKLEOTIDEN UND METHODEN ZU DEREN ANWENDUNG**
NUCLEOTIDE CONJUGATES AND METHODS OF USES THEREOF
CONJUGUÉS DE NUCLÉOTIDES ET MÉTHODES D'UTILISATION ASSOCIÉES

(30) Priorität: 26.10.2009 DE 102009050640
(43) Veröffentlichungstag der Anmeldung: 05.09.2012
(73) Patentinhaber: AGCT GmbH, 23562 Lübeck (DE)
(72) Erfinder: Becker, Claus, 76470 Ötigheim (DE); Cerkasov, Dmitry, 35039 Marburg (DE)
(74) Vertreter: Schulz Junghans Patentanwälte PartGmbB
(86) Internationale Anmeldenummer: PCT/EP2010/006523
(87) Internationale Veröffentlichungsnummer: WO 2011/050938

(56) Entgegenhaltungen:
- EP-A1- 1 186 669
- WO-A2-2005/044836
- WO-A2-2006/097320
- WO-A2-2008/043426
- DE-A1-102004 009 704
- DE-A1-102006 012 317
- DE-A1-102007 045 173

## Beschreibung

### Einleitung

### 1.1 Stand der Technik und Aufgaben der Erfindung

Bei der Analyse von Nukleinsäureketten handelt es sich oft um einen Nachweis einer spezifischen Sequenz aus einem Organismus, z.B. Erregernachweis in einem klinischen Material anhand einer spezifischen Sequenz. Diese Sequenz wird oft als Zielsequenz bezeichnet. Solche Zielsequenzen liegen oft nicht isoliert vor, sondern sind in eine Matrize mehr oder weniger eingebettet, das Material. Als Material kann Patientenmaterial, Nahrungsmittel, Teil eines Organismus sein. Die Überprüfung eines Materials auf Vorliegen der Zielsequenz stellt dabei die Zielsetzung der Analyse dar.

Üblicherweise erfolgt eine Extraktion von Nukleinsäuren aus einem Material und eine selektive Vermehrung / Vervielfältigung der Zielsequenz. Dem Fachmann sind viele Methoden zur Isolierung und Vervielfältigung eines spezifischen Abschnittes aus einem Organismus bekannt. Es sind auch Verfahren beschrieben, die einen Direktnachweis aus einem Material ermöglichen, ohne eine vorherige Isolierung der Nukleinsäuren. Die Kontrolle einer erfolgreichen und spezifischen Amplifikation einer Zielsequenz erfolgt üblicherweise durch Hybridisierung einer fluoreszenzmarkierten Sonde (s.g. Real-Time PCR) oder durch andere Hybridisierungsverfahren, z.B. auf einem Microarray, oder mittels Sequenzierung.
Bei bekannten Vorteilen dieser Kontrollmethoden, haben sie auch erhebliche Nachteile, bestehend unter anderem darin, dass sie oft aufwendige apparative Ausstattung voraussetzen und nur spezialisierten Laboratorien vorbehalten werden. Eine Entwicklung der Analysetechniken für robuste Bedingungen (Feldanalysen) stoßen an die Grenzen der aktuellen Nachweismethoden.

Die Aufgabe dieser Anmeldung ist, diesen Engpass zu lösen und Komponenten sowie Verfahren bereitzustellen, die es einem Fachmann ermöglichen, eine spezifische Zielsequenz unter einfachen Bedingungen (ohne Einsatz von Realtime PCR Apparatur oder eines Sequenziergerätes) schnell nachweisen zu können.

Eine weitere Aufgabe der vorliegenden Erfindung ist die Bereitstellung von Nukleotid-Konjugaten die ihre Substrateigenschaften für Polymerasen beibehalten.

Eine weitere Aufgabe der Erfindung ist die Bereitstellung von Verfahren zur Markierung von Nukleinsäuren mit erfindungsgemäß modifizierten Nukleotid-Konjugaten.

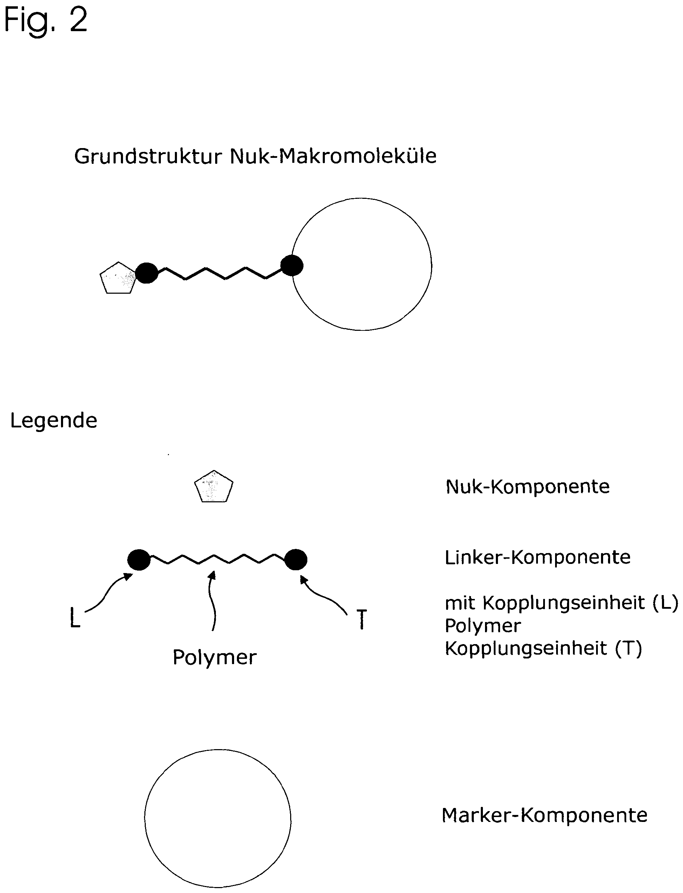

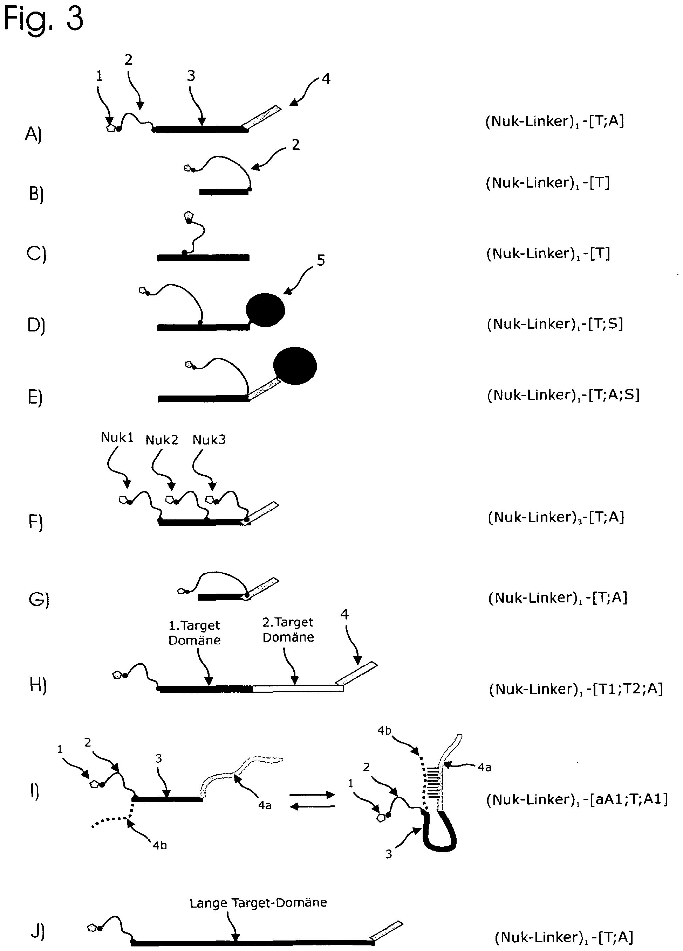

Es werden Nuk-Makromoleküle mit neuen Strukturen der Marker-Kompontente und mit neuen Funktionen bereitgestellt (Fig. 3). Die Nukleotid-Strukturen stellen neue Varianten von Nuk-Makromolekülen mit der Grundstuktur, beschrieben in Anmeldungen Cherkasov et al WO2005044836, Cherkasov et al WO2006097320, Cherkasov et al WO2008043426, Cherkasov et al DE 10356837, Cherkasov et al DE 102004009704 (Fig. 2).

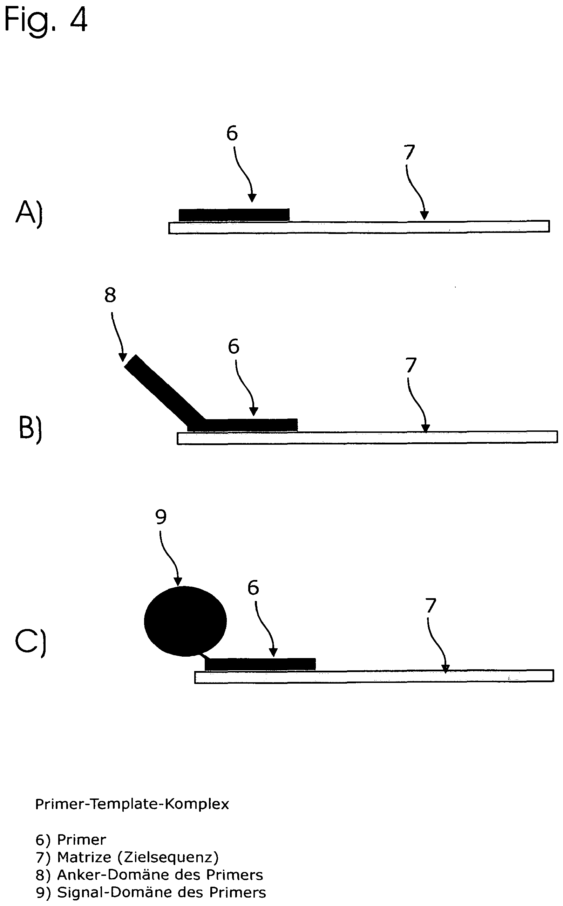

Überraschenderweise ermöglichen diese Strukturen in Kombination mit anderen Assay-Komponenten eine neue Art der Markierungseffizienz, Markierungsspezifität, sowie neue Art für Detektion bei Multiplex-Analysen. Die vorliegende Erfindung offenbart solche vorteilhafte Kombinationen für eine Markierungsreaktion von Zielsequenzen (z.B. Primer, modifizierte Primer, Zielsequenz usw.), (Fig. 4), so dass neue Assays für die Nukleinsäureanalysen aufgebaut werden können. Die erfindungsgemäßen Verfahren erlauben eine größere Vielfalt von Molekülen an die Nukleinsäureketten sequenzspezifisch zu koppeln. Dadurch können viele Methoden in der Nukleinsäureketten-Analytik profitieren.

### 1.2 Kurze Beschreibung der Gegenstände der Erfindung

Der Gegenstand dieser Anmeldung schließt Verfahren zum Nachweis einer oder mehrerer Nukleinsäureketten (eine oder mehrere Zielsequenzen) ein, bei dem ein Konjugat aus mindestens einem Nukleotid-Triphosphat und einem Oligonukleotid verwendet wird, wobei das Oligonukleotid sequenzspezifisch an die Zielsequenz binden kann und das Nukleotid-Triphosphat in durch eine Polymerase in den wachsenden Strang eingebaut werden kann.

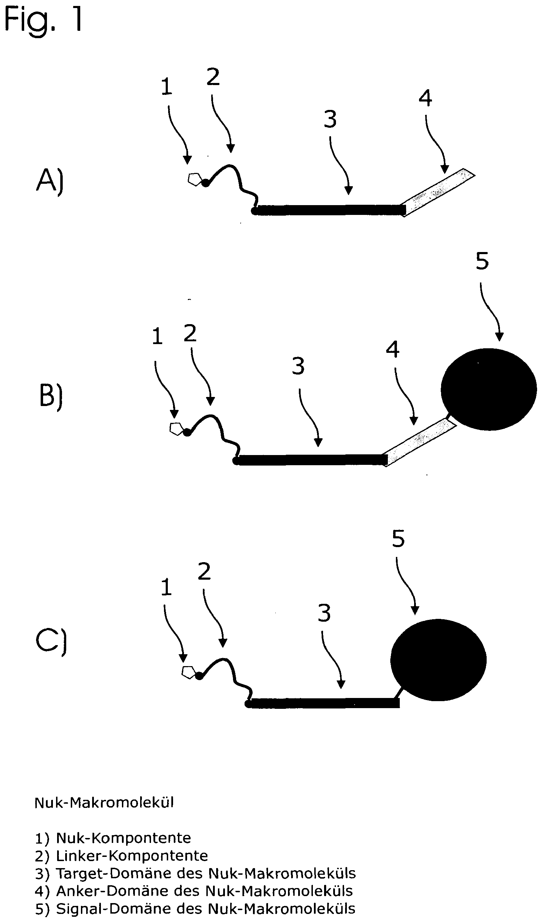

Der Gegenstand dieser Anmeldung schließt Verfahren zum Nachweis einer oder mehrerer Nukleinsäureketten (eine oder mehrere Zielsequenzen) ein, bei denen modifizierte Nukleotide (Nuk-Makromoleküle, Fig. 1) mit einer der folgenden Struktur eingesetzt werden:
(Nuk-Linker)ₙ-Marker
wobei:
Nuk - ein Nukleotid (Nuk-Komponente) ist
Linker - eine Linker-Komponente, die Nuk-Komponente und makromolekulare
Marker-Komponente vebindet ist
Marker - eine Marker-Komponente ist, die mindestens eine zur Zielsequenz vollständig oder teilweise komplementäre Nukleinsäuresequenz einschließt, z.B. ein Oligonukleotid, die sogenannte Target-Domäne
n - eine Zahl von 1 bis 1000 ist.

Ein solches Nuk-Makromolekül ist in der Lage, entsprechend seinen komplementären Eigenschaften sequenzspezifisch an eine Zielsequenz zu binden. Durch eine solche Bindung der Target-Domäne an die Zielsequenz wird eine spezifische Markierungreaktion der zur Zielsequenz komplementären Stränge durch eine Polymerase stark begünstigt: es wird bevorzugt die Nuk-Komponente des gebundenen Nuk-Makromoleküls eingebaut.

Die erfindungsgemäßen Nuk-Makromoleküle unterscheiden sich von den natürlichen Nukleotiden in mehreren Aspekten:
- In einer vorteilhaften Ausführungsform des Verfahrens werden Nuk-Makromoleküle eingesetzt, die zumindest einen Oligonukleotid-Anteil einschließen. Dieser Oligonukleotid-Anteil ist erfindungsgemäß in der Lage, an einer Stelle in der Zielsequenz (Matrize) unter Ausbildung eines komplementären Doppelstranges zu binden. Ein solcher Oligonukleotid-Anteil wird als Target-Domäne bezeichnet und abgekürzt "T-Domäne" genannt (Fig. 1).
- In einer weiteren vorteilhaften Ausführungsform des Verfahrens werden Nuk-Makromoleküle eingesetzt, die zumindest eine Target-Domäne einschließen, sowie zumindest eine Anker-Domäne (abgekürzt "A-Domäne") (Fig. 1). Über eine Anker-Domäne kann ein Nuk-Makromolekül an eine feste Phase gebunden werden. Beispiele für eine Anker-Domäne stellen Biotin oder Oligonukleotide dar. Über Biotin oder Oligonukleotid kann ein Nuk-Makromolekül an eine feste Phase gebunden werden, wenn diese Phase Streptavidin oder ein komplementäres Oligonukleotid einschließt.
- In einer weiteren vorteilhaften Ausführungsform des Verfahrens werden Nuk-Makromoleküle eingesetzt, die zumindest eine Target-Domäne einschließen, sowie zumindest eine Signal-Domäne (abgekürzt "S-Domäne") (Fig. 1). Über eine Signal-Domäne kann ein Nuk-Makromolekül eindeutig identifiziert werden. Beispiele für Signal-Domäne stellen Fluoreszenzfarbstoffe dar.

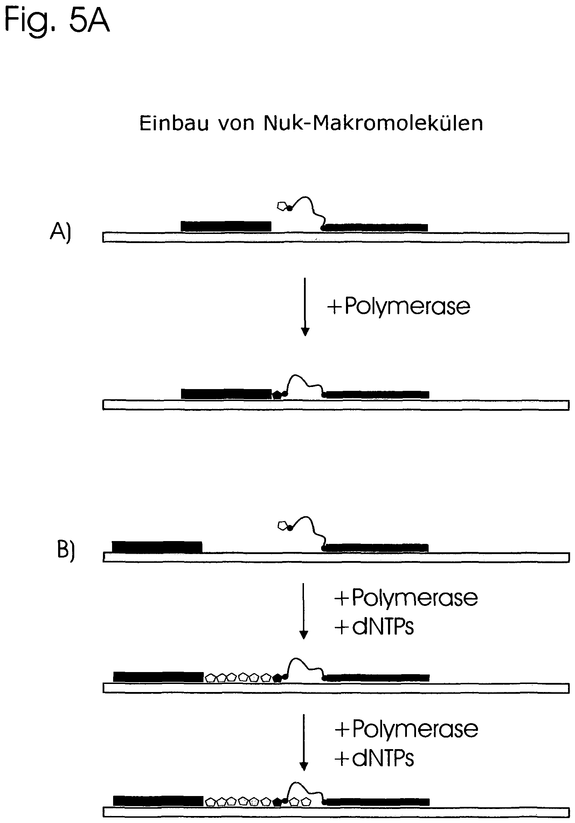

Eine Markierungsreaktion nach einem erfindungsgemäßen Verfahren wird beispielsweise wie folgt durchgeführt (Fig. 5A): Es werden mindestens eine Zielsequenz (eine Nukleinsäuren-Matrize), mindestens ein Primer, mindestens eine Polymerase, sowie mindestens eine Art der oben genannten Nuk-Makromoleküle vorgelegt und unter Bedingungen inkubiert, die einen enzymatischen Einbau der Nuk-Kompontente des Nuk-Makromoleküls in den wachsenden Strang erlauben. Durch eine spezifische Bindung der Nuk-Makromoleküle an die Zielsequenz wird der komplementäre Strang spezifisch Markiert. Die enzymatische Kopplung eines solchen Nuk-Makromoleküls in den wachsenden Nukleinsäurenstrang führt zur Herstellung einer Bindung zwischen dem verlängerten Strang und unterschiedlichen Domänen eines Nuk-Makromoleküls.

In einer Ausführungsform der vorliegenden Anmeldung werden Nuk-Makromoleküle verwendet, die mindestens eine Domäne (Target-Domäne) einschließen, die an die Zielsequenz sequenzspezifisch binden kann, und zumindest eine weitere Domäne, die an einen Bindungspartner an einer festen Phase binden kann (Anker-Domäne). Durch den enzymatischen Einbau eines solchen Nuk-Makromoleküls wird auch die Anker-Domäne zielsequenzspezifisch an die Nukleinsäurekette gebunden. Bei einer Inkubation der nun markierten Nukleinsäureketten mit einer festen Phase, die zumindest einen Bindungspartner für die Anker-Domän einschließt, können markierte Nukleinsäureketten spezifisch an die feste Phase binden.

Wie oben dargestellt, kann ein Nuk-Makromolekül Strukturen einschließen, die komplementär zur Zielsequenz sind. Daher ist es vorteilhaft, die Einbaureaktion unter Bedingungen durchzuführen, die eine Bindung dieser Anteile an die komplementäre Position in der Matrize erlauben. Dadurch kann eine selektive Markierung von ausgewählten Zielsequenzen erreicht werden.

In einer vorteilhaften Ausführungsform können Nuk-Makromoleküle mit natürlichen Nukleotiden (z.B. dNTPs oder NTPs) zusammen im Ansatz verwendet werden. Die erfindungsgemäßen Nuk-Makromoleküle und ensprechend zur Matrize komplementäre dNTPs konkurrieren miteinander um das Einbauereignis durch die Polymerase am 3'-Ende des wachsenden komplementären Stranges. Ein Nuk-Makromolekül kann durch die spezifische Bindung an die Zielsequenz über die Target-Domäne einen Vorteil gegenüber den freien Nukleotiden in der Lösung erzielen: es kann bevorzugt durch die Polymerase eingebaut werden. Dadurch können die Zielsequenen auch in Anwesenheit von natürlichen dNTPs mit Nuk-Makromolekülen spezifisch markiert werden.

Die Kopplung von Nuk-Makromolekülen erfolgt bevorzugt in der Nähe ihrer Bindung an die Matrize. Sequenzbereiche, die von diesem Ort weit entfernt sind. oder andere, in einem Ansatz vorkommende Sequenze ("nicht-Zielsequenzen") werden nicht markiert. In einer vorteilhaften Ausführugnsform der Erfindung, bleiben solche "Nicht-Zielseuenzen" unmarkiert. Dies kann durch die fehlende oder nur schwache Bindung von Nuk-Makromolekülen an diese "nicht Zielsequenzen" erreicht werden: sie werden mit dNTPs verlängert und werden nur schwach oder gar nicht mit Nuk-Makromolekülen markiert.

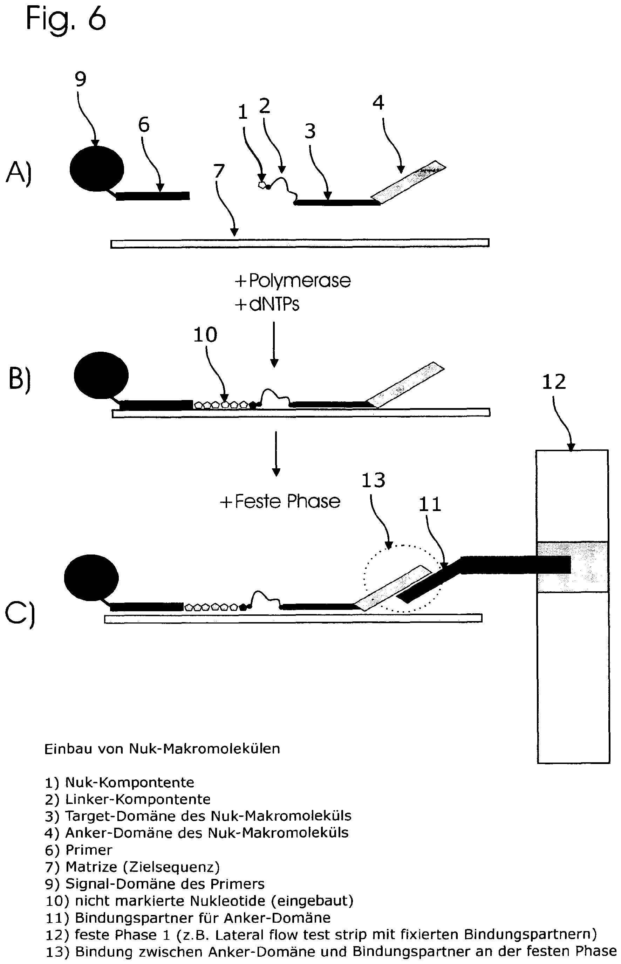

Durch die vorgegebene Zusammensetzung der Nuk-Makromoleküle kann der verlängerte Nukleinsäurestrang die Affinitätseigenschaften der Anker-Domäne erlangen. Für eine Analyse kann eine feste Phase bereitgestellt werden, die einen zur Anker-Domäne eines Nuk-Makromoleküls einen passenden Bindungspartner einschließt. Durch eine anschließend an die Markierungsreaktion erfogende Bindung von markierten Zielsequenzen an einen spezifischen, an einer festen Phase fixierten Bindungspartner (z.B. Oligonukleotid-Oligonukleotid-Paar oder in einem Biotin-Streptavidin-Paar, oder ein Antikörper-Hapten-Paar), kann der gesamte verlängerte Nukleinsäurestrang an diesen immobilisierten Bindungspartner gebunden werden (Fig. 6).

Besonders vorteilhaft sind Ausführungsformen, in denen mehrere unterschiedliche Matrizen mit jeweils unterschiedlichen Primern vorliegen und Nuk-Makromoleküle mit entsprechenden für die jeweilige Matrize spezifischen Oligonukleotid-Anteilen (Target-Domänen) und spezifischen Anker-Domänen verwendet werden. Dies erlaubt jeweils einen sequenzspezifischen Einbau von Nuk-Makromolekülen in die komplementäre Stränge zu den jeweiligen Zielsequenzen, sowie eine anschließende selektive Bindung an die feste Phase unter Verwendung unterschiedlicher, aber jeweils spezifischer Anker-Domänen. Besonders vorteilhaft sind Ausführungsformen, in denen spezifische Bindungspartner für Anker-Domäne auf einer festen Phase in einer adressierbaren Anordnung immobilisiert sind. Dies ermöglicht eine spezifische Verteilung von verlängerten Nukleinsäuresträgen auf der festen Phase aufgrund ihrer Sequenzzusammensetzung.

Die Bindungseigenschaften der Anker-Domänen an die Bindungspartner können optimiert werden. Zum einen, können die Anker-Domänen dermassen gestaltet werden, dass sie keine komplementären Bereiche zu Zielsequenz(en) einschließen. Zum anderen, kann ihre Bindungsstärke und Spezifität unabhängig von Zielsequenz(en) optimiert werden.

Ein weiterer Gegenstand der Anmeldung sind Verfahren zur enzymatischen Synthese von Nukleinsäureketten und Komponenten zur Ausführung dieser Verfahren zur Markierung mit erfindungsgemäßen Nuk-Makromolekülen.

Ein weiterer Gegenstand der Anmeldung sind die Kompositionen aus einzelnen Komponenten, die eine selektive Markierung von Zielsequenzen mit Nuk-Makromolekülen erlauben.

Die vorliegende Erfindung bietet mehrere Vorteile für die Entwicklung von Nachweisverfahren von Nukleinsäureketten.

Ein Vorteil dieser Ausführungsform besteht unter anderem darin, dass die Bindungseigenschaften der Anker-Domäne unabhängig von der Zielsequenz an eine jeweilige Nachweismethode angepasst bzw. optimiert werden können. Die Bedingungen unter denen die spezifische Bindung zwischen der Anker-Domäne und der festen Phase stattfinden können dadurch viel robuster gestaltet werden, ohne an Spezifität und Sensitivität der Bindung zu verlieren.

Ein weiterer Vorteil besteht darin, dass für die Markierung dieselben Enzyme verwendet werden können, die auch für die Vervielfältigung eingesetzt werden.

Ein weiterer Vorteil besteht darin, dass im Unterschied zu konventionell markierten Nukleotiden, wie dUTP-Biotin, dUTP-Digoxigenin oder farbstoffmarkierten Nukleotiden, ein zielsequenzgerichteter Einbau durch die Präsenz der Target-Domäne stark begünstigt wird.

Ein weiterer Vorteil besteht darin, dass mehrere unterschiedlich gebaute Nuk-Makromoleküle für einen spezifsichen Nachweis einer Zielsequenz gleichzeitig verwendet werden können.

### Detaillierte Beschreibung der Erfindung

### 1.3 Begriffe und Definitionen:

Im Folgenen wird Aufbau von Nuk-Makromolekülen besprochen, sowie deren strukrurelle Elemente erklärt. Dieser Abschnitt hat folgendes Inhaltsverzeichnis:
**1.3.1 Makromolekulare Verbindung**
**1.3.2 Niedermolekulare Verbindung**
**1.3.3 Ein Nuk-Makromolekül**
   **1.3.3.1 Nuk-Komponente**
      1.3.3.1.1 Variationen am Phosphat
      1.3.3.1.2 Variationen an der Base
      1.3.3.1.3 Variationen am Zucker
      1.3.3.1.4 Kopplung von NT und Linker
      1.3.3.1.5 Zahl der gekoppelten Nuk-Komponenten
   **1.3.3.2 Linker-Komponente**
      1.3.3.2.1 Linker-Bestandteile
      1.3.3.2.2 Linker-Länge
      1.3.3.2.3 Linker-Kopplung in einem Nuk-Makromolekül
   **1.3.3.3 Marker-Komponente**
      1.3.3.3.1 Target-Domäne.
      1.3.3.3.2 Anker-Domäne eines Nuk-Makromoleküls und Kombination mit der festen Phase
      1.3.3.3.3 Signal-Domäne (Funktionen und Zusammensetzung)
         1.3.3.3.3.1 Struktur der signalgebenden oder der signalvermittelnden Einheiten der Signal-Domäne
            1.3.3.3.3.1.1 Strukturen mit niedriger Molmasse:
            1.3.3.3.3.1.2 Strukturen mit hoher Masse (Makromoleküle)
      1.3.3.3.4 Kern-Komponente des Markers
      1.3.3.3.5 Kopplung der Marker-Einheiten oder Domänen
      1.3.3.3.6 Kopplung zwischen Linker und Marker
      1.3.3.3.7 Das Verhältnis der Nuk- Komponenten in einem Nuk-Makromolekül
      1.3.3.3.8 Das Verhältnis von Marker-Einheiten in einem Nuk-Makromolekül
   **1.3.3.4 Substrateigenschaften der Nuk-Makromoleküle**
**1.3.4 Niedermolekularer Marker**
**1.3.5. Konventionell modifiziertes Nukleotid**
**1.3.6. Enzyme (Polymerasen)**
**1.3.7. Spaltbare Verbindung -**
**1.3.8 DNA -**
**1.3.9 RNA -**
**1.3.10 PNA**
**1.3.11 LNA**
**1.3.12 Nukleotide:**
   - 1.3.12.1 dNTP
   - 1.3.12.2 NTP
   - 1.3.12.3 Abkürzung "NT"
**1.3.13 NSK -** Nukleinsäurekette. DNA oder RNA, PNA, LNA
**1.3.14 Gesamtsequenz**
**1.3.15 NSKF**
**1.3.16 Primerbindungstelle (PBS)**
**1.3.17 Referenzsequenz**
**1.3.18 Tm -** Schmelztemperatur
**1.3.19 Sterisches Hindernis,**
**1.3.20 Feste Phase zur Analyse**
**1.3.21 Zielsequenz oder Targetsequenzen**
**1.3.22 Primer**
**1.3.23 Hybridisierungssonde**
**1.3.24 Amplifikationsmethoden:**
**1.3.25 Nachweismethoden**
**1.3.26 weitere Enzyme**

**1.3.1 Makromolekulare Verbindung -** ein Molekül, oder ein Molekülkomplex, oder ein Nanokristall oder ein Nanoteilchen, dessen Masse zwischen 2kDa und 20kDa, 2kDa und 50kDa, 2kDa und 100kDa, 100kDa und 200kDa, 200kDa und 1000kDa oder 1MDa und 100MDa oder 100 MDa und 100 GDa liegt. Beispiele für makromolekulare Verbindungen sind Nukleinsäuren, wie Oligonukleotide mit einer Länge von mehr als 10 Nukleotiden, Polynukleotide, Polypeptide, Proteine oder Enzyme, Quantum Dots, Polymere wie PEG, Mowiol, Dextran, Polyacrylat, Nanogold-Partikel aber auch Komplexe, die aus mehreren Makromolekülen bestehen.

**1.3.2 Niedermolekulare Verbindung -** ein Molekül oder ein Molekülkomplex, dessen Masse kleiner 2000 Da (2kDa) beträgt, z.B. Biotin, natürliche Nukleotide, dATP, dUTP, viele Farbstoffe, wie Cy3, Rhodamine, Fluorescein und konventionell modifizierte Nukleotide, wie Biotin-16-dUTP.

**1.3.3 Ein Nuk-Makromolekül** im Sinne dieser Anmeldung ist eine chemische Struktur (ein Nukleotid-Analog, ein Nukleotid-Konjugat), die eine oder mehrere Nuk-Komponenten, eine oder mehrere Linker-Komponenten und zumindest eine Marker-Komponente einschließt (Fig. 1-3):
(Nuk-Linker)ₙ-Marker
wobei:
Nuk - eine Nuk-Komponente ist
Linker - eine Linker-Komponente ist
Marker - eine Marker-Komponente ist
n - eine Zahl von 1 bis 10000 ist

Nuk - ein Nukleotid- oder ein Nukleosid-Monomer ist (Nuk-Komponente)
Linker - Seine Zusammensetzung ist nicht eingeschränkt, solange die Substrat-Eigenschaften der Nukleotide nicht verloren gehen. Seine Länge liegt zwischen 5 und 10000 Kettenatome.
Marker - eine Marker-Komponente, die eine oder mehrere Domänen einschließen kann. Beisielsweise Target-Domäne, Anker-Domäne, Signal-Domäne.
n - eine Zahl von 1 bis 10000, wobei (n) einen durchschnittlichen Wert darstellen kann.

In einer weiteren Ausführungsform besteht die Linker-Komponente aus einer Kopplungseinheit L für die Kopplung des Linkers an die Nuk-Komponente, aus einem hydrophilen, wasserlöslichen Polymer und aus einer Kopplungseinheit T für die Kopplung des Linkers an die Marker-Komponente. In dieser bevorzugten Ausführungsform hat ein Nuk-Makromolekül folgende Struktur, Fig. 1 oder 2:
(Nuk-(L- Polymer-T))ₙ-Marker
wobei:
Nuk - ein Nukleotid- oder ein Nukleosid-Monomer ist (Nuk-Komponente)
Linker - einschließlich der Kopplungseinheit (L), hydrophilem Polymer, und Kopplungseinheit (T). Wobei (L) ein Teil des Linkers ist, das die Verbindung zwischen Nuk und dem Linkerrest darstellt (Kopplungseinheit L), und (T) ein Teil des Linkers ist, das die Verbindung zwischen dem Linkerrest und dem Marker darstellt (Kopplungseinheit T). Polymer ist ein Teil des Linkers, das ein hydrophiles, wasserlösliches Polymer mit einer durchschnittlichen Länge zwischen 5 und 100000 Atome ist
   (Kopplungseinheit L, Polymerlinker, Kopplungseinheit T sind bei dieser Ausführungsform als Linker-Komponente zusammengefaßt)
Marker - eine Marker-Komponente, die eine oder mehrere Domänen einschließen kann. Beisielsweise Target-Domäne, Anker-Domäne, Signal-Domäne.
n - eine Zahl von 1 bis 10000, wobei (n) einen durchschnittlichen Wert darstellen kann.

Beispiele für Synthesen von Nuk-Makromolekülen sind in Anmeldungen Cherkasov et al WO 2005044836, Cherkasov et al WO2006097320, Cherkasov et al WO 2008043426, Cherkasov et al DE 10356837, Cherkasov et al DE 102004009704 angegeben.

### 1.3.3.1 Nuk-Komponente

Nuk-Komponente ist ein Substrat für Nukleotid- bzw. Nukleosid-akzeptierendes Enzym.

Nuk-Komponente kann sowohl ein Nukleotid als auch ein Nukleosid darstellen. Im Folgenden werden bei der Beschreibung Nukleotide stellvertretend für beide Klassen betrachtet. Nukleotiside können mittels entsprechender Enzyme oder chemischer Methoden in eine Nukleotidform umgewandelt werden.

In einer Ausführungsform ist Nuk-Komponente ein Nukleotid- oder ein Nukleosid-Monomer, das mit der Linker-Komponente gekoppelt ist. Prinzipiell können alle als Substrat für Nukleotid-akzeptierende Enzyme geeigneten konventionellen Nukleotid-Varianten als Nuk-Komponente des Nuk-Makromoleküls dienen, so dass sowohl natürliche als auch modifizierte Nukleotide (Nukleotid-Analoga) für die Nuk-Komponente in Frage kommen. Bei modifizierten Nukleotiden können Base-, Zucker- oder Phosphat-Teile modifiziert werden. Viele Beispiele für Nukleotid-Modifikationen sind dem Fachmann bekannt ("Nucleoside Triphosphates and their Analogs", Morteza Vaghefi, 2005, ISBN 1-57444-498-0; "Deoxynucleoside analogs in cancer therapy" Godefridus J. Peters, 2006, ISBN 1-58829-327-0; "Chemistry of nucleosides and nucleotides" Leroy B. Townsend, 1991, ISBN 0-306-43646-9; "Advanced organic chemistry of nucleic acids", 1994, Shabarova, ISBN 3-527-29021-4; "Nucleotide Analogs" Scheit, 1980, ISBN 0-471-04854-2; "Nucleoside and Nucleic Acid Chemistry", Kisakürek 2000, "Anti-HIV Nucleosides" Mitsuya, 1997, "Nucleoside Analogs in cancer therapy", Cheson, 1997) im Text werden auch weitere Beispiele für die Modifikationen der Nukleotide angegeben.

Die Nuk-Komponente schließt vorzugsweise eine Base-Komponente (Base), eine Zucker-Komponente (Zucker) und wahlweise eine Phosphat-Komponente (Phosphat) ein. Base, Zucker und Phosphat können modifiziert sein, d.h. die Grundstruktur sieht den natürlichen Nukleotiden oder Nukleosiden ähnlich, trägt aber beispielsweise zusätzliche chemische Gruppen. Beispiele für Kombinationen aus verschiedenen Komponenten sind dem Fachmann bekannt. Solche Nuk-Komponenten können in vielen enzymatischen und chemischen Reaktionen eingesetzt werden (G. Wright et al. Pharmac.Ther. 1990, V. 47, S. 447-).

In einer bevorzugten Ausführungsform, ist die Nuk-Komponente ein Substrat für DNA-Polymerasen. In einer anderen Ausführungsform, ist die Nuk-Komponente ein Substrat für RNA-Polymerasen. Variationen von Nukleotiden, die solche Substrat-Eigenschaften zulassen, können als Nuk-Komponente eingesetzt werden. Beispielsweise, Substrate für Nukleotid-akzeptierende Enzyme, denen ein Bestandteil eines konventionellen Nukleotids fehlt, z.B. azyklische Nukleotid-analoga, können ebenfalls als Nuk-Komponente eingesetzt werden.

### 1.3.3.1.1 Variationen am Phosphat

In einer Ausführungsform stellt die Nuk-Komponente ein Nukleosid dar. In einer anderen Ausführungsform stellt die Nuk-Komponente ein Nukleosid-Monophosphat dar. In einer anderen Ausführungsform stellt die Nuk-Komponente ein Nukleosid-Diphosphat dar. In einer weiteren Ausführungsform stellt die Nuk-Komponente ein Nukleosid-Triphosphat dar. Auch höhere Phosphat-Derivate (Tetraphosphat, Pentaphosphate usw.) können verwendet werden.

Die genannten Phosphatmodifikationen können wie bei Nukleosid-Triphosphaten an der 5'-Position oder auch an anderen Positionen des Zucker-Teils des Nukleotides sitzen, beispielsweise an der 3'-Position.

Wahlweise kann die Phosphat-Komponente Modifikationen einschließen, solche Modifikationen schließen beispielsweise in einer Ausführungsform einen Linker ein (D. Jameson et al. Methods in Enzymology 1997, V. 278, S. 363-, A. Draganescu et al. J. Biol.Chem. 2000 v.275, 4555-). In einer weiteren Ausführungsform schließt die Phosphat-Komponente der Nuk-Komponente Thiotriphosphat-Verbindungen ein (Burges et al. PNAS 1978 v. 75, S. 4798-).

In einer weiteren Ausführungsform schließt die Phosphat-Komponente der Nuk-Komponente geschützte Phosphat-Gruppen (z.B. Phosphoroamidite) ein.

In einer Ausführungsform stellt die Phosphat-Komponente die Verbindung zwischen der Nuk-Komponente und der Linker-Komponente der Nuk-Makromoleküle dar.

### 1.3.3.1.2 Variationen an der Base

Die Nuk-Komponente kann ein in der Natur in den Nukleinsäuren vorkommendes Nukleotid oder Nukleosid oder seine Analoga darstellen, vorzugsweise die an Watson-Crick-Paarbildung teilnehnem, beispielsweise Adenin, Guanin, Thymin, Cytosin, Uracil, Inosin, oder eine modifizierte Base, wie z.B. 7-Deazaadenin, 7-Deazaguanin, 6-Thioadenin, einschließen, Literatur s. oben. Wahlweise kann die Base Modifikationen einschließen, solche Modifikationen schließen beispielsweise in einer Ausführungsform einen an die Base gekoppelten Linker wie beispielsweise ein Amino-propargyl-Linker oder ein Amino-Allyl-Linker ein, weitere Beispiele für die Linker sind bekannt (Ward et al. US Pat 4711955, G. Wright et al. Pharmac.Ther. 1990, V. 47, S. 447-, Hobbs et al. US Patent 5.047.519 oder andere Linker z.B. Klevan US Pat. 4,828,979, Seela US pat. 6211158, US pat. 4804748, EP 0286028, Hanna M. Method in Enzymology 1996 v.274, S.403, Zhu et al. NAR 1994 v.22 S.3418, Jameson et al. Method in Enzymology, 1997, v. 278, S. 363-, Held et al. Nucleic acid research, 2002, v. 30 3857-, Held et al. Nucleosides, nucleotides & nucleic acids, 2003, v. 22, S. 391, Short US Pat. 6579704, Odedra WO 0192284). In einer Ausführungsform stellt der an die Base gekoppelte Linker die Verbindung zwischen der Nuk-Komponente und der Linker-Komponente der Nuk-Makromoleküle dar. Weitere Modifikationen an der Base sind beispielsweise im Katalog von Trilink Biotechnologies, Inc. San Diego, USA, dargestellt bzw. in "Nucleoside triphosphates and their analogs", Morteza Vaghefi, 2005 ISBN 1-57444-498-0.

### 1.3.3.1.3 Variationen am Zucker

Dem Fachmann sind verschiedene Variationen der Zucker-Komponente der Nukleotide bekannt, die z.B. in der Diagnostik, Therapie oder Forschung eingesetzt werden. Solche Variationen schließen z.B. Ribose , 2'-Deoxyribose oder 2',3'-Dideoxyribose ein. Wahlweise kann die Zucker-Komponente Modifikationen einschließen (M. Metzger et al. Nucleic Acid Research 1994, V. 22, 4259-, Tsien WO 91/06678), solche Modifikationen schließen beispielsweise in einer Ausführungsform einen Linker ein. Die modifizierende Gruppe oder Linker kann beispielsweise reversibel an die Zucker-Komponente gekoppelt sein (Hovinen et al. J.Chem.Soc.Prking Trans. 1994, S. 211-, Canard US Pat. 5798210, Kwiatkowski US Pat. 6255475, Kwiatkowski WO 01/25247, Ju et al. US Pat. 6664079, Fahnestock et al. WO 91066678, Cheeseman US Pat. 5302509, Parce et al. WO 0050642, Milton et al. WO 2004018493, Milton et al. 2004018497).

In einer Ausführungsform stellt der an die Zucker-Komponente gekoppelte Linker die Verbindung zwischen der Nuk-Komponente und der Linker-Komponente der Nuk-Makromoleküle dar.

In einer weiteren Ausführungsform schließt die Zuker-Komponente z.B. folgende Modifikationen ein: wahlweise kann die 3'- oder die 2'-OH-Gruppen durch folgende Atome oder Gruppen ersetzt werden: Halogenatome, Wasserstoff, Amino-, Merkapto- oder Azido-Gruppe (Beabealashvilli et al. Biochem Biophys Acta 1986, v.868, 136-, Yuzhanov et al. FEBS Lett. 1992 v. 306, 185-).

In einer weiteren Ausführungsform schließt die Nuk-Komponente azyklische Nukleotid- oder Nukleosid-Modifikationen ein (A. Holy Current Pharmaceutical Design 2003 v. 9, 2567-, G. Wright et al. Pharmac.Ther. 1990, V. 47, S. 447-). In einer weiteren Ausführung kann die Zucker-Komponente eine Doppeltbindung einschließen.
In der Anmeldung werden 2'-Deoxynukleotide, beispielsweise 2'-Deoxyuridin-Triphosphat, 2'-Deoxycytidin-Triphosphat, 2'-Deoxyadenosin-Triphosphat, 2'-Deoxyguanosin-Triphosphat als dUTP, dCTP, dATP und dGTP bezeichnet.

### 1.3.3.1.4 Kopplung der Nuk-Komponente und Linker

Die Nuk-Komponente ist an einer Kopplungsstelle mit dem Linker verbunden. Die Kopplungsstelle des Linkers an die Nuk-Komponente kann sich an der Base, am Zucker (Ribose bzw. Deoxyribose), oder am Phosphat-Teil befinden.

Die Verbindung zwischen der Linker-Komponente und der Nuk-Komponente ist vorzugsweise kovalent.
Wenn die Kopplungsstelle an der Base liegt, so befindet sie sich vorzugsweise an den Positionen 4 oder 5 bei Pyrimidin-Basen und an den Positionen 6,7,8 bei den Purin-Basen (Ward et al. US Pat 4711955, G. Wright et al. Pharmac.Ther. 1990, V. 47, S. 447-, Hobbs et al. US Patent 5.047.519 oder andere Linker z.B. Klevan US Pat. 4,828,979, Seela US pat. 6211158, US pat. 4804748, EP 0286028, Hanna M. Method in Enzymology 1996 v.274, S.403, Zhu et al. NAR 1994 v.22 S.3418, Jameson et al. Method in Enzymology, 1997, v. 278, S. 363-, Held et al. Nucleic acid research, 2002, v. 30 3857-, Held et al. Nucleosides, nucleotides & nucleic acids, 2003, v. 22, S. 391, Short US Pat. 6579704, Odedra WO 0192284). Weitere Beispiele für Modifikationen an der Base sind in "Nucleoside triphosphates and their analogs", Morteza Vaghefi, 2005 ISBN 1-57444-498-0; angegeben. Am Zucker können die Positionen 2', 3' , 4' oder 5'die Kopplungsstellen darstellen. Die Kopplung an die Phosphatgruppen kann beispielweise an der alpha, beta, oder gamma-Phosphatgruppe erfolgen. Beispiele für die Kopplungsstelle an der Base sind in Short WO 9949082, Balasubramanian WO 03048387, Tcherkassov WO 02088382 (siehe auch kommerziell erhältliche Nukleotide (Amersham, Roche), an der Ribose in Herrlein et al. Helvetica Chimica Acta, 1994, V. 77, S. 586, Jameson et al. Method in Enzymology, 1997, V. 278, S. 363, Canard US Pat. 5798210, Kwiatkowski US Pat. 6255475, Kwiatkowski WO 01/25247, Parce WO 0050642., an Phosphatgruppen in Jameson et al. Method in Enzymology, 1997, V. 278, S. 363 beschrieben.

Die Position der Kopplungsstelle hängt vom Einsatzgebiet der Nuk-Makromoleküle ab. So werden beispielsweise für Markierungen von Nukleinsäuren, die am Nukleinsäurestrang verbleiben soll, vorzugsweise Kopplungsstellen am Zucker oder an der Base verwendet.

Die Kopplung an die Gamma- oder Beta-Phosphatgruppen kann beispielsweise dann erfolgen, wenn die Markierung beim Einbau des Nuk-Makromoleküls freigesetzt werden soll.

Die Verbindung zwischen der Nuk-Komponente und der Linker-Komponente erfolgt beispielsweise über eine Kopplungseinheit (L), die ein Teil der Linker-Komponente ist.

Die Verbindung zwischen der Nuk-Komponente und dem Linker kann in einer Ausführungsform widerstandsfähig sein, z.B. bei Temperaturen bis zu 130°C, für pH-Bereiche zwischen 1 und 14, und/oder resistent gegen hydrolytische Enzyme (z.B. Proteasen, Esterasen) sein. In einer anderen Ausführungsform der Erfindung ist die Verbindung zwischen der Nuk-Komponente und dem Linker unter milden Bedingungen spaltbar.

Diese spaltbare Verbindung ermöglicht die Entfernung der Linker- und der Marker-Komponenten. Dies kann beispielsweise in den Verfahren der Sequenzierung durch die Synthese von Bedeutung sein, wie Pyrosequencing, BASS (Canard et al. US Patent 5.798.210, Rasolonjatovo Nucleosides & Nucleotides 1999, V.18 S.1021, Metzker et al. NAR 1994, V.22, S.4259, Welch et al. Nucleosides & Nucleotides 1999, V.18, S.19, Milton et al. WO 2004018493, Odedra at al. WO 0192284) oder single molecule sequencing, Tcherkassov WO 02088382. Ihre Wahl ist nicht eingeschränkt, sofern sie unter den Bedingungen der enzymatischen Reaktion stabil bleibt, keine irreversible Störung der Enzyme (z.B. Polymerase) verursacht und unter milden Bedingungen abgespalten werden kann. Unter "milden Bedingungen" sind solche Bedingungen zu verstehen, die zum Beispiel Nukleinsäure-Primer-Komplexe nicht zerstören, wobei z.B. der pH-Wert vorzugsweise zwischen 3 und 11 und die Temperatur zwischen 0°C und einem Temperaturwert (x) liegt. Dieser Temperaturwert (x) hängt von der Tm des Nukleinsäure-Primer-Komplexes (Tm ist der Schmelzpunkt) und wird beispielsweise als Tm (Nukleinsäure-Primer-Komplex) minus 5°C errechnet (z.B. Tm ist 47°C, dann liegt die maximale Temperatur bei 42°C; unter diesen Bedingungen eignen sich besonders Ester-, Thioester-, Acetale, Phosphoester-, Disulfid-Verbindungen und photolabile Verbindungen als spaltbare Verbindungen).

Vorzugsweise gehört die genannte spaltbare Verbindung zu chemisch oder enzymatisch spaltbaren oder photolabilen Verbindungen. Als Beispiele von chemisch spaltbaren Gruppen sind Ester-, Thioester-, Tartrat-, Disulfid-, Acetal-Verbindungen bevorzugt (Short WO 9949082, "Chemistry of protein conjugation and crosslinking" Shan S. Wong 1993 CRC Press Inc., Herman et al. Method in Enzymology 1990 V.184 S.584, Lomant et al. J.Mol.Biol. 1976 V.104 243, "Chemistry of carboxylic acid and esters" S.Patai 1969 Interscience Publ., Pierce Katalog). Beispiele für photolabile Verbindungen können in folgenden Literaturstellen gefunden werden: Rothschild WO 9531429, "Protective groups in organic synthesis" 1991 John Wiley & Sons, Inc., V. Pillai Synthesis 1980 S.1, V. Pillai Org. Photochem. 1987 V.9 S.225, Dissertation "Neue photolabile Schutzgruppen für die lichtgesteuerte Oligonucleotidsynthese" H.Giegrich, 1996, Konstanz, Dissertation "Neue photolabile Schutzgruppen für die lichtgesteuerte Oligonucleotidsynthese" S.M.Bühler, 1999, Konstanz)..

### 1.3.3.1.5 Zahl der gekoppelten Nuk-Komponenten

In einer Ausführungsform der Erfindung ist pro ein Nuk-Makromolekül nur eine Nuk-Komponente gekoppelt. In einer anderen Ausführungsform der Erfindung sind mehrere Nuk- Komponenten pro Nuk-Makromolekül gekoppelt. Mehrere Nuk-Komponenten können einheitlich oder unterschiedlich sein, wobei beispielsweise durchschnittlich 2 bis 5, 5 bis 10, 10 bis 25, 25 bis 50, 50 bis 100, 100 bis 250, 250 bis 500, 500 bis 1000, 1000 bis 100000 Nuk-Komponenten pro ein Nuk-Makromolekül gekoppelt sein können.

### 1.3.3.2 Linker-Komponente

Die Funktion des Linkers ist, unter anderem, eine Nuk-Komponente und eine Marker-Komponente in Art und Weise zu verbinden, dass die Substrateigenschaften der Nuk-Komponente für nukleotid-akzeptierende Enzyme trotz Kopplung eines makromolekularen Markers nicht verloren gehen. Die Bezeichnung Linker oder Linker-Komponente wird synonym in der Anmeldung verwendet und bezieht sich auf den gesamten strukturellen Abschnitt des Nuk-Makromoleküls zwischen der Nuk-Komponente und der Marker-Komponente. Die genaue Linkerzusammensetzung ist nicht eingeschänkt und kann variieren. In einer Ausführungsform ist der Linker vorzugsweise hydrophil.

### 1.3.3.2.1 Linker-Länge

Die durchschnittliche Linkerlänge schließt folgende Bereiche ein: zwischen 2 und 5, 5 und 10, 10 bis 20, 20 bis 30, 30 bis 40, 40 bis 50, 50 bis 60, 60 bis 70, 70 bis 80, 80 bis 90, 90 bis 100, 50 bis 100, 100 bis 200, 200 bis 500, 500 bis 1000, 1000 bis 2000, 2000 bis 10.000, 10000 bis 100000 Atome (gezählt werden Ketten-Atome), somit beträgt die durchschnittliche Linker-Länge zwischen 2 und 5, 5 und 10, 10 bis 20, 20 bis 30, 30 bis 40, 40 bis 50, 50 bis 60, 60 bis 70, 70 bis 80, 80 bis 90, 90 bis 100, 50 bis 100, 100 bis 200, 200 bis 500, 500 bis 1000, 1000 bis 2000, 2000 bis 10.000, 10000 bis 100000 Angström (gemessen an einem potenziell maximal ausgestrecktem Molekül).

Falls ein Nuk-Makromolekül mehrere Linker-Komponenten einschließt, können diese Linker-Komponenten untereinander gleiche oder auch unterschiedlich lang sein.

Einige Abschnitte des Linkers können rigide Bereiche enthalten und andere Abschnitte können flexible Bereiche enthalten.

### 1.3.3.2.2 Kurzer Linker

In einer bevorzugten Form haben Nuk-Makromoleküle einen kurzen Linker. Seine Länge scjließt Bereiche zwischen 2 und 50 Kettenatomen ein. Solche Linker können funktionelle Gruppen tragen, wie beispielsweise Amino-, Carboxy-, Mercapto-, Hydroxygruppen, Alkyn-, Isothiocyanat-, Aldehyd- oder Azid-Gruppe. Solche Gruppen können in einer reaktiven Form, z.B. NHS-Ester für Carboxy-Gruppe, bereitgestellt werden. An diese Gruppen können weitere Moleküle gekoppelt werden. In einer Ausführungsform werden Cross-Linker an den kurzen Linker der Nuk-Komponente gekoppelt, so dass die resultierende Nuk-Komponente an weitere Substanzen gebunden werden kann, z.B. an makromolekulre Linker-Komponente oder Marker-Komponenten. Beispiele von kurzen Linkern gekoppelt an Nukleotide sind dem Fachmann bekannt ("Nucleoside triphosphates and their analogs", Morteza Vaghefi, 2005 ISBN 1-57444-498-0, Ward et al. US Pat 4711955, G. Wright et al. Pharmac.Ther. 1990, V. 47, S. 447-, Hobbs et al. US Patent 5.047.519 oder andere Linker z.B. Klevan US Pat. 4,828,979, Seela US pat. 6211158, US pat. 4804748, EP 0286028, Hanna M. Method in Enzymology 1996 v.274, S.403, Zhu et al. NAR 1994 v.22 S.3418, Jameson et al. Method in Enzymology, 1997, v. 278, S. 363-, Held et al. Nucleic acid research, 2002, v. 30 3857-, Held et al. Nucleosides, nucleotides & nucleic acids, 2003, v. 22, S. 391, Short US Pat. 6579704, Odedra WO 0192284). Der Linker kann eine oder mehrere Einheiten von Polymeren enthalten, wie beispielsweise Aminosäuren, Zucker, PEG-Einheiten oder Carbonsäuren. Weitere Beispiele für kurze Linker kann die Kopplungseinheit (L) eines langen Linkers dienen s.u. Beispiele für Cross-Linker sind einem Fachmann bekannt ("Chemistry of protein conjugation and crosslinking" Shan S. Wong 1993). Viele Cross-Linker sind kommerziell erhältlich, z.B. von Invitrogen (Lifescience Technologies, Pierce Biotech, Iris-Biotech). Beispiele von Kopplungen von verschiedenen Substanzen an Makromoleküle, z.B. an Oligonukleotide sind ebenfalls bekannt (Y.Singh et al Chem.Soc.Rev.2010, 39, 2054-). Es ist für einen Fachmann offensichtlich, dass der Linker zwischen der Nuk-Komponente und der Marker-Komponente in mehreren chemischen Schritten aufgebaut werden kann.

### 1.3.3.2.3 Langer Linker

In einer weiteren bevorzugten Ausführungsform der Erfindung wird ein langer Linker eingesetzt, mit einer Länge von mehr als 50 Ketten-Atomen. Die Linker-Komponente hat in ihrer Struktur beispielsweise folgende Bestandteile:
1) Kopplungseinheit L
2) hydrophiles bzw. wasserlösliches Polymer
3) Kopplungseinheit T

Die Aufteilung der Linker-Komponente in einzelne Bestandteile ist rein funktionell und soll lediglich der Anschaulichkeit der Struktur dienen. Je nach Betrachtungsweise können einzelne Strukturen des Linkers zum einen oder zum anderen Bestandteil gerechnet werden.

Die Kopplungseinheit L hat die Funktion, die Linker-Komponente und die Nuk-Komponente zu verbinden. Bevorzugt sind kurze, unverzweigte Verbindungen, von 1 bis 20 Atome in der Länge. Die jeweilige Struktur der Kopplungseinheit L hängt von der Kopplungsstelle des Linkers an das Nukleotid und von dem jeweiligen Polymer des Linkers ab. Einige Beispiele für die Kopplungseinheiten L sind in Beispielen 1 bis 33 angegeben. Viele konventionell modifizierte Nukleotide tragen einen kurzen Linker, diese kurzen Linker dienen als weitere Beispiele für die Kopplungseinheit L, z.B. kurze Linker an der Base: Short WO 9949082, Balasubramanian WO 03048387, Tcherkassov WO 02088382(siehe auch kommerziell erhältliche Nukleotide (Amersham, Roche), kurze Linker an der Ribose in Herrlein et al. Helvetica Chimica Acta, 1994, V. 77, S. 586, Jameson et al. Method in Enzymology, 1997, V. 278, S. 363, Canard US. Pat. 5798210, Kwiatkowski US Pat. 6255475, Kwiatkowski WO 01/25247, Ju et al. US Pat. 6664079, Parce WO 0050642., kurze Linker an Phosphatgruppen in Jameson et al. Method in Enzymology, 1997, V. 278, S. 363 .
Noch weitere Beispiele für die Kopplungseinheit L sind nachfolgend angegeben:
R₆-NH-R₇, R₆-O-R₇, R₆-S-R₇, R₆-SS-R₇, R₆-CO-NH-R₇, R₆-NH-CO-R₇, R₆-CO-O-R₇, R₆-O-CO-R₇, R₆-CO-S-R₇, R₆-S-CO-R₇, R₆-P(O)₂-R₇, R₆-Si-R₇, R₆-(CH₂)ₙ-R₇, R₆-(CH₂)ₙ-R₇, R₆-A-(CH₂)ₙ-R₇, R₆-(CH₂)ₙ-B-R₇,
   R₆-(CH=CH-)ₙ-R₇, R₆-(A-CH=CH-)ₙ-R₇, R₆-(CH=CH-B-)ₙ-R₇, R₆-A-CH=CH-(CH₂-)ₙ-R₇, R₆-(-CH=CH-CH₂)ₙ-B-R₇, R₆-(-CH=CH-CH₂-CH₂)ₙ-B-R₇, R₆-(C≡C-)ₙ-R₇, R₆-(A-C≡C-)ₙ-R₇, R₆-(C≡C-B-)ₙ-R₇, R₆-A-C≡C-(CH₂-)ₙ-R₇, R₆-(-C≡C-CH₂)ₙ-B-R₇, R₆-(-C≡C-CH₂-CH₂)ₙ-B-R₇,
wobei R₆ - die Nuk-Komponente ist, R₇ - de Polymer ist und A und B folgende strukturelle Elemente einschließen: -NH-, -O-, -S-, -SS-, -CO-NH-, -NH-CO-, - CO-O-, -O-CO-, -CO-S-, -S-CO-, -P(O)₂-, -Si-, -(CH₂)ₙ-, eine photolabile Gruppe, wobei n - gleich 1 bis 5 ist

Die Kopplungseinheit L ist auf einer Seite mit der Nuk-Komponente kovalent verbunden. Auf der anderen Seite können weitere Teile des Linkers, z.B. ein hydrophiles Polymer oder direkt die Kopplungseinheit T oder direkt der Marker gebunden werden.

Im Folgenden, wird die Kopplung eines Polymers, als Bestandteil des Linkers exemplarisch erläutert. Die Art der Kopplung hängt von der Art des Polymers ab. In bevorzugter Form hat das Polymer an seinen Enden reaktive Gruppen, beispielsweise NH2 (Amino), OH (Hydroxy), SH (Mercapto), COOH (Carboxy), CHO (Aldehyd), Acryl- oder Maleimid, Halogen-, Alkyn-, Isothiocyanat- oder Azid-Gruppe. Solche Gruppen können in einer reaktiven Form, z.B. NHS-Ester für Carboxy-Gruppe, bereitgestellt werden. Solche Polymere sind käuflich erwerblich (z.B. Fluka, Iris-Biotech, Nanocs inc, Pierce Biotech). Einige Varianten für die Kopplung von Polymeren an die Kopplungseinheiten sind unter Beispielen angegeben.

Das wasserlösliche Polymer bildet in einer bevorzugten Ausführungsform den Hauptanteil der Linker-Komponente. Es ist ein Polymer, vorzugsweise hydrophil, bestehend aus gleichen oder unterschiedlichen Monomeren. Beispiele für geeignete Polymere stellen Polyethylen-glycol (PEG), Polyamide (z.B. Polypeptide), Polysaccharide und deren Derivate, Dextran und seine Derivate, Polyphosphate, Polyacetate, Poly(alkyleneglycole), Kopolymere aus Ethylenglycol und Propylenglycol, Poly(olefinische Alkohole), Poly(Vinylpyrrolidone), Poly(Hydroxyalkylmethacrylamide), Poly(Hydroxyalkylmethacrylate), Poly(x-Hydroxy-Säuren), Poly-Acrylsäure und ihre Derivate, Poly-Acrylamide in ihre derivate, Poly(Vinylalkohol), Polylactat Säure, polyglycolic Säure, Poly(epsilon-caprolactone), Poly(beta-hydroxybutyrate), Poly(beta-hydroxyvalerate), Polydioxanone, Poly(ethylene terephthalate), Poly(malic Säure), Poly(tartronic Säure), Poly(ortho ester), Polyanhydride, Polycyanoacrylate, Poly(phosphoester), Polyphosphazene, Hyaluronidate, Polysulfones.

Dieses Polymer schließt in einer Ausführungsform verzweigte oder weiteren Ausführungsform nicht verzweigte Polymere ein. Das Polymer kann aus mehreren unterschiedlich langen Abschnitten bestehen, wobei jeder Abschnitt aus gleichen Monomeren besteht und Monomere in unterschiedlichen Abschnitten unterschiedlich sind. Einem Fachmann sollte naheliegend erscheinen, dass für einen makromolekularen Linker meistens nur eine durchschnittliche Masse bestimmt werden kann, so dass die Angaben zu den Molmassen einen Mittelwert darstellen ("Makromoleküle, Chemische Struktur und Synthesen", Band 1, 4, H. Elias, 1999, ISBN 3-527-29872-X). Aus diesem Grund kann für Nuk-Makromoleküle oft keine exakte Massenangabe gemacht werden.

In einer bevorzugten Ausführungsform der Erfindung schließt die Linker-Komponente ein lineares, nicht verzweigtes Polymer ein und trägt keine sterisch anspruchsvollen chemischen Strukturen, wie beispielsweise Farbstoffe, Fluoreszenzfarbstoffe, Liganden. Solche Linker ermöglichen eine geringe sterische Hinderung der enzymatischen Erkennung der Nuk-Komponenten.

In einer weiteren bevorzugten Ausführungsform der Erfindung ist das Polymer der Linker-Komponente linear, die Linker-Komponente trägt jedoch eine oder mehrere sterisch anspruchsvollen chemischen Strukturen, wie beispielsweise Farbstoffe. Die Einführung der sterisch anspruchsvollen Gruppen erlaubt bei manchen Verfahren eine Kontrolle der enzymatischen Reaktion (Tcherkassov WO 02088382). Weitere Beispiele für sterisch anspruchsvolle Gruppen sind im Abschnitt 1.3.19 angegeben.

Sterisch anspruchsvollen Liganden bzw. Strukturen können an unterschiedliche Linker-Abschnitte gekoppelt sein. Die durchschnittliche Zahl der an den Linker gekoppelten sterisch anspruchsvollen Liganden kann variieren und liegt beispielsweise zwischen 1 und 3, 3 und 5, 5 und 20, 20 und 50. Die Kopplung von sterisch anspruchsvollen Gruppen sollte die Tatsache berücksichtigen, dass eine raumfordernde Struktur in der Nähe der Nukleotid-Komponente zur Aufhebung der Substrateigenschaften führen kann. Sterisch anspruchsvolle Liganden können gleichmäßig oder zufällig über die gesamte Länge des Linkers gekoppelt sein, oder in einem bestimmten Abstand von der Nuk-Komponte am Linker gekoppelt sein. Der Abstand zwischen der Nuk-Komponente und dem sterischen Hindernis beträgt beispielsweise 10 bis 15, 15 bis 20, 20 bis 25, 25 bis 30, 30 bis 35, 35 bis 40, 40 bis 45, 45 bis 50, 50 bis 55, 55 bis 60, 60 bis 70, 70 bis 80, 80 bis 90, 90 bis 100, 100 bis 200, 200 bis 1000, 1000 bis 5000 Kettenatome. Die sterisch anspruchsvolle Gruppe kann als Teil des Linkers oder als Teil des Markers betrachtet werden. Die Betrachtugnsweise kann beispielsweise davon abhängen, ob sterisch anspruchsvolle Gruppe gewisse Signaleigenschaften aufweist oder nicht.

### 1.3.3.2.4 Linker-Kopplung in einem Nuk-Makromolekül

Der Linker ist an einer Seite an die Nuk-Komponente und auf der anderen Seite an die Marker-Komponente gebunden. Dazu kann der Linker an seinen Enden Kopplungseinheiten haben, die diese Funktion erfüllen. Die Verbindung mit der Nuk-Komponenten wurde oben erörtert. Die Verbindung zwischen dem Linker und der Marker-Komponenten erfolgt über Kopplungseinheit T. Bevorzugt sind kurze, unverzweigte Verbindungen, bis max. 20 Atome in der Länge. Die jeweilige Struktur der Kopplungseinheit T hängt von der Kopplungsstelle an der Marker-Komponente und von dem jeweiligen Polymer des Linkers. Die Kopplungseinheit T ist mit dem Polymer kovalent verbunden. Die Art der Kopplung hängt von der Art des Polymers ab. In bevorzugter Form hat das Polymer an seinen Enden reaktive Gruppen, beispielsweise NH2 (Amino), OH (Hydroxy), SH (Mercapto), COOH (Carboxy), CHO (Aldehyd), Acryl- oder Maleimid, Halogen-, Alkyn-, Isothiocyanat- oder Azid-Gruppe. Solche Gruppen können in einer reaktiven Form, z.B. NHS-Ester für Carboxy-Gruppe, bereitgestellt werden.. Solche Polymere sind kommerziell erhältlich (z.B. Sigma-Aldrich, Iris-Biotech, Nanocs Inc., ). Einige Beispiele für die Kopplungseinheiten L sind angegeben in Cherkasov et al WO 2005044836, Cherkasov et al WO2006097320, Cherkasov et al WO 2008043426, Cherkasov et al DE 10356837, Cherkasov et al DE 102004009704. Weitere Beispiele für die chemische und affine Kopplungen s. in der Literatur: "Nucleoside triphosphates and their analogs", Morteza Vaghefi, 2005 ISBN 1-57444-498-0; "Chemistry of protein conjugation and crosslinking" Shan S. Wong 1993, "Bioconjugation: protein coupling techniques for the biomedical sciences", M. Aslam, 1996.

Der Linker kann auch andere funktionelle Gruppen, bzw.Abschnitte enthalten, beispielsweise eine oder mehrere unter milden Bedingungen spaltbare Gruppen s. Anmeldungen Cherkasov et al WO 2005044836, Cherkasov et al WO2006097320, Cherkasov et al WO 2008043426, Cherkasov et al DE 10356837, Cherkasov et al DE 102004009704.

Eine spaltbare Verbindung innerhalb des Linkers ermöglicht eine Entfernung eines Teils des Linkers und der Marker-Komponente. Nach der Spaltung verbleibt ein LinkerRest an der Nuk-Komponente, Beispiele für spaltbare Verbindungen sind im Abschnitt 1.3.3.1.4 angegeben.

### 1.3.3.3 Marker-Komponente

Die Struktur der Marker-Komponente ist vorzugsweise an ihre Funktionen angepasst. In der vorliegenden Anmeldung hat die Marker-Kompontente Vorzugsweise eine oder mehrere folgender Funktionen: a) Erkennung einer oder mehrerer Zielsequen, b) Bindung an eine feste Phase, c) Generierung eines spezifischen Signals. Diese Funktionen werden von einzelnen Teilen der Marker-Komponetene erfüllt.

In dieser Anmeldung wird Begriff "Domäne" verwendet. Dieser Begriff dient zur Beschreibung eines Teils oder einer Gruppe von Teilen des Nuk-Makromoleküls mit einer gemeinsamen Funktion. Einzelne Teile der Domänen können auch als Marker-Einheiten bezeichnet werden. Dabei kann eine Domäne aus einer oder mehreren Marker-Einheiten mit gleicher Funktion gebildet werden. Die Bezeichnung Domäne soll einem Fachmann eine bessere Übersicht über die möglichen Kombinationen einzelner Strukturen und ihre Funktionen vermitteln. Sie dient nicht zur Einschränkung auf bestimmte Strukturen.

Begriff "Marker-Einheit" wird deskriptiv/ beschreiben für Strukturen verwendet, die eine Funktion erfüllen und gut mit für einen Fachmann bekannten Strukturen korrelieren, z.B. Biotin, Farbstoff, Fluoreszenzfarbstoff, Oligonukleotid, Quantum-Dot, Nanoteilchen, eine reaktive Gruppe usw.. Beispielsweise ein Farbstoff oder ein Mikroteilchen oder eine Amino-Gruppe stellen für den Fachmann bekannte Elemente dar. Ähnlich wie im Bereich der Polymer-Chemie stellen einzelne Marker-Einheiten die Bausteine einer makromolekularen Struktur dar. Für eine anschauliche Beschreibung, können Marker-Einheiten mit ähnlichen Funktionen zu Domänen zusammengefasst werden. Im einfachsten Fall schließt eine Domäne nur eine Marker-Einheit ein, z.B. eine Anker-Domäne mit einem Biotin. Es können aber mehrere Biotin-Moleküle zu einer Anker-Domäne zusammengefasst werden.

Erfindungsgemäß kann eine Marker-Komponente der Nuk-Makromoleküle folgende Domäne einschließen: eine oder mehrere Target-Domänen, eine oder mehrere Anker-Domänen, eine oder mehrere Signal-Domänen. In einer weiteren Ausführungsform schließen Nuk-Makromoleküle entsprechende Antagonisten ein.

**Target-Domäne:** Die Erkennung einer Zielsequenz oder mehreren Zielsequenzen wird von einem Teil der Marker-Kompontente gewährleistet, einer sogenannten Target-Domäne. Beispiele für Target-Domäne sind Nukleinsäureketten oder deren Analoga, die an die Zielsequenz binden können. Unterschiedliche Zielsequenzen können durch unterschiedliche, spezifische Target-Domänen oder durch eine Target-Domäne mit breitem Sequenzerkennungsspektum erkannt werden.

**Anker-Domäne:** Die Bindung an eine feste Phase wird von einem weiteren Teil der Marker-Komponente ermöglicht, einer sogenannten Anker-Domäne. Diese Anker-Domäne kann an eine feste Phase über eine affine oder kovalente Bindung binden. Beispiele für Anker-Domäne stellen ebenfalls Nukleinsäureketten, z.B. Oligonukleotide, die an die komplementäre Partner, fixiert an einer festen Phase, binden können (komplementäre Bindung von Nukleinsäureketten, oder Aptamer-Protein-Bindung). Weitere Beispiele für Anker-Domäne stellen Biotin oder Haptene (z.B. Farbstoffe, Digoxigenin, DNP), oder Proteine mit Fähigkeit, andere Moleküle zu binden (z.B. Streptavidin (SA), Antikörper, Lektine usw.). Einem Fachmann sind viele Bespiele für Bindugnspartner für affine Bindungen bekannt. Generell, ein Partner aus einem solchem Bindungspaar ist ein Bestandteil der Marker-Komponente (Anker-Domäne), ein weiterer Partner - Bestandteil der festen Phase (Bindungspartner).

**Signal-Domäne** der Markerkompontente vermittelt die spezifische Erkennung bzw. Nachweis der Nuk-Makromoleküle. Mehrere Beispiele sind unten angegeben und sind einem Fachmann bekannt.

### Antagonisten

In einer Ausführungsform schließt die Marker-Kompontente Strukturen ein, die Eigenschaften bzw. die Funktion einer Domäne reversibel blockieren kann. Diese Struktur wird als Antagonist zu der jeweiligen Domäne bezeichnet. Die Wirkung des Antagonisten ist reversibel, so dass die Eigenschaften der Domänen wiederhergestellt werden können.

### Antagonist der Anker-Domäne:

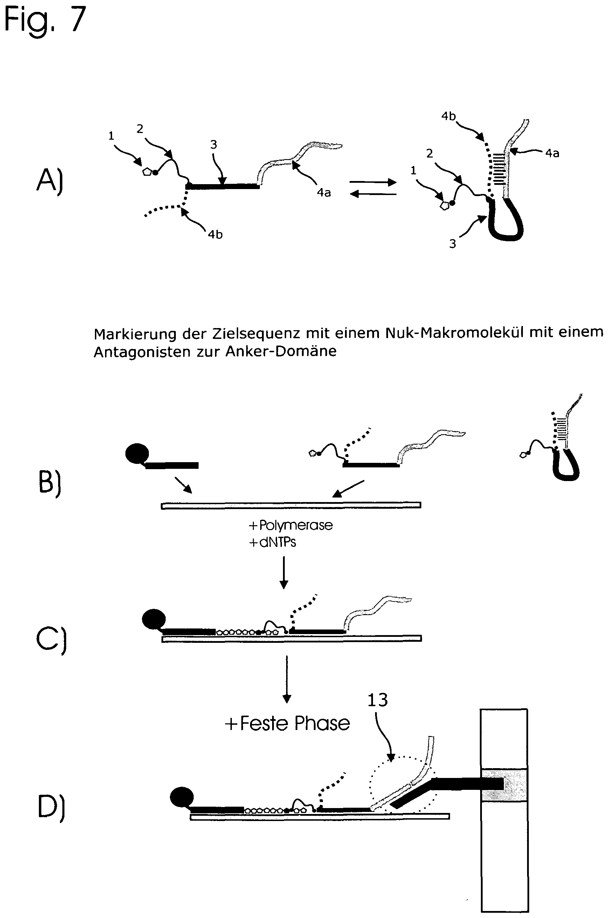

Anker-Domäne hat die Aufgabe, Bindung an die feste Phase zu vermitteln. In einigen Ausführungsformen schließt die Anker-Domäne Oligonukleotide ein, die durch Hybridisierung an einen komplementären, immobilisierten Bindungspartner binden. Ein Antagonist für eine solche Anker-Domäne stellt beispielsweise ein komplementäres Oligonukleotid innerhalb des Nuk-Makromoleküls dar, das die Anker-Domäne reversibel binden kann und dadurch die Interaktion mit dem Bindungspartner an der festen Phase verhindert (Fig. 3i). Die Wirkung des Antagonisten wird beispielsweise bei der Bindung eines Nuk-Makromoleküls an die Zielsequenz und den Einbau der Nuk-Komponente dauerhaft aufgehoben (Fig. 7). Durch die frei gewordene Anker-Domäne kann nun die Bindung an die feste Phase stattfinden. In einer weiteren Ausführungsform der Erfindung, kann ein Antagonist der Anker-Domäne vom Nuk-Makromolekül durch chemische oder enzymatische Reaktion abgespalten werden.

### Antagonist der Signal-Domäne:

Quencher-Moleküle stellen für Signal-Domäne mit Fluoreszenzeigenschaften Beispiele von Antagonisten dar. Diese Antagonisten können beispielsweise über eine spaltbare chemische Bindung bzw. über eine Harrnadel-ähnliche Struktur der Nukleinsäureketten ("molecular beacons", FRET-Paare) in die Nähe der Marker-Einheiten gebracht werden, die Fluoreszeigenschaften haben. Dadurch wird das Signal gemindert oder aufgehoben. Nach der Abspaltung der Quencher oder durch Zerstörung der Haarnadel-Struktur der Nukleinsäuren, erhöhet sich der Abstand zwischen einem Quencher und dem Fluoreszenzfarbstoff. Es kommt zu Steigerung des Signals.

Bei der Beschreibung werden die Antagonisten zusammen mit Domänen betrachtet, deren Wirkung sie reversibel blockieren.

Die einzelnen Domänen und ihre Antagonisten sind intergriert zu einer Marker-Kompontente. Die Intergration kann durch Kopplung einzelner Domänen aneinander stattfinden oder sie werden mittels Linker verknüpft oder einzelne Domänen sind an eine weitere Struktur gekoppelt, z.B. an eine Kern-Kompontente des Markers.

Einzelne Domänen können aus einer oder mehreren Marker-Einheiten bestehen. In einer Ausführungsform schließt eine Domäne nur eine Marker-Einheit ein. Beispielsweise, eine Target-Domäne schließt nur eine Oligonukleotidsequenz ein, die an eine Zielsequenz binden kann. Beispiel für eine Anker-Domäne mit nur einer Struktur-Einheit ist Biotin oder auch ein Oligonukleotid. Beispiel für eine Signal-Domäne mit nur einer Struktur-Einheit ist ein Fluoreszenzfarbstoff oder ein Quantum-Dot.

In einer weiteren Ausführungsform kann eine Domäne mehrere Marker-Einheiten einschließen. Beispielsweise, mehrere gleiche oder unterschiedliche Oligonukleotidsequenzen können in einem Nuk-Makromolekül gekoppelt werden und an den gleichen Abschnitt in der Zielsequenz binden. In diesem Fall bilden die einzelnen Oligonukleotidsequenzen die Marker-Einheiten und ihre Gesamtheit stellt eine Target-Domäne dar. Ähnlich können auch mehrere Oligonukleotide oder mehrere Biotin-Molelüle zu einer Anker-Domäne zusammengefasst werden.

Ein Nuk-Makromolekül kann auch mehrere Target-Domänen oder mehrere Anker-Domänen oder mehrere Signal-Domänen einschließen. Dies ist dann der Fall, wenn sich die Domänen einer Gruppe in ihrer Funktion unterscheiden. Beispielsweise binden unterschiedliche Target-Domänen eines Nuk-Makromoleküls an unterschiedliche Abschnitte in einer Zielsequenz oder an unterschiedliche Zielsequenzen. Die Funktion der Bindung an die feste Phase kann ebenfalls durch nur eine oder auch mehrere unterschiedliche Anker-Domänen erfüllt werden.

Ein Nuk-Makromolekül kann eine Marker-Komponente einschließen, die nur eine einzelne Funktion oder auch eine Kombination aus zwei oder mehr Funktionenen hat. Je nach Funktion unterscheidet sich auch die Zusammensetzung einzelner Nuk-Makromolekül-Arten.

Die Darstellungsweise für die Zusammensetzung des Markers kann dabei beispielseise folgende sein: eine Target- und eine Signal-Domänen = [T1;S1], zwei verschiedene Anker-Domänen und eine Signal-Domäne = [A1;A2;S1]; vier verschiedene Target-Domänen und eine Anker-Domäne = [T1;T2;T3;T4;A1], drei gleiche Target-Domänen, eine Anker-Domäne und eine Signal-Domäne = [3×T1;A1;S1] usw.

Die Antagonisten einzelner Domänen können beispielsweise durch einen zusätzlichen Buchstaben gekennzeichnet werden, beispielswiese ein Antagonist zu Anker-Domäne wird ein [aA], ein Antagonist zu Signal-Domäne mit [aS] bezeichnet. Die Beschreibung der Zusammensetzung der Marker kann dabei wie folgt aussehen: [aA1;T1;A1]. Der Antagonist [aA1] blockiert reversibel die Bindungseigenschaften der Anker-Domäne [A1].

Bei einer allgemeinen Struktur für Nuk-Makromoleküle, z.B. (Nuk-Linker)ₙ-Marker, kann die detailiertere Zusammensetzung von verschiedenen Arten von Nuk-Makromolekülen beispielsweise folgendermassen dargestellt werden:
(Nuk-Linker)ₙ-Marker steht für eine generelle Beschreibung von Nuk-Makromolekülen.
(Nuk-Linker)ₙ -[T1;S1] steht für generelle Beschreibung von Nuk-Makromolekülen mit einer Target-Domäne und einer Signal-Domäne.
dUTP-Linker-[T1;S1] steht für generelle Beschreibung von Nuk-Makromolekülen mit dUTP als Nuk-Komponente und einer Target-Domäne und einer Signal-Domäne.
(dCTP-Linker)₅-[T1;S1] steht für generelle Beschreibung von Nuk-Makromolekülen mit fünf dCTP-Linker-Einheiten und einer Target-Domäne und einer Signal-Domäne. Bei Bedarf kann die Beschreibung für einzelne Bestandteile in mehr oder weniger Details dargestellt werden. Da alle Nuk-Makromoleküle einen Linker zwischen der Nuk-Kompontente und Marker-Komponente einschließen, kann seine Angabe bei der Aufzählung von Kompontenten von Nuk-Makromolekülen unterlassen werden, z.B.
(Nuk1)-[T1;A1], (Nuk2)-[T2;A2] usw.

Nachfolgend sind einige Beispiele für die Zusammensetzung von Nuk-Makromolekülen mit genereller Formel (Nuk-Linker)ₙ-Marker präsentiert (Fig 3).
(Nuk-Linker)ₙ -[T]
(Nuk-Linker)ₙ -[A]
(Nuk-Linker)ₙ -[T; S]
(Nuk-Linker)ₙ-[T; A]
(Nuk-Linker)ₙ-[S; A]
(Nuk-Linker)ₙ -[T; A; S]
(Nuk-Linker)ₙ -[T_{Q}]
(Nuk-Linker)ₙ -[A_{R}]
(Nuk-Linker)ₙ -[T_{Q}; S_{P}]
(Nuk-Linker)ₙ -[T_{Q}; A_{R}]
(Nuk-Linker)ₙ -[S_{P}; A_{R}]
(Nuk-Linker)ₙ -[T_{Q}; A_{R}; S_{P}]

Die Anteile, die zum Marker gehören, sind in [ ] eingeschlossen: T= Target-Domäne; A= Anker-Domäne; S= Signal-Domäne.
wobei die Anzahl von Nuk-Komponenten (gekoppelt über einen Linker) (n) schließt in einer Ausführungsform der Erfindung Bereiche von 1 bis 100, in einer anderen Ausführungsform Bereiche zwischen 100 und 10000 ein. Diese Anzahl kann oft einen Durchschnittswert der Population einer Art von Nuk-Makromolekülen darstellen.
Die Anzahl (P), (Q) und (R) für einzelne Domänen schließen unabhängig von einander in einer Ausführungsform der Erfindung Bereiche von 1 bis 100, in einer anderen Ausführungsform Bereiche zwischen 100 und 10000 ein. Wobei die Eigenschaften einzelner Domäne innerhalb einer Gruppe (z.B. 100 Anker-Domänen) gleich oder unterschiedlich sein können. Dadurch kann beispielsweise die effektive Konzentration einzelner Domänen oder deren Vielfalt oder beides erhöht werden. Die Anzahl kann oft einen Durchschnittswert der Population einer Art von Nuk-Makromolekülen darstellen.

Ein Marker kann folgende Domäne einschließen: In einer Ausführungsform mindestens eine Target-Domäne, oder mindestens eine Anker-Domäne, oder mindestens eine Signal-Domäne. In einer weiteren Ausführungsform schließt der Marker ein: entweder eine Kombination aus mindestens einer Target-Domäne und mindestens einer Signal-Domäne, oder eine Kombination aus mindestens einer Target-Domäne und mindestens einer Anker-Domäne, oder eine Kombination aus mindestens einer Anker-Domäne und mindestens einer Signal-Domäne. In einer weiteren Ausführungsform schließt der Marker eine Kombination aus allen drei Funktionen ein, wobei mindestens eine Target-Domäne, mindestens eine Anker-Domäne, mindestens eine Signal-Domäne kombiniert werden. In einer weiteren Ausführungsform schließt der Marker ein: entweder eine Kombination aus mindestens einer Target-Domäne und mindestens einer Signal-Domäne und mindestens einem Antagosisten zu Signal-Domäne (z.B. ein Quencher), oder eine Kombination aus mindestens einer Target-Domäne und mindestens einer Anker-Domäne und einem Antagonisten zu Anker-Domäne.

Kombinationen zwischen einer zielsequenzspezifischen Target-Domäne und einer Anker-Domäne innerhalb einer Art von Nuk-Makromolekülen können dermassen gestaltet werden, dass die Eigenschaften einer Anker-Domäne spezifisch, einzigartig und charakteristisch an die Merkmale der jeweilige Target-Domäne kombiniert sind. Durch eine solche einzigartige charakteristische Kombination wird eine Anker-Domäne zielsequenzzugeordnet, z.B. Nuk1-[T1;A1].

Die einzelnen Domänen können untereinander direkt oder mit Hilfe der Kern-Komponente verbunden sein.

Im Folgenden, werden einige Beispiele für die Strukturen und Funktionen einzelner Domänen der Marker-Kompontente ausführlicher besprochen.

### 1.3.3.3.1 Target-Domäne.

Die Target-Domäne hat die Aufgabe, das gesamte Nuk-Makromolekül sequenzspezifisch an die Zielsequenz zu binden. Nuk-Makromoleküle können mittels einer Target-Domäne sequenzspezifisch vor oder während der enzymatischen Reaktion an die Zielsequenz binden, ähnlich wie ein Primer oder eine Sonde in einer Real-Time PCR an die Nukleinsäurekette während eines Zyklus bindet. Die Nuk-Kompontente der gebundenen Nuk-Makromoleküle kann durch eine Polymerase in den wachsenden Strang eingebaut werden (Fig. 5A).

Die Nukleobasen wie Adenin, Cytosin, Guanin, Thymin, Uracil (abgekürzt als A, C, G, T, U) oder deren Analoga gekoppelt an einen Zucker-Phosphatrückgrad in Form von DNA oder RNA oder deren Analoga, z.B. PNA, LNA, können sequenzspezifisch an die Nukleinsäurestränge binden. Verschiedene Nukleinsäureketten, z.B. DNA, RNA, Proteinnukleinsäuren (PNA), Morpholino und deren Analoga können den Nukleinsäure-Anteil der Target-Domäne darstellen. Generell gesprochen, eignen sich für Target-Domäne Substanzen, die eine sequenzspezifische Bindung mit einer einzelsträngigen oder einer doppelsträngigen Nukleinsäurekette eingehen können. Gewöhnlich haben solche Substanzen Nukleobasen (A, C, T, G, U) oder deren Abkömmlinge, die eine sequenzspezifische Bindung ermöglichen. Der Rückgrad kann dabei eine natürliche Zusammensetzung haben (Zucker-Phosphatrückgrad) oder eine Abwandlung davon, z.B. PNA. Einzelne Target-Domänen innerhalb einer Art von Nuk-Makromolekülen können dabei durchgehend aus einer Art der Bausteine bestehen, z.B. nur DNA oder nur PNA, oder auch ein gemischtes, zusammengesetztes Polymer darstellen, wo stellenweise DNA, RNA, PNA, Morpholino, LNA oder weitere Modifikationen zu einer Kette zusammengefasst sind. Falls mehrere Target-Domänen innerhalb einer Art von Nuk-Makromolekülen vorkommen, können einzelne Domänen unterschiedliche Arten von Bausteinen vorweisen, z.B. eine Target-Domäne besteht aus DNA, die andere aus PNA, noch eine weitere aus RNA.

Zur Vereinfachung der Darstellung werden Nukleinsäureketten in Form der DNA in Details besprochen. Andere Arten der Nukleinsäureketten können entsprechend den einem Fachmann bekannten Regeln nach dem Muster der DNA-Oligonukleotide konstruiert und eingesetzt werden.

Die Kopplung der Target-Domäne im Nuk-Makromolekül erfolgt in einer Ausführungsform an einem der beiden Enden der Target-Domäne, z.B. am 5'-Ende oder am 3'-Ende. Beispiele für Kopplung eines Oligonukleotides an einem seiner Enden sind einem Fachmann bekannt. In einer anderen Ausführungsform der Erfindung erfolgt die Kopplung im inneren Bereich der Target-Domäne, beispielsweise über einen an eine Base gekoppelten Linker.
Die Target-Domäne kann über einen Linker direkt an die Nuk-Komponente gekoppelt sein oder sie kann an die Kern-Komponente des Nuk-Makromoleküls gekoppelt sein.

In einer Ausführungsform der Anmeldung kann die Target-Domäne durch die 5'-3'-Exonukleasenaktivität einer Polymerase komplett oder teilweise abgebaut werden. Die Struktur der Nuk-Makromolekülen kann dabei dermassen gestaltet werden, dass die eingebaute Nuk-Komponente mit dem Rest des Nuk-Makromoleküls im verlängerten komplementären Strang verbleibt. Dies kann beispielsweise dadurch erreicht werden, dass die Kopplung der Target-Domäne an den Rest des Nuk-Makromoleküls am 3'-Ende der Target-Domäne erfolgt oder in seiner Nähe.

In einer weiteren Ausführungsform der Anmeldung ist die Target-Domäne resistent gegenüber einer 5'-3'-Exonuklease-Aktivität. Dies kann beispielsweise durch den Einsatz von PNA oder weiterer Modifikationen erreich werden.

Eine Zielsequenz kann in Form einer einzelsträngigen oder einer doppelsträngigen DNA oder RNA vorliegen. Die Bindung der Target-Domäne an die Zielsequenz kann in einer Ausführungsform unter Ausbildung von Doppelsträngen (nach den Regeln der Watson-Crick Basenpaarung) erfolgen, in einer anderen Ausführungsform werden Triple-Stränge (nach Hoogsteen Regeln) gebildet. Beispiele für eine sequenzspezifische Bindung von Nukleinsäureketten sind einem Fachmann bekannt. Die Länge und die Zusammensetzung der Target-Domäne wird dermassen angepasst, dass die Target-Domäne an die Zielsequenz unter jeweiligen Reaktionsbedingungen erfolgen kann.

Im Folgenden, wird die Bindung an einen Einzelstrang als Beispiel betrachtet. Die Zusammensetzung einer Target-Domäne kann eine vollständige Komplementarität zur Zielsequenz aufweisen ("perfect Match") oder sich an einigen Stellen unterscheiden ("Miss-Match").
Bei einer gegebenen Zielsequenz können mehrere einem Fachmann bekannte Methoden zur Wahl einer passenden Sequenz für die Target-Domäne verwendet werden. Da die Target-Domäne innerhalb der Zielsequenz binden soll, können beispielsweisen die Regel angewandt werden, die bei der Wahl einer Real-Time-PCR-Sonde eingesetzt werden (Literatur siehe Abschnitt Amplifikationsmethoden) Andererseits sind einem Fachmann auch Regeln bekannt nach denen die Microarray-Oligonukleotide konstruiert werden. Bei einer gewünschten Bindung an eine einzelsträngige Zielsequenz werden beispielsweise koomplementäre Sequenzen einer Target-Domäne in der Länge von ca. 10 bis 50 Nukleobasen oder 15 bis 30 Nukleobasen ausgewählt.

Vorzugsweise schließen eine Target-Domäne von Nuk-Makromolekülen Nukleinsäureketten mit der Länge in folgenden Bereichen ein: von 3 bis 6, 6 bis 9, 9 bis 12, 12 bis 14, 14 bis 16, 16 bis 18, 18 bis 20, 20 bis 25, 25 bis 30, 30 bis 40, 40 bis 50, 50 bis 60, 60 bis 70, 70 bis 100, 100 bis 200, von 200 bis 500 Nukleobasen. Falls mehrere Target-Domänen innerhalb eines Nuk-Makromoleküls integriert sind, können sie unterschiedliche Längen haben. Einzelne Target-Domänen können innerhalb einer kontinuierlichen Nukleinsäurekette zusammengefasst sein oder auch als einzelstehendeMarker-Einheiten in einem Nuk-Makromolekül vorkommen.

In einer Ausführungsform der Anmeldung sind die Sequenzen einer Target-Domäne nur für eine Zielsequenz komplementär und können nur die Zielsequenz binden.

In einer weiteren Ausführungsform der Anmeldung ist die Sequenz einer Target-Domäne in der Lage eine Gruppe von unterschiedlichen Zielsequenzen zu binden.

In einer weiteren Ausführungsform der Anmeldung schließt ein Nuk-Makromolekül mehrere Target-Domäne bestehend aus jeweils unterschiedlichen Sequenzen ein, wobei diese Target-Domänen in der Lage sind, unterschiedliche Zielsequenzen zu binden.

In einer bevorzugten Ausführungsform der Anmeldung werden Sequenzen der Target-Domäne dermassen ausgewählt, dass sie nicht in der Lage sind, unter verwendeten Reaktionsbedinungen an die Anker-Domänen oder an die auf einer bereitgestellten festen Phase fixierten Bindungspartner zu binden.

In einer weiteren Ausführungsform der Anmeldung wird die Sequenz der Target-Domäne einer Art von Nuk-Makromolekülen dermassen ausgewählt, dass sie nicht in der Lage ist, unter verwendeten Reaktionsbedingungen an weitere Target-Domäne oder an Anker-Domäne oder weitere Bestandteile innerhalb derselben Art von Nuk-Makromolekülen zu binden.

In einer weiteren Ausführungsform der Anmeldung wird die Sequenz der Target-Domäne einer Art von Nuk-Makromolekülen dermassen ausgewählt, dass sie nicht in der Lage ist, unter verwendeten Reaktionsbedingungen an weitere Target-Domäne oder an Anker-Domäne oder weitere Bestandteile anderer Nuk-Makromoleküle zu binden die im selben Ansatz verwendet werden.

In einer weiteren Ausführungsform der Anmeldung wird die Länge und die Sequenz der Target-Domäne einer Art von Nuk-Makromolekülen dermassen gestaltet (sowie die Reaktionsbedingungen, z.B. Reaktionstemperatur, "stringente Bedingungen"), dass sie Abweichungen in der Zielsequenz diskriminieren kann. Diese Abweichungen können beispielsweise Nukleotid-Austausch (z.B. Adenosin statt Guanosin oder statt Cytosin), Nukleotid-Deletionen oder Nukleotid-Additionen sein. Beispielsweise kann die Target-Domäne unter gegebenen Reaktionsbedingungen die Abweichungen in der Zielsequenz unterscheiden, die folgende Bereiche einschließt: von 1 bis 2 Nukleotiden, oder 2 bis 5 Nukleotiden, oder 5 bis 10 Nukleotiden, oder 10 bis 20 Nukleotiden, oder 20 bis 50 Nukleotiden. Diese Abweichungen können an einer Position in der Zielsequenz vokommen oder über mehrere Stellen der Zielsequenz verteilt sein.

In einer weiteren Ausführungsform der Anmeldung wird die Länge und die Sequenz der Target-Domäne einer Art von Nuk-Makromolekülen dermassen gestaltet (sowie die Reaktionsbedingungen, z.B. Reaktionstemperatur, weniger "stringente Bedingungen"), dass sie Abweichungen in der Zielsequenz tolerieren kann. Diese Abweichungen können beispielsweise Nukleotid-Austausch (z.B. Adenosin statt Guanosin oder statt Cytosin), Nukleotid-Deletionen oder Nukleotid-Additionen sein. Beispielsweise kann die Target-Domäne unter gegebenen Reaktionsbedingungen die Abweichungen in der Zielsequenz tolerieren, die folgende Bereiche einschließt: von 1 bis 2 Nukleotiden, oder 2 bis 5 Nukleotiden, oder 5 bis 10 Nukleotiden, oder 10 bis 20 Nukleotiden, oder 20 bis 50 Nukleotiden. Diese Abweichungen können an einer Position in der Zielsequenz vokommen oder über mehrere Stellen der Zielsequenz verteilt sein. In dieser Ausführungsform werden somit nicht nur Zielsequenzen markiert, sonder auch andere, einer zielsequenz-ähnliche Nukleinsäureketten.

Eine Target-Domäne kann aus mehreren einzelnen Oligonukleotiden bestehen, die in einem Nuk-Makromolekül integriert sind. Auch mehrere Target-Domäne können in einem Nuk-Makromolekül vorkommen, dabei werden beispielsweise Oligonukleotide mit unterschiedlichen Sequenzen in einem Nuk-Makromolekül intergriert. Mehrere Target-Domänen können auch innerhalb einer Oligonukleotidkette integriert sein. Dabei können sie unmittelbar ineinander übergehen oder deren Sequenzen können überlappend gestalltet werden oder die Domäne durch eine weitere Sequenz getrennt werden.

Die einzelnen Target-Domänen können innerhalb einer Art von Nuk-Makromolekülen von gleicher oder von unterschiedlicher Nukleinsäureart sein. Beispielsweise können DNA-basierte Target-Domänen mit PNA-basierten Target-Domänen innerhalb einer Art von Nuk-Makromolekülen kombiniert sein. Die Synthese von gemischten Nukleinsäureketten bestehend aus z.B. DNA und PNA ist bekannt.

Eine Target-Domäne kann zusätzliche Modifikationen tragen, wie beispielsweise signalgebende oder signalvermittelnde Moleküle, z.B. Farbstoffe, Fluoresenzfarbstoffe oder Biotin, oder makromolekulare Substanzen, wie Enzyme oder Nanokristalle. Modifizierte Oligonukeotide kann man kommerziell erwerben z.B. MWG-Biotech. Vorzugsweise ist das 3'-Ende einer solchen Target-Domäne (z.B. DNA-Oligonukleotid) nicht enzymatisch aktiv, d.h. eine Polymerase ist nicht in der Lage ein Nukleotid an das 3'-Ende eines solchen Oligonukleotides zu koppeln. Dies kann beispielsweise durch Kopplung einer Gruppe erreicht werden, z.B. einer Phasphat oder Amino oder einer weiteren Gruppe.

Eine Target-Domäne kann die Funktion einer Kern-Komponente erfüllen und Vebindung zwischen einzelnen Teilen des Markers eines Nuk-Makromoleküls darstellen. Die Linker-Komponente kann direkt an die Target-Domäne gekoppelt sein.

Bezüglich der chemischen Synthese von Oligonukleotiden, die Target-Domäne bilden, und deren Modifikation kann ein Fachmann an folgende Quellen verwiesen werden: Singh et al Chem Soc Rev, 2010, v.39, 2054-, "Oligonucleotide synthesis, methods and applications" Piet Herdewijn, 2004, ISBN 1-58829-233-9, Protocols for oligonucleotide conjugates, synthesis and analytical techniques" Sudhir Agrawal, 1993, ISBN 0-89603-252-3, "Protocols for oligonucleotide conjugates, synthesis and properties" Sudhir Agrawal, 1993, ISBN 0-89603-247-7, "The aptamer handbook" Sven Klussmann, 2006, ISBN 10: 3-527-31059-2, "Pharmaceutical aspects of oligonucleotides" Patrick Couvreur, 2000, ISBN 0-748-40841-X,
"Triple Helix forming Oligonucleotides"Claude Malvy, 1999, ISBN 0-7923-8418-0, "Artificial DNA, methods and applications" Yury E. Khudyakov, ISBN 0-8493-1426-7

In einer weiteren Ausführungsform der Anmeldung werden Sequenzen der Target-Domäne dermassen ausgewählt, dass sie in der Lage sind, einen doppelsträngigen Abschnitt oder Haarnadel-Strukturen innerhalb einer Target-Domäne zu bilden.

In einer weiteren Ausführungsform der Anmeldung werden Sequenzen der Target-Domäne dermassen ausgewählt, dass sie nicht in der Lage sind, unter verwendeten Reaktionsbedinungen einen doppelsträngigen Abschnitt oder Haarnadel-Strukturen innerhalb einer Target-Domäne zu bilden.

In einer weiteren vorteilhaften Ausführungsform der Anmeldung sind die Target-Domäne und weitere Domänen (z.B. Anker-Domänen und/ oder Signal-Domänen) innerhalb einer Nukleinsäurekette positioniert. Die Target-Domäne kann beispielsweise den Anteil der Nukleinsäurekette am oder in der Nähe von 5'-Ende bilden und die Anker-Domäne oder Signal-Domäne den Anteil der Nukleinsäurekette am oder in der Nähe von 3'-Ende. Eine solche Anordnung Domänen erlaubt eine gleichzeitige Synthese von beiden Domänen während einer Synthese des Oligonukleotid-Anteils des Nuk-Makromoleküls. In weiteren Ausführungsformen kann beispielsweise eine oder mehrere Target-Domänen von mehreren Anker-Domänen oder Signal-Domänen innerhalb einer Nukleinsäurekette umgeben sein. Als weiteres Beispiel, können eine oder mehrere Signal-Domäne auch mehren Target-Domänen umgeben sein.

In einer vorteilhabten Ausführungsform der Erfindung sind Sequenzen der Target-Domäne und die der Anker-Domäne oder Signal-Domäne überlappend. In dieser Ausführungsform sind einige Nukleobasen Bestandteile von mindestens beiden Domänen. Die Länge der Sequenz, die das gemeinsame Fragment für die Target- und die Anker-Domäne oder die Target- und die Signal-Domäne bildet, kann beispielsweise von 5 bis 80% der Sequenz einer der Domänen einschließen.

In einer weiteren vorteilhaften Ausführungsform der Erfindung sind Sequenzen der Target-Domäne und die der Anker-Domäne oder die der Signal-Domäne durch eine oder mehrere Spacer-Sequenzen getrennt. In einer Ausführungsform der Erfindung schließt eine solche Spacer-Sequenz die Funktion mit signalvermittelnden Eigenschaften ein. In einer weiteren Ausführungsform der Erfindung schließt eine Spacer-Sequenz Sequenzen ein, die komplementär zu einer Target-Domäne oder zu einer Anker-Domäne oder zu Signal-Domäne sind. Diese Abschnitte innerhalb der Spacer-Sequenz können vollständig oder nur teilweise komplementär zu einer der Domänen sein. In solchen Ausführungsformen hat eine Spacer-Sequenz die Rolle eines Antagonisten.

Vorzugsweise ist das 3'-Ende einer Target-Domäne (z.B. DNA-Oligonukleotid) nicht enzymatisch aktiv und ist beispielsweise blockiert, d.h. eine Polymerase ist nicht in der Lage ein Nukleotid an das 3'-Ende eines solchen Oligonukleotides zu koppeln. Dies kann beispielsweise durch Kopplung einer Gruppe erreicht werden, z.B. einer Phasphat-Gruppe oder einer Amino- oder einer weiteren Gruppe.

Die Target-Domäne kann die Funktion einer Kern-Komponente erfüllen und Vebindung zwischen einzelnen Teilen des Markers eines Nuk-Makromoleküls darstellen. Beispielsweise kann die Linker-Komponente direkt an die Target-Domäne gekoppelt sein.

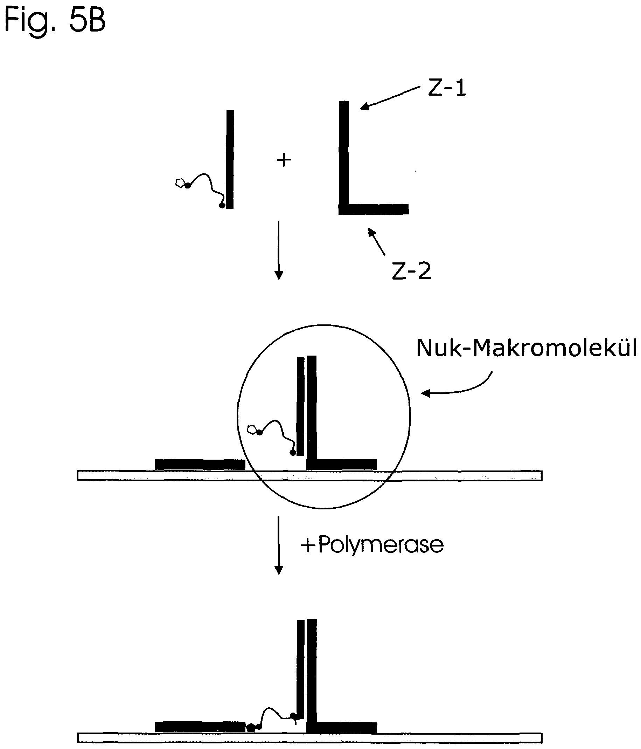

In einer weiteren Ausführungsform bindet die Target-Domäne des Nuk-Makromoleküs vor oder während der Reaktion an eine Hybridisierungssonde, (Z), statt an die Zielsequenz (Fig. 5B). Eine solche Sonde schließt vorzugsweise ein Oligonukleotid ein. Dieses Oligonukleotid kann sowohl an die Target-Domäne des Nuk-Makromoleküls spezifisch binden (Sequenzabschnitt Z-1) als auch an die Zielsequenz (Sequenzabschnitt Z-2). Sequenzabschnitt Z-2 ist somit einer für die Zielsequenzspezifische Target-Domäne. In einer weiteren Ausführungsform wird eine Art von Nuk-Makromolekülen mit einer für die Hybridisierungssonde spezifischen Target-Domäne in Kontakt mit mehreren Hybridisierungsonden gebracht, die jeweils gleiche Bindungsstellen (Z-1) für die Target-Domäne eines Nuk-Makromoleküls aber unterschiedliche Bindunsstellen für die Zielsequenz (Z2).
Dadurch ist es möglich mit einer einheitlichen Art von Nuk-Makromolekülen unterschiedliche Zielsequenzen zu markieren.
Vorzugsweise ist die Bindung zwischen dem Nuk-Makromolekül und der Hybridiserungssonde unter Bedingungen einer Markierungsreaktion stabil. Dies kann beispielsweise durch die Sequenzwahl des Sequenzabschnitts (Z-1) und der Target-Domäne der Nuk-Makromoleküle mit einer höheren Tm erreichen, verglichen mit der Tm des Abschnitts Z-2 der Hybridisierungssonde.

Das gesamte Konstrukt, einschließlich kovalent aneinader gebundenen Nuk-Komponente, des Linkers, des Oligonukletids und der damit durch eine Affinitätsbindung (durch Basenpaarung entstandene Bindung zwischen zwei Nukleinsäuresträngen) gekoppelten Oligonukleotide (Hybridisierungssonde in Fig. 5B) als Nuk-Makromolekül bezeichnet werden. Die Synthese / die Ausbildung eines reaktionsfähigen Nuk-Makromoleküls im Sinne dieser Anmeldung erfolgt somit unmittelbar vor oder sogar während der Markierungsreaktion. Das Oligonukleotid, das mit der Nuk-Komponente in kovalenter Verbindung steht, kann auch als eine Kern-Komponente bezeichnet werden (s.u.), weils es die Rolle eines Vermittlers zwischen zwei Funktionalitäten, der Nuk-Komponente und der Target-Domäne, erfüllt.

### Antagonisten der Target-Domäne

Ein Antagonist der Target-Domäne blockiert reversibel ihre Funktion der Bindung an die Ziel-Sequenz. Die Blockade kann vollständig sein oder unvollständig aber dennoch die Fähigkeit der Target-Domäne zur Bindung an die Zielsequenz stark reduzieren.

In einer Ausführungsform schließen Nuk-Makromoleküle Nukleinsäureketten ein, die zur Target-Domäne vollständig oder teilweise komplementär sind. Diese Nukleinsäureketten können beispielsweise aus DNA, RNA oder PNA sein und zwischen 5 und 90% der Sequenz einer Target-Domäne binden. Bevorzugt sind Ausführungsformen, in denen die Target-Domäne unter Reaktionsbedingungen einer Markierungsreaktion in offener Form vorliegt.

Die Antagonisten werden im Abschnitt Anker-Domäne ausführlicher besprochen.

### 1.3.3.3.2 Die Anker-Domäne eines Nuk-Makromoleküls und Kombination mit der festen Phase

Die Anker-Domäne hat die Aufgabe, eine Art von Nuk-Makromolekül oder einen Nukleinsäurestrang, was mit einem solchen Molekül markiert ist, spezifisch an eine feste Phase zu binden. Nuk-Makromoleküle oder die mit Nuk-Makromolekülen markierte Nukleinsäurestränge (z.B. Zielsequenzen oder deren Äquivalente) können mittels einer Anker-Domäne vor oder während oder nach der enzymatischen Reaktion an eine feste Phase gebunden werden.

Erfindungsgemäß können in einigen Ausführungsformen feste Phasen für die Analyse bereitgestellt werden, die in der Lage sind, mindestens eine Anker-Domäne spezifisch zu binden. Die Bindung zwischen einer Anker-Domäne und einer festen Phase kann affin oder kovalent erfolgen. Es gibt viele Bindungspaare, wie z.B. Biotin-Streptavidin oder Biotin-Avidin, oder Antigen-Antikörper, oder zwei komplementäre Nukleinsäuresträge, oder Aptamer-Zielprotein usw. Substanzen, die an die Anker-Domäne spezifisch binden können werden als Bindungspartner genannt.

Die Bindung eines Bindungspartner einer Anker-Domäne an die feste Phase ist einem Fachmann bekannt. Die Bindungspartner können in einer bestimmten Anordnung an die feste Phase gebunden werden, so dass eine eindeutige räumliche Zuordnung der Bindungsereignisse stattfinden kann. Beispiele für eine räumlich geordnete Bindung von Bindungspartnern für Anker-Domänen an eine feste Phase stellen feste zweidimensionalle Microarrays (erhältlich z.B. von Affymetrix, Eppendorf) oder feste monodimensionalle Teststreifen (z.B. Westernblots von Dako oder DNA-Streife vertrieben von Firma Innogenetics). Bindungspartner können an einer festen Phase gebunden werden, wobei die Zuordnung der festel Phase zu einem bestimmten Bindungspartner und entsprechend zu einer Anker-Domäne über andere, spezifische Kodierung erfolgen kann. Beispiele dafür stellen so genannte flüssige Bead-Arrays (erhältlich z.B. von Luminex oder Illumina). Weitere Beispiele sind einem Fachmann bekannt (Hinweise auf Literarur siehe unten).

Die feste Phase kann in unterschiedlicher Form bereitgestellt sein. Beispielsweise, können Reaktionsgefäße, plane Oberfläche, Kügelchen, Gele als feste Phase verwendet werden. Die feste Phase mit fixierten Bindungspartnern kann als ein Teil einer Vorrichtung zum Flüssigkeitsaustausch bereitgestellt sein. Solche festen Phasen sind einem Fachmann bekannt. Beispiele dafür stellen Lateral-Flow-Devices, Mikrofluidic-Vorrichtungen, Mirotiter-Platten usw. (Hinweise auf Literarur siehe unten)

Erfindungsgemäß werden eine oder mehrere solcher festen Phase bereitgestellt, die möglichst geringe unspezifische Bindung von Nukleinsäureketten haben.

In einer Ausführungsform schließt eine Anker-Domäne mindestens ein Biotin ein. Die feste Phase schließt entsprechend Streptavidin oder Avidin ein.

In einer weiteren Ausführungsform schließt eine Anker-Domäne mindestens Streptavidin oder Avidin ein. Die feste Phase schließt entsprechend Biotin ein.

In einer weiteren Ausführungsform schließt eine Anker-Domäne mindestens ein kleines Antigen, Hapten (z.B. Farbstoff (z.B. Fluoreszein), oder Digoxigenin oder DNP) oder größeres Antigen (z.B. ein Protein) ein. Die feste Phase schließt entsprechend einen passenden Antikörper ein.

Falls in einer Reaktion unterschiedliche Nuk-Makromoleküle eingesetzt werden, können ihre Anker-Domäne unterschiedliche, aber jeweils für eine Art von Nuk-Makromolekülen spezifische Strukturen (z.B. Antigene, Haptene, oder OligonukleotidSequenzen) einschließen. Durch Verwendung einer festen Phase mit mehreren räumlich angeordnet, getrennt gebundenen, gegen besagte Strukturen gerichtete Bindungspartner (z.B. Antikörpern oder komplementäre Oligonukleotide), können die besagten unterschiedlichen Nuk-Makromoleküle oder die mit ihnen markierten Nukleinsäureketten an unterschiedliche Positionen auf einer festen Phase binden. Beispiele stellen Immunoassay, die nach dem Prinzip der "Lateral-Flow-Vorrichtungen" zusammengesetzt sind.

In einer weiteren Ausführungsform schließt eine Anker-Domäne mindestens ein Oligonukleotid ein. Die feste Phase schließt entsprechend ein komplementäres Oligonukleotid ein. Falls in einer Reaktion unterschiedliche Nuk-Makromoleküle eingesetzt werden, können ihre Anker-Domäne unterschiedliche, aber jeweils für eine Art von Nuk-Makromolekülen spezifische Oligonukleotide einschließen. Durch Verwendung einer festen Phase mit mehreren räumlich angeordnet, getrennt gebundenen zu Anker-Domäne spezifisch komplementären Nukleinsäureketten, können die besagten unterschiedlichen Nuk-Makromoleküle oder die mit ihnen markierten Nukleinsäureketten an unterschiedliche Positionen auf einer festen Phase binden.

In einer weiteren Ausführungsform schließt eine Anker-Domäne mindestens ein Oligonukleotid mit einer Aptamer-Funktion ein. Die feste Phase schließt entsprechend ein für dieses Aptamer spezifisches Protein ein. Falls in einer Reaktion unterschiedliche Nuk-Makromoleküle eingesetzt werden, können ihre Anker-Domäne unterschiedliche, aber jeweils für eine Art von Nuk-Makromolekülen spezifische Aptamere (Oligonukleotide) einschließen. Durch Verwendung einer festen Phase mit mehreren räumlich angeordnet, getrennt gebundenen und zu der jeweligen Anker-Domäne passenden Proteinen, können die besagten unterschiedlichen Nuk-Makromoleküle oder die mit ihnen markierten Nukleinsäureketten an unterschiedliche Positionen auf einer festen Phase binden. Die Anker-Domäne können auch Proteine einschließen, gegen welche auf einer festen Phase gerigete Aptamere gerichtet sind.

In einer weiteren Ausführungsform der Anmeldung wird die Zusammensetzung der Anker-Domäne (z.B. Länge und die Sequenz der Oligonukleotide) der verwendeten Nuk-Makromoleküle und die der entsprechenden Bindungspartner dermassen gestaltet (sowie die Reaktionsbedingungen, z.B. Reaktionstemperatur, "stringente Bedingungen"), dass jeweiligen Anker-Domänen von ihren entsprechenden Bindungspartnern spezifisch erkannt und gebunden werden.

In einer weiteren Ausführungsform der Anmeldung wird die Zusammensetzung (z.B. Länge und die Sequenz) der Anker-Domäne der verwendeten Nuk-Makromoleküle und die der entsprechenden Bindungspartner dermassen gestaltet (sowie die Reaktionsbedingungen, z.B. Reaktionstemperatur, weniger "stringente Bedingungen"), dass mehrere unterschiedliche Anker-Domänen an einen Bindungspartner binden können. Die Zusammensetzung der Anker-Domänen und die der Bindungspartner wird dermassen gestaltet (z.B. durch Variation in der Sequenz der Oligonukleotide) dass mindestens 2 oder mindestens 3 bis 5 oder 5 bis 10 Anker-Domäne an einen Bindungspartner binden können.

Im Folgenden werden Beispiele mit Oligonukleotiden als eine Anker-Domäne ausführlicher dargestellt. Eine solche Anker-Domäne schließt beispielsweise folgende Varianten der Bindugnspartner auf einer festen Phase ein: Oligonukleotid der Anker-Domäne bindet an ein komplementäres Oligonukleotid auf der festen Phase, oder Oligonukleotid der Anker-Domäne bindet als Aptamer an ein spezifisches Protein auf der festen Phase.

Zunächst wird die Struktur der Oligonukleotide als Anker-Domänen besprochen. Generell gesprochen, eignen sich für die Zusammensetzung einer Anker-Domäne Substanzen, die eine sequenzspezifische Bindung mit einer Nukleinsäurekette, die an einer festen Phase fixiert ist (Bindungspartner), eingehen können. Gewöhnlich haben solche Substanzen Nukleobasen (A, C, T, G, U) oder deren Abkömmlinge, die eine spezifische Bindung ermöglichen. Der Rückgrand kann dabei eine natürliche Zusammensetzung haben (Zucker-Phosphatrückgrad) oder eine Abwandlung davon, z.B. Protein-Rückgrad bei PNA. Einzelne Nukleinsäureketten innerhalb einer Art von Nuk-Makromolekülen können dabei durchgehend aus einer Art der Bausteine bestehen, z.B. nur DNA oder nur PNA, oder auch ein gemischtes, zusammengesetztes Polymer darstellen, wo stellenweise DNA, RNA, PNA, Morpholino, LNA oder weitere Modifikationen zu einer Kette zusammengefasst sind. Da sich die einzelnen Arten der Nukleinsäureketten in ihrem Bindungsverhalten unterscheiden, kann es sinnvoll sein, eine Kombination aus unterschiedlichen Bausteinen einzusetzen. Dadurch können bessere Bindungs-Eigenschaften erzielt werden.

Zur Vereinfachung der Darstellung werden Nukleinsäureketten in Form der DNA in Details besprochen. Andere Arten der Nukleinsäureketten können entsprechend den bekannten Regeln nach dem Muster der DNA-Oligonukleotide konstruiert und eingesetzt werden.

Die Länge der Nukleinsäureketten in einer Anker-Domäne liegt vorzugsweise in folgenden Bereichen von 8 bis 10, 10 bis 12, 12 bis 14, 14 bis 16, 16 bis 18, 18 bis 20, 20 bis 25, 25 bis 30, 30 bis 40, 40 bis 50, 50 bis 60, 60 bis 70, 70 bis 100, 100 bis 200 Nukleotiden.
Die Kopplung der Anker-Domäne im Nuk-Makromolekül erfolgt in einer Ausführungsform an einem der beiden Enden der Anker-Domäne, z.B. am 5'-Ende oder am 3'-Ende. Beispiele für Kopplung eines Oligonukleotides an einem seiner Enden sind einem Fachmann bekannt. In einer anderen Ausführungsform der Erfindung erfolgt die Kopplung im inneren Bereich der Anker-Domäne, beispielsweise über einen an eine Base gekoppelten Linker. In einer vorteilhaften Ausführungsform kann die Anker-Domäne über einen Linker an die Nuk-Komponente gekoppelt sein.
In einer weiteren vorteilhaften Ausführungsform der Anmeldung sind die Anker-Domäne und die Target-Domäne innerhalb einer Nukleinsäurekette positioniert. Die Target-Domäne kann beispielsweise den Anteil der Nukleinsäurekette am oder in der Nähe von 5'-Ende bilden und die Anker-Domäne den Anteil der Nukleinsäurekette am oder in der Nähe von 3'-Ende. Eine solche Anordnung der Anker-Domäne und der Target-Domäne erlaubt eine gleichzeitige Synthese von beiden Domänen während einer Festphasensynthese des Oligonukleotid-Anteils des Nuk-Makromoleküls. In weiteren Ausführungsformen kann eine oder mehrere Target-Domänen von mehreren Anker-Domänen innerhalb einer Nukleinsäurekette umgeben sein. Eine oder mehrere Anker-Domäne können auch mehren Target-Domänen umgeben sein.

In einer vorteilhabten Ausführungsform der Erfindung sind Sequenzen der Target-Domäne und die der Anker-Domäne überlappend. In dieser Ausführungsform sind einige Nukleobasen Bestandteile von beiden Domänen. Die Länge der Sequenz, die das gemeinsame Fragment für die Target- und die Anker-Domäne bildet kann beispielsweise von 5 bis 80% der Sequenz einer der Domänen darstellen.

In einer weiteren vorteilhaften Ausführungsform der Erfindung sind Sequenzen der Target-Domäne und die der Anker-Domäne durch eine Spacer-Sequenz getrennt. In einer Ausführungsform der Erfindung schließt eine solche Spacer-Sequenz die Funktion mit signalvermittelnden Eigenschaften ein. In einer weiteren Ausführungsform der Erfindung schließt eine Spacer-Sequenz Sequenzen ein, die komplementär zu einer Target-Domäne oder zu einer Anker-Domäne sind. Diese Abschnitte innerhalb der Spacer-Sequenz können vollständig oder nur teilweise komplementär sein. In solchen Ausführungsformen hat eine Spacer-Sequenz die Rolle eines Antagonisten und hemmt die Bindungseigenschaften der weiteren Domänen.

In einer vorteilhaften Ausführungsform der Anmeldung ist die Anker-Domäne resistent gegenüber einer 5'-3'-Exonuklease-Aktivität. Dies kann beispielsweise durch den Einsatz von PNA oder weiterer Modifikationen erreicht werden.

Ein Bindungspartner auf der festen Phase kann in Form einer einzelsträngigen oder einer doppelsträngigen Nukleinsäureketten, z.B. DNA oder RNA oder PNA vorliegen. Die Bindung der Anker-Domäne an den Bindungspartner kann in einer Ausführungsform unter Ausbildung von Doppelsträngen (nach den Regeln der Watson-Crick Basenpaarung) erfolgen, in einer anderen Ausführungsform werden Triple-Stränge (nach Hoogsteen Regeln) gebildet. In einer weiteren Ausführungsform bindet die Anker-Domäne an ein Protein als ein Aptamer.

Beispiele für eine sequenzspezifische Bindung von Nukleinsäureketten sind einem Fachmann bekannt (siehe Literatur zu Microarray und PCR).

Im Folgenden, wird die Bindung an einen Einzelstrang als Beispiel betrachtet. Die Zusammensetzung einer Anker-Domäne kann eine vollständige Komplementarität zur Bindungspartner aufweisen ("perfect Match") oder sich an einigen Stellen unterscheiden ("Miss-Match").

Die Länge eines Nukleinsäurestranges eines fixierten auf einer festen Phase Bindungspartner kann vorzugsweise in folgenden Bereichen liegen 8 bis 10, 10 bis 12, 12 bis 14, 14 bis 16, 16 bis 18, 18 bis 20, 20 bis 25, 25 bis 30, 30 bis 40, 40 bis 50, 50 bis 60, 60 bis 70, 70 bis 100, 100 bis 200, Nukleotiden.

In einer Ausführungsform der Anmeldung sind die Sequenzen einer Anker-Domäne nur für eine Sequenz eines Bindugnspartner komplementär und können nur diesen einen Partner binden.

In einer weiteren Ausführungsform der Anmeldung schließt ein Nuk-Makromolekül mehrere Anker-Domänen bestehend aus unterschiedlichen Sequenzen ein, wobei eine jeweilige Anker-Domäne in der Lage ist, einen für sie spezifischen Bindungspartner an der festen Phase zu binden.

In einer weiteren Ausführungsform der Anmeldung werden Sequenzen der Anker-Domäne dermassen ausgewählt, dass sie nicht in der Lage sind, unter verwendeten Reaktionsbedinungen an die Zielsequenz zu binden.

In einer weiteren Ausführungsform der Anmeldung werden Sequenzen der Anker-Domäne dermassen ausgewählt, dass sie in der Lage sind, einen doppelsträngigen Abschnitt oder Haarnadel-Strukturen innerhalb einer Anker-Domäne zu bilden.

In einer weiteren Ausführungsform der Anmeldung werden Sequenzen der Anker-Domäne dermassen ausgewählt, dass sie nicht in der Lage sind, unter verwendeten Reaktionsbedinungen einen doppelsträngigen Abschnitt oder Haarnadel-Strukturen innerhalb einer Anker-Domäne zu bilden.

In einer weiteren Ausführungsform der Anmeldung wird die Sequenz der Anker-Domäne einer Art von Nuk-Makromolekülen dermassen ausgewählt, dass sie nicht in der Lage ist, unter verwendeten Reaktionsbedingungen an weitere Target-Domäne oder an Anker-Domäne oder weitere Bestandteile innerhalb derselben Art von Nuk-Makromolekülen zu binden.

In einer weiteren Ausführungsform der Anmeldung wird die Sequenz der Anker-Domäne einer Art von Nuk-Makromolekülen dermassen ausgewählt, dass sie nicht in der Lage ist, unter verwendeten Reaktionsbedingungen an weitere Target-Domäne oder an Anker-Domäne oder weitere Bestandteile anderer Nuk-Makromoleküle zu binden die im selben Ansatz verwendet werden.

Die Anker-Domäne kann aus mehreren einzelnen Oligonukleotiden bestehen, die in einem Nuk-Makromolekül integriert sind. Auch mehrere Anker-Domänen können in einem Nuk-Makromolekül vorkommen, dabei werden beispielsweise Oligonukleotide mit unterschiedlichen Sequenzen in einem Nuk-Makromolekül intergriert. Mehrere Anker-Domänen können auch innerhalb einer Nukleinsäurekette integriert sein. Dabei können sie unmittelbar ineinander übergehen oder deren Sequenzen können überlappend gestalltet werden oder die Domänen sind durch eine weitere Sequenz getrennt.

Die einzelnen Anker-Domänen können innerhalb einer Art von Nuk-Makromolekülen von gleicher oder von unterschiedlicher Nukleinsäureart sein. Beispielsweise, können DNA-basierte Anker-Domänen mit PNA-basierten Anker-Domänen innerhalb einer Art von Nuk-Makromolekülen kombiniert sein.

Vorzugsweise ist das 3'-Ende einer solchen Anker-Domäne (z.B. DNA-Oligonukleotid) nicht enzymatisch aktiv und ist beispielsweise blockiert, d.h. eine Polymerase ist nicht in der Lage ein Nukleotid an das 3'-Ende eines solchen Oligonukleotides zu koppeln. Dies kann beispielsweise durch Kopplung einer Gruppe erreicht werden, z.B. einer Phasphat-Gruppe oder einer Amino- oder einer weiteren Gruppe.

Die Anker-Domäne kann zusätzliche Modifikationen tragen, wie beispielsweise freie Aminogruppen, sowie andere signalgebende oder signalvermittelnde Moleküle, z.B. Farbstoffe, Fluoresenzfarbstoffe oder Biotin, oder makromolekulare Stoffe, wie Enzyme oder Nanokristalle. Modifizierte Oligonukeotide kann man kommerziell erwerben z.B. MWG-Biotech oder Trilink-Biotechnolgies.

Auch anderen Marker-Einheiten, wie Zucker und Lektine; Wachstumsfaktoren, Hormone, sequenz-erkennende Proteine, wie z.B. Transkriptions-Faktoren, oder auch entsprechende Rezeptor-Moleküle können als spezifische Bindungspartner für eine Anker-Domäne eines Nuk-Makromoleküls eingesetzt werden.

In einer vorteilhaften Ausführungsform ist der Bindungspartner für eine Anker-Domäne eine eine feste Phase gekopplet. Falls mehrere Bindungspartner für eine oder mehrere Anker-Domänen an einer festen Phase gekoppelt sind, ist ihre Anordnung vorzugsweise so gewählt, dass eine eindeutige räumliche Zuordnung der Bindungsereignisse der Anker-Domänen an die Bindungspartner möglich ist. Dies kann beispielsweise durch die Bereitstellung eines Arrays mit immobilisierten Bindungspartnern erreicht werden.

Die Anker-Domäne kann die Funktion einer Kern-Komponente erfüllen und Vebindung zwischen einzelnen Teilen des Markers eines Nuk-Makromoleküls darstellen. Die Linker-Komponente kann direkt an die Anker-Domäne gekoppelt sein.

### Antagonisten der Anker-Domäne

Ein Antagonist der Anker-Domäne blockiert reversibel ihre Funktion der Bindung an die feste Phase. Die Blockade kann vollständig sein oder unvollständig aber dennoch die Fähigkeit der Anker-Domäne zur Bindung an die feste Phase stark reduzieren.

Beispielsweise schließen die Nuk-Makromoleküle einzelsträngige Oligonukleotide ein, die als Anker-Domäne auftreten. Ihre Bindungspartner - beispielsweise komplementäre einzelsträngige Oligonukleotide oder Proteine (falls Aptamer-Protein Interaktion gewählt wurde) - sind an der festen Phase fixiert. Die spezifische Bindung der Anker-Domäne an ihre Bindungspartner erfolgt dann, wenn die Oligonukleotide der Anker-Domäne in einzelsträngiger Form vorliegen.
Durch Kopplung eines komlementären Oligonukleotides (Antagonist in 4b in Fig. 3i) z.B. in der Nähe der Anker-Domäne (4a in Fig. 3i), innerhalb des Markers des Nuk-Makromoleküls kann dieses an die Anker-Domäne komlementär binden und einen Doppelstrang bilden. Durch diese Bindung ist die Anker-Domäne in ihrer Bindungsfähigkeit an einen Bindungspartner gehindert. Eine solche Struktur der Nuk-Makromoleküle wird beispielsweise folgendermassen errecht: Die Anker-Domäne und ihr Antagonist werden von beiden Seiten einer Target-Domäne plaziert, so dass es zu intramolekularen Bildung einer Haarnadel-Struktur kommt (Fig. 3i). Dadurch wird die Anker-Domäne solange blockiert, bis sich Nuk-Makromoleküle sich in Lösung im freien Zustand befinden und Reaktionsbedingungen (Temperatur, Salz) die Bildung des Doppelstranges zwischen der Anker-Domäne und ihrem Antagonisten begünstigen. Die Aufhebung der Bindung des Antagonisten an die Anker-Domäne kann beispielswiese durch Einsatz höherer Temperaturen und / oder durch die Bindung der Target-Domäne an die Zielsequenz und / oder durch enzymatischen Abbau durch eine Endonuklease oder Exonuklease (z.B. 5'-3'-Exonuklease einer Polymerase) erreicht werden. Dadurch wird die Anker-Domäne wierder in den einzelsträngigen Zustand überführt und kann ihre Funktion der Bindung an einen Bindungspartner wahrnehmen.

Die Stärke der Bindung des Antagonisten an die Anker-Domäne kann durch die Base-Zusammensetzung des Antagonisten-Oligonukleotids moduliert werden. Bevorzugt werden dafür Oligonukleotide eingesetzt, deren Länge zwischen 6 und 10, 10 und 15, 15 und 20, 20 und 25, 25 und 30, 30 und 40, 40 und 50 Nukleotide beträgt.
Die Anker-Domäne kann in ihrer Gesamtlänge oder nur teilweise blockiert werden. Beispielsweise schließt Blockade durch den Antagonisten 5 bis 90% der Anker-Sequenz ein. Die Natur eines Antagonisten schließt beispielsweise DNA, RNA, PNA oder eine gemischte Sequenz ein. Die Kopplung des Antagonisten innerhalb des Nuk-Makromoleküls kann in ähnlicher weise realisiert werden, wie auch die Kopplung einer Anker-Domäne selbst. Beispielsweise können die verschiedenen Domänen und Antagonisten innerhalb eines Nukleinsäurestranges vorkommen: Die Anker-Domäne, die Target-Domäne und Antagonist zu Anker-Domäne. Die Target-Domäne bildet dabei den Abstandhalter zwischen der Anker-Domäne und dem Antagonisten (Fig. 3 und 7).

Eine Art von Nuk-Makromolekülen, die solche Anker-Domäne und ihre Antagonisten einschließt, kann demnach in mindestens zwei Zuständen vorliegen: erster Zustand mit einer offenen Anker-Domäne, die fähig ist, an ihren an der festen Phase immobilisierten Bindungspartner zu binden; zweiter Zustand mit einer blockierten Anker-Domäne, die Bindung an die feste Phase ist durch den Antagonisten verhindert.

Die beiden Zustände können durch Änderung der Reaktionsbedingungen (reversible Änderung der Antagonisierung z.B. Temeratur, Salz, pH, organische Lösungsmittel, bzw. irreversible Aufhebung der Antagonisierung durch enzymatischen Abbau des Antagonisten oder seine Abspaltung) moduliert werden. Eine irreversible Aufhebung der Antagonisierung kann beispielsweise folgendermassen erreicht werden: die Anker-Domäne wird aus DNA zusammengesetzt und der Antagonist aus RNA. Durch den Einsatz einer entsprechenden Rnase wird der RNA-Anteil abgebaut und die Anker-Domäne aus DNA ist nicht mehr blockiert.

Einsatz von Nuk-Makromolekülen mit Antagonisten kann einige Vorteile bringen. Beispielsweise, in einer Ausführungsform der Anmeldung wird die unerwünschte Bindung von überschüssigen Nuk-Makromolekülen an die feste Phase herabgesetzt (Fig. 7). Die Bindung der markierten Nukleinsäuren an die feste Phase wird unter Bedingungen durchgeführt, bei denen die Anker-Domänen der nicht eingebauten Nuk-Makromolekülen vom Antagonisten blockiert sind. Solche Bedingungen schließen beispielsweise Temperaturen deutlich unter der Tm der Bindung des Antagonisten an die Anker-Domäne ein. Die Nukleinsäureketten, die mit solchen Nuk-Makromolekülen markiert sind, enthalten offene, nicht blockierte Anker-Domänen (Fig. 7) und können an die feste Phase binden. Dadurch ist die Bindung der markierten Zielsequenzen in Anwesenheit eines Überschusses von nicht eingebauten Nuk-Makromolekülen möglich und ein zusätzlicher Reinigungsschritt von markierten Zielsequenzen kann eingespart werden.

Solche Antagonisten können auch für Target-Domäne und für Signal-Domäne innerhalb eines Nuk-Makromoleküls vorkommen (siehe dort).

Bezüglich der Synthese von Oligonukleotiden, die als Anker-Domänen auftreten oder deren Bindungspartner an der festen Phase oder als deren Antagonisten bilden, und deren Modifikation kann ein Fachmann an folgende Quellen verwiesen werden:
"Oligonucleotide synthesis, methods and applications" Piet Herdewijn, 2004, ISBN 1-58829-233-9
"Protocols for oligonucleotide conjugates, synthesis and analytical techniques" Sudhir Agrawal, 1993, ISBN 0-89603-252-3
"Protocols for oligonucleotide conjugates, synthesis and properties" Sudhir Agrawal, 1993, ISBN 0-89603-247-7
"The aptamer handbook" Sven Klussmann, 2006, ISBN 10: 3-527-31059-2
"Pharmaceutical aspects of oligonucleotides" Patrick Couvreur, 2000, ISBN 0-748-40841-X
"Triple Helix forming Oligonucleotides"Claude Malvy, 1999, ISBN 0-7923-8418-0 "Artificial DNA, methods and applications" Yury E. Khudyakov, ISBN 0-8493-1426-7

Bezüglich der Veteilung von Bindungspartner auf einer festen Phase, Bildung räumlich angeordneten Arrays von Bindungspartnern, Bindung von biologisch aktiven Molekülen an die feste Phase, Vorrichtungen zum Austausch von Flüssigkeiten usw. kann ein Fachmann an folgende Quellen verwiesen werden:
"Microfluidic Lab-on-a-chip for chemical and biological Analysis and discovery" Paul C.H.Li, 2006, ISBN 1-57444-572-3
"Microfluidic for biological applications" Wei-Cheng Tian, 2008, ISBN 978-0-387-09479-3
"Preparation, Microfluidics, Detection methods and Biological applications" Kilian Dill, 2009, ISBN 978-0-387-72716-5
"DNA Microarray, a practical approach" M. Shena, 2002, ISBN 0-19-963777 6
"DNA Microarrays" David Bowtell, 2003, ISBN 0-87969-624-9
"Lateral flow Immunoassays" Raphael Wong, 2009, ISBN 978-1-58829-908-6
"Nucleic Acid Hybridization" M.L.M.Anderson, 1999, ISBN 1-85996-007-3

### 1.3.3.3.3 Signal-Domäne (Funktionen und Zusammensetzung)

### Funktion einer Signal-Domäne

Die Signal-Domäne kann in einer Ausführungsform eine signalgebende Funktion haben. In einer anderen Ausführungsform hat sie eine signalvermittelnde Funktion. In einer weiteren Ausführungsform hat sie eine katalytische Funktion. In einer weiteren Ausführungsform hat die Signal-Domäne mehr als nur eine Funktion, sondern z.B. vereinigt sowohl signalgebende als auch eine signalvermitteldne Funktion. Weitere Kombinationen sind nahe liegend. Weitere Beispiele für Nachweismethoden sind im Kapitel 1.3.25 angegeben.

Bei der signalgebenden Funktion enthält die Signal-Domäne Bestandteile, die bereits während der chemischen Synthese an Nuk-Makromoleküle gebunden werden, s. Beispiele in Anmeldungen Cherkasov et al WO 2005044836, Cherkasov et al WO2006097320, Cherkasov et al WO 2008043426, Cherkasov et al DE 10356837, Cherkasov et al DE 102004009704.

Bei der signalvermittelnden Funktion trägt die Signal-Domäne, die erst durch eine Reaktion mit signalgebenden Molekülen ihre Signaleigenschaften entfalten. Beispielsweise können mehrere Biotin-Moleküle, z.B. 100 Biotin-Moleküle, den Marker-Teil bilden. Nach dem Einbau der Nuk-Makromoleküle erfolgt eine Nachweisreaktion mit modifizierten Streptavidin-Molekülen. In einem anderen Beispiel haben die Nukleinsäureketten die signalvermittelnde Funktion. Nach dem Einbau von Nuk-Makromoleküle, erfolgt eine Hybridisierung von einheitlichen Oligonukleotiden mit detektierbaren Einheiten, z.B. Fluoreszenzfarbstoffen (MWG-Biotech) an die Marker-Komponente. In einem weiteren Beispiel haben Amino- oder Merkapto-Gruppen, beispielsweise 50 Aminogruppen pro Marker, die signalvermittelnde Funktion. Nach dem Einbau der Nuk-Makromoleküle in die Nukleinsäurekette erfolgt eine chemische Modifikation mit reaktiven Komponenten, z.B. mit Farbstoffen, wie beispielsweise für eingebaute Allyl-Amino-dUTP beschrieben, Diehl et al. Nucleic Acid Research, 2002, V. 30, Nr. 16 e79.

In einer anderen Ausführungsform hat die Signal-Domäne eine katalytische Funktion (in Form eines Enzyms oder Ribozyms). Dabei können unterschiedliche Enzyme verwendet werden, z.B. Peroxidase oder alkalische Phosphatase. Dank der Kopplung an die Nuk-Komponente kann das jeweilige Enzym durch den Einbau von Nuk-Makromolekülen an den Nukleinsäurestrang kovalent gebunden werden.

Eine Signal-Domäne schließt in einer Ausführung eine niedermolekulare Marker-Einheit ein. In einer weiteren Ausführungsform schließt die Signal-Domäne eine makromolekulare Marker-Einheit ein. In einer weiteren Ausführungsform schließt die Signal-Domäne mehrere niedermolekulare Marker-Einheiten ein. In einer weiteren Ausführungsform schließt die Signal-Domäne mehrere makromolekulare Marker-Einheiten ein. In einer weiteren Ausführungsform schließt die Signal-Domäne eine Kombination aus niedermolekularen und makromolekularen Einheiten ein. Die Signal-Domäne können eine signalgebende oder signalvermittelnde Funktion haben.

Diese Einheiten können Moleküle mit geringer Molekularmasse, z.B. unter 2000 Da, oder auch selbst Makromoleküle sein. Dabei schließt die Zahl der signalgebenden oder signalvermittelnden Einheiten, die zu einer Signal-Domäne zusammengefaßt sind, folgende Bereichen ein: 1 und 2, 2 und 5, 5 und 20, 20 und 50, 50 und 100, 100 und 500, 500 und 1000. 1000 und 10000, 10000 und 100000.

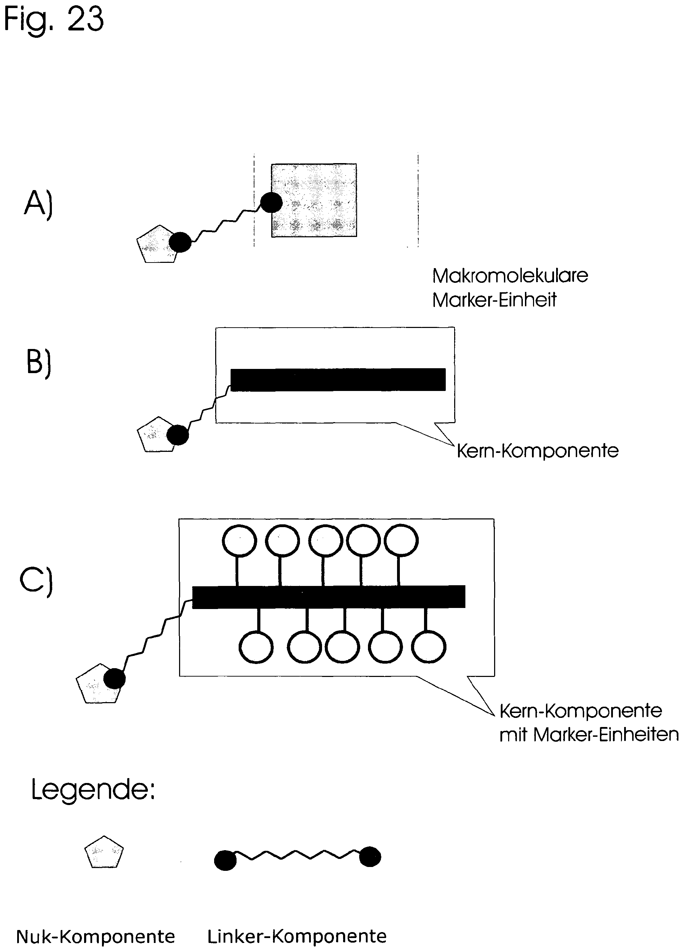

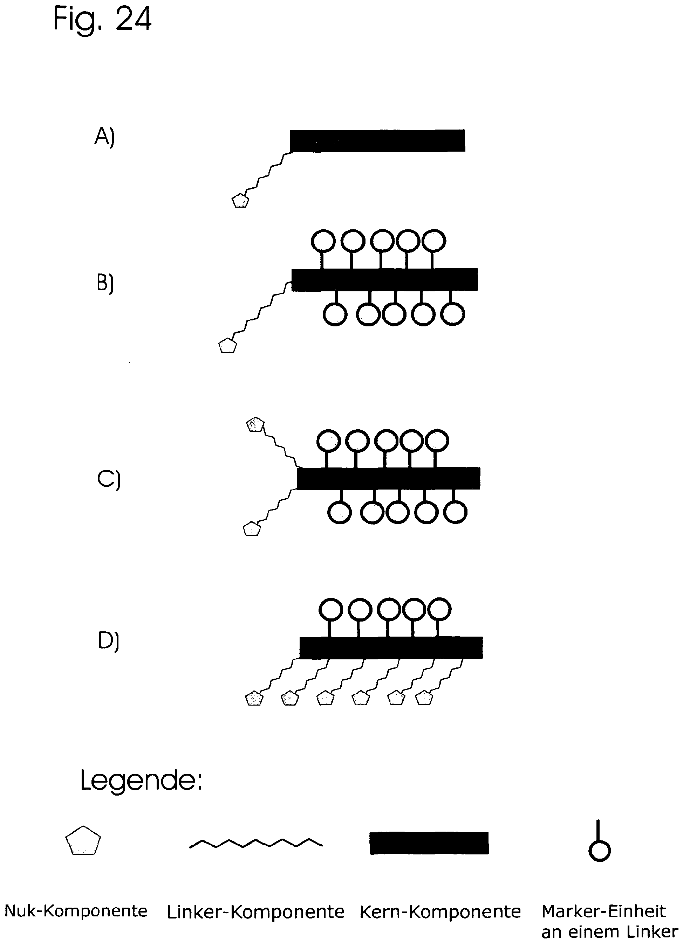

Falls mehrere Marker-Einheiten zu einer Signal-Domäne zusammengefasst sind, sind diese Einheiten in einer Ausführungsform an ein Gerüst gebunden, die Kern-Komponente des Markers (Fig. 23). Diese Kern-Komponente verbindet die Einheiten untereinander. Die Kern-Komponente kann die Verbindung zu einem oder mehreren Nuk-Linker-Komponenten herstellen (Fig 24). Die Kern-Komponente kann niedermolekulare oder makromolekulare Verbindungen einschließen.

### 1.3.3.3.3.1 Struktur der signalgebenden oder der signalvermittelnden Einheiten der Signal-Domäne

Die strukturellen Marker-Einheiten schließen folgende Gruppen ein:

### 1.3.3.3.3.1.1 Strukturen mit niedriger Molmasse:

Biotin-Moleküle, Hapten-Moleküle (z.B. Digoxigenin oder Dinitrophenol (DNP)), radioaktive Isotope (z.B. P³², J¹³¹), oder deren Verbindungen, seltene Erden , Farbstoffe, Fluoreszenzfarbstoffe, Fluoreszenz-Quencher (z.B. Dabsyl) (viele dieser Moleküle sind kommerziell erhältlich z.B. von Molecular Probes, Inc., oder Sigma-Aldrich) mit gleichen oder unterschiedlichen spektralen Eigenschaften, Farbstoffe, die untereinander FRET eingehen. Thermochrome, photochrome oder chemolumineszente Substanzen (erhältlich z.B. bei Sigma-Aldrich), Chromogene Substanzen (z.B. als Substrate für Peptidasen beschrieben in "Proteolytic enzymes Tools and Targets",E. Sterchi, 1999, ISBN 3-540-61233-5).

Auch chemisch reaktive Gruppen, wie beispielsweise Amino-, Carboxy-, Merkapto-, Aldehyd-, Jodacetat-, Acryl-, Dithio-, Thioester-Verbindungen können als signalvermittelnde strukturelle Einheiten dienen. Nach dem Einbau können diese reaktiven Gruppen mit signalgebenden Elementen, wie beispielsweise Farbstoffe mit entsprechenden reaktiven Gruppen (beispielsweise NHS-Ester, Merkapto-, AminoGruppen) modifiziert werden. Allgemeine Grundlagen für die Wahl eines passenden Paares von reaktiven Gruppen sind in "Chemistry of protein conjugation and crosslinking" Shan S. Wong 1993 dargestellt.

In einer speziellen Ausführungsform erfühlt die Kombination aus einer Nuk-Einheit, einer markomolekularen Linker-Komponente und einer Signal-Domäne mit niedriger Molmasse bereits die Anforderungen der vorliegenden Erfindung. Solche Verbindungen sind ebenfalls Gegenstand dieser Erfindung. Sie können sowohl als Zwischenprodukte für die chemische Synthese von Nuk-Makromolekülen mit einem makromolekularen Marker, z.B. dUTP-PEG-Biotin, als auch selbständig in den enzymatischen Reaktionen verwendet werden, wie beispielsweise mit nur einem Farbstoff markierten Nukleotid.

Als Farbstoffe können verschiedene Fluoreszenzfarbstoffe auftreten, ihre Wahl ist nicht eingeschränkt, solange sie die enzymatische Reaktion nicht wesentlich beeinflußen. Beispiele für Farbstoffe sind Rhodamine (Rhodamin 110, Tetramethylrhodamin, erhältlich bei Fluka-Sigma), Cyanin-Farbstoffe (Cy2, Cy3, Cy3.5, Cy5, Cy5.5, Cy7 von Amersham Bioscience), Coumarine, Bodipy, Fluorescein, Alexa-Farbstoffe: z.B. Alexa 532, Alexa 548, Alexa 555 (Molecular Probes). Viele Farbstoffe sind kommerziell erhältlich, beispielsweise von Molecular Probes Europe, Leiden, Niederlande (im weiteren als Molecucular Probes bezeichnet) oder von Sigma-Aldrich-Fluka (Taufkirchen, Deutschland).

In einer Ausführungsform schließt die Signal-Domäne mehrere Marker-Einheiten ein. Dabei können die Marker-Einheiten untereinander gleiche oder verschiedene Eigenschaften haben. Beispielsweise können Fluoreszenzfarbstoffe mit unterschiedlichen Spektraleigenschaften eingesetzt werden. In einer Ausführungsform werden Fluoreszenzfarbstoffe eingesetzt, die FRET-Paare bilden.

### 1.3.3.3.3.1.2 Strukturen mit hoher Masse (Makromoleküle)

### Nanokristalle

Nanokristalle, z.B. Quantum Dots können als Marker-Einheiten der Signal-Domäne auftreten. Dabei können in einer Marker-Komponente Quantum Dots mit gleichen oder unterschiedlichen spektralen Eigenschaften verwendet werden, US Pat. 6.322.901, US Pat. 6.423.551, US Pat. 6.251.303, US Pat. 5.990.479).

### Nano oder Mikro-Teilchen.

Nano oder Mikro-Teilchen können als Marker-Einheiten der Signal-Domäne auftreten, wobei die größten Ausmaße dieser Teilchen von 1nm bis 2nm, von 2nm bis 5nm, von 5nm bis 10nm, von 10nm bis 20 nm, von 20nm bis 50nm, von 50nm bis 100nm, von 100 nm bis 200nm, von 200nm bis 500nm, von 500nm bis 1000nm, von 1000 nm bis 5000 nm betragen. Das Material der Teilchen können beispielsweise reine Metalle wie Gold, Silber, Aluminium (beispielsweise Teilchen mit surface plasmon resonance), - Protein-Au-Konjugate: J. Anal.Chem. 1998, V. 70,S. 5177, - Nukleinsäure-Au-Konjugaten: J. Am. Chem. Soc. 2001, V. 123, S.5164, J. Am. Chem. Soc. 2000, V. 122, S. 9071 , Biochem. Biophys. Res. Commun 2000, V. 274, S. 817, Anal. Chem. 2001, V. 73, S. 4450), - Latex (z.B. Latex-Nanopartikel, Anal. Chem. 2000,V. 72, S. 1979) - Kunststoff (Polystyrene), - paramagnetische Verbindungen / Gemische Zhi ZL et al. Anal Biochem. 2003 Jul 15;318(2):236-43, Dressman D et al. Proc Natl Acad Sci U S A. 2003 Jul 22;100(15):8817-22" - Metall-Teilchen, - magnetische Verbindungen / Gemische Jain KK. Expert Rev Mol Diagn. 2003 Mar;3(2): 153-61, Patolsky F et al. Angew Chem Int Ed Engl. 2003 May 30;42(21):2372-2376, Zhao X et al. Anal Chem. 2003 Jul 15;75(14):3144-51, Xu H et al. J Biomed Mater Res. 2003 Sep 15;66A(4):870-9, JOSEPHSON Lee et al. US Pat. 2003092029, KLICHE KAY-OLIVER et al. WO0119405.

### Proteinmoleküle,

Proteinmoleküle können als Marker-Einheiten der Signal-Domäne auftreten, wobei sie folgende Gruppen schließen: Enzyme (z.B. Peroxidase, alkalische Phosphotase, Urease, beta-Galaktosidase, Proteinasen), - fluoreszierenden Proteine (z.B. aus GFP-Protein-Familie oder Phycobiliproteine (z.B. Phycoerythrin, Phycocyanin), erhältlich z.B. von Molecular Probes Inc.), Antigen-bindende Proteine (z.B. Antikörper, Tetramere, Affibodies (Nord et. Al Nature Biotechnology, V. 15 (S.772-777) oder deren Bestandteile (z.B. Fab-Fragmente), Nukleinsäurebindende Proteine (z.B. Transcriptionsfaktor)

### Nukleinsäureketten,

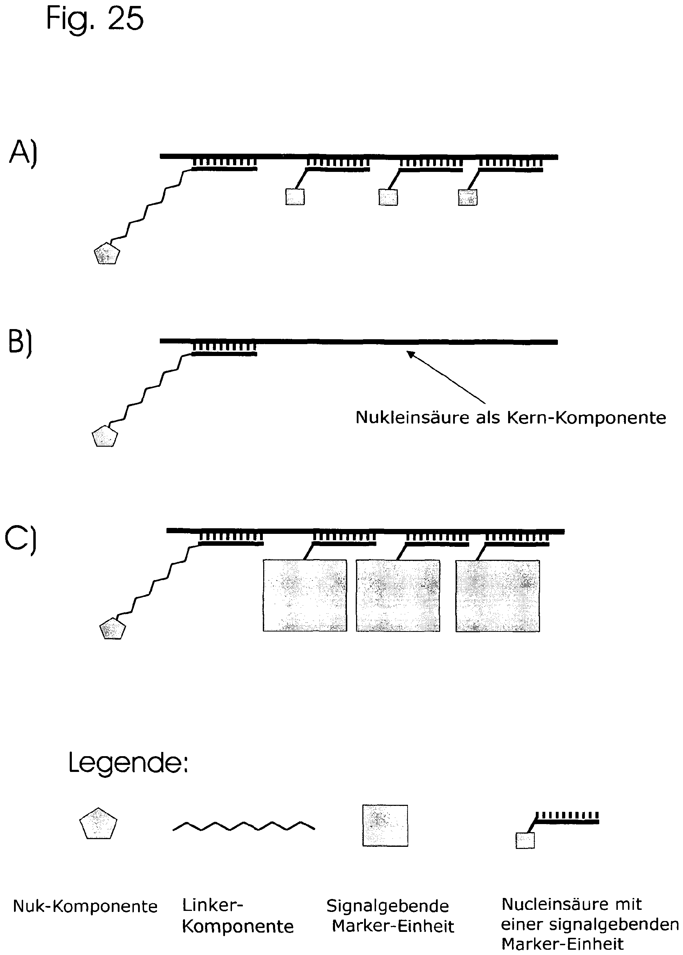

Die Nukleinsäureketten einschließlich der Gruppe, Oligonukleotide, modifizierte Oligonukleotide, können als Marker-Einheiten der Signal-Domäne auftreten. Die Länge der Nukleinsäureketten liegt vorzugsweise in folgenden Bereichen von 10 bis 20, von 20 bis 50, von 50 bis 100, von 100 bis 200, von 200 bis 500, von 500 bis 1000, 1000 bis 5.000, von 5.000 bis 10.000, von 10.000 bis 100.000 Nukleotiden. Es können DNA, RNA, PNA-Moleküle verwendet werden. Nukleinsäureketten können zusäzliche Modifikationen tragen, wie beispielsweise freie Aminogruppen, Farbstoffe und andere signalgebende Moleküle, z.B. makromolekulare Stoffe, Enzyme oder Nanokristalle (Fig. 25). Modifizierte Nukleinsäureketten sind kommerziell zugänglich, z.B. MWG-Biotech oder TrilLink-Biotechnologies.
Weitere Beispiele für Makromoleküle oder Makromolekülkomplexe, die im Rahmen der vorliegenden Erfindung als Marker oder Marker-Einheiten in der Marker-Komponente für die Signalverstärkung eingesetzt werden können, sind in US Pat. 4882269, US Pat. 4687732, WO 8903849, US Pat. 6017707, US Pat. 6627469 beschrieben. Vorzugsweise ist das 3'-Ende der Signal-Domäne blockiert, so dass die Target-Domäne nicht als Primer für eine Polymerase dienen kann.

Vorzugsweise schließt die Signal-Domäne keine komplementären Sequenzen zur Zielsequenz oder zu Target-Domäne oder zu Anker-Domäne oder zu den verwendeten Primern.

### 1.3.3.3.4 Die Kern-Komponente des Markers

Die Kernkomponente hat die Funktion, mehrere strukturelle Elemente der Nuk-Makromoleküle zu binden. Beispielsweise bindet die Kern-Komponente mehrere Marker-Einheiten zusammen oder einzelne Domänen können mittels Kern-Komponente verbunden sein. In einer weiteren Ausführungsform können Linker-Komponenten an die Kern-Komponente gebunden werden. Der Begriff der Kern-Komponente ist funktionell und dient zur Erläuterung von möglichen Strukturen von Nuk-Makromolekülen. Unterschiedliche chemische Strukturen, die Linker und Marker-Einheiten zusammenhalten, können als Kern-Komponente bezeichnet werden.
Im Folgenden sind Beispiele für Bestandteile der Kernkomponente angegeben.

### Bestandteile der Kernkomponente

In einer Ausführungsform besteht die Kern-Komponente aus einer oder mehreren niedermolekularen Verbindungen. Sie haben die Funktion, die Marker-Einheiten untereinander zu verbinden. Die Verbindung zwischen der Kern-Komponente und den Marker-Einheiten kann kovalent oder affin sein. Bei kovalenter Bindung können beispielsweise Verbindungen mit allgemeiner Struktur-Formel (F)ₘ-R-(H)ₙ als Ausgangsmateriel dienen, wo (F) und (H) - reaktive Gruppen darstellen, und R - eine Verbindungskomponente, wobei Zahl solcher Gruppen und ihre Anordnung stark variieren kann. Viele Beispiele sind dem Fachmann bekannt, z.B. Verbindungen aus der Gruppe Cross-Linker ("Chemistry of protein conjugation and crosslinking" Shan S. Wong 1993 CRC Press Inc.). Die Struktur ist nicht eingeschränkt. Sie ist vorzugsweise wasserlöslich. Beispielsweise schließen (F) und (H) unabhängig von einander folgende Gruppen ein: NH2 (Amino), OH (Hydroxy), SH (Mercapto), COOH (Carboxy), CHO (Aldehyd), Acryl- oder Maleimid. Beispiele für (R) stellen wasserlösliche Polymere wie PEG oder Polypeptid-Ketten oder kurze aliphatische Ketten dar.

In einer weiteren Ausführungsform besteht die Kern-Komponente aus einem hydrophilen, wasserlöslichen Polymer, dabei kann das Polymer aus gleichen oder unterschiedlichen Monomeren bestehen.

Beispiele für den Kern-Komponennte stellen folgende Polymere und deren Derivate dar: Polyamide (z.B. Polypeptide wie Poly(glutamin) oder Poly(Glutamin säure)) und deren Derivate, Poly-Acrylsäure und deren Derivate, natürliche oder synthetische Polysaccharide (z.B. Stärke, Hydroxy-Ethyl-Strärke), Dextran und seine Derivate (z.B. Aminodextran, Carboxy-Dextran), Dextrin, Poly-Acrylamide und deren Derivate (z.B. N-(2-hydroxypropyl)-methacdylamid), Poly-Vinylalkohole und deren Derivate, Nukleinsäureketten, Proteine. Diese Polymere können linear, globulär, z.B. Streptavidin oder Avidin, oder verzweigt sein, z.B. Dendrimere. Auch vernetzte lösliche Polymere, wie beispielswiese vernetzte Poly-Acrylamide (Crosslinker Bisacrylamid in Kombination mit Poly-Acrylamid), eingesetzt werden.

Da sowohl die Linker-Komponente als auch die Marker-Komponente wasserlösliche Polymere enthalten können, kann in einer Ausführungsform ein solches Polymer sowohl als Linker als auch als Kern-Komponente dienen. In diesem Fall kann ein Abschnitt eines solchen Polymers als Linker, ein anderer Abschnitt als Kern-Komponente betrachtet werden.

In einer vorteilhaften Ausführungsform der Erfindung werden lineare oder wenig verzweigte Polymere als Kernkomponenten eingesetzt, beispielsweise Polyamide (z.B. Polypeptide), Poly-Acrylsäure, Polysaccharide, Dextran, Poly-Acrylamide, Poly-Vinylalkohole. Das Polymer kann aus einheitlichen oder unterschiedlichen Monomeren bestehen. Insbesondere in dieser Ausführungsform kann die Linker-Komponente weniger als 50 Kettenatome aufweisen. So können auch Linker-Längen von ca. 5 bis 10, 10 bis 20 oder 20 bis 50 Kettenatome ausreichend sein, um die Substrateigenschaften der Nuk-Makromoleküle für Enzyme zu bewahren. Eine solche Kern-Komponente des Markers erfüllt die Funktion der Linker-Komponente: sie schafft räumlichen Abstand zwischen sterisch anspruchsvollen Marker-Einheiten und aktiven Zentren der jeweiligen Enzyme.

Die wasserlöslichen Polymere haben vorzugsweise eine durchschnittliche Ketten-Länge von 20 bis 10.000 Kettenatomen. Beispielsweise liegt die durchschnittliche Kettenlänge zwischen 20 und 100, 100 und 500, 500 und 5000, 5000 und 10000 Kettenatome.
In einer Ausführungsform liegt das Polymer in wässriger Phase bei pH zwischen 4 und 10 vorwiegend in einer neutralen Form vor (z.B. Dextran oder Polyacrylamid). In einer anderen Ausführungsform liegt das Polymer in einer wässrigen Phase bei pH zwischen 4 und 10 in einer geladen Form vor. Es kann sowohl positive (z.B. Polylysin) oder negative Ladungen (z.B. Polyacrylsäure) tragen.
Die Kopplung von Marker-Einheiten an ein wasserlösliches Polymer hängt von der Art des Polymers ab. Die für die Kopplung notwendigen reaktiven Gruppen können bereits im Polymer vorhanden sein (z.B. Polylysin oder Polyacrylsäure) oder in einem getrennten Schritt in das Polymer eingeführt werden. Beispielsweise bei Dextran sind viele Varianten der Einführung von reaktiven Gruppen und chemischen Kopplungen bekannt (Molteni L. Methods in Enzymology 1985, v.112, 285, Rogovin A.Z. et al. J.Macromol Sci. 1972, A6, 569, Axen R. et al. Nature 1967, v. 214, 1302, Bethell G.S. et al. J. Biol.Chem. 1979, v.254, 2572, Lahm O. et al. Carbohydrate Res. 1977, v. 58, 249, WO 93/01498, WO 98/22620, WO 00/07019).

In einer weiteren Ausführungsform besteht die Kern-Komponente aus einem Nano- oder Mikroteilchen. Beispielsweise, Polystyren oder Latex oder Dextran-Teilchen, die reaktive Gruppen, wie Amino, Carboxy-Gruppe tragen, oder auch Streptavidin können als Kern-Komponente eingesetzt werden. Solche Teilchen sind kommerziell erhältlich. Nano oder Mikro-Teilchen der Kern-Komponente können unterschiedliche Größe haben. Die Ausmaße dieser Teilchen können in folgenden Bereichen liegen: von 1nm bis 2nm, von 2nm bis 5nm, von 5nm bis 10nm, von 10nm bis 20 nm, von 20nm bis 50nm, von 50nm bis 100nm, von 100 nm bis 200nm, von 200nm bis 500nm, von 500nm bis 1000nm, von 1000 nm bis 5000 nm.
Das Material der Teilchen können beispielsweise reine Metalle wie Gold, Silber, Aluminium (beispielsweise Teilchen mit surface plasmon resonance), - Protein-Au-Konjugate: J. Anal.Chem. 1998, V. 70,S. 5177, - Nukleinsäure-Au-Konjugaten: J. Am. Chem. Soc. 2001, V. 123, S.5164, J. Am. Chem. Soc. 2000, V. 122, S. 9071 , Biochem. Biophys. Res. Commun 2000, V. 274, S. 817, Anal. Chem. 2001, V. 73, S. 4450), - Latex (z.B. Latex-Nanopartikel, Anal. Chem. 2000,V. 72, S. 1979) - Kunststoff (Polystyrene), - paramagnetische Verbindungen / Gemische Zhi ZL et al. Anal Biochem. 2003 Jul 15;318(2);236-43, Dressman D et al. Proc Natl Acad Sci U S A. 2003 Jul 22;100(15):8817-22" - Metall-Teilchen, - magnetische Verbindungen / Gemische Jain KK. Expert Rev Mol Diagn. 2003 Mar;3(2): 153-61, Patolsky F et al. Angew Chem Int Ed Engl. 2003 May 30;42(21):2372-2376, Zhao X et al. Anal Chem. 2003 Jul 15;75(14):3144-51, Xu H et al. J Biomed Mater Res. 2003 Sep 15;66A(4):870-9, JOSEPHSON Lee et al. US Pat. 2003092029, KLICHE KAY-OLIVER et al. WO0119405.

Die Kern-Komponennte hat vorzugsweise mehrere Kopplungsstellen, an die weitere Elemente gebunden werden können, z.B. strukturelle Marker-Einheiten oder Nuk-Linker-Komponente.

Beispielsweise Poly-Lysin-Moleküle haben multiple freie Aminogruppen, an die mehrere Farbstoffmoleküle, Biotinmoleküle, Haptenmoleküle oder Nukleinsäureketten gekoppelt werden können. Poly-Lysine haben unterschiedliche Molmasse, z.B. 1000-2000, 2000-10000, 10000-50000 Da können käuflich erworben werden.

Als weiteres Beispiel für die Kernkomponente dienen Nukleinsäurenstränge, wobei die Nukleinsäureketten eine Länge von 10 bis 20, von 20 bis 50, von 50 bis 100, von 100 bis 200, von 200 bis 500, von 500 bis 1000, 1000 bis 5000, von 5000 bis 10000 Nukleotiden haben. Diese Nukleinsäuren dienen als Bindungspartner für sequenzkomplementäre Markereinheiten. In einer Ausführugnsfrom bindet die Kernkomponente nicht an die zu markierende Zielsequenz. Die an die Kern-Komponente gebundenen Marker-Einheiten können beispielsweise Target-Domäne oder Anker-Domäne einschließen. In solchen Ausführungsformen sind solche Marker-Einheiten durch eine affine Bindung zwischen Basenpaaren gebunden (genannt Hybridisierung). Vorzugsweise ist die Bindung zwischen der Kern-Komponente und den Marker-Einheiten unter Reaktionsbedingungen stabil.

In einer weiteren Ausführungsform besteht die Kern-Komponente aus einem Dendrimer, z.B. Polypropylenimine, Polyaminoamine. Beispiele für andere Dendrimere sind bekannt: Cientifica "Dendrimers", 2003, Technology white papers Nr. 6, Klajnert et al. Acta Biochimica Polonica, 2001, v. 48, S. 199, Manduchi et al. Physiol. Genomics 2002, V.10, S.169, Sharma et al. Electrophoresis. 2003, V. 24, S. 2733, Morgan et al. Curr Opin Drug Discov Devel. 2002 Nov;5(6):966-73, Benters et al. Nucleic Acids Res. 2002 Jan 15;30(2):E10, Nilsen et al. J Theor Biol. 1997 Jul 21;187(2):273-84. Viele Dendrimere sind kommerziell erwerblich (Genisphere, www.genisphere.com, Chimera Biotech GmbH).

Weitere Kombinationen für die Kern-Komponennte aus den oben dargestellten Bestandteilen sind einem Fachmann naheliegend.

### 1.3.3.3.5 Kopplung der Marker-Einheiten oder Domänen

Marker-Einheiten oder Domänen können an die Kern-Komponente oder an die Linker-Komponente durch eine kovalente Bindung, beispielsweise über einen Cross-linker (Chemistry of protein conjugation and cross-linking, S. Wang,1993, ISBN 0-8493-5886-8, "Bioconjugation: protein coupling techniques for the biomedical sciences", M. Aslam, 1996, ISBN 0-333-58375-2), oder über eine affine Kopplung erfolgen, beispielsweise Biotin-Streptavidin-Verbindung oder Hybridisierung von Nukleinsäuresträngen oder Antigen-Antikörper-Wechselwirkung ("Bioconjugation: protein coupling techniques for the biomedical sciences", M. Aslam, 1996, ISBN 0-333-58375-2).

Die Kopplung von Marker-Einheiten an die Kern-Komponente erfolgt in einer Ausführungsform bereits während der Synthese der Nuk-Makromoleküle.

In einer anderen Ausführungsform erfolgt zunächst die chemische Synthese von Nuk-Makromoleküle, bei denen die Markerkomponente nur aus der Kern-Komponente besteht. Die Kopplung von Marker-Einheiten an die Kern-Komponente erfolgt erst nach dem Einbau von Nuk-Makromoleküle in die Nukleinsäurekette. Durch eine große Zahl der potenziellen Bindungsstellen am Kernteil ist die Wahrscheinlichkeit der Bindung der Marker-Einheiten an die Kern-Komponente und somit an die eingebaute Nuk-Komponente wesentlich größer im Vergleich zu konventionellen Nukleotid-Strukturen. Die Kopplungschemie im einzelnen hängt von der Struktur der Marker-Einheiten und der Struktur der Kern-Komponente ab.

**Kovalente Kopplung:** Die Verbindung zwischen den Marker-Einheiten und der Kern-Komponente kann in einer Ausführungsform widerstandsfähig sein, z.B. bei Temperaturen bis zu 100°C, für pH-Bereiche zwischen 3 und 12, und/oder resistent gegen hydrolytische Enzyme (z.B. Esterasen) sein. In einer anderen Ausführungsform der Erfindung ist die Verbindung zwischen der Nuk-Komponente und dem Linker unter milden Bedingungen spaltbar.
Beispiele für die Kopplung von Nukleinsäuren an Dendrimere (entspricht einer Kopplung von Marker-Einheiten an eine Kernkomponente) sind z.B. in Shchepinov et al. Nucleic Acids Res. 1999 Aug 1;27(15):3035-41, Goh et al. Chem Commun (Camb). 2002 Dec 21;(24):2954 dargestellt.

### 1.3.3.3.6 Kopplung zwischen Linker und Marker

Die Verbindung zwischen der Linker-Komponente und dem Marker hängt von der jeweiligen Struktur der Marker-Einheiten bzw. der Struktur der Kern-Komponente ab. In einer Ausführungsform ist die Linker-Komponente direkt an die signalgebende oder signalvermittelnde Marker-Einheit gebunden. Dabei kann der Marker aus nur einer oder mehreren Marker-Einheiten bestehen.
In einer weiteren Ausführungsform sind ein oder mehrere Linker-Komponenten an die Kern- Komponente des Markers gebunden.
Die Bindung zwischen der Linker-Komponente und dem Marker kann sowohl kovalent als auch affine erfolgen. Viele Beispiele sind dem Fachmann bekannt, s. z.B. "Bioconjugation: protein coupling techniques for the biomedical sciences", M. Aslam, 1996, ISBN 0-333-58375-2. "Chemistry of protein conjugation and crosslinking" Shan S. Wong 1993 CRC Press Inc.).

**Kovalente Kopplung:** Die Verbindung zwischen der Linker-Komponente und dem Marker kann in einer Ausführungsform widerstandsfähig sein, z.B. bei Temperaturen bis zu 130°C, für pH-Bereiche zwischen 1 und 14, und/oder resistent gegen hydrolytische Enzyme (z.B. Proteasen, Esterasen)sein . In einer anderen Ausführungsform der Erfindung ist die Verbindung zwischen der Nuk-Komponente und dem Linker unter milden Bedingungen spaltbar.

Die für die Markierung von Nukeotiden erfindugnsgemäß eingesetzten Makromolekulare Verbidungen schließen in einigen Ausführungsformen wasserlösliche Polymere ein (s.o.). Die Linker der Nukmakromoleküle schließen ebenfalls wasserlösliche Polymere ein (s.o.). Es ist für den Fachmann erkennbar, dass die Zuordung einzelner Polymere zum Linker oder zum Marker einen beschreibenden Charakter hat.

### 1.3.3.3.7 Das Verhältnis der Nuk- Komponenten in einem Nuk-Makromolekül

Ein Nuk-Makromolekül kann durchschnittlich 1 bis 2, 2 bis 5, 5 bis 10, 10 bis 30, 30 bis 100, 100 bis 1000, mehr als 1000 Nuk-Komponenten einschließen.

In einer Ausführungsform haben alle Nuk-Makromoleküle eine gleiche Anzahl an Nuk-Komponenten pro ein Nuk-Makromolekül. Beispielsweise können pro ein Strepavidin-Molekül maximal 4 Biotin-Moleküle gebunden werden, bei sättigender Konzentration von Nuk-Linker- Komponenten, eine einheitliche Population an Nuk-Makromolekülen entsteht.

In einer anderen Ausführungsform haben die Nuk-Makromoleküle einer Population eine definierte durchschnittliche Anzahl von Nuk- Komponenten pro Nuk-Makromolekül, in der Population selbst findet allerdings eine Verteilung der tatsächlichen Besetzung der Nuk-Makromoleküle mit Nuk- Komponenten statt. Die Verteilungsangaben von Nuk- Komponenten pro Nuk-Makromolekül stellen in diesem Fall einen Mittelwert dar.

### 1.3.3.3.8 Das Verhältnis von Marker-Einheiten in einem Nuk-Makromolekül

Die Zahl der Marker-Einheiten in einem Nuk-Makromolekül schließt folgende Bereiche ein: 1 und 2, 2 und 5, 5 und 20, 20 und 50, 50 und 100, 100 und 500, 500 und 1000. 1000 und 10000, 10000 und 100000, mehr als 100000.
In einer Ausführungsform der Erfindung haben Nuk-Makromoleküle eine feste Anzahl von signalgebenden Einheiten pro Marker.
In einer anderen Ausführungsform kann die Verteilung von Marker-Einheiten in einer Nuk-Makromolekül-Population schwanken und muß nicht notwendigerweise einen konstanten Wert für jedes einzelne Nuk-Makromolekül darstellen.

In einer Ausführungsform haben alle Nuk-Makromoleküle eine gleiche Anzahl der Marker-Einheiten pro ein Nuk-Makromolekül. Beispielsweise können pro ein Strepavidin-Molekül maximal 4 Biotin-Moleküle gebunden werden, Avidin-Biotin-Technology, Methods in Enzymology v.184, 1990.

In einer anderen Ausführungsform haben die Nuk-Makromoleküle einer Population eine definierte durchschnittliche Zahl der Marker-Einheiten pro Nuk-Makromolekül. In der Population selbst findet allerdings eine Verteilung der tatsächlichen Besetzung der Nuk-Makromoleküle mit Marker-Einheiten statt. Bei sättigenden Konzentrationen bei der Synthese von Marker-Komponenten findet eine zunehmend einheitlichere Besetzung der Nuk-Makromoleküle mit Marker-Einheiten statt.

So spielt beispielsweise in Bereichen, wo es um den qualitativen Nachweis geht, die exakte Zahl der Marker-Einheiten pro Nuk-Makromolekül eine untergeordnete Rolle. In solchen Fällen ist das Vorhandensein eines stabilen Signals an sich von Bedeutung.

Es sollte einem Fachmann naheliegend erscheinen, dass die angeführten Marker-Komponenten eine beträchtlich größere Molekül-Größe und -Masse haben, als die jeweilige Nuk-Komponente selbst. Weiterhin sollten andere Beispiele für makromolekulare Marker-Komponenten einem Fachmann naheliegend erscheinen.

### 1.3.3.4 Substrateigenschaften der Nuk-Makromoleküle

### 1.3.3.4.1 Substrat-Eigenschaften der Nuk-Komponente

Die Nuk-Komponente integriert in ein Nuk-Makromolekül kann als Substrat für unterschiedliche Enzyme dienen. Beispielsweise dient die Nuk-Komponente, in dieser Ausführungsform als Nukleosid-Triphosphat, als Substrat für Polymerasen, so dass die Nuk-Komponente in einen wachsenden Strang durch eine Polymerase eingebaut werden kann und somit das gesamte Nuk-Makromolekül an den Strang kovalent gekoppelt werden kann.

Zum einen, bestimmen die Substrateigenschaften der Nuk-Komponente(n) die Substrateigenschaften der Nuk-Makromoleküle. Beispielsweise kann die Nuk-Komponente ein 2'-deoxynucleosid-triphosphat sein und ist somit potenzielles Substrat für DNA Polymerasen oder ein ribonucleosid-triphosphat und ist somit ein potenzielels Substrat für RNA Polymerasen. Weiterhin, kann die Nuk-Komponente beispielweise als ein Terminator dienen, so dass nur ein einziges Nuk-Makromolekül eingebaut werden kann. In einer anderen Ausführungsform dient die Nuk-Komponente als ein reversibler Terminator, der die Durchführung einer schrittweise kontrollierten Verlängerungsreaktion erlaubt, wie z.B. in Ju et al. US Pat. 6664079, Tcherkassov WO 02088382 dargestellt ist.

Zum anderen, kann der Marker (z.B. Target-Domäne) einen großen Einfluss auf die Eigenschaften von Nuk-Komponenten ausüben: durch die Bindung der Target-Domäne an die Zielsequenz kann sich die lolake Konzentration der Nuk-Komponente stark erhöhen. Diese Erhöhung in der lokalen Konzentration kann einen Einfluss auf Akzeptanz der Nuk-Kompontente des an die Zielsequenz gebundenen Nuk-Makromoleküls durch die Polymerase haben. Beispielsweise kann die Diskiminierungsfähigkeit der Polymerase bezüglich der Nukleobase der Nuk-Komponente oder weiterer Modifikationen verändert sein. Ebenfalls kann die Wettbewerbssituation zwischen frei in der Lösung befindlichen natürlichen Nukleotiden (z.B. dNTPs) und der Nuk-Kompontente der an die Zielsequenz gebundenen Nuk-Makromolekülen zugunsten des Einbaus der Nuk-Komponente verschoben sein.
Diese Verändungen in der lokalen Konzentration der Nuk-Komponente - starke Erhöhung in der Nähe der gebundenen Target-Domäne - machen es möglich, eine viel größere Bandbreite an Nukleotid-Analoga als Nuk-Komponente zu verwenden, beispielsweise auch solche, die unter gewöhnlichen Reaktionsbedingungen eine nur sehr geringe Einbau-Effizienz haben.

Ebenfalls bietet die Target-Domäne die Möglichkeit, einer Selektion der Markierung zugunsten von Zielsequenzen an. Dies ist insbesondere in Anwesenheit einer starken Kontamination mit fremdartigen Nukleinsäureketten, z.B. störende DNA, von Vorteil: Reaktion kann viel spezifischer Ablaufen, als mit bekannten, konventionell markierten Nukleotiden.
Kopplung von antiviralen Nukleotid-Analoga als Nuk-komponente innerhalb des Nuk-Makromoleküls ermöglicht eine selektive Unterdrückung von viralen Polymerasen.

Die Substrat-Eigenschaften von Nuk-Makromolekülen können durch Anwesenheit von natürlichen Nukleotiden stark beeinflußt werden. Falls keine Bindung der Target-Domäne an die Zielsequenz erfolgt, kann die Präsenz von konkurrierenden Nukleotiden in der Reaktion den Einbau von Nuk-Makromolekülen in den wachsenden Strang verhindern oder stark reduzieren. Umgekehrt, falls die Target-Domäne an die Zielsequenz gebunden hat, können Nuk-Komponenten der Nuk-Makromoleküle in den wachsenden Strang auch in Anwesenheit von hoch konzentrierten konkurrierenden Nukleotiden eingebaut werden.

### 1.3.3.4.2 Substrat-Eigenschaften der Target-, Anker-, Signal-Domänen oder deren Antagonisten

Die Nukleinsäure-Bestandteile eines Nuk-Makromoleküls (z.B. Target-Domäne, Anker-Domäne, Signal-Domäne oder deren Antagonisten) können ebenfalls als Substrat für Enzyme dienen. Beispielsweise können Angatonisten durch Nukleasen (z.B. sequenzspezifische Endonukleasen, oder Exonukleasen, z.B. 5'-3'-Exonuklease-Aktivitäten von Polymerasen) während der Reaktion abgebaut werden.

**1.3.4. Niedermolekularer Marker** eines konventionell markiertes Nukleotides - eine zum Stand der Technik gehörende Markierung von Nukleotiden beispielsweise mit einem oder zwei Biotin-Molekülen, einem oder zwei Farbstoff-Molekülen, einem oder zwei Hapten-Moleküln (z.B. Digoxigenin).

**1.3.5. Konventionell modifiziertes Nukleotid -** ein Nukleotid mit einem kurzen Linker (durchschnittliche Länge zwischen 5 und 30 Atomen) und einem niedermolekularem Marker. Üblicherweise trägt ein konventionell modifiziertes Nukleotid einen niedermolekularen Marker, z.B. ein Farbstoff- oder ein Biotinmolekül oder ein Hapten-Molekül (z.B. DNP, oder Digoxigenin).
Diese Modifikationen können als Signal-Domäne oder Anker-Domäne verwendet werden. Mittels Biotin können beispielsweise Nukleinsäureketten an die feste Phase gebunden werden (Funktion einer Anker-Domäne), oder ein mit einem Enzym oder mit einem Farbstoff markiertes Streptavidin kann an das Biotin gekoppelt werden (Funktion einer Signal-Domäne).

### 1.3.6. Enzyme

### 1.3.6.1 Polymerasen

In einer Ausführungsform können die Nuk-Makromoleküle als Substrate für Enzyme eingesetzt werden. Polymerasen stellen in Anwendungen oft eingesetzte Enzyme, die Nukleotide als Substrate verwerden. Sie werden im weiteren beispielhaft und stellvertretend für andere Nukleotid-umsetzende Enzyme betrachtet. Eine der zentralen Fähigkeit der Polymerasen besteht in kovalenten Kopplung von Nukleotid-Monomeren zu einem Polymer. Dabei kann die Synthese sowohl matrizen-abhängig (wie z.B. DNA-oder RNA-Synthese mit DNA- oder RNA-abhängigen Polymerasen) als auch matrizenunabhängig, z.B. durch Terminale Transferasen ("J Sambrook "Molecular Cloning" 3. Ed. CSHL Press 2001).

Falls RNA als Substrat (z.B. mRNA) in die Sequenzierungsreaktion eingesetzt wird, können handelsübliche RNA-abhängige DNA-Polymerasen eingesetzt werden, z.B. AMV-Reverse Transcriptase (Sigma), M-MLV Reverse Transcriptase (Sigma), HIV-Reverse Transcriptase ohne RNAse-Aktivität. Auch für Klenow Fragment DNA Polymerase ist die Tätigkeit als Reverse Transcriptase beschrieben. Für bestimmte Anwendungen, können Reverse Transcriptasen von RNAse-Aktivität weitgehend frei sein ("Molecular cloning" 1989, Ed. Maniatis, Cold Spring Harbor Laboratory), z.B. bei mRNA-Makrierung für Hybridisierungsexperimente.

Falls DNA als Substrat (z.B. cDNA) verwendet wird, eignen sich als Polymerasen prinzipiell alle DNA-abhängigen DNA-Polymerasen mit oder ohne 3'-5' ExonukleaseAktivität (DNA-Replication" 1992 Ed. A.Kornberg, Freeman and company NY), z.B. modifizierte T7-Polymerase z.B. vom Typ "Sequenase Version 2" (Amersham Pharmacia Biotech), Klenow Fragment der DNA-Polymerase I mit oder ohne 3'-5' Exonukleaseaktivität (New England Biolabs), T4 DNA Polymerase, phi29 DNA Polymerase, Polymerase Beta verschiedenen Ursprungs (Animal Cell DNA Polymerases" 1983, Fry M., CRC Press Inc., kommerziell erhältlich bei Chimerx) thermostabile Polymerasen wie beispielsweise Taq-Polymerase (New England Biolabs), Vent Polymerase, Vent exo minus Polymerase, Deep Vent Polymerase, Deep Vent exo minus Polymerase, Pfu Polymerase, Tli Polymerase, Tfl Polymerase, Tth Polymerase, Thermosequenase, Pwo-Polymerase, Terminator, Terminator I, Terminator II, Terminator III, Bst DNA Polymerase, Bst DNA Polymerase, Large Fragment, Phusion® High-Fidelity DNA Polymerase, Phusion® High-Fidelity Hot Start DNA Polymerase, Phire® Hot Start DNA Polymerase, Phire® Hot Start II DNA Polymerase, Phusion® Flash High-Fidelity DNA Polymerase, Crimson Taq DNA Polymerase, DyNAzyme™ EXT DNA Polymerase, DyNAzyme™ II Hot Start DNA Polymerase, 9°Nₘ DNA Polymerase usw. (von z.B. New England Biolabs oder von Promega oder von Roche oder von Qiagen).

Durch moderne gentechnische Methoden ist es möglich, Polymerasen zu konstruieren, die sich in ihren Fähigkeiten von in der Natur vorkommenden Enzymen unterscheiden, z.B. durch Fehlen bestimmter Aktititäten oder durch verbesserte enzymatische Parameter, wie z.B. Präzision, Prozessivität usw. Eine zunehmende Anzahl von Firmen stellt solche thermolabile und thermostabile Polymerase her, die als optimierte Enzyme für PCR oder andere Amplifikationsverfahren bzw. Markierungsverfahren vermarktet werden. Die Grundfunktionen der Polymerasen bleiben allerdings beibehalten: sie sind in der Lage Nukleotide einzubauen und dadurch komplementäre Stränge synthetisieren. Solche Polymerasen können ebenfalls für die beschriebenen Methoden eingesetzt werden. Eine entsprechende Optimierung der Reaktionsbedingungen ist einem Fachmann bekannt.

In einer Ausführungsform der Anmeldung werden Polymerasen ohne eine 5'-3-exonuklease-Aktivität bevorzugt, z.B. Vent exo minus, Klenow exo minus.

In einer Ausführungsform der Anmeldung werden Polymerasen mit einer 5'-3-exonuklease-Aktivität bevorzugt, z.B. Taq-Polymerase.

In einer Ausführungsform der Anmeldung werden Polymerasen ohne eine Strang-Verdrängungs-Aktivität (keine Strand-Displacement) bevorzugt, z.B. Vent exo minus.

In einer Ausführungsform der Anmeldung werden Polymerasen mit einer Strang-Verdrängungs-Aktivität (keine Strand-Displacement) bevorzugt, z.B. Klenow exo minus.

Auch DNA-abhängige RNA-Polymerasen können verwendet werden, z.B. E.coli RNA-Polymerase, T7-RNA-Polymerase, SP6 RNA Polymerase.

RNA-abhängige RNA-Polymerasen (RNA-Replikase) kann zur Vermehrung und Markierung von RNA verwendet werden, phi6 RNA Polymerase (z.B. Q-beta replicase, Polio-Replicase, 3Dpol, oder Replicase von hepatitis C virus, NS5b).

In der Anmeldung werden DNA-abhängige DNA-Polymerasen als Beispiele für Polymerasen betrachtet.

Weiterführende Literatur mit Beispielen einer Wahl einer richtigen Polymerase, Reaktionsbedingungen usw. ist im Abschnitt "Amplifikationsmethoden" angegeben.

### 1.3.7. Spaltbare Verbindung

Eine unter milden Bedingungen spaltbare Verbindung. Diese Verbindung kann einen Abschnitt im Linker darstellen und kann an einer oder an mehreren Stellen spaltbar sein. Es kann sich um eine chemisch spaltbare Verbindung, wie z.B. eine Disulfid-, eine Ester-, eine Acetal-, oxidativ spaltbare Verbindungen (z.B. Linker, die eine Tartrat-Verbindung einschließen) oder eine Thioesterverbindung (Short WO 9949082, Tcherkassov WO 02088382) handeln. Es kann auch eine photochemisch spaltbare Verbindung, wie in (Rothschild WO 9531429) dargestellt sein. Es kann auch eine enzymatisch spaltbare Verbindung (beispielsweise eine Peptid- oder Polypeptide-Bindung, Odedra WO 0192284), spaltbar durch Peptidasen, eine Poly- oder Oligosaccharid-Bindung, spaltbar durch Disaccharidasen) sein, wobei die Spaltung durch ein spezifisches Enzym zwischen bestimmten Monomeren der spaltbaren Stellen stattfinden kann.

Mehrere Beispiele für spaltbare Verbindungen sind bekannt. Die Kopplung einer solchen Verbindung ist beispielsweise beschrieben in (Tcherkassov 02088382, Metzker et al. Nucleic Acid Research 1994, V.22, S. 4259, Canard et al. Gene, 1994, V. 148, 1, , Kwiatkowski US Pat. 6255475, Kwiatkowski WO 01/25247, Parce WO 0050642.). Eine Spaltbare Verbindung kann ein Teil des Linkers sein oder die Kopplungsstelle des Linkers an das Nukleotid bilden, oder die Verbindung zwischen der Linker-Komponente und der Makrer-Komponente, oder die Verbindung zwischen den Marker-Einheiten und der Kern-Komponente.

### 1.3.8 DNA - Deoxyribonukleinsäure verschiedenen Ursprungs und unterschiedlicher Länge (z.B. Oligonukleotide, Polynukleotide, Plasmide, genomische DNA, cDNA, ssDNA, dsDNA)

### 1.3.9 RNA - Ribonukleinsäure

### 1.3.10 PNA - Peptide Nucleic Acid

### 1.3.11 LNA - locked nucleic acids

### 1.3.12 Nukleotide:

- **dNTP -** 2'-deoxi-Nucleosid-Triphosphate oder deren Analoga, Substrate für DNA-Polymerasen und Reverse-Transkriptasen, z.B. dATP, dGTP, dUTP, dTTP, dCTP.
- **NTP -** Ribonukleosid-Triphosphate oder deren Analoga, Substrate für RNA-Polymerasen, UTP, CTP, ATP, GTP.
- Abkürzung "NT" wird auch bei der Längenangabe einer Nukleinsäuresequenz verwendet, z.B. 1.000 NT. In diesem Fall steht "NT" für Nukleosid-Monophosphate.

Im Text wird bei Abkürzungen die Mehrzahl durch Verwendung des Suffixes "s" gebildet, "NT" steht zum Beispiel für "Nukleotid", "NTs" steht für mehrere Nukleotide.

### 1.3.13 NSK - Nukleinsäurekette. DNA oder RNA, PNA, LNA

**1.3.14 Gesamtsequenz -** Summe aller Sequenzen zu analysierenden Nukleinsäureketten im Ansatz, sie kann ursprünglich aus einer oder mehreren NSKs bestehen. Dabei kann die Gesamtsequenz Teile oder Äquivalente einer anderen Sequenz oder von Sequenz-Populationen darstellen (z.B. mRNA, cDNA, Plasmid-DNA mit Insert, BAC, YAC) und aus einer oder unterschiedlichen Spezies stammen. Gesamtsequenz kann eine oder mehrere Zielsequenzen einschließen.

**1.3.15 NSKF** - Nukleinsäurekettenfragment (DNA oder RNA), das einem Teil der Gesamtsequenz entspricht, NSKFs - Nukleinsäurekettenfragmente. Die Summe der NSKFs bildet ein Äquivalent zur Gesamtsequenz. Die NSKFs können beispielsweise Fragmente von DNA- oder RNA-Gesamtsequenz sein, die nach einem Fragmentierungsschritt entstehen.

### 1.3.16 Primerbindungstelle (PBS) - Teil der Zielsequenz, an den ein Primer binden kann

**1.3.17 Referenzsequenz** - eine bereits bekannte Sequenz, zu der die Abweichungen in der zu untersuchenden Sequenz bzw. in den zu untersuchenden Sequenzen (z.B. Gesamtsequenz) ermittelt werden. Als Referenzsequenzen können in Datenbanken zugängliche Sequenzen verwendet werden, wie z.B. aus der NCBI-Datenbank.

### 1.3.18 Tm - Schmelztemperatur

**1.3.19 Sterisches Hindernis**, sterisch anspruchsvolle Gruppe oder Ligand Sterisch anspruchsvolle Gruppe oder Ligand, die durch ihre chemische Struktur die Eigenschaften der mit dieser Gruppe gekoppelten Nukleotide so verändert, dass diese durch eine Polymerase in einer Extensionsreaktion nicht nacheinander eingebaut werden können. Eine oder mehrere an die Base gekoppelte sterisch anspruchsvolle Gruppen können zur Aufhenung der Synthese oder zu Behinderung der weiteren Synthese führen. Viele in der modernen Forschung eingesetzten Marker stellen ein sterisches Hindernis für die Enzyme dar. Biotin, Digoxigenin und Fluoreszenzfarbstoffe wie Fluoreszein, Tetramethylrhodamine, Cy3-Farbstoff stellen Beispiele einer solchen sterisch anspruchsvollen Gruppe dar (Zhu et al. Cytometry 1997, v.28, S.206, Zhu et al. NAR 1994, v.22, S.3418, Gebeyehu et al., NAR 1987, v.15, S.4513, Wiemann et al. Analytical Biochemistry 1996, v.234, S.166, Heer et al. BioTechniques 1994 v.16 S.54). Weitere sterisch anspruchsvollen Gruppen können beispielsweise lineare oder verzweigte Polymere mit einer kompakten drei-dimensionalen Struktur, wie z.B. Proteine oder Dendrimere darstellen.

Anderes Beispiel für sterisches Hindernis und seine Applikationen siehe Cherkasov et al WO 2008043426.

### 1.3.20 Feste Phase zur Analyse

Es wird feste Phase zur Bindung von markierten Zielsequenzen bereitgestellt. Es wird zwischen einer direkten und einer indirekten, d.h. vermittelten Bindung von Zielsequenen an die feste Phase unterschieden.

Als eine direkte Bindung von Zielsequenzen oder deren Äquivalenten wird in dieser Anmeldung eine Bindung bezeichnet, bei der Zielsequenzen oder deren Äquivalte selbst am Bindungsereignis durch Ausbildung von komplementären Strängen teilnehmen. In einer Ausführungsform wird eine feste Phase bereitgestellt, die in der Lage ist, mindestens eine Zielsequenz oder deren Äquivalente sequenzspezifisch zu binden. Eine solche feste Phase schließt beispielsweise Nukleinsäureketten ein, die Zielsequenz durch Ausbildung eines komplementären Doppelstranges spezifisch binden können. Beispiele für solche feste Phasen sind einem Fachmann bekannt (siehe Literatur zu Microarrays). In einer Ausführungsform kann die feste Phase sequenzspezifsich eine oder mehrere Zielsequenzen binden. Beispiele der festen Phasen mit räumlich angeordneten fixierten Oligonukleotiden sind einem Fachmann bekannt (adressierbare Arrays). Solche feste Phasen sind in der Lage, Zielsequenzen oder deren Äquivalente in Art und Weise zu binden, dass eine räumliche Trennung der Zielsequenzen erfolgen kann. Verwendung von solchen Methoden erlaubt einem Fachmann, Differenzierung zwischen Zielsequenzen vorzunehmen. Ein weiteres Beispiel stellt eine feste Phase bestehend aus Kügelschen oder Teilchen. Solche feste Phasen schließen jeweils spezifische Oligonukeotide pro eine Art der feste Phase (Bead-Array).

Bei einer "indirekten Bindung" der Zielsequenzen an eine feste Phase wird jegliche andere Art der spezifischen Bindung an eine feste Phase verstanden, z.B. solche, welche durch eine Anker-Domäne eines Nuk-Makromoleküls oder Anker-Domäne eines Primers oder Anker-Domäne einer Hybridisierungssonde oder durch konventionelle modifizierte Nukleotide vermittelt wird. Dementsprechend wird in einer weiteren Ausführungsform eine feste Phase bereitgestellt, die in der Lage ist, mindestens eine Anker-Domäne spezifisch zu binden. Die Bindung zwischen einer Anker-Domäne und einer festen Phase kann affin oder kovalent erfolgen. Viele affine Bindungen zwischen zwei spezifischen Partnern sind bekannt, dabei kann man von einem mehr oder weniger spezifischen Bindungspaar sprechen, wie z.B. Biotin-Streptavidin oder Biotin-Avidin, oder Antigen-Antikörper (z.B. Hapten-Antikörper), oder zwei komplementäre Nukleinsäuresträge, oder Aptamer-Zielprotein usw. Die Anker-Domäne kann dabei als Bestandteil eines Nuk-Makromoleküls oder eines Primers oder Hybridisierungssonde integriert sein. Beispiele für Primer mit Anker-Domänen sind einem Fachmann bakannt. Beispiele für Nuk-Makromoleküle wurden oben besprochen.

Die Bindung bzw. Immobilisierung eines der beiden Partner (Bindungspartner einer Anker-Domäne genannt) an die feste Phase ist einem Fachmann bekannt. Die Bindungspartner können in einer bestimmten Anordnung an die feste Phase gebunden werden, so dass eine eindeutige räumliche Zuordnung der Bindungsereignisse stattfinden kann. Beispiele für eine räumlich geordnete Bindung von Bindungspartnern an eine feste Phase sind einem Fachmann bekannt.

Die feste Phase kann in unterschiedlicher Form bereitgestellt sein. Beispielsweise, können Reaktionsgefäße, plane Oberfläche, Kügelchen, Teilchen, Gele als feste Phase verwendet werden. Die feste Phase mit gebundenen Bindungspartnern kann beispielweise als ein Teil einer Vorrichtung zum Flüssigkeitsaustausch und / oder Lichtleitung bereitgestellt sein. Solche festen Phasen sind einem Fachmann bekannt. Beispiele dafür stellen Lateral-Flow-Devices, Mikrofluidic-Vorrichtungen, Mirotiter-Platten usw. Es können unterschiedliche Arrays als feste Phase verwendet werden, wobei Arrays adressierbare Positionen für einzelne Bestandteile der Reaktion vorweisen können, z.B. Bindungspartner zu Anker-Domänen sind in einem bestimmten Muster an der Festphase in Form eines Arrays fixiert. Auch Beads können beispielseise durch Farb-, bzw. durch Durchmesser in einer festimmten Art und Weise kodiert werden, so dass eine Zuordnung von Bindungsereignissen anhand von charakteristischen Merkamalen von Beads möglich ist.

In einer Ausführungsform der Erfindung wird die feste Phase bereitgestellt, z.B. ein Reaktionsgefäß, die einen Bereich einschließt, in dem die Markierungsreaktion durchgeführt wird und einen weiteren, separaten Bereich einschließt, in dem die Bindung der Reaktionsprodukte an die feste Phase erfolgt. Die Überführung der Flüssigkeit aus dem ersten Bereich in den zweiten erfolgt vorzugsweise ohne Öffnung des Gefäßes. Die Bindungspartner der Anker-Domäne sind vorzugsweise an der festen Phase in einer räumlichen Anordnung als Array fixiert.

Erfindungsgemäß werden eine oder mehrere solche feste Phase bereitgestellt, die möglichst geringe unspezifische Bindung von Nukleinsäureketten haben.

Einem Fachmann sollten die technischen Lehren, die in folgenden Literaturquellen aufgeführt sind, helfen, Kombinationen von bekannten Vorrichtungen mit einer festen Phase mit hier beschriebenen Ausführungsformen der Erfindung zu realisieren. Bezüglich der Synthese von Oligonukleotiden, die als Anker-Domänen auftreten oder deren Bindungspartner an der festen Phase oder als deren Antagonisten bilden, und deren Modifikation kann ein Fachmann an folgende Quellen verwiesen werden:
"Oligonucleotide synthesis, methods and applications" Piet Herdewijn, 2004, ISBN 1-58829-233-9
"Protocols for oligonucleotide conjugates, synthesis and analytical techniques" Sudhir Agrawal, 1993, ISBN 0-89603-252-3
"Protocols for oligonucleotide conjugates, synthesis and properties" Sudhir Agrawal, 1993, ISBN 0-89603-247-7
"The aptamer handbook" Sven Klussmann, 2006, ISBN 10: 3-527-31059-2
"Pharmaceutical aspects of oligonucleotides" Patrick Couvreur, 2000, ISBN 0-748-40841-X
"Triple Helix forming Oligonucleotides"Claude Malvy, 1999, ISBN 0-7923-8418-0
"Artificial DNA, methods and applications" Yury E. Khudyakov, ISBN 0-8493-1426-7

Bezüglich der Veteilung von Bindungspartner auf einer festen Phase, Bildung räumlich angeordneten Arrays von Bindungspartnern, Bindung von biologisch aktiven Molekülen an die feste Phase, Vorrichtungen zum Austausch von Flüssigkeiten usw. kann ein Fachmann an folgende Quellen verwiesen werden:
"Microfluidic Lab-on-a-chip for chemical and biological Analysis and discovery" Paul C.H.Li, 2006, ISBN 1-57444-572-3
"Microfluidic for biological applications" Wei-Cheng Tian, 2008, ISBN 978-0-387-09479-3
"Preparation, Microfluidics, Detection methods and Biological applications" Kilian Dill, 2009, ISBN 978-0-387-72716-5
"DNA Microarray, a practical approach" M. Shena, 2002, ISBN 0-19-963777 6
"DNA Microarrays" David Bowtell, 2003, ISBN 0-87969-624-9
"Lateral flow Immunoassays" Raphael Wong, 2009, ISBN 978-1-58829-908-6
"Nucleic Acid Hybridization" M.L.M.Anderson, 1999, ISBN 1-85996-007-3

### 1.3.21 Zielsequenz oder Targetsequenzen

Zielsequenez (Targetsequenz) ist eine Sequenz einer Nukleinsäurekette, die analysiert werden soll. In der modernen Biotechnologie und Medizin sind viele Beispiele für die Analyse von ausgewählten Sequenzen bekannt. In einer Ausführungsform, besteht die Analyse im Nachweis der Präsenz einer bestimmten oder mehrer Zielsequenzen. In einer anderen Ausführungsform besteht die Analyse in der Detektion der Basenabfolge in der Zielsequenz, wobei diese Abfolge vom Interesse ist. In einer anderen Ausführungsform besteht die Analyse in der Bestimmung der Menge der Zielsequenz,

Die Zielsequenz von Organismen kann als DNA oder RNA vorliegen. In der modernen Forschung und Industrie werden auch modifizierte Nukleinsäureketten eingesetzt, so dass eine Zielsequenz auch eine künstliche Sequenz mit oder ohne Modifikationen sein kann.

In einem analytischen Ansatz können auch mehrere Zielsequenzen vorkommen. Beispiele für komplexe Gemische von Zielsequenzen stellen mRNA oder cDNA Gemische, oder Zielsequenzen, die in einer Multiplex-PCR generiert wurden, oder Gemische von Fragmenten einer genomischen DNA. Virale Nukleinsäureketten, die ein Gemisch von Virusvarianten, z.B. HIV-Sequenzen, können ebenfalls ein Gemisch an Zielsequenzen darstellen. Isolate vom Patienten bieten oft ein Gemisch an Nukleinsäureketten, in denen mehrere Zielsequenzen vorkommen können, z.B. virale und bakterielle Zielsequenzen.

Oft wird für einen Nachweis oder eine Analyse einer Zielsequenz oder mehrerer Zielsequenzen eine Vervielfältigung dieser Sequenzen durchgeführt (z.B. mittels einer Polymerase-Chain-Reaction, PCR, Ligase-Chain-Reaction, LCR, oder isothermische Amplifikation). Dadurch werden Äquivalente einer Zielsequenz generiert, z.B. als PCR-Fragmente, oder LCR-Fragmente. Diese Vervielfältigungsmethoden sind dermassen konstruiert, dass die Äquivalente dieselbe Information einschließen, wie auch die ursprünglichen Zielsequenzen. Dies erlaubt in späteren Analyseschritten die Rückschlüsse auf die Zielsequenzen zu ziehen. Aus diesem Grund werden die vervielfältigten Nukleinsäureketten, abgeleitet von einer Zielsequenz, als auch Zielsequenzen oder Zielsequenzäquivalente bezeichnet.

Wegen einer großen Länge kann eine Zielsequenz in mehreren Abschnitten vervielfältig werden. In einer Ausführungsform kann die Summe von einzelnen Abschnitten kann auch als Zielsequenz bezeichnet werden. In einer weiteren Ausführungsform können einzelne Abschnitte als unabhängige Zielsequenzen betrachtet werden.

Ähnliche Situation kennt ein Fachmann auch mit der Umschreibung einer Zielsequenz aus einem Format, z.B. DNA, in ein anderes Format, z.B. RNA (Transcription) oder umgekehrt, wie im Beispiel von Generierung von cDNA aus mRNA mittels Reversen Transcriptase. Solche Sequenzen, die als Folge einer Umschreibung entstanden sind, werden auch als Zielsequenzen oder deren Äquivalente bezeichnet.

Zusammenfassend kann man als Zielsequenzen oder deren Äquivalente alle Sequenzen bezeichnen, die von der Ausgangszielsequenz abgeleitet worden sind oder ursprüngliche Information der Ausgangszielsequenz beibehalten haben und daher Rückschlüsse auf diese Ausgangszielsequenz erlauben.

Die Zielsequenzen (Target-Sequenzen) können aus verschiedenen Spezies abstammen, bzw. unterschiedliche genetische Bereiche erfassen. Viele Organismen wurden in den letzten Jahren intensiv untersucht. Die daraus abgeleiteten Zielsequenzen können beispielsweise PCR Produkte darstellen oder mRNA-Gemische oder small-RNA oder Plasmide usw. Viele Amplifikationstechniken und Isolierungsmethoden sind etabliert worden, um spezifische. genetische Bereiche anzureichern. Die Länge der Targetsequenzen kann varieren. So können beispielswiese ganze Genome eine Zielsequenz darstellen (z.B. HIV-Genom), oder mehrere mRNA in einem Transcriptionsprofil. Andererseits können auch einzelne Basenvariationen an einer Stelle im Genom vom Interesse sein (SNP-Analyse). In einem solchen Fall wird oft ein Abschnitt der Sequenz um die interessierte Stelle (SNP) ausgewählt und amplifiziert. Dieser Abschnitt kann dann als Zielsequenz bezeichnet werden.

Oft besteht ein Bedarf in der Analyse mehrerer Zielsequenzen, z.B. bei der Suche nach einem Krankheiterreger. In einem solchen Fall sucht man nach Vorkommen einzelner Zielsequenzen in einem Material.

Die Wahl der Zielsequenz hängt von der Aufgabenstellung ab. Wie oben erwähnt, können Sequenzen unterschiedlichen Ursprungs als Targetzequenzen dienen. Nachfolgend sind einige Beispiele für Organismen angegeben, von denen die Zielsequenzen ausgewählt werden können.

Ursprung der Zielsequenz kann jeder beliebige Organismus sein, z.B. Viren, Prokarionten, Archea, Eukarionen. Bei Eukarionen können Einzeller, Mehrzeller, wie Tiere, Pflanzen, Fische usw. als Ursprung für eine Zielsequenz dienen.
In einer vorteilhaften Ausführungsform der Anmeldung sind Zielsequenzen aus folgenden Organismen ausgewählt: Mensch, darunter beispielsweise Sequenzen von proteinkodierenden und / oder regulatorischen Bereichen des Genoms, beispielsweise für Rezeptoren, Onkogenen, MHC, Blutgruppen. Weiterhin als Ursprung für Zielsequenzen sind Nutztiere, Forschungstiere und Haustiere wichtig, z.B. Ring, Schwein, Pferd, Hund, Katze, Maus, Ratte, Kaninchen, Affen. Fische können als Ursprung der Zielsequenzen dienen. Sequenzen von Bäumen und Pflanzen wie in der natürlichen Form als auch gentechnisch modifizierte können als Zielsequenzen vom Interesse sein, z.B. Reis, Mais, Weizen, Raabs.
Pilze und Bakterien, die von humanmedizinischer, veterinärer, landwirtschaftlicher, industrielle oder militärischer Bedeutung sind, können den Ursprung der Zielsequenzen darstellen. Beispiele für Bakterien stellen Staphylokokkus aureus, Streptokokkus pyogenes, Streptokokkus pneumoniae, Pseudomonaden, Salmonellen, Shigellen, Yersinien, Campylobacter, Helicobacer, Legionellen, Mykobakterien, Chlamydien, Gonokokken, Yersinien, Francisella tularensis, B. antracis, Aspergillus fumigatus. Beispiele für Viren stellen Humanpathogen: HIV, HSV, HPV, CMV, HBV, HCV, Influenza, SARS, FSME. Beispiele für Parasiten stellen Erreger der Malaria (Plasmodien), Leishmanien, Toxoplasmen.
Die Zielsequenzen können aus einem genomischen Abschnitt abstammen oder von Plasmiden oder von mobilen genetischen Elementen.
In einer Ausführungsform werden Sequenzen aus Genen ausgewählt, die für Antibiotikaresistenz verantwortlich sind. Beispiele dafür stellen Organismen wie MRSA, VRE oder Träger von ESBL-Resistenzen, Chinolon-Resistenzen, In einer weiteren Ausführungsform werden Gene als Zielsequenzen ausgewählt, die für Pathogenitästfaktoren, wie Toxin-Kodierung zuständig sind, oder Invasine oder Adhesine, z.B. Diphterotoxin, Shiga-Toxin, TSST.

In einer weiteren Ausführungsform werden Sequenzen aus Organismen als Zielsequenzen ausgewählt, die in der Nahrungsmittelindustrie eine Rolle spielen, z.B. Bierhefen oder bei Milchprodukten, wie Joghurt-Kulturen oder Käsekulturen.

In vielen Ansätzen werden Kontrollsequenzen mitgeführt. Dies dient der Qualitätssicherung der Reaktion. Solche Kontrollsequenzen können auch als Zielsequenzen bezeichnet werden.

Für Beispiele für Anwendungen, Aufbau von diagnostischen Assay, wo die erfindungsgemäßen Nukleotide und Methoden von Vorteil sein können, wird ein Fachmann an folgende Literatur verwiesen:
"PCR Protocols for emerging infectious Diseases", 1996, ISBN 1-55581-108-6
"Molecular Diagnostic PCR Handbook" Gerrit J. Viljoen, 2005 ISBN 1-4020-3403-2
"PCR Detection of microbial Pathogens" Konrad Sachse, 2003, ISBN 1-58829-049-2
"Clinical Applications of PCR" Y.M. Dennis Lo, 2006, ISBN 1-58829-348-3
"Microarrays in Clinical Diagnostics" Thomas O. Joos, 2005, ISBN 1-58829-394-7
"Molecular Diagnostics" William B. Coleman, 2006, ISBN 1-58829-356-4
"Single nucleotide polymorphisms, methods and protocols" Pui-Yan Kwok, 2003, ISBN 0-89603-968-4
"Molecular Microbiology, diagnostic principles and practice" Fred C. Tenover, 2004, ISBN 1-55581-221-X
"Rapid detection of infectious Agents", Steven Specter, 1998, ISBN 0-306-45848-9
"Nucleic acid amplification technologies, applications to disease diagnosis" H. Lee, 1997, ISBN 1-881299-04-X
"PCR Primers, a laboratory manual" Carl W. Dieffenbach, 2003, ISBN 0-87969-653-2
"Real-Time PCR, current technology and applications" Julie Logan, 2009, ISBN 978-1-904455-39-4
"Rapid cycle Real-Time PCR, methods and applications" S. Meuer, 2001, ISBN 3-540-66736-9
"PCR Primer Design" Anton Yuryev, 2004, ISBN 978-1-58829-725-9
"PCR Troubleshooting, the essential guide" Michael L. Altshuler, 2006, ISBN 1-904455-07-7
"PCR in Bioanalysis" Stephen J. Meltzer, 1998, ISBN 0-89603-497-6
"PCR Protocols" John M.S.Bartlett, ISBN 0-89603-642-1
"PCR Technology current innovations" Thomas Weissensteiner, 2004, ISBN 0-8493-1184-5

### 1.3.22 Primer

Als Primer wird oft ein Oligonukleotid verwendet, der an die komplementäre Position in der Zielsequenz binden kann und von einer Polymerase erkannt werden kann. An das 3'-Ende eines solchen Primers werden Nukleotide angeknüpft.
Einem Fachmann sind viele Beispiele für Primer bekannt. Sie werden für Amplifikationsreaktionen von Nukleinsäureketten und für Markierungsreaktenen eingesetzt. Sie können sequenzspezifisch an die Nukleinsäureketten binden. Durch Einführung einheitlicher Primer-Bindungsstellen und Verwendung entsprechender einheitlicher Primer können viele verschiedene Nukleinsäureketten in einem Ansatz amplifiziert oder markiert werden. Hexamer-Primer stellen Beispiel für unspezifische Markierung dar. Weitere Beispiele sind einem Fachmann bekannt (siehe Literatur zu PCR und Microarrays). Die Primer können mehr oder weniger spezifisch an die Zielsequenzen binden. In einer Ausführungsform ist ein Primer vollständig komplementär zur Zielsequenz und bindet nur an eine solche Zielsequenz. In einer anderen Ausführungsform schließt Primer Sequenzen ein, die an mehrere Zielsequenzen binden können.

In dieser Anmeldung können Primer für die Markierungsreaktion und für die Amplifikation verwendet werden. Je nach Anwendung können Primer nur für eine Aufgabe (z.B. entweder Markierung oder Amplifikation) oder für beide eingesetzt werden. Einem Fachmann sollte es naheliegend erscheinen, in welchen Situationen welche Primer eingesetzt werden sollten.

Ein Primer kann durch Kopplung weiterer Strukturen modifiziert werden. Diese Strukturen können beispielsweise für die Signalgebung bzw. für die Bindung an die feste Phase eingesetzt werden. Ein solcher modifizierter Primer schließt beispielsweise mindestens eine Signal-Domäne oder mindestens eine Anker-Domäne.
Die Strukturen der Anker-Domänen bzw. der Signal-Domänen eines modifizierten Primers können nach selben Prinzipien zusammengesetzt werden, wie für Domänen eines Nuk-Makromoleküls beschrieben (siehe Abschnitt zu Anker-Domäne und Signal-Domäne).
In einer Ausführungsform kann beispielsweise eine Anker-Domäne, gestalltet als eine Nukleinsäurekette (z.B. als DNA, PNA, LNA), an das 5'-Ende vom Primer gekoppelt werden. In einer anderen Ausführungsform kann beispielseise ein Biotin-Molekül oder ein Farbstoff-Molekül (als Signal- oder Anker-Domäne) an den Primer gekoppelt werden.

Durch Verwendung von modifizierten Primern lassen sich die Nukleinsäuketten beispielsweise an die feste Phase binden. Mit einer Signal-Domäne markierte Primer können für die Detektion verwendet werden. Einige Beispiele werden unten angegeben.

Für Beispiele für Primer-Design wird ein Fachmann an folgende Literaur-Quellen verwiesen:
"Nucleic acid amplification technologies, applications to disease diagnosis" H. Lee, 1997, ISBN 1-881299-04-X
"PCR Primers, a laboratory manual" Carl W. Dieffenbach, 2003, ISBN 0-87969-653-2
"Real-Time PCR, current technology and applications" Julie Logan, 2009, ISBN 978-1-904455-39-4
"Rapid cycle Real-Time PCR, methods and applications" S. Meuer, 2001, ISBN 3-540-66736-9
"PCR Primer Design" Anton Yuryev, 2004, ISBN 978-1-58829-725-9
"PCR Troubleshooting, the essential guide" Michael L. Altshuler, 2006, ISBN 1-904455-07-7
"PCR in Bioanalysis" Stephen J. Meltzer, 1998, ISBN 0-89603-497-6
"PCR Protocols" John M.S.Bartlett, ISBN 0-89603-642-1
"PCR Technology current innovations" Thomas Weissensteiner, 2004, ISBN 0-8493-1184-5
"PCR Protocols for emerging infectious Diseases", 1996, ISBN 1-55581-108-6
"Molecular Diagnostic PCR Handbook" Gerrit J. Viljoen, 2005 ISBN 1-4020-3403-2
"PCR Detection of microbial Pathogens" Konrad Sachse, 2003, ISBN 1-58829-049-2
"Clinical Applications of PCR" Y.M. Dennis Lo, 2006, ISBN 1-58829-348-3
"Microarrays in Clinical Diagnostics" Thomas O. Joos, 2005, ISBN 1-58829-394-7
"Molecular Diagnostics" William B. Coleman, 2006, ISBN 1-58829-356-4
"Single nucleotide polymorphisms, methods and protocols" Pui-Yan Kwok, 2003, ISBN 0-89603-968-4
"Molecular Microbiology, diagnostic principles and practice" Fred C. Tenover, 2004, ISBN 1-55581-221-X
"Rapid detection of infectious Agents", Steven Specter, 1998, ISBN 0-306-45848-9

Beispiele für modifizierten Oligonukleotide, die als Primer verwendet werden oder an Primer angekoppelt werden sind in folgenden Quellen zu finden:
"Oligonucleotide synthesis, methods and applications" Piet Herdewijn, 2004, ISBN 1-58829-233-9
"Protocols for oligonucleotide conjugates, synthesis and analytical techniques" Sudhir Agrawal, 1993, ISBN 0-89603-252-3
"Protocols for oligonucleotide conjugates, synthesis and properties" Sudhir Agrawal, 1993, ISBN 0-89603-247-7
"The aptamer handbook" Sven Klussmann, 2006, ISBN 10: 3-527-31059-2
"Pharmaceutical aspects of oligonucleotides" Patrick Couvreur, 2000, ISBN 0-748-40841-X
"Triple Helix forming Oligonucleotides"Claude Malvy, 1999, ISBN 0-7923-8418-0
"Artificial DNA, methods and applications" Yury E. Khudyakov, ISBN 0-8493-1426-7

### 1.3.23 Hybridisierungssonde

Eine Hybridisierungssonde ist ein Oligonukleotid, das in der Lage ist, an die Zielsequenz sequenzspezifisch zu binden.

Eine Hybridisierungssonde kann als zusätzliches Mittel für eine Nachweisreaktion oder für die Kopplung an die feste Phase eingesetzt werden. Eine Hybridisierunssonde kann ähnlich wie ein Nuk-Makromolekül aufgebaut sein, allerdings ohne eine Nuk-Komponente.

Eine Hybridisierungssonde schließet mindestens eine Target-Domäne und wahlweise mindestens eine Signal-Domäne und / oder mindestens eine Anker-Domäne.

Die Strukturen der Target-Domänen, der Anker-Domänen und der Signal-Domänen von einer Hybridisierungssonde können nach selben Prinzipien zusammengesetzt werden, wie für Domänen eines Nuk-Makromoleküls beschrieben (siehe Abschnitt Signal-Domäne).

In einer Ausführungsform kann beispielsweise eine Anker-Domäne, gestalltet als eine Nukleinsäurekette (z.B. als DNA, PNA, LNA), an das 5'-wende oder 3'-Ende von der Hybridisierungssonde gekoppelt werden. In einer anderen Ausführungsform kann beispielseise ein Biotin-Molekül oder ein Farbstoff-Molekül (als Signal-Domäne) an die Hybridisierungssonde gekoppelt werden.

In einer Ausführungsform kann eine Hybridisierungssonde (bestehend aus einer Target-Domäne und einer Anker-Domäne) einen mit einem Nuk-Makromolekül (bestehend aus einer Target-Domäne und einer Signal-Domäne) markierten Nukleinsäurestrang binden und seine Bindung an eine feste Phase vermitteln.

In einer weiteren Ausführungsform kann eine Hybridisierungssonde (bestehend aus einer Target-Domäne und einer Signal-Domäne) einen mit einem Nuk-Makromolekül (bestehend aus einer Target-Domäne und einer Anker-Domäne) markierten Nukleinsäurestrang binden und ein spezifisches Signal vermitteln.

In einer Ausführungsform der Erdingung hat die Hybridisierungssonde Sequenzen, die komplementäre Bindung mit der Kern-Komponente des Nuk-Makromoleküls eingehen können.

### 1.3.24 Amplifikationsmethoden für Nukleinsäureketten

In der modernen Analytik der Nukleinsäuren sind viele Verfahren bekannt, wie man Nukleinsäureketten vervielfältigen kann. Beispiele stellen isothermale Amplifikation oder PCR und ihre verschiedene Modifikationen wie Hotstart-PCR, Multiplex PCR usw.

Zielsequenzen können als solche oder in Form von Zielsequenzequivalenten (s.o.) oder in Form von Amplifikaten (Äquivalente der Zielsequenzen) in einem analytischen Ansatz bereitgestellt werden.

In dieser Anmeldung werden einige Methoden genannt. Sie dienen lediglich zur Veranschaulichung der Erfindung und nicht zu einer Einschränkung.

Beispiele für Amplifikationstechniken von Zielsequenzen oder deren Äquivalenten sind einem Fachmann bekannt. Mehrere wissenschaftliche Artikel beschreiben Amplifikation von Zielsequenzen mittels einer PCR oder einer isothermischen Amplifikation und können in Datenbanken gefunden werden. Beispielweise in der NCBI-Datenbank, PubMed. Dabei sind sowohl die Primer-Sequenzen, als auch Amplifikationsbedingungen angegeben. Viele Zielsequenzen werden mit kommerziell erhältlichen Kits amplifiziert. Am Beispiel der Real-Time-PCR können auch Sondensequenzen ausgewählt werden. Diese Techniken sind einem Fachmann bekannt.

Viele Beispiele für potenzielle Amplifikationsmethoden, die für Vermehrung von Zielsequenzen verwendet werden können, bzw. mit einer Markierungsreaktion kombiniert werden können sind in nachfolgenden Quellen dargestellt.
"Nucleic acid amplification technologies, applications to disease diagnosis" H. Lee, 1997, ISBN 1-881299-04-X
"PCR Primers, a laboratory manual" Carl W. Dieffenbach, 2003, ISBN 0-87969-653-2 "Real-Time PCR, current technology and applications" Julie Logan, 2009, ISBN 978-1-904455-39-4
"Rapid cycle Real-Time PCR, methods and applications" S. Meuer, 2001, ISBN 3-540-66736-9
"PCR Primer Design" Anton Yuryev, 2004, ISBN 978-1-58829-725-9

"PCR Troubleshooting, the essential guide" Michael L. Altshuler, 2006, ISBN 1-904455-07-7
"PCR in Bioanalysis" Stephen J. Meltzer, 1998, ISBN 0-89603-497-6
"PCR Protocols" John M.S.Bartlett, ISBN 0-89603-642-1
"PCR Technology current innovations" Thomas Weissensteiner, 2004, ISBN 0-8493-1184-5
"PCR Protocols for emerging infectious Diseases", 1996, ISBN 1-55581-108-6
"Molecular Diagnostic PCR Handbook" Gerrit J. Viljoen, 2005 ISBN 1-4020-3403-2
"PCR Detection of microbial Pathogens" Konrad Sachse, 2003, ISBN 1-58829-049-2
"Clinical Applications of PCR" Y.M. Dennis Lo, 2006, ISBN 1-58829-348-3
"Microarrays in Clinical Diagnostics" Thomas O. Joos, 2005, ISBN 1-58829-394-7
"Molecular Diagnostics" William B. Coleman, 2006, ISBN 1-58829-356-4
"Single nucleotide polymorphisms, methods and protocols" Pui-Yan Kwok, 2003, ISBN 0-89603-968-4
"Molecular Microbiology, diagnostic principles and practice" Fred C. Tenover, 2004, ISBN 1-55581-221-X
"Rapid detection of infectious Agents", Steven Specter, 1998, ISBN 0-306-45848-9

### 1.3.25 Nachweismethoden

Einem Fachmann sind viele Nachweismethoden bekannt, die heute in der Analytik eingesetzt werden. Dabei können direkte Nachweismethoden (Signalgebende Verfahren) und indirekte (signalvermittelnde Verfahren), einstufige oder mehrstufige Methoden, physikalische, enzymatische, chemische oder elektrochemische Methoden verwendet werden. Viele Signal-Amplifikaitons-Methoden sind ebenfalls bekannt. Einem Fachmann steht frei zu wählen, welche Nachweismethode für eine bestimmte Anwendung besser geeignet ist. In dieser Anmeldung werden einige Beispiele angegeben. Diese Beispiele sollen lediglich zu Demonstration dienen, nicht zur Einschränkung der potenziellen Vielfalt der Nachweismethoden, die mit beschriebenen Verfahren und Strukturen der Nuk-Makromoleküle vereinbar sind.
Es können beispielsweise fluoreszenzbasierte Methoden, Methoden mit Farbgenerierung basierend auf enzymatischen Verfahren (ähnlich wie ELISA) oder Teilchen-basiere Verfahren (z.B. kolloidales Gold, Agglutination) verwendet werden. Weitere Beispiele sind im Abschnitt Signal-Domäne des Markers von Nuk-Makromolekülen angegeben.

Viele Nachweismethoden sind in der Literaur zur festen Phase (s.dort) und Amplifikationstechniken (s. dort) angeführt. Nachfolgend sind noch weitere Beispiele angegeben, wo die modernen Nachweismethoden im Detail beschrieben sind.
"Fluorescent Energy Transfer Nucleic Acid Probes" Vladimir V. Didenko, 2006, ISBN 1-58829-380-7
"Protocols for nucleic acid analysis by nonradioactive probes" Elena Hilario, 2006, ISBN 1-58829-430-7
"Nonisotopic DNA Probe Techniques" Larry J. Kricka, 1992, ISBN 0-12-426295-3
"Handbuch Immunchemische Färbemethoden", 2003, DakoCytomation, ISBN 3-00-011868-3

### 1.3.26 Weitere Enzyme:

In der modernen Diagnostik und Forschung werden noch weitere Enzyme mit Markierungs- bzw. Amplifikationsreaktionen verwendet. Diese Enzyme und deren Wirkungen auf die Reaktionen sind einem Fachmann bekannt. Deren Einsatz kann auch mit in dieser Anmeldung beschriebenen Methoden von Vorteil sein. Hier sind einige Beispiele:
**Uracil-N-Glycosidase** wird oft zur Vermeidung von Cross-Kontaminationen verwendet. Das Enzym ist thermolabil und wird durch PCR-Bedingungen inaktiviert.
**Ligasen** werden zur Verknüpfung von Nukleinsäuresträngen verwendet. Ligasen können als thermolabile oder thermostabile Varianten zur Reaktion zugegeben werden.
**Pyrophosphatasen** wirken unterstützend durch die Hydrolyse des Reaktionsproduktes beim Einbau von Nukleotiden - Phyrophosphat. Thermolabile oder thermophile Formen können kommerziel erworben werden.
**Helicase** entfalten den Doppelstrang. Deren Einsatz wird bei Amplifikationsmethoden unter isothermischen Bedingungen bevorzugt.
**Single Strand Bindung Protein** bindet die einzelsträngige DNA und verhindert die Ausbildung von Sekundärstrukturen

Manche von diesen Proteinen bzw. Enzymen benötigen Kofaktoren bzw. energieliefernde Moleküle, z.B. ATP. Einem Fachmann ist es naheliegend, dass solche Stoffe in die entsprechende Reaktion zugegeben werden müssen.
Bei Optimierung der Reaktionen können diese Proteine bzw. Enzyme und deren Substrate / Co-Faktoren eingesetzt werden.

In einer Ausführungsform schließen die Kits für die Markierungsreaktionen diese Enzyme und deren Substrate / Co-Faktoren ein.

### 1.4. Nachfolgend sind Aspekte der Erfindung sowie ferner beschriebene Aspekte zusammengefasst

Die Erfindung beschreibt neue Verfahren zur Markierung und Nachweis von Nukleinsäureketten, sowie eine neue Gruppe von makromolekularen Nukleotidverbindungen.
1. Aspekt der Erfindung betrifft ein Verfahren zur enzymatischen Synthese von Nukleinsäureketten, bei dem Nukleotide in den komplementären Strang zu mindestens einer Zielsequenz oder deren Äquivalenten enzymatisch inkorporiert werden, wobei zumindest ein der verwendeten Nukleotide folgende Struktur aufweist:
   (Nuk-Linker)n-Marker
   wobei:
   Nuk - ein Nukleotid (Nuk-Komponente)
   Linker - eine Linker-Komponente, die Nuk-Komponente und makromolekulare Marker-Komponente vebindet
   Marker - eine Marker-Komponente ist, die mindestens eine zur Zielsequenz komplementäre Nukleinsäuresequenz einschließt, die sogenannte Target-Domäne
   n - eine Zahl von 1 bis 1000 ist
2. Aspekt der Erfindung betrifft ein Verfahren zur enzymatischen Synthese von Nukleinsäureketten, bei dem Nukleotide in den komplementären Strang zu mindestens einer Zielsequenz oder deren Äquivalenten enzymatisch inkorporiert werden, wobei zumindest ein der verwendeten Nukleotide folgende Struktur aufweist:
   (Nuk-Linker)ₙ-Marker
   wobei:
   Nuk - ein Nukleotid (Nuk-Komponente)
   Linker - eine Linker-Komponente, die Nuk-Komponente und makromolekulare Marker-Komponente vebindet
   Marker - eine Marker-Komponente ist, die mindestens eine Target-Domäne, sowie mindestens eine Anker-Domäne einschließt.
   n - eine Zahl von 1 bis 1000 ist
3. Aspekt der Erfindung betrifft ein Verfahren zur enzymatischen Synthese von Nukleinsäureketten, bei dem Nukleotide in den komplementären Strang zu mindestens einer Zielsequenz oder deren Äquivalenten enzymatisch inkorporiert werden, wobei zumindest ein der verwendeten Nukleotide folgende Struktur aufweist:
   (Nuk-Linker)ₙ-Marker
   wobei:
   Nuk - ein Nukleotid (Nuk-Komponente)
   Linker - eine Linker-Komponente, die Nuk-Komponente und makromolekulare Marker-Komponente vebindet
   Marker - eine Marker-Komponente ist, die mindestens eine Target-Domäne, sowie mindestens eine Signal-Domäne einschließt.
   n - eine Zahl von 1 bis 1000 ist
4. Aspekt der Erfindung betrifft ein Verfahren zur enzymatischen Synthese von Nukleinsäureketten, bei dem Nukleotide in den komplementären Strang zu mindestens einer Zielsequenz oder deren Äquivalenten enzymatisch inkorporiert werden, wobei zumindest ein der verwendeten Nukleotide folgende Struktur aufweist:
   (Nuk-Linker)ₙ-Marker
   wobei:
   Nuk - ein Nukleotid (Nuk-Komponente)
   Linker - eine Linker-Komponente, die Nuk-Komponente und makromolekulare Marker-Komponente vebindet
   Marker - eine Marker-Komponente ist, die mindestens eine Target-Domäne, sowie mindestens eine Anker-Domäne und mindestens eine Signal-Domäne einschließt.
   n - eine Zahl von 1 bis 1000 ist
5. Ein ferner beschriebener Aspekt betrifft ein Verfahren zur enzymatischen Synthese von Nukleinsäureketten, bei dem Nukleotide in den komplementären Strang zu mindestens einer Zielsequenz oder deren Äquivalenten enzymatisch inkorporiert werden, wobei zumindest ein der verwendeten Nukleotide folgende Struktur aufweist:
   (Nuk-Linker)ₙ-Marker
   wobei:
   Nuk - ein Nukleotid (Nuk-Komponente)
   Linker - eine Linker-Komponente, die Nuk-Komponente und makromolekulare Marker-Komponente vebindet
   Marker - eine Marker-Komponente ist, die mindestens Anker-Domäne einschließt
   n - eine Zahl von 1 bis 1000 ist
6. Aspekt der Erfindung betrifft ein Verfahren zur Synthese von Nukleinsäureketten nach einem der Verfahren von einem der Aspekte 1 bis 5 unter Verwendung von mindestens einer Polymerase
7. Aspekt der Erfindung betrifft ein Verfahren zur Synthese von Nukleinsäureketten nach einem der Verfahren von einem der Aspekte 1 bis 6 unter Verwendung von mindestens einem Primer
8. Aspekt der Erfindung betrifft ein Verfahren zur Synthese von Nukleinsäureketten nach einem der Verfahren von einem der Aspekte 1 bis 7 unter Verwendung von mindestens einer weiteren Art von Nukleotiden
9. Aspekt der Erfindung betrifft ein Verfahren zur Synthese von Nukleinsäureketten, wobei das Verfahren folgende Schritte einschließt:
   a) Synthese zumindest eines komplementären Stranges zu mindestens einer Zielsequenz oder deren Äquivalenten nach einem der Verfahren von einem der Aspekte 1 bis 8
   b) Trennung des verlängerten komplementären Stranges von der Zielsequenz oder deren Äquivalenten
   c) gegebenenfalls Wiederholung der Syntheseschritte a) bis b)
10. Aspekt der Erfindung betrifft ein Verfahren zur Synthese von Nukleinsäureketten, das folgende Schritte einschließt:
   a) Synthese zumindest eines komplementären Stranges zu mindestens einer Zielsequenz oder deren Äquivalenten nach einem der Verfahren von einem der Aspekte 1 bis 8 unter Verwendung von mindestens einem Primer, einer Polymerase und einer Nukleotidkomposistion, die mindestens eine Art von Nuk-Makromolekülen sowie mindesten eine weitere Art von Nukleotiden (mit Nukleobasen A, C, T, G oder deren Äquivalenten) einschließt
   b) Trennung des verlängerten komplementären Stranges von der Zielsequenz oder deren Äquivalenten
   c) gegebenenfalls Wiederholung der Syntheseschritte a) und b)
11. Aspekt der Erfindung betrifft ein Verfahren zur Analyse von Nukleinsäureketten, das folgende Schritte einschließt:
   a) Synthese zumindest eines komplementären Strangs zu mindestens einer Zielsequenz oder deren Äquivalenten nach einem der Verfahren von einem der Aspekte 1 bis 10, wobei eine Art von Nuk-Makromolekülen pro eine Zielsequenz mit mindestens einer zielsequenzzugeordneten Anker-Domäne eingesetzt wird
   b) Bereitstellung einer festen Phase, welche die jeweilige Anker-Domäne der unter (a) verwendeten Art der Nuk-Makromoleküle spezifisch binden kann
   c) Inkubation der in (a) synthetisierten Nukleinsäurestrange mit der in (b) bereitgestellten festen Phase unter Bedingungen, die eine spezifische Bindung der jeweiligen Anker-Domäne an die feste Phase zulassen.
   d) Detektion des Bindungsereignisses der Nukleinsäurestränge in (c)
12. Aspekt der Erfindung betrifft ein Verfahren zur Analyse von Nukleinsäureketten, das folgende Schritte einschließt:
   a) Synthese zumindest eines komplementären Strangs zu mindestens einer Zielsequenz oder deren Äquivalenten nach einem der Verfahren von einem der Aspekte 1 bis 10, wobei mehrere Arten von Nuk-Makromolekülen pro eine Zielsequenz mit unterschiedlichen für die jeweilige Art spezifischen, zielsequenzzugeordneten Anker-Domänen eingesetzt werden
   b) Bereitstellung einer festen Phase, welche die jeweiligen Anker-Domäne der unter (a) verwendeten Arten der Nuk-Makromoleküle spezifisch und adressierbar binden kann, wobei die Bindung auf räumlich getrennten, optisch unterscheidbaren Bereirchen der festen Phase stattfindet.
   c) Inkubation der in (a) synthetisierten Nukleinsäurestrange mit der in (b) bereitgestellten festen Phase unter Bedingungen, die eine spezifische Bindung der jeweiligen Anker-Domänen an die feste Phase zulassen.
   d) Detektion des Bindungsereignisses der Nukleinsäurestränge in (c)
13. Aspekt der Erfindung betrifft ein Verfahren zur Analyse von Nukleinsäureketten, das folgende Schritte einschließt:
   a) Synthese zumindest eines komplementären Strangs zu mindestens einer Zielsequenz oder deren Äquivalenten nach einem der Verfahren von einem der Aspekte 1 bis 10, wobei eine Art von Nuk-Makromolekülen pro eine Zielsequenz mit mindestens einer Signal-Domäne eingesetzt wird
   b) Bereitstellung einer festen Phase, welche die mit Nuk-Makromolekülen markierten Nukleinsäurestränge aus dem Schritt (a) spezifisch und adressierbar binden kann, wobei die Bindung auf räumlich getrennten, optisch unterscheidbaren Bereirchen der festen Phase stattfindet. Diese Bindung kann direkt über die Hybridisierung des neu synthetisierten, markierten Strangs an die feste Phase oder indirekt, vermittelt über eine Anker-Domäne eines Nuk-Makromoleküls, oder eines markierten Primers, oder einer markierten Hybridisierungssonde oder durch markierte Nukleotide erfolgen.
   c) Inkubation der in (a) synthetisierten Nukleinsäurestrange mit der in (b) bereitgestellten festen Phase unter Bedingungen, die eine spezifische Bindung der synthetisierten Stränge an die feste Phase zulassen.
   d) Detektion des Bindungsereignisses der Nukleinsäurestränge in (c)
14. Aspekt der Erfindung betrifft ein Verfahren zur Analyse von Nukleinsäureketten, das folgende Schritte einschließt:
   a) Synthese zumindest eines komplementären Strangs zu mindestens einer Zielsequenz oder deren Äquivalenten nach einem der Verfahren von einem der Aspekte 1 bis 10, wobei mehrere Arten von Nuk-Makromolekülen pro eine Zielsequenz mit mindestens einer Signal-Domänen eingesetzt werden
   b) Bereitstellung einer festen Phase, welche die mit Nuk-Makromolekülen markierten Nukleinsäurestränge aus dem Schritt (a) spezifisch und adressierbar binden kann, wobei die Bindung auf räumlich getrennten, optisch unterscheidbaren Bereirchen der festen Phase stattfindet. Diese Bindung kann direkt über die Hybridisierung des neu synthetisierten, markierten Strangs an die feste Phase oder indirekt, vermittelt über eine Anker-Domäne eines jeweiligen Nuk-Makromoleküls, oder eines jeweiligen markierten Primers, oder einer jeweiligen Hybridisierungssonde erfolgen.
   c) Inkubation der in (a) synthetisierten Nukleinsäurestrange mit der in (b) bereitgestellten festen Phase unter Bedingungen, die eine spezifische Bindung der synthetisierten Stränge an die feste Phase zulassen.
   d) Detektion des Bindungsereignisses der Nukleinsäurestränge in (c)

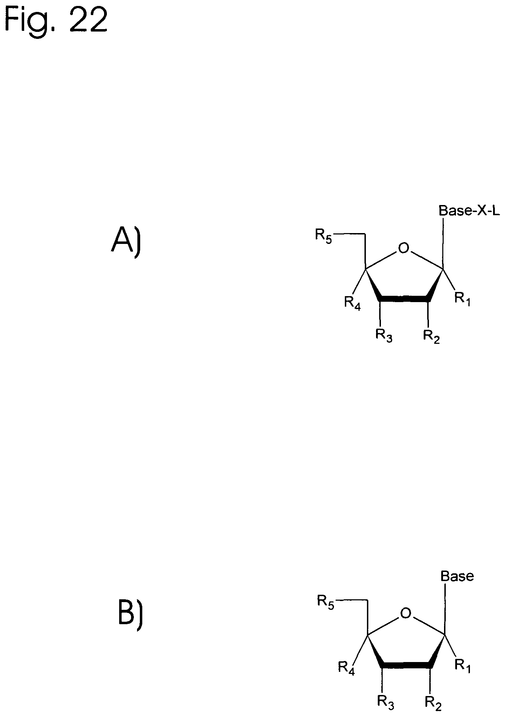

Ein ferner beschriebener Aspekt 15 betrifft makromolekulare Nukleotid-Verbindungen nach einem der Aspekte 1 bis 11, wobei die Nuk-Komponente folgende Strukturen einschließt (Fig. 22A):
Wobei:
   Base - ist unabhängig gewählt aus der Gruppe von Adenin, oder 7-Deazaadenin, oder Guanin, oder 7-Deazaguanin, oder Thymin, oder Cytosin, oder Uracil, oder deren Modifikationen, wobei L die Verbindung zwischen der Nuk-Komponente und der Linker-Komponente darstellt (Kopplungseinheit L) und X die Kopplungsstelle der Kopplungseinheit L an der Base ist

   R₁ - ist H
   R₂ - ist unabhängig gewählt aus der Gruppe H, OH, Halogen, NH₂, SH oder geschützte OH-Gruppe
   R₃ - ist unabhängig gewählt aus der Gruppe H, OH, Halogen, PO₃, SH, N₃, NH₂, O-R₃₋₁, P(O)ₘ-R₃₋₁ (m ist 1 oder 2 ist), NH-R₃₋₁, S-R₃₋₁, Si-R₃₋₁ wobei R₃₋₁ eine chemisch, photochemisch oder enzymatisch spaltbare Gruppe ist, oder eine der folgenden Modifikationen einschließt: -CO-Y, -CH₂-O-Y, -CH₂-S-Y, -CH₂-N₃, -CO-O-Y, -CO-S-Y, -CO-NH-Y, -CH₂-CH=CH₂, wobei Y ein Alkyl ist, beispielsweise (CH₂)ₙ-CH₃ wobei n zwischen 0 und 4 liegt, oder ein substituiertes Alkyl ist, beispielsweise mit Halogen, Hydroxy, Amino, Carboxy-Gruppen).
   R₄ - ist H oder OH
   R₅ - ist unabhängig gewählt aus der Gruppe OH, oder eine geschützte OH-Gruppe, eine Monophosphat-Gruppe, oder eine Diphosphat-Gruppe, oder eine Triphosphat-Gruppe, oder eine Alpha-Thiotriphosphat-Gruppe ist.

Ein ferner beschriebener Aspekt 16 betrifft makromolekulare Nukleotid-Verbindungen nach einem der Aspekte 1 bis 11, wobei die Nuk-Komponente folgende Strukturen einschließt (Fig. 22B):
Wobei:
   Base - ist unabhängig gewählt aus der Gruppe von Adenin, oder 7-Deazaadenin, oder Guanin, oder 7-Deazaguanin, oder Thymin, oder Cytosin, oder Uracil, oder deren zu enzymatischen Reaktionen fähigen Modifikationen

   R₁ - ist H
   R₂ - ist unabhängig gewählt aus der Gruppe H, OH, Hal, NH₂, SH oder geschützte OH-Gruppe
   R₃ - ist unabhängig gewählt aus der Gruppe O-R₃₋₂-L, P(O)ₘ-R₃₋₂-L, wobei m 1 oder 2 ist, NH-R₃₋₂-L, S-R₃₋₂-L, Si-R₃₋₂-L oder, wobei R₃₋₂ die Kopplungsstelle des Linkers an das Nukleotid ist und L - die Kopplungseinheit des Linkers (L) ist.
   R₄ - ist H oder OH
   R₅ - ist unabhängig gewählt aus der Gruppe OH, oder eine geschützte OH-Gruppe, eine Monophosphat-Gruppe, oder eine Diphosphat-Gruppe, oder eine Triphosphat-Gruppe, oder eine Alpha-Thiotriphosphat-Gruppe ist.

Ein ferner beschriebener Aspekt 17 betrifft makromolekulare Nukleotid-Verbindungen nach einem der Aspekte 1 bis 11, wobei die Nuk-Komponente folgende Strukturen einschließt (Fig. 22B):
Wobei:
   Base - ist unabhängig gewählt aus der Gruppe von Adenin, oder 7-Deazaadenin, oder Guanin, oder 7-Deazaguanin, oder Thymin, oder Cytosin, oder Uracil, oder deren zu enzymatischen Reaktionen fähigen Modifikationen

   R₁ - ist H
   R₂ - ist unabhängig gewählt aus der Gruppe H, OH, Hal, NH2, SH oder geschützte OH-Gruppe
   R₃ - ist unabhängig gewählt aus der Gruppe H, OH, Hal, PO₃, SH, NH₂, O-R₃₋₁, P(O)m-R₃₋₁, NH-R₃₋₁, S-R₃₋₁, Si-R₃₋₁ wobei R₃₋₁ eine chemisch, photochemisch oder enzymatisch spaltbare Gruppe ist und m 1 oder 2 ist.
   R₄ - ist H oder OH
   R₅ - ist unabhängig gewählt aus der Gruppe O-R₅₋₁-L, oder P-(O)₃-R₅₋₁-L(modifizierte Monophosphat-Gruppe), oder P-(O)₃-P-(O)₃-R₅₋₁-L (modifizierte Diphosphat-Gruppe) oder P-(O)₃-P-(O)₃-P-(O)₃-R₅₋₁-L (modifizierte Triphosphat-Gruppe), wobei R₅₋₁ die Kopplungsstelle der Kopplungseinheit L an das Nukleotid ist und Kopplungseinheit L die Verbindung zwischen der Nuk-Komponente und der Linker-Komponente ist.

Ein ferner beschriebener Aspekt 18 betrifft makromolekulare Nukleotid-Verbindungen nach Aspekten 15 bis 17, wobei Kopplungseinheit L folgende strukturelle Elemente einschließt:
R₆-NH-R₇, R₆-O-R₇, R₆-S-R₇, R₆-SS-R₇, R₆-CO-NH-R₇, R₆-NH-CO-R₇, R₆-CO-O-R₇, R₆-O-CO-R₇, R₆-CO-S-R₇, R₆-S-CO-R₇, R₆-P(O)₂-R₇, R₆-Si-R₇, R₆-(CH₂)ₙ-R₇,
   R₆-(CH₂)ₙ-R₇, R₆-A-(CH₂)ₙ-R₇, R₆-(CH₂)ₙ-B-R₇,
   R₆-(CH=CH-)ₙ-R₇, R₆-(A-CH=CH-)ₙ-R₇, R₆-(CH=CH-B-)ₙ-R₇, R₆-(CH=CH-CH₂-B-)ₙ-R₇, R₆-A-CH=CH-(CH₂-)ₙ-R₇, R₆-(-CH=CH-CH₂)ₙ-B-R₇,
   R₆-(C≡C-)ₙ-R₇, R₆-(A-C≡C-)ₙ-R₇, R₆-(A-C≡C-CH₂)ₙ-R₇, R₆-(C≡C-B-)ₙ-R₇, R₆-(C≡C-CH₂-B-)ₙ-R₇, R₆-A-C≡C-C-(CH₂-)ₙ-R₇, R₆-(-C≡C-CH₂)ₙ-B-R₇,
   R₆-(-C≡C-CH₂-CH₂)ₙ-B-R₇
wobei R₆ - die Nuk-Komponente ist, R₇ - der Linkerrest ist und A und B unabhängig folgende strukturelle Elemente einschließen: -NH-, -O-, -S-, -SS-, -CO-NH-, -NH-CO-, -CO-O-, -O-CO-, -CO-S-, -S-CO-, -P(O)₂-, -Si-, -(CH₂)ₙ-, eine photolabile Gruppe, wobei n - gleich 1 bis 5 ist

Ein ferner beschriebener Aspekt 19 betrifft makromolekulare Nukleotid-Verbindungen nach Aspekten 15 bis 17 wobei die Linker-Komponente ein hydrophiles, wasserlösliches Polymer einschließt.

Ein ferner beschriebener Aspekt 20 betrifft ein Verfahren nach einem der Aspekten 1 bis 14, wobei die feste Phase unabhängig aus der folgenden Gruppe ausgewählt ist: Papier, Metalle, Silicon, Glas, Keramik, Kunststoffe, Gele oder deren Modifikationen

Ein ferner beschriebener Aspekt 21: Kit zur Markierung von Nukleinsäureketten nach dem Verfahren nach einem der Ansprüche 1 bis 20, das folgende Elemente einschließt:
∘ Eine oder mehrere Arten der Polymerasen
∘ Zumindes eins der Nukleotid-Analoga (Nuk-Makromoleküle), nach Ansprüchen 1 bis 19
∘ Eine feste Phase zu Bindung von markierten Nukleinsäureketten

Ein ferner beschriebener Aspekt 22: Kit zur Markierung von Nukleinsäureketten nach dem Verfahren von einem der Ansprüche 1 bis 21, das einer oder mehrere der folgenden Kompositionen - vorliegend als Lösung in konzentrierter oder in verdünter Form oder auch als Gemisch von trockenen Substanzen - aus der folgenden Liste einschließt:
∘ Eine oder mehrere Arten der Polymerasen
∘ Zumindes eins der Nukleotid-Analoga (Nuk-Makromoleküle), nach einem der Ansprüche 1 bis 19
∘ Lösungen zur Durchführung von enzymatischen Reaktionen
∘ Komposition für Einbaureaktion, einschließlich mindestens einer erforderliche Art von weiteren Nukleosid-Triphosphaten
∘ Komposition für die Bindung von markierten Nukleinsäureketten an die feste Phase
∘ Komposition für Waschen der festen Phase nach der Einbaureaktion
∘ Komposition für optische Detektion der Signale an der festen Phase

Ein ferner beschriebener Aspekt 23: Kit zur Amplifikation und Markierung von Nukleinsäureketten nach einem der Ansprüche 1 bis 22, das eine oder mehrere Elemente aus der folgende Liste einschließt:
∘ Eine oder mehrere Arten der Polymerasen
∘ Eine oder mehrere Primer für die Amplifikation von Nukleinsäureketten
∘ Zumindes eins der Nukleotid-Analoga (Nuk-Makromoleküle), nach einem der Ansprüche 1 bis 19
∘ Lösungen zur Durchführung von enzymatischen Reaktionen
∘ Komposition aus vier dNTPs oder NTPs
∘ Komposition für die Bindung von markierten Nukleinsäureketten an die feste Phase
∘ Komposition für Waschen der festen Phase nach der Einbaureaktion
∘ Komposition für optische Detektion der Signale an der festen Phase

Ein ferner beschriebener Aspekt 24: Kit zur Amplifikation und Markierung von Nukleinsäureketten nach einem der Ansprüche von 1 bis 23, das eine oder mehrere Polymerasen aus der folgenden Liste einschließt:
∘ Reverse Transcriptasen: M-MLV, RSV, AMV, RAV, MAV, HIV
∘ DNA Polymerasen: Klenow Fragment DNA Polymerase, Klenow Fragment exo minus DNA Polymerase, T7 DNA Polymerase, Sequenase 2, Vent DNA Polymerase, Vent exo minus DNA Polymerase, Deep Vent DNA Polymerase, Deep Vent exo minus DNA Polymerase, Taq DNA Polymerase, Tli DNA Polymerase, Pwo DNA Polymerase, Thermosequenase DNA Polymerase, Pfu DNA Polymerase

Ein ferner beschriebener Aspekt 25: Kit zur Markierung von Nukleinsäureketten nach einem der Ansprüche 1 bis 24 in dem die Bestandteile der Kompositionen breits vorgemischt vorliegen.

Ein weiterer Aspekt 26: Kit zur Markierung von Nukleinsäureketten nach einem der Ansprüche 1 bis 25, das eine oder mehrere feste Phasen zur Bindung von markierten Nukleinsäureketten aus der folgenden Liste einschließt:
∘ Plane, transparente feste Phase
∘ Plane, transparente feste Phase, die als Bestandteil einer Flow-Cell oder eines Chips bereitgestellt ist
∘ Feste Phase in Form von Nano- oder Mikrokügelchen
∘ Feste Phase in Form von Nano- oder Mikrokügelchen, die paramagnetisch sind
∘ Mikrotiterplatte
∘ Steifentest bzw. Lateral-Flow-Device

27. Aspekt der Erfindung betrifft ein Verfahren zur enzymatischen Synthese von Nukleinsäureketten, bei dem Nukleotide in den komplementären Strang zu mindestens einer Zielsequenz oder deren Äquivalenten enzymatisch inkorporiert werden, wobei zumindest ein der verwendeten Nukleotide folgende Struktur aufweist:
   (Nuk-Linker)ₙ-Marker
   wobei:
   Nuk - ein Nukleotid (Nuk-Komponente)
   Linker - eine Linker-Komponente, die Nuk-Komponente und makromolekulare Marker-Komponente vebindet
   Marker - eine Marker-Komponente ist, die mindestens ein zur jeweiligen Zielsequenz komplementäres Oligonukleotid einschließt, die sogenannte Target-Domäne
   n - eine Zahl von 1 bis 1000 ist

Ein ferner beschriebener Aspekt 28 betrifft ein Verfahren zur enzymatischen Synthese von Nukleinsäureketten, bei dem Nukleotide in den komplementären Strang zu mindestens einer Zielsequenz oder deren Äquivalenten enzymatisch inkorporiert werden, wobei zumindest ein der verwendeten Nukleotide folgende Struktur aufweist:
(Nuk-Linker)ₙ-Marker
wobei:
Nuk - ein Nukleotid (Nuk-Komponente)
Linker - eine Linker-Komponente, die Nuk-Komponente und makromolekulare Marker-Komponente vebindet
Marker - eine Marker-Komponente ist, die mindestens ein zur jeweiligen Zielsequenz komplementäres Oligonukelotid, sowie mindestens ein weiteres Oligonukleotid zur Bindung an die feste Phase oder zur Signalgenerierung einschließt.
n - eine Zahl von 1 bis 1000 ist

Ein ferner beschriebener Aspekt 29 betrifft ein Verfahren zur enzymatischen Synthese von Nukleinsäureketten, bei dem Nukleotide in den komplementären Strang zu mindestens einer Zielsequenz oder deren Äquivalenten enzymatisch inkorporiert werden, wobei zumindest ein der verwendeten Nukleotide folgende Struktur aufweist:
(Nuk-Linker)ₙ-Marker
wobei:
Nuk - ein Nukleotid (Nuk-Komponente)
Linker - eine Linker-Komponente, die Nuk-Komponente und makromolekulare Marker-Komponente vebindet
Marker - eine Marker-Komponente ist, die mindestens ein zur jeweiligen Zielsequenz komplementäres Oligonukelotid, sowie mindestens ein Biotin-Rest zur Bindung an die feste Phase oder zur Signalgenerierung einschließt.
n - eine Zahl von 1 bis 1000 ist

30. Aspekt der Erfindung betrifft ein Verfahren zur enzymatischen Synthese von Nukleinsäureketten, bei dem Nukleotide in den komplementären Strang zu mindestens einer Zielsequenz oder deren Äquivalenten enzymatisch inkorporiert werden, wobei zumindest ein der verwendeten Nukleotide folgende Struktur aufweist:
   (Nuk-Linker)ₙ-Marker
   wobei:
   Nuk - ein Nukleotid (Nuk-Komponente)
   Linker - eine Linker-Komponente, die Nuk-Komponente und makromolekulare Marker-Komponente vebindet
   Marker - eine Marker-Komponente ist, die mindestens ein zur jeweiligen Zielsequenz komplementäres Oligonukelotid, sowie mindestens einen charakteristischen Farbstoff zur Signalgenerierung einschließt.
   n - eine Zahl von 1 bis 1000 ist

   Ein ferner beschriebener Aspekt 31 betrifft ein Verfahren zur enzymatischen Synthese von Nukleinsäureketten, bei dem Nukleotide in den komplementären Strang zu mindestens einer Zielsequenz oder deren Äquivalenten enzymatisch inkorporiert werden, wobei zumindest ein der verwendeten Nukleotide folgende Struktur aufweist:
   (Nuk-Linker)ₙ-Marker
   wobei:
   Nuk - ein Nukleotid (Nuk-Komponente)
   Linker - eine Linker-Komponente, die Nuk-Komponente und makromolekulare Marker-Komponente vebindet
   Marker - eine Marker-Komponente ist, die mindestens ein Oligonukleotid einschließt, das in der Lage ist, an die feste Phase zu binden, die sogenannte Anker-Domäne
   n - eine Zahl von 1 bis 1000 ist
32. Ein Aspekt der Erfindung betrifft ein Verfahren zu enzymatischen Synthese von Nukleinsäureketten, dieses Verfahren schließt ein:
   Durchführung einer enzymatischen Synthese von mindestens einer Nukleinsäurekette (Zielsequenz) mit mindestens einer Polymerase, mit mindestens einem Primer, das in der Lage ist, an die jeweilige Zielsequenz zu binden, mit mindestens einem Konjugat (einem Nuk-Makromolekül),
   das genannte Konjugat (Nuk-Makromolekül) schließt mindestens ein Nukleosid-triphosphat oder sein Analog, dass in der Lage ist von einer Polymerase als Substrat erkannt zu werden, sowie mindestens ein Oligonukleotid ein, wobei das Oligonukleotid in der Lage ist, an die Zielsequenz spezifisch zu hybridisieren,
   die genannte Polymerase baut das Nukleosid-Triphosphat oder sein Analog während der enzymatischen Synthese in den wachsenden, zur Zielsequenz komplementären Strang,
   Detektion des Einbauereignisses des genannten Konjugats (Nuk-Makromoleküls)
33. Ein Aspekt der Erfindung betrifft ein Verfahren zu enzymatischen Synthese von Nukleinsäureketten, dieses Verfahren schließt ein:
   Durchführung einer enzymatischen Synthese von mindestens einer Nukleinsäurekette (Zielsequenz) mit mindestens einer Polymerase, mit mindestens einem Primer, das in der Lage ist, an die jeweilige Zielsequenz zu binden, mit mindestens einem Konjugat (einem Nuk-Makromolekül),
   das genannte Konjugat (Nuk-Makromolekül) schließt mindestens ein Nukleosid-triphosphat oder sein Analog, dass in der Lage ist von einer Polymerase als Substrat erkannt zu werden, sowie mindestens ein Oligonukleotid ein, wobei das Oligonukleotid in der Lage ist, an die Zielsequenz von der 3'-Seiten des Primers spezifisch zu hybridisieren,
   die genannte Polymerase baut das Nukleosid-Triphosphat oder sein Analog während der enzymatischen Synthese in den wachsenden, zur Zielsequenz komplementären Strang,
   Detektion des Einbauereignisses des genannten Konjugats (Nuk-Makromoleküls)
34. Ein Aspekt der Erfindung betrifft ein Verfahren zu enzymatischen Synthese von Nukleinsäureketten, dieses Verfahren schließt ein:
   Durchführung einer enzymatischen Synthese von mindestens einer Nukleinsäurekette (Zielsequenz) mit mindestens einer Polymerase, mit mindestens einem Primer, das in der Lage ist, an die jeweilige Zielsequenz zu binden, mit mindestens einem Konjugat (einem Nuk-Makromolekül), mindestens einer Hybridisierungssonde
   das genannte Konjugat (Nuk-Makromolekül) schließt mindestens ein Nukleosid-triphosphat oder sein Analog, dass in der Lage ist von einer Polymerase als Substrat erkannt zu werden, sowie mindestens ein Oligonukleotid ein, wobei das Oligonukleotid in der Lage ist, an die Zielsequenz von der 3'-Seite des Primers spezifisch zu hybridisieren,
   die genannte Hybridisierungssonde schließt mindestens ein zur Zielsequenz komplementären Sequenzabschnitt ein, der an die Zielsequenz hybridisiert werden kann von der 3'-Seite des genannten Oligonukleotides des Konjugats (Nuk-Makromolekül)
   die genannte Polymerase baut das Nukleosid-Triphosphat oder sein Analog während der enzymatischen Synthese in den wachsenden, zur Zielsequenz komplementären Strang,
   Detektion des Einbauereignisses des genannten Konjugats (Nuk-Makromoleküls)
35. Ein Aspekt der Erfindung betrifft ein Verfahren zu enzymatischen Synthese von Nukleinsäureketten, dieses Verfahren schließt ein:
   Kontaktierung von mindestens einer Nukleinsäurekette (Zielsequenz) mit mindestens einer Polymerase, mit mindestens einem Primer, das in der Lage ist, an die jeweilige Zielsequenz spezifisch zu binden, mit mindestens einem Konjugat (einem Nuk-Makromolekül),
   das genannte Konjugat (Nuk-Makromolekül) schließt mindestens ein Nukleosid-triphosphat oder sein Analog, dass in der Lage ist von einer Polymerase als Substrat erkannt zu werden, sowie mindestens ein Oligonukleotid ein, wobei das Oligonukleotid in der Lage ist, an die Zielsequenz spezifisch zu hybridisieren,
   Inkubation unter Bedingungen, die eine spezifische Hybridisierung von Primer und / oder Konjugat (und / oder optionsweise Hybridisierungssonde) an die Nukleinsäurekette (Zielsequenz) zulassen
   Inkubation unter Bedingungen, die eine enzymatische Kopplung des Konjugates durch die Polymerase zulassen. Die genannte Polymerase baut das Nukleosid-Triphosphat oder sein Analog während der enzymatischen Synthese in den wachsenden, zur Zielsequenz komplementären Strang,
   Detektion des Einbauereignisses des genannten Konjugats (Nuk-Makromoleküls)

Ein ferner beschriebener Aspekt 36 betrifft ein Verfahren nach einem der Aspekte 32 - 35, wobei sich die genannte Nukleinsäurekette (Zielsequenz) in einer Probe befindet.

Ein ferner beschriebener Aspekt 37 betrifft ein Verfahren nach Aspekt 36, wobei die genannte Probe ein biolofisches oder ein diagnostisches Mateterial oder eine Lebensmittelprobe oder eine Umweltprobe oder eine analytische Probe ist.

Ein ferner beschriebener Aspekt 38 betrifft ein Verfahren nach einem der Aspekte 32 - 37, wobei die genannte Nukleinsäurekette (Zielsequenz) in einzelsträngiger Form bereitgestellt wird

Ein ferner beschriebener Aspekt 39 betrifft ein Verfahren nach einem der Aspekte 32 - 37, wobei die genannte Nukleinsäurekette (Zielsequenz) in doppelsträngiger Form bereitgestellt wird

Ein ferner beschriebener Aspekt 40 betrifft ein Verfahren nach einem der Aspekte 32 - 37, wobei die genannte Nukleinsäurekette (Zielsequenz) in doppelsträngiger Form bereitgestellt wird und Verfahren schließt Komponenten oder Schritte ein, die diese doppelsträngige Form der Nukleinsäurekette in eine einzelsträngige Form umwandeln.

Ein ferner beschriebener Aspekt 41 betrifft ein Verfahren nach einem der Aspekte 1-40, wobei das genannte Oligonukleotid (Target-Domäne des Nuk-Makromoleküls) nicht durch eine Polymerase extendiert werden kann.
42. Ein Aspekt der Erfindung betrifft ein Verfahren nach einem der Aspekte 32 - 41, wobei das Verfahren unter Bedingungen durchgeführt wird, die eine selektive Bindung des genannten Oligonukleotides (Target-Domäne) an die Zielsequenz zulassen.
43. Ein Aspekt der Erfindung betrifft ein Verfahren nach einem der Aspekte 32- 41, wobei das Verfahren unter Bedingungen durchgeführt wird, die eine vorwiegend selektive Bindung des genannten Oligonukleotides (Target-Domäne) an die Zielsequenz zulassen.
44. Ein Aspekt der Erfindung betrifft ein Verfahren nach einem der Aspekte 32-43, wobei mindestens einer der Komponenten (Primer und / oder Konjugat (Nuk-Makromolekül) und / oder Zielsequenz und / oder Hybridisierungssonde und / oder Polymerase) des Verfahrens an eine feste Phase gebunden ist

Ein ferner beschriebener Aspekt 45 betrifft ein Verfahren nach einem der Aspekte 32 - 45, wobei die enzymatische Synthese in mehreren Schritten erfolgt.

Ein ferner beschriebener Aspekt 46 betrifft ein Verfahren nach einem der Aspekte 32 - 45, wobei die enzymatische Synthese in mehreren Schritten erfolgt und diese Schritte unter unterschiedlicher Temperatur durchgeführt werden.

Ein ferner beschriebener Aspekt 47 betrifft ein Verfahren nach einem der Aspekte 32 - 45, wobei die enzymatische Synthese in mehreren Schritten erfolgt und mindestens eines dieser Schritte schließt eine Denaturiereung der doppelstränge von Nukleinsäureketten.

Ein ferner beschriebener Aspekt 48 betrifft ein Verfahren nach einem der Aspekte 32-47, wobei die enzymatische Synthese in mehreren Schritten erfolgt und diese Schritte werden zyklisch wiederholt

Ein ferner beschriebener Aspekt 49 betrifft ein Verfahren nach einem der Aspekte 1-48, wobei das Verfahren zur Vervielfältigung von mindestens einer Nukleinsäurekette eingesetzt wird

Ein ferner beschriebener Aspekt 50 betrifft ein Verfahren nach einem der Aspekte 1-48, wobei das Verfahren zur Markierung von mindestens einer Nukleinsäurekette eingesetzt wird

Ein ferner beschriebener Aspekt 51 betrifft ein Verfahren nach einem der Aspekte 1-48, wobei das Verfahren zum Nachweis von mindestens einer Nukleinsäurekette in einem Ansatz eingesetzt wird

Ein ferner beschriebener Aspekt 52 betrifft ein Verfahren nach einem der Aspekte 1-48, wobei das Verfahren zur Vervielfältigung und gleichzeigiter Markierung von mindestens einer Nukleinsäurekette eingesetzt wird.

Ein ferner beschriebener Aspekt 53 betrifft ein Verfahren nach einem der Aspekte 32-48, wobei das Verfahren zum Nachweis der Präsenz von mindestens einer Nukleinsäurekette in einem Ansatz durch die Vervielfältigung und Markierung eingesetzt wird.

Ein ferner beschriebener Aspekt 54 betrifft ein Verfahren nach einem der Aspekte 32-48, wobei das Verfahren zum Nachweis von mindestens zwei unterschiedlichen Nukleinsäureketten in einem Ansatz eingesetzt wird.

Ein ferner beschriebener Aspekt 55 betrifft ein Verfahren nach einem der Aspekte 32-54, wobei das Verfahren für den Nachweis und / oder Markierung und / oder Amplifikation von mindesten zwei Nukleinsäureketten die für die jeweilige Nukleinsäurekette spezifischen Komponenten, d.h. mindestens einen spezifischen Primer und / oder mindestens ein spezifisches Konjugat (Nuk-Makromoleküle) und / oder mindestens eine spezifische Hybridisierungssonde verwendet.

Ein ferner beschriebener Aspekt 56 betrifft ein Verfahren nach einem der Aspekte 32-54, wobei das Verfahren für den Nachweis und / oder Markierung und / oder Amplifikation von mindesten zwei Nukleinsäureketten die für mindestens zwei Nukleinsäureketten gemeinsame Komponenten, d.h. mindestens einen gemeinsamen Primer und / oder mindestens ein gemeinsames Konjugat (Nuk-Makromoleküle) und / oder mindestens eine gemeinsame Hybridisierungssonde verwendet.

Ein ferner beschriebener Aspekt 57 betrifft ein Verfahren nach einem der Aspekte 32-56, wobei im Verfahren für den Nachweis und / oder Markierung und / oder Amplifikation von Nukleinsäureketten mindesten zwei Arten von Konjugaten (Nuk-Makromolekülen) verwendet und diese Konjugate Oligonukleotide (Target-Domäne), die an unterschiedliche Positionen in der Zielsequenz binden können.

Ein ferner beschriebener Aspekt 58 betrifft ein Verfahren nach einem der Aspekte 1-57, wobei das Verfahren zur Vervielfältigung eine Polymerasen-Ketten-Reaktion (PCR) einschließt

Ein ferner beschriebener Aspekt 59 betrifft ein Verfahren zur Vervielfältigung eine Polymerasen-Ketten-Reaktion (PCR) ist und folgendes einschließt:
Bereitstellung eines PCR-Ansatzes mit einer Probe mit mindestens einer Nukleinsäurekette (Zielsequenz) mit mindestens einer Polymerase, mit mindestens zwei PCR-Primer, die in der Lage sind, an die jeweilige Zielsequenz spezifisch zu binden, mit mindestens einem Konjugat (einem Nuk-Makromolekül),
das genannte Konjugat (Nuk-Makromolekül) schließt mindestens ein Nukleosid-triphosphat oder sein Analog, dass in der Lage ist von der genannten Polymerase als Substrat erkannt zu werden, sowie mindestens ein Oligonukleotid ein, wobei das Oligonukleotid in der Lage ist, an die Zielsequenz spezifisch zwischen beiden PCR-Primern zu hybridisieren,
Bereitstellung von Oligonukleotid-Primern, wobei ein Primer eine zu einem Strand der Zielsequenz komplementäre Sequenz hat und eine Primer-Extension Reaktion starten kann und der andere Primer eine zum anderen Strand der Zielsequenz komplementäre Sequenz hat und eine Primer-Extension Reaktion starten kann, wobei beide Primer dermassen gewählt sind, dass ihre Bindungsstellen innerhalb der Zielsequenz auf beiden Seiten der Bindungsstelle des genannten Konjugates (Nuk-Makromoleküls) liegen
Vervielfältigung der Zielsequenz, falls diese in der Probe vorhanden ist, unter Verwendung einer Polymerase und Bedingungen einer PCR-Reaktion: (a) Hybridisierung der Primer und des Konjugates an die Zielsequenz, (b) Verlängerung der Primer und Einbau von Konjugaten (c) gegebenenfalls Trennung der gebildeten Doppelstränge der Zielsequenz (d) gegebenenfalls Wiederholung der Schritte (a) bis (c) mehrmals
Detektion des Einbauereignisses des genannten Konjugats (Nuk-Makromoleküls)

Ein ferner beschriebener Aspekt 60 betrifft ein Verfahren nach einem der Aspekte 1-57, wobei das Verfahren zur Vervielfältigung eine Transkription ist

Ein ferner beschriebener Aspekt 61 betrifft ein Verfahren nach einem der Aspekte 1-57, wobei das Verfahren zur Vervielfältigung eine isothermische Amplifikation ist

Ein ferner beschriebener Aspekt 62 betrifft ein Verfahren nach einem der Aspekte 1-57, wobei das Verfahren zur Vervielfältigung eine Ligasenketten-Ketten-Reaktion (LCR) ist
63. Ein Aspekt der Erfindung betrifft ein Verfahren nach einem der Aspekte 1-59, wobei das Verfahren zur Markierung von mindestens einer amplifizierten Nukleinsäurekette eingesetzt wird
64. Ein Aspekt der Erfindung betrifft ein Verfahren nach einem der Aspekte 1-59, wobei das Verfahren zum Nachweis von mindestens einer amplifizierten Nukleinsäurekette in einem Ansatz eingesetzt wird

Ein ferner beschriebener Aspekt 65 betrifft ein Verfahren nach einem der Aspekte 1-64, wobei das Verfahren zur Quantifizierung von mindestens einer amplifizierten Nukleinsäurekette in einem Ansatz eingesetzt wird

Ein ferner beschriebener Aspekt 66 betrifft ein Verfahren nach einem der Aspekte 1-64 zur Quantifizierung einer unbekannten Menge einer Zielsequenz, wobei ein Kontrollexperiment mit einer bekannten Menge einer gleichen oder einer unterschiedlicher Zielsequenz durchgeführt wird und die Ergebnisse der Messung der Signalintensität aus beiden Experimenten miteinader verglichen werden.

Ein ferner beschriebener Aspekt 67 betrifft ein Verfahren nach einem der Aspekte 1-66, wobei im Verfahren für den Nachweis und / oder Markierung und / oder Amplifikation und / oder Quantifizierung von Nukleinsäureketten die markierten Nukleinsäureketten (Zielsequenzen oder deren Äquivalente) von anderen Komponenten der Reaktion isoliert werden, bevor die Signal-Messung durchgeführt wird.
68. Ein Aspekt der Erfindung betrifft ein Verfahren nach einem der Aspekte 32-67, wobei das genannte Konjugat mindestens eine der Strukturen nach Aspekten 1 bis 31 einschließt
69. Ein Aspekt der Erfindung betrifft ein Verfahren nach einem der Aspekte 32-67, wobei der Primer und / oder das Konjugat und / oder Hybridisierungssonde einen Reporter (eine Signal-Domäne) einschließt.
70. Ein Aspekt der Erfindung betrifft ein Verfahren nach einem der Aspekte 32-69, wobei die Signalintensität des am Primer und / oder das Konjugat und / oder Hybridisierungssonde gekoppelten Reporters (eine Signal-Domäne) nach dem Einbau des Konjugats in den wachsenden Strang gemessen wird.

Ein ferner beschriebener Aspekt 71 betrifft ein Verfahren nach einem der Aspekte 32-70, wobei die Menge der Zielsequenz im Ansatz durch die Messung des Signals vom genannten Reporter quantifiziert wird.

Ein ferner beschriebener Aspekt 72 betrifft ein Verfahren nach einem der Aspekte 32-71, wobei der Primer und / oder das Konjugat und / oder Hybridisierungssonde eine Modifikation (eine Anker-Domäne) einschließen, die in der Lage ist, eine affine Bindung mit einem Bindungspartner einzugehen.
73. Ein Aspekt der Erfindung betrifft ein Verfahren nach einem der Aspekte 1-72, wobei eine feste Phase bereitgestellt wird, die in der Lage ist, mindestens eine der genannten Modifikationen (Anker-Domäne) vom Primer und / oder das Konjugat (Nuk-Makromolekül) und / oder Hybridisierungssonde affin zu binden.
74. Ein Aspekt der Erfindung betrifft ein Verfahren nach einem der Aspekte 1-72, wobei eine feste Phase bereitgestellt wird, die in der Lage ist, spezifisch mindesten eine Zielsequenz oder deren Äquivalente affin zu binden.
75. Ein Aspekt der Erfindung betrifft ein Verfahren nach einem Aspekt 74, wobei eine feste Phase bereitgestellt wird, die in der Lage ist, mindesten eine Zielsequenz oder deren Äquivalente durch Hybridisierung zu binden.

Ein ferner beschriebener Aspekt 76 betrifft ein Verfahren nach einem der Aspekte 73-75, das mindestens folgende Schritte einschließt:
- Kontaktierung der Reaktionslösung im Anschluss an die enzymatische Reaktion mit der genannten festen Phase, und
- Inkubation unter Bedingungen, die es zulassen, dass Zielsequenzen oder deren gegebenenfalls markieret Äquivalente an diese feste Phase binden können, und
- gegebenenfalls Waschen der festen Phase

Ein ferner beschriebener Aspekt 77 betrifft ein Verfahren nach einem der Aspekte 32-72, wobei der Primer und / oder das Konjugat eine weitere Modifikation (eine Kern-Komponente) einschließen, die in der Lage ist, eine kovalente oder eine affine Bindung mit weiteren Teilen des Konjugats (Linker, Nuk-Komponenten, Signal-Domänen, Anker-Domänen, Target-Domänen) einzugehen.

Ein ferner beschriebener Aspekt 78 betrifft ein Verfahren nach einem der Aspekte 1-77, wobei das genannte Konjugat mindestens ein Nukleosid-Triphosphat einschließt, das über einen Linker an das 5'-Enden des genannten Oligonukleotids gekoppelt ist.

Ein ferner beschriebener Aspekt 79 betrifft ein Verfahren nach einem der Aspekte 1-78, wobei das Oligonukleotid des Konjugats mindestens einen weiteren Sequenzabschnitt einschließt, das nicht zur Zielsequenz komplementär ist.

Ein ferner beschriebener Aspekt 79 betrifft ein Verfahren nach einem der Aspekte 1-79, wobei das Oligonukleotid mit sich selbst keine Hybridisierung eingeht.
80. Ein Aspekt der Erfindung betrifft ein Verfahren nach einem der Aspekte 32-79, wobei mindestens eine Art von weiteren Nukleotiden (nicht Nuk-Makromoleküle) in der Reaktion verwendet werden.

Ein ferner beschriebener Aspekt 81 betrifft ein Verfahren nach Aspekt 80, wobei diese weiteren Nukleotide aus der Gruppe von natürlichen Nukleotiden (z.B. dATP, dGTP, dCTP, dTTP, dUTP, ATP, GTP, CTP, UTP) und / oder modifizierten Nukleotiden (z.B. Nukleotide markiert mit Biotin, wie beispielsweise dUTP-Biotin, dCTP-Biotin, oder Terminatoren, wie beispielweise ddTTP, ddCTP, ddATP, ddGTP, oder Fluoreszenzfarbstoffmarkierte Nukleotide, wie beispielweise dUTP-Cy3 oder dUTP-TAMRA) ausgewählt werden.

Ein ferner beschriebener Aspekt 82 betrifft ein Verfahren nach einem der Aspekte 1-81, bei dem die genannte Polymerase eine thermolabile Polymerase ist

Ein ferner beschriebener Aspekt 83 betrifft ein Verfahren nach einem der Aspekte 1-81, bei dem die genannte Polymerase eine thermostabile Polymerase ist

Ein ferner beschriebener Aspekt 84 betrifft ein Verfahren nach einem der Aspekte 1-81, bei dem die genannte Polymerase eine reversible inaktivierte thermostabile Polymerase ist (eine Hotstart-Polymerase)

Ein ferner beschriebener Aspekt 85 betrifft ein Verfahren nach einem der Aspekte 1-81, bei dem die genannte Polymerase eine strand-displacement Aktivität hat

Ein ferner beschriebener Aspekt 86 betrifft ein Konjugat (Nuk-Makromolekül), dass mindestens folgende Komponenten einschließt:
Mindestens ein Nukleotid
Mindestens ein Oligonukleotid
Mindestens einen Linker
wobei das Nukleotid ein Nukleosid-Triphosphat oder sein Analog ist, das in der Lage ist an einen Primer durch eine Polymerase gekoppelt zu werden
das Oligonukleotid in der Lage ist, an eine Zielsequenz zu binden.
der Linker zwischen dem Nukleosid-Triphosphat und dem Oligonukleotid liegt

Ein ferner beschriebener Aspekt 87 betrifft ein Konjugat (Nuk-Makromolekül) nach Aspekt 86, wobei das Konjugat in einem der Verfahren nach einem der Aspekte 32-85 verwendet wird.

Ein ferner beschriebener Aspekt 88 betrifft ein Konjugat (Nuk-Makromolekül) nach Aspekt 86, wobei das Konjugat mindestens einen Reporter (eine Signal-Domäne) einschließt.

Ein ferner beschriebener Aspekt 89 betrifft ein Konjugat (Nuk-Makromolekül) nach einem der Aspekte 86-88, wobei das Konjugat mindestens eine Modifikation (eine Anker-Domäne) einschließen, die in der Lage ist, eine affine Bindung mit einem Bindungspartner einzugehen.

Ein ferner beschriebener Aspekt 90 betrifft ein Konjugat (Nuk-Makromolekül) nach einem der Aspekte 86-89, wobei das Konjugat mindestens eine weitere Modifikation (eine Kern-Komponente) einschließen, die in der Lage ist, eine kovalente oder eine affine Bindung mit weiteren Teilen des Konjugats (Linker, Nuk-Komponenten, Signal-Domänen, Anker-Domänen, Target-Domänen) einzugehen.

Ein ferner beschriebener Aspekt 91 betrifft ein Konjugat (Nuk-Makromolekül) nach einem der Aspekte 86-90, wobei das genannte Oligonukleotid (Target-Domäne des Nuk-Makromoleküls) nicht durch eine Polymerase extendiert werden kann.

Ein ferner beschriebener Aspekt 92 betrifft ein Konjugat (Nuk-Makromolekül nach einem der Aspekte 86-91, wobei mindestens ein Oligonukleotid aus einer oder mehreren Arten von Nukleinsäuren besteht, ausgewählt aus der Gruppe: DNA, RNA, PNA, LNA
93. Ein Aspekt der Erfindung betrifft ein Konjugat (Nuk-Makromolekül) nach einem der Aspekte 86-92, wobei mindestens ein Oligonukleotid die Länge von mindestens 6 Nukleotiden hat
94. Ein Aspekt der Erfindung betrifft ein Konjugat (Nuk-Makromolekül nach einem der Aspekte 86-92, wobei mindestens ein Oligonukleotid die Länge von mindestens 15 Nukleotiden hat

Ein ferner beschriebener Aspekt 95 betrifft ein Konjugat (Nuk-Makromolekül nach einem der Aspekte 86-94, wobei mindestens ein Oligonukleotid, das mit sich selbst keine Hybridisierung eingeht.

Ein ferner beschriebener Aspekt 96 betrifft ein Konjugat (Nuk-Makromolekül nach einem der Aspekte 86-95, wobei das Oligonukleotid des Konjugats mindestens einen weiteren Sequenzabschnitt einschließt, das nicht zur Zielsequenz komplementär ist.

Ein ferner beschriebener Aspekt 97 betrifft ein Konjugat (Nuk-Makromolekül nach einem der Aspekte 86-96, wobei das genannte Konjugat mindestens ein Nukleosid-Triphosphat oder sein Analog einschließt, das über einen Linker an das 5'-Ende des genannten Oligonukleotids gekoppelt ist.

Ein ferner beschriebener Aspekt 98 betrifft ein Konjugat (Nuk-Makromolekül) nach einem der Aspekte 86-96, wobei das genannte Konjugat mindestens ein Nukleosid-Triphosphat oder sein Analog einschließt, das über einen Linker an das 3'-Ende des genannten Oligonukleotids gekoppelt ist.

Ein ferner beschriebener Aspekt 99 betrifft ein Konjugat (Nuk-Makromolekül) nach einem der Aspekte 86-96, wobei das genannte Konjugat mindestens ein Nukleosid-Triphosphat oder sein Analog einschließt, das über einen Linker an eine interne Sequenz-Position im genannten Oligonukleotids gekoppelt ist.

Ein ferner beschriebener Aspekt 100 betrifft ein Konjugat (Nuk-Makromolekül) nach einem der Aspekte 86-99, wobei mindestens ein Linker zwischen einem Nukleotid und einem Oligonukleotid im genannten Konjugat eine Länge von mindestens 5 Kettenatome hat

Ein ferner beschriebener Aspekt 101 betrifft ein Konjugat (Nuk-Makromolekül) nach einem der Aspekte 86-100, wobei mindestens ein Linker zwischen einem Nukleotid und einem Oligonukleotid im genannten Konjugat eine Länge von mindestens 20 Kettenatome hat

Ein ferner beschriebener Aspekt 102 betrifft ein Konjugat (Nuk-Makromolekül) nach einem der Aspekte 86-101, wobei das Oligonukleotid des Konjugats mindestens einen weiteren Sequenzabschnitt einschließt, das nicht zur Zielsequenz komplementär ist.

Ein ferner beschriebener Aspekt 103 betrifft ein Konjugat (Nuk-Makromolekül) nach einem der Aspekte 86-102, wobei mindestens ein Nukleotid eine Art von Nukleobasen einschließt, die in der Lage ist eine Basenpaarung mit einer der Nukleobasen in der Zielsequenz (Adenin, Guanin, Cytosine, Thymine, Uracil) einzugehen.

Ein ferner beschriebener Aspekt 103 sind Verfahren nach einem der vorheringen Aspekte bei denen die Markierung von komplementären Strängen der Zielsequenzen mit Nuk-Makromolekülen parallel zur Amplifikation von Nukleinsäuresträngen stattfindet.

Ein ferner beschriebener Aspekt 104 sind Verfahren nach einem der vorheringen Aspekte bei denen die Markierung von komplementären Strängen der Zielsequenzen mit Nuk-Makromolekülen parallel zur Amplifikation einer Zielsequenz stattfindet.

Ein ferner beschriebener Aspekt 105 sind Verfahren nach einem der vorheringen Aspekte bei denen die Markierung von komplementären Strängen der Zielsequenzen mit Nuk-Makromolekülen parallel zur Amplifikation der Zielsequenzen stattfindet, wobei weitere, nicht zur Zielsequenz gehörende Nukleinsäureketten im Ansatz vorhanden sind.

Ein ferner beschriebener Aspekt 106 sind Verfahren nach einem der vorheringen Aspekte bei denen die Markierung von komplementären Strängen der Zielsequenzen mit Nuk-Makromolekülen parallel zur Amplifikation dieser Zielsequenzen stattfindet, wobei mehr als eine Zielsequenz im Ansatz vorkommt und entsprechend mehr als zwei spezifische Nuk-Makromoleküle verwendet werden.

Ein ferner beschriebener Aspekt 107 sind Verfahren nach einem der vorheringen Aspekte bei denen die Markierung von komplementären Strängen der Zielsequenzen mit Nuk-Makromolekülen parallel zur Amplifikation dieser Zielsequenzen stattfindet, wobei mehr als eine Zielsequenz im Ansatz vorkommt und diese Zielsequenzen durch ein einheitliches und spezifisches Nuk-Makromolekül erkannt werden.

Ein ferner beschriebener Aspekt 108 sind Verfahren nach einem der vorheringen Aspekte bei denen Amplifikation und Markierung der Nukleinsäureketten durch zyklische Schritte stattfindet, z.B. PCR.
Solche Verfahren schließen einige der folgenden Schritte ein:
- Denaturierung der Zielsequenzen
- Bindung der Primer und der Nuk-Makromoleküle zu Zielsequenzen
- Inkubation des Ansatzes unter Bedingungen, die eine Verlängerung der gebundenen Primer und Einbau der Nuk-Makromoleküle zulassen
- Denaturierung der neu synthetisierten Stränge
- Wiederholung des Vorganges mehrmals
Diese Vorgänge sind beispielsweise mit der Änderung der Temperatur des Ansatzes verbunden, wie es für die PCR bekannt ist.

Ein ferner beschriebener Aspekt 109 sind Verfahren nach einem der vorheringen Aspekte bei denen Amplifikation und Markierung der Nukleinsäureketten durch zyklische Schritte stattfindet, wobei mehrere Enzyme mit unterschiedichen Substrateigenschaften in einem Ansatz kombiniert sind, z.B. Reverse Transcriptasen und DNA polymerasen.

Ein ferner beschriebener Aspekt 110 sind Verfahren nach einem der vorheringen Aspekte bei denen Amplifikation und Markierung der Nukleinsäureketten durch zyklische Schritte stattfindet, wobei die verwendete Polymerase in reversibel inaktivierter Form im Ansatz bereitgestellt wird und erst während der Reaktion aktiviert wird, z.B. eine Hot-Start-Polymerase.

Ein ferner beschriebener Aspekt 111 sind Verfahren nach einem der vorheringen Aspekte bei denen Amplifikation und Markierung der Nukleinsäureketten durch zyklische Schritte stattfindet, wobei die verwendeten Primer in reversibel inaktivierter Form im Ansatz bereitgestellt werden und erst während der Reaktion aktiviert werden, z.B. eine Hot-Start-Primer

Ein ferner beschriebener Aspekt 112 sind Verfahren nach einem der vorheringen Aspekte bei denen Amplifikation und/oder Markierung der Nukleinsäureketten unter Verwendung einer Polymerase stattfinden, die zur Strang-Vertdrängung (Strand-Displacement) fähig ist.

Ein ferner beschriebener Aspekt 113 sind Verfahren nach einem der vorheringen Aspekte bei denen Amplifikation und/oder Markierung der Nukleinsäureketten in einem Reaktionsgemisch stattfinden, das ein oder mehrere Proteine und entsprechende Substrate einschlißet, die in der Lage ist, eine doppelsträngige Nukleinsäureketten (Zielsequenzen) in einzelsträngige Form zu überführen. Beispiel für solche Proteine stellen ATP-abhängige Helicasen.

Ein ferner beschriebener Aspekt 115 sind Verfahren und Komponenten zur Ausführung dieser Verfahren nach einem der vorheringen Aspekte bei denen markierte Nukleinsäureketten an eine feste Phase spezifisch gebunden werden.

Ein ferner beschriebener Aspekt 116 sind Verfahren nach einem der vorheringen Aspekte und Komponenten zur Ausführung dieser Verfahren, bei denen markierte Nukleinsäureketten an eine feste Phase spezifisch gebunden werden und spezifisch detektiert werden.

Ein ferner beschriebener Aspekt 117 sind Verfahren nach einem der vorheringen Aspekte und Komponenten zur Ausführung dieser Verfahren, bei denen markierte Nukleinsäureketten an eine feste Phase spezifisch gebunden werden und durch räumliche Anordnung der Signale spezifisch detektiert werden.

Ein ferner beschriebener Aspekt 118 sind Verfahren nach einem der vorheringen Aspekte und Komponenten zur Ausführung dieser Verfahren, bei denen markierte Zielsequenzen an eine feste Phase spezifisch gebunden werden und spezifisch detektiert werden, wobei weitere, nicht zur Zielsequenz gehörende Nukleinsäureketten im Ansatz vorhanden sind.

Ein ferner beschriebener Aspekt 119 sind Verfahren nach einem der vorheringen Aspekte bei denen die Markierung von komplementären Strängen der Zielsequenzen mit Nuk-Makromolekülen parallel zur Amplifikation dieser Zielsequenzen stattfindet, wobei die Zielsequenzen verschiedenen biologischen Organismen gehören, die unabhängig aus der Gruppe von Viren, Bakterien, Pilzen, einzelligen Parasieten, mehrzelligen Parasieten, Säugetieren, Pflanzen, Fischen ausgewällt sind.

Ein ferner beschriebener Aspekt 120 sind Verfahren nach einem der vorheringen Aspekte bei denen die Nukleinsäurekette (Zielsequenz) eine virale Nukleinsäurekette oder ein Fragment davon ist.

Ein ferner beschriebener Aspekt 121 sind Verfahren nach einem der vorheringen Aspekte bei denen die Nukleinsäurekette (Zielsequenz) eine bakterielle Nukleinsäurekette oder ein Fragment davon ist.

Ein ferner beschriebener Aspekt 122 sind Verfahren nach einem der vorheringen Aspekte bei denen die Nukleinsäurekette (Zielsequenz) eine fungale Nukleinsäurekette oder ein Fragment davon ist.

Ein ferner beschriebener Aspekt 123 sind Verfahren nach einem der vorheringen Aspekte bei denen die Nukleinsäurekette (Zielsequenz) eine Nukleinsäurekette eines Säugetieres oder ein Fragment davon ist.

Ein ferner beschriebener Aspekt 124 sind Verfahren nach einem der vorheringen Aspekte bei denen die Nukleinsäurekette (Zielsequenz) eine Nukleinsäurekette einer Pflanze oder ein Fragment davon ist.

Ein ferner beschriebener Aspekt 125 sind Verfahren nach einem der vorheringen Aspekte bei denen die Nukleinsäurekette (Zielsequenz) eine Nukleinsäurekette eines einzelligen Parasiten oder ein Fragment davon ist.

Ein ferner beschriebener Aspekt 126 sind Verfahren nach einem der vorheringen Aspekte bei denen die Nukleinsäurekette (Zielsequenz) eine Nukleinsäurekette eines mehrzelligen Parasiten oder ein Fragment davon ist.

Ein ferner beschriebener Aspekt 127 sind Verfahren nach einem der vorheringen Aspekte zur Identifizierung bzw. Nachweis der infektiösen Organismen im biologischen Material menschlicher Herkunft.

Ein ferner beschriebener Aspekt 128 sind Verfahren nach einem der vorheringen Aspekte zur Identifizierung bzw. Nachweis der infektiösen Organismen im biologischen Material tierischer Herkunft.

Ein ferner beschriebener Aspekt 129 sind Verfahren nach einem der vorheringen Aspekte zur Identifizierung bzw. Nachweis der infektiösen Organismen im biologischen Material tierischer Herkunft, wobei Tiere aus der folgenden Gruppe gewählt sind: Nutztiere (Säugetiere verschiedener Arten, z.B. Rind, Schwein, Pferd, Hund, Katze, Schaf, Kamel, Vogel verschiedener Arten, Huhn, Trutan, Fische verschiedener Arten).

Ein ferner beschriebener Aspekt 130 sind Verfahren nach einem der vorheringen Aspekte zur Identifizierung bzw. Nachweis der infektiösen Organismen im biologischen Material pflanzlicher Herkunft, wobei Pflanzen aus der folgenden Gruppe gewählt sind: Getreide (z.B. Weizen), Reis, Kartofel, Mais, Raps, Hopfen, Weintrauben).

Ein ferner beschriebener Aspekt 131 sind Verfahren nach einem der vorheringen Aspekte zur Identifizierung bzw. Nachweis der malignen bzw. entarteten Zellen im biologischen Material menschlicher oder tierischer Herkunft.

Ein ferner beschriebener Aspekt 132 sind Verfahren nach einem der vorheringen Aspekte zur Identifizierung bzw. Nachweis Variationen in Nukleinsäuresequenzen im biologischen Material menschlicher Herkunft

Ein ferner beschriebener Aspekt 133 sind Verfahren nach einem der vorheringen Aspekte zur Identifizierung bzw. Nachweis Variationen in Nukleinsäuresequenzen im biologischen Material menschlicher Herkunft, wobei die Zielsequenzen innerhalb der Sequenzen von Rezeptoren, Membran-Proteinen, Transcriptionsfaktoren gewählt werden oder in ihrer Nähe.

Ein ferner beschriebener Aspekt 134 sind Verfahren nach einem der vorheringen Aspekte zur Identifizierung bzw. Nachweis Variationen in Nukleinsäuresequenzen im biologischen Material menschlicher Herkunft, wobei Methylierungsmuster der DNA analysiert werden.

Ein ferner beschriebener Aspekt 135 sind Verfahren nach einem der vorheringen Aspekte zur Identifizierung bzw. Nachweis Variationen in Nukleinsäuresequenzen im biologischen Material menschlicher Herkunft, wobei SNP in der DNA analysiert werden.

Ein ferner beschriebener Aspekt 136 sind Verfahren nach einem der vorheringen Aspekte zur Identifizierung bzw. Nachweis Variationen in Nukleinsäuresequenzen im biologischen Material menschlicher Herkunft, wobei Mutationen (z.B. Translokationen, Delitionen, Insertionen) in der DNA analysiert werden.

Ein ferner beschriebener Aspekt 137 sind Verfahren nach einem der vorheringen Aspekte zur Identifizierung bzw. Nachweis von Nukleinsäuresequenzen im biologischen Material, wobei genetische Abschnitte von Organismen analysiert werden, die im Zusammenhang mit Toxin-Produktion oder Antibiotika-Resistenzen assozieiert sind. Beispiele für solche Toxine stellen Shiga-Toxin, Toxic Shok Syndrom Toxin (TSST), C.dificile Toxin. Beispiele für Resistenz-Assoziierte Gene sind MecA-Gen bei S. aureus, oder Beta-Iactamasen.

Ein ferner beschriebener Aspekt 138 sind Verfahren nach einem der vorheringen Aspekte zur quantitativen Analyse der Nukleinsäuresequenzen im biologischen Material, wobei die Signal-Intensität von mindestens einer mit einem Konjugat markierten Nukleinsäureketten gemessen wird.

Ein ferner beschriebener Aspekt 139 sind Verfahren nach einem der vorheringen Aspekte zur quantitativen Analyse der Nukleinsäuresequenzen im biologischen Material, wobei die Signal-Intensität von mindestens zwei mit einem Konjugat markierten Nukleinsäureketten gemessen wird und die Signalintensitäten miteinader verglichen werden.

Ein ferner beschriebener Aspekt 140 sind Verfahren nach einem der vorheringen Aspekte zur Analyse bzw. Nachweis von Nukleinsäuresequenzen in Lebensmittel.

Ein ferner beschriebener Aspekt 141 sind Verfahren nach einem der vorheringen Aspekte zur Analyse bzw. Nachweis von Nukleinsäuresequenzen in Wasser.

Ein ferner beschriebener Aspekt 142 sind Nukleotid-Analoga mit einer Struktur:
Nukleotid-Linker-(X)
wobei (X) eine Amino-Reaktive Gruppe ist

Ein ferner beschriebener Aspekt 143 sind Nukleotid-Analoga, die an der Base einen Linker und eine reaktive N-Hydroxisuccinimidyl-Gruppe (NHS-Ester) oder deren Derivate (z.B. Sulfo-NHS) einschließen.
Ein ferner beschriebener Aspekt 144 sind Nukleotid-Analoga, die an der Base einen Linker und eine reaktive Isothiocyanat-Gruppe oder deren Derivate einschließen.

Ein ferner beschriebener Aspekt 145 sind Verfahren zur Modifikation von Makromolekülen oder Teilchen mit Nukleotiden, wobei Makromoleküle oder Teilchen mindestens eine Amino-Gruppe einschließen und die Modifikation mit einem modifizierten Nukleotid mit der Formel Nukleotid-Linker-(X) erfolgt.

Ein ferner beschriebener Aspekt 146 sind Verfahren zur Modifikation von Makromolekülen oder Teilchen mit Nukleotiden, wobei Makromoleküle aus folgender Liste gewählt sind: Proteine oder deren Derivate, Dendrimere, Nukleinsäureketten oder deren Derivate (z.B. PNA, LNA), Oligo- und Polysaccharide oder deren Derivate, Lipide.

Ein ferner beschriebener Aspekt 147 sind die Kits, die Komponenten einschließen, um erfindungsgemäße Verfahren nach einem der vorheringen Aspekte durchführen zu können. Solche Kits schließen Konjugate (Nuk-Makromoleküle) nach einem der vorherigen Aspekte und mindestens eine der folgenden Komponenten ein: Puffer, Polymerasen, dNTPs, Primer, Kotrollsequenzen und feste Phase ein.

Ein ferner beschriebener Aspekt 148 sind Kits mit Komponenten zur Herstellung von erfindungsgemäßen Nuk-Makromolekülen. Diese Komponenten schließen ein:
- Mindestens reaktive Nuk-Komponente oder Nuk-Linker-Komponente, die an die Oligonukleotide gekoppelt werden können.
   Beispielsweise Bestand eines solchen Kits sind Nuk-Komponenten oder Nuk-Linker-Komponente, mit einer Amino-Reaktiven-Gruppe (z.B. Nuk-Komponenten mit einem NHS-Ester, oder einem Isothiocyanat), oder einer Thiol-Reaktiven Gruppe (z.B. Nuk-komponenten mit einer Maleimid-Gruppe).
- Mindestens ein Oligonukleotid mit einer Sequenz die an die Zielsequenz spezifisch binden kann Puffer-Lösungen zur Kopplung von Nuk-Komponenten an die Oligonukleotide

### 1.5 Beispiele für Ausführungsformen

1.5.1 Allgemeiner Ablauf einer Markierungsreaktion
1.5.2 Beispiele Nukleinsäurekette / Ausgangsmaterial / Zielsequenzen:
1.5.3 Beispiele für Lösungen:
1.5.4 Polymerasen für Markierungsreaktion:
1.5.5 Primer für die Markierungsreaktion
1.5.6 Zielsequenzspezifische Hybridisierungssonden
1.5.7 Nukleotide
1.5.8 Nukleotidkompositionen
1.5.9 Bindung von markierten Nukleinsäuren an die feste Phase und Detektion
   1.5.9.1 Verwendung von Nuk-Makromolekülen mit einer einheitlichen Anker-Domäne.
   1.5.9.2 Verwendung von Nuk-Makromolekülen mit unterschiedlichen Anker-Domänen.
      1.5.9.2.1 mehrere Nuk-Makromoleküle mit unterschiedlichen spezifischen Kombinationen von Target-Anker-Domänen
      1.5.9.2.2 mehrere Nuk-Makromolküle mit unterschiedlichen spezifischen Kombinationen Nuk-Komponente - Anker-Domäne
   1.5.9.3 Bindung an die feste Phase durch einen sequenzspefischen modifizierten Primer
   1.5.9.4 Bindung an die feste Phase durch eine modifizierten Hybridisierungssonde
   1.5.9.5 Bindung an die feste Phase durch ein modifiziertes Nukleotid
   1.5.9.6 Direkte Bindung der markierten Zielsequenz oder deren Äquivalente an die feste Phase mit fixierten, adressierbaren, der Zielsequenz komplementären Nukleinsäureketten
1.5.10 Nachweis von gebundenen markierten Nukleinsäureketten
   1.5.10.1 Nuk-Makromoleküle mit T-A-S-Domänen
   1.5.10.2 Nuk-Makromoleküle mit T-S-Domänen bzw. S-Domänen
   1.5.10.3 Signalgebung durch einen sequenzspefischen modifizierten Primer
   1.5.10.4 Signalgebung durch eine modifizierten Hybridisierungssonde
   1.5.10.5 Signalgebung durch ein modifiziertes Nukleotid
1.5.11 Verfahrensvarianten: Verwendung von Nuk-Makromolekülen zur Markierung
   1.5.11.1 Markierungsreaktion durch Primerextension
   1.5.11.2 mehrere Nuk-Makromoleküle und eine Zielsequenz
   1.5.11.3 Mehrere Nukleinsäureketten
   1.5.10.4 Matrkierung in mehreren Zyklen bei isothermischen vs. zyklischen Änderung von Temperaturbedingungen
   1.5.10.5 Amplifikation mit Markierung
   1.5.10.6 Nachweis einer spezifischen Zielsequenz in einem Material
   1.5.10.7 Beispiele für Nachweis von Sequenzvarianten in einer spezifischen Zielsequenz (Unterscheidung von verwandten Zielsequenzen, z.B. SNP-Nachweis
1.5.11 Beispiele für Kits
1.5.12 Weitere Enzyme für Verwendung von Nuk-Makromolekülen
1. 5.13 Hinweise für chemische Kopplung
1.5.14 Beispiele von Synthesen von Nuk-Makromolekülen
1.5.15 Beispiele von enzymatischen Markierungsreaktionen von Zielsequenzen mit Nuk-Makromolekülen

### 1.5.1 Allgemeiner Ablauf der Markierungsreaktion:

Als Beispiel kann die Markierung einer einzelsträngigen DNA mit Nuk-Makromolekülen betrachtet werden. Die zu markierenden Nukleinsäureketten werden bereitgestellt und mit einem Primer und einer Polymerase und mindesten einer Art von Nuk-Makromolekülen in Kontakt gebracht und unter Bedingungen inkubiert, die es zulassen, dass der Primer mittels Polymerase verlängert wird. Durch den Einbau von Nuk-Makromolekülen in den wachsenden Strand werden Domäne der Nuk-Makromoleküle an die wachsenden Stränge der Nukleinsäureketten gebunden.

In einer vorteilhaften Ausführungsform der Anmeldung werden Nukleinsäureketten mit Nuk-Makromolekülen markiert, die mindestens eine zielsequenzspezifische Target-Domäne einschließen. Diese Target-Domäne bindet an die zu markierende Nukleinsäurekette auf der 3'-Seite des Primers und erlaubt den Nuk-Makromolekülen, an die zu markierende Nukleinsäurekette sequenzspezifisch zu binden.

Die Nuk-Komponente des Nuk-Makromoleküls ist vorzugsweise so gewählt, dass sie mindestens ein komplementäres Basenpaar mit einem Nukleotid in der Zielsequenz bilden kann. Ein solches Nukleotid liegt vorzugsweise auf der 3'-Seite des hybridisierten Primers, so dass eine Polymerase währed der Verlängerung des 3'-Endes des Primers in der Lage ist diese Nuk-Komponente in den wachsenden Strang einzubauen.

Wegen hoher lokaler Konzentrationen der Nuk-Komponente in der Nähe der Target-Domäne ist es allerdings auch möglich, eine Nuk-Komponente einzusetzen, die kein Basenpaar mit einem Nukleotid in der Zielsequenz bildet und trotzdem durch eine Polymerase unter Mißachtung einer korrekten Basenpaarung eingebaut wird.

In einer weiteren vorteilhaften Ausführungsform der Anmeldung werden Nukleinsäureketten mit Nuk-Makromolekülen markiert, die mindestens eine Target-Domäne und mindestens eine Anker-Domäne einschließen. Die Anker-Domäne kann an einen Bindungspartner an einer festen Phase binden. Nach einer Markierungsreaktion wird eine feste bereitgestellt, die solche Bindungspartner einschließt. Durch Inkubation mit einer solchen festen Phase können markierte Nukleinsäureketten an diese feste Phase spezifisch gebunden werden.

In einer weiteren vorteilhaften Ausführungsform der Anmeldung werden Nukleinsäureketten mit Nuk-Makromolekülen markiert, die mindestens eine Target-Domäne und mindestens eine Signal-Domäne einschließen. Nach einer Markierungsreaktion kann die markierte Nukleinsäurekette anhand des spezifischen Signals der Signal-Domäne detektiert werden.

Im Folgenden werden einzelne Komponenten der Markierungsreaktion und Beispiele für deren Kombinationen einzeln besprochen.

### 1.5.2 Beispiele Nukleinsäurekette / Ausgangsmaterial / Zielsequenzen:

Es können unterschiedliche Nukleinsäuren als Matrize für die Synthese von komplementären Strängen verwendet werden. Viele Methoden sind einem Fachmann bekannt, wie man aus einem Material Nukleinsäuren isoliert und daraus eine Zielsequenz bereitstellt.

Es können sowohl DNA oder RNA als Matrize dienen. Durch Verwendung entsprechender Amplifikationstechniken, können Zielsequenzen in ausreichend hohen Konzentrationen bereitgestellt werden (siehe Abschnitt "Amplifikation").

Die Länge der Zielsequenzen liegt beispielsweise in Bereichen zwischen 20 und 50, 50 und 200, 200 und 500, 500 und 2000, 2000 und 10000, 10000 und 1000000 Nukleotiden, größer 1000000 Nukleotide. Es können einzelne Abschnitte eines Gens oder ein komplettes Genom als Zielsequenz definiert werden.

Es können sowohl doppelsträngige Nukleinsäureketten als auch einzelsträngige Nukleinsäureketten oder auch Gemische aus doppel- und einzelsträngigen Nukleinsäurekette vorliegen.

In einer bevorzugten Ausführungsform der Erfindung, wird nur eine Zielsequenz bereitgestellt.

In einer weiteren bevorzugten Ausführungsform der Erfindung werden mehrere Zielsequenzen bereitgestellt. Die Anzahl an unterschiedlichen Zielsequenzen liegt vorzugsweise in Bereichen zwischen 2 bis 5, 5 bis 10, 10 bis 20, 20 bis 50, 50 bis 100, 100 bis 500, 500 bis 1000, 1000 bis 10000, mehr als 10000.

In einer weiteren bevorzugten Ausführungsform der Erfindung werden zusätzlich zu Zielsequenzen noch weitere Kontrollsequenzen zugegeben, um beispielsweise Qualitäs der Analyse zu kontrollieren. Einsatz solcher Kontrollsequenzen ist einem Fachmann bekannt.

In einer weiteren bevorzugten Ausführungsform der Erfindung werden mehrere Zielsequenzen bereitgestellt, deren Anzahl größer 1000 ist. Ein solches Gemisch kann beispielsweise ein mRNA-Gemisch oder ein cDNA-Gemisch darstellen.

Die Zielsequenzen können mit verschiedenen Techniken vervielfältig werden (z.B. durch PCR, LCR, Isothermische Amplifikation oder durch TMA (transcription mediated amplification). Die Vervielfältigung der Zielsequenzen kann dabei in der Lösung erfolgen oder an einer festen Phase.
In einer Ausführungsform der Erfindung werden die Zielsequenzen für eine Markierungsreaktion bereits in einer amplifizierten Form bereitgestellt, z.B. Produkte einer PCR Reaktion oder einer LCR-Reaktion oder einer isothermischen Amplifikation oder als Plasmide. Die bereitgestellen zu markierenden Nukleinsäureketten können in einzelsträngiger oder auch doppelsträngiger Form bereitgestellt werden. Weitere Beispiele für die Vermehrung von Nukleinsäuren sind einem Fachmann bekannt.

In einer Ausführungsform erfolgt die Vervielfältigung und die Markierung von Zielsequenzen mit Nuk-Makromolekülen in getrennten Ansätzen.

In einer weiteren bevorzugten Ausführungsform erfolgt die Vervielfältigung und die Markierung von Zielsequenzen mit Nuk-Makromolekülen im gleichen Ansatz.

Vor der Analyse können Zielsequenzen mittels weiterer Techniken, z.B. durch Hybridisierung an Microarrays, vorselektiert werden.

Die Zielsequenzen können in einer Lösung oder auch an einer festen Phase fixiert bereitgestellt werden. Die Fixierung an einer festen Phase kann dabei kovalent oder affine sein. Die feste Phase kann beispielseise in Form von planen Oberflächen oder Kügelchen oder Nanoteilchen oder Gelen vorliegen. Die fixierten Zielsequenzen können mit fester Phase in bestimmen Kombinationen eingesetzt werden, beispielsweise sie können in einer bestimmen Anordnung an der festen Phase fixiert sein, oder die feste Phase kann Kodierungselemente einschließen, die eine spätere Zuordnung der festen Phase und Zielsequenz erlauben. Die Kodierung kann bespielsweise farblich erfolgen.

In einer Ausführungsform der Erfindung werden Zielsequenzen in gereinigter Form bereitgestellt. Mehrere Verfahren und Techniken sind einem Fachmann bekannt, wie man Nukleinsäureketten reinigt (siehe Abschnitt "Amplifikation"). In einer weiteren Ausführungsform werden Zielsequenzen als Teil eines Untersuchungsmaterial, z.B. Blut, Sekrete, Reaktionsgemisches usw. bereitgestellt. Solche Materialien werden oft als biologische Matrix bezeichnet, in welche die zu untersuchenden Zielsequenzen integriert sind.
Solche biologische Matrix kann außer Zielsequenzen auch weitere Nukleinsäureketten einschließen. Durch Isolierung von Zielsequenzen werden oft auch diese, Nicht-Zielsequenzen mitisoliert. Solche Nukleinsäureketten (Nicht-Zielsequenzen) können auch als begleitende Nukleinsäureketten oder Kontamination betrachtet werden. Auch Nebenprodukte einer Amplifikationsreaktion können eine solche Kontamination darstellen.

In einer Ausführungsform der Erfindung schließt ein Ansatz zur Markierung von Zielsequenzen auch weitere Nukleinsäureketten (begleitende Nukleinsäureketten), die nicht markiert bzw. nicht detektiert werden sollen. Als solche Nukleinsäureketten kann z.B. DNA oder RNA aus einem biologischen Material sein.

Die unterschiedlichen Kombinationen von Komponenten werden unten ausführlicher dargestellt.

### 1.5.3 Beispiele für Lösungen:

Die verwendbaren Lösungen sollten einen enzymatischen Einbau von Nukleotiden in den wachsenden Strang von Nukleinsäureketten ermöglichen. Als Lösungen für die Markierungsreaktion werden wässrige Puffer-Lösungen bevorzugt. Viele Puffer sind in konzentrierter Form, z.B. 10x, kommerziell erhältlich (z.B. von Nue England Biolabs, Roche Molecular Diagnostics, Abbott, Qiagen usw.). Als Puffer Substanzen kommen werden beispielsweise Tris, Phosphat und HEPES eingesetzt. Der pH-Wert liegt üblicherweise zwischen 7 und 9, wobei viele Polymerasen können auch unter pH 5 und 10 arbeiten. Weitere monovalente Kationen, wie Li+, Na+, K+, NH4+ in Kombination mit Anionen, wie Cl-, SO4²⁻ werden oft zugegeben. Divalente Kationen, wie Mg2+ oder Mn2+ werden zusammen mit Anionen zugegeben. Organische Zusätze, wie DMSO, Glycerol, Detergentien (z.b. Tween), Betaine, PEG, antioxidanten (wie z.B. DTT) werden ebenfalls oft zu Reaktionen zugegeben. Zur Bindung von Schwermetallen wird oft auch EDTA in geringen Konzentrationen eingesetzt.

Die Zusammensetzungen der Lösungen können variieren, so beispielseise die Konzentraionen einzelner Komponenten kann oft durch Titrierung optimal eingestellt werden.

Die entsprechenden Puffer sind vorzugsweise Bestandteil eines Kits zur Markierung von Zielsequenzen mit Nuk-Makromolekülen. Sie werden vorzugsweise in konzentrierer Form bereitgestellt oder in trockener Zubereitung bereitgestellt.

### 1.5.4 Polymerasen für Markierungsreaktion:

Zur Markierungsreaktion können DNA-abhängige DNA Polymerasen, RNA-Abhängige DNA Polymerasen (reverse Transcriptasen), DNA-abhängige RNA Polymerasen und RNA-abhängige RNA Polymerasen verwendet werden. Beispiele für Polymerasen sind im Abschnitt Begriffe und Definitionen angegeben.

In einer bevorzugten Ausführungsform werden Polymerasen ohne 3'-5'Exonuklease Aktivität verwendet. In einer bevorzugten Ausführungsform werden Polymerasen mit 3'-5' Exonuklease Aktivität verwendet.

In einer weiteren Ausführungsform werden Polymerasen mit einer 5'-3'Exonuklease-Aktivität verwendet. In einer weiteren Ausführungsform werden Polymerasen ohne eine 5'-3' Exonuklease-Aktivität verwendet.

In einer Ausführungsform werden thermolabile Polymerase, z.B. Klenow Fragment eingesetzt, In einer weiteren Ausführungsform werden thermostabile Polymerasen, z.B. Taq Polymerase oder Vent exo minus Polymerase eingesetzt.

Es können sogenannte Hotstart-Polymerasen verwendet werden. Es handelt sich um Polymerasen, deren Aktivität durch einen Antikörper oder eine chemische Modifikation reversibel inaktiviert ist. Die Polymerasen werden beispielsweise durch Erhitzen aktiviert.

Es können auch Gemische aus mehreren Polymerasen verwendet werden. Beispielseise schließt ein solches Gemisch Polymerasen mit unterschiedlichen Substrat-Eigenschaften, z.B. eine reverse Transcriptase und eine DNA-Abhängige Polymerase oder es werden thermilabile und thermostabile Enzyme kombiniert.

Die Polymerasen können in gelöster Form oder in trockener Form bereitgestellt werden. Sie können in mit anderen Substanzen als Kompositionen bereitgestellt werden, z.B. zwecks Aufbewahrung kombiniert mit stabilisierenden Substanzen, z.B. Glycerol oder PEG. Es können auch Zugebereitungen von Polymerasen verwendet werden, die für Aufbewahrung bei 4°C oder bei Raumtemperatur vorgesehen sind, solche Zubereitungen sind kommerziell erhältlich, z.B. von GE Healthcare.

In einer bevorzugten Ausführungsform der Anmeldung werden Polymerasen eingesetzt, die Bestandteil einer Zubereitung sind, wobei diese Zubereitung im trockenen Zustand bereitgestellt wird. Die Aktivierung der Polymerasen kann dabei durch Zugabe von Flüssigkeit erfolgen.

In einer bevorzugten Ausführungsform der Anmeldung sind eine oder mehrere Polymerasen oder deren Zubereitungen ein Bestandteil des Kits und werden vorzugsweise in konzentrierer Form bereitgestellt.

In einer Ausführungsform werden für die Amplifikation und für die Markierungsreaktion von Zielsequenzen dieselben Polymerasen verwendet.

Die entsprechenden Polymerasen sind vorzugsweise Bestandteil eines Kits zur Markierung von Zielsequenzen mit Nuk-Makromolekülen.

### 1.5.5 Primer für die Markierungsreaktion

In einer vorteilhaften Ausführungsform der Erfindung wird zumindest ein Oligonukleotid als Primer für die enzymatische Markierungsreaktion von Zielsequenzen eingesetzt. Der Primer muss von der verwendeten Polymerase akzeptiert werden. Beispiele für Oligonukleotide mit einer Primerfunktion sind einem Fachmann bekannt.

Ein Primer für die Markierungsreaktion wird vorzugswise in Form von DNA bereitgestellt. In einer weiteren Ausführugnform wird Primer in Form von RNA bereitgestellt.

Die Länge des Primers liegt vorzugsweise zwischen 6 und 10, 10 und 15, 15 und 20, 20 und 25, 25 und 30, 30 und 40, 40 und 50, 50 und 100, oder auch länger als 100 Nukleotiden. Ein Primer schließt zumindest ein Teil in seiner Sequenz ein, der an die Zielsequenz unter Ausbildung eines Doppelstranges unter Berücksichtigung von Watson-Crick Basenpaarung binden kann. Vorzugsweise liegt dieser Teil des Primer am 3'-Ende des Primers, so dass nach der Bindung des Primers an die Zielsequenz eine enzymatische Einbaureaktion stattfinden kann.

In einer Ausführungsform ist ein Primer vollständig koomplementär zu der Zielsequenz.

Da Sequenzvarianten in der Natur oft vorkommen, ist es in manchen Anwendungen sinnvoll, wenn man mit dem Primer die Sequenzvarianten unterscheiden kann. In einer weiteren Ausführungsform kann die Zusammensetzung des Primers von der ideal komplementären Zusammensetzung der Zielsequenz abweichen, so dass ein Miss-Match in der Nähe des 3'-Endes des Primers auftreten kann (z.B. ein oder mehrere Basenpaare sind im Primer nicht komplementär zu einer Zielsequenzvariante). Ein solcher Miss-Matsch kann zur Differenzierung zwischen Sequenzvarianten herangezogen werden.

Die Positionierung des Primers innerhalb der Zielsequenz kann variabel gestalltet werden. In einer Ausführungsform liegt der Primer an einem Ende der Zielsequenz. In einer weiteren Ausführungsform liegt der Primer innerhalb der Zielsequenz.
In einer weiteren Ausführungsform wird mindestens ein zielsequenzspezifisches Primer-Paar zur Amplifikation und zur Markierung eingesetzt.
In einer weiteren Ausführungsform wird in einem solchen Primer-Paar mindestens ein Primer mit einer Signal-Domäne markiert. In einer weiteren Ausführungsform wird in einem solchen Primer-Paar mindestens ein Primer mit einer Anker-Domäne versehen.

Bei Analyse mehrerer Zielsequenzen kann ein gemeinsamer Primer oder auch mehrere unterschiedliche Primer verwendet werden.

In einer weiteren Ausführungform werden mehrere Primer pro eine Zielsequenz für die Markierungsreaktion eingesetzt, wobei mehrere Primer an einen Strang oder an beide Stränge der Zielsequenz binden können. In einer Ausführungsform schließt ein solches Primergemisch Primer-Sequenzen, die ähnliche Bindungsstelle in der Zielsequenz haben und durch Basen-Unterschiede in Primern verschiedene Varianten der Zielsequenz binden können. Solche Primer können z.B. für SNP-Analysen verwendet werden. In einer anderen Ausführungsform schließt ein solches Primergemisch Primer-Sequenzen, die unterschiedliche Bindungsstellen entlang der Zielsquenz haben. Solche Primer können z.B. für multiple Markierung von Zielsequenzen eingesetzt werden.

In einer weiteren Ausführungform werden mehrere Zielsequenzen in einer Markierungsreaktion mit Nuk-Makromolekülen markiert, wobei jede Zielsequenz mindestens mit einem für spezifischen Primer kombiniert wird.

In einer weiteren Ausführungform werden mehrere Zielsequenzen in einer Markierungsreaktion mit Nuk-Makromolekülen markiert, wobei mindestens ein Primer an zwei oder mehr Zielsequenzen binden kann.

In einer weiteren Ausführungsform werden für unterschiedliche Zielsequenzen einheitliche Primer verwendet. Beispiele für solche Primer stellen Oligo-dT-Sequenzen bei einer cDNA-Synthese. Ein weiteres Beispiel stellt die Einführung einer einheitlichen Primer-Bindungsstelle an alle Zielsequenzen beispielsweise mittels Ligation dar und eine anschließende Markierungsreaktion mit einem einheitlichen Primer.

Die Tm des Primers und die Tm von Target-Domäne der verwendeten Nuk-Makromolekülen werden in einer Ausführungsform aneinader angepaßt, so dass beispielsweise Unterschiede in Tm liegen nicht weiter auseinaner als +/- 5°C.

In einer weiteren Ausführungsform wird die Sequenz des Primers (oder eines Primer-Paars oder eines Sets von Primern) dermassen gestalltet, dass Primer-Tm über der Tm der Target-Domäne des zu verwendenden Nuk-Makromoleküls liegt, wobei der Unterschied beispielsweise größer als 5° bis größer als 50°C beträgt.

In einer weiteren Ausführungsform wird die Sequenz des Primers (oder eines Primer-Paars oder eines Sets von Primern) dermassen gestalltet, dass Primer-Tm unter der Tm der Target-Domäne des zu verwendenden Nuk-Makromoleküls liegt, wobei der Unterschied beispielsweise größer als 5° bis größer als 50°C beträgt.

Durch Variationen in Tm von Primer und Target-Domänen der Nuk-Makromoleküle können die einzelnen Bindungsereignisse (Primer-Bindung und Nuk-Makromolekül-Bindung) durch Temperatur-Veränderungen gesteuert werden. Da die DNA-Polymerasen nur gebunde Primer verlängern, können somit auch die Prozesse der Primer-Verlängerung gesteuert werden. Die Bindung der Target-Domäne eines Nuk-Makromoleküls an die Zielsequenz begünstigt sein Einbau in den Primer. Durch Anpassung der Tm der Target-Domäne an die Reaktionsbedinungen können auch Einbauereignisse der Nuk-Makromoleküle gesteuert werden.

### Modifikatinen

In einer weiteren Ausführungsform schließen Oligonukleotide mit Primerfunktion eine oder mehrere Modifikationen ein. Beispiele für Modifikationen stellen Farbstoffe, Haptene (Antigene), Biotin, weitere Oligonukleotidsequenzen, Proteinsequenzen, PNA Sequenzen dar.

In einer weiteren Ausführungsform schließt ein Primer mindestens eine Anker-Domäne ein. In einer weiteren Ausführungsform schließt ein Primer mindestens eine Signal-Domäne ein. Die verwendbaren Strukturen der Anker-Domäne und Signal-Domäne der Primer können mit denen der Nuk-Makromoleküle identisch sein oder sich auch unterscheiden. Beispiele für modifizierte Primer sind einem Fachmann bekannt.

In einer Ausführungsform werden die Primer für die Markierungsreaktion in einer Lösung bereitgestellt.
In einer weiteren Ausführungsform werden die Primer bereitgestellt, wobei die Primer an einer festen Phase immobilisiert sind. Die fixierten Primer können in Kombinationen mit der festen Phase bereitgestellt werden, bei denen eine eindeutige Zuordnung der Primersequenzen zu einer bestimmen Eigenschaft der festen Phase möglich ist, beispielsweise zu einer Position an der festen Phase, z.B. bei einer planen festen Phase, oder zu einer Farbe oder einem Durchmesser, z.B. bei Kügelchen.

In einer Ausführungsform unterscheiden sich die Primer für die Markierungsreaktion von denen, die für die Amplifikation von Zielsequenzen verwendet werden.

In einer weiteren Ausführungsform werden für die Markierungsreaktion dieselben Primer verwendet, wie für die Amplifikation von Zielsequenzen.

Die entsprechenden Primer sind vorzugsweise Bestandteil eines Kits zur Markierung von Zielsequenzen mit Nuk-Makromolekülen.

### 1.5.6 Zielsequenzspezifische Hybridisierungssonden

Eine Hybridisierungssonde ist ein Oligonukleotid, das in der Lage ist, an die Zielsequenz sequenzspezifisch zu binden.

In einer Ausführungsform der Erfindung können Hybridisierungssonden mit einer Signal-Domäne verwendet werden. Durch eine spezifische Hybridisierung dieser markierten Oligonukleotide an die jeweilige mit Nuk-Makromolekülen markierte Zielsequenz kann ein signalgebendes oder signalvermittelndes Molekül gebunden werden.

In einer weiteren Ausführungsform der Erfindung werden Hybridisierunssonden eingesetzt, die eine Anker-Domäne einschließen. Mit dieser Anker-Domäne, z.B. Hapten, Biotin, Oligonukleotid, können sie an die feste Phase spezifisch binden. Durch solche Oligonukleotide können auch mit Nuk-Makromolekülen markierte Zielsequenzen an die feste Phase spezifisch gebunden werden.

### 1.5.7 Nukleotide

In einer bevorzugten Ausführungsform der Anmeldung werden Nuk-Makromoleküle bei enzymatischen Markierungsreaktion eingesetzt, die folgende Komponenten (Domäne) einschließen:
Nuk-Linker-(Target-Domäne-1)-(Anker-Domäne-1)
   - Nuk - ist eine Nuk-Komponente
   - Linker - ist eine Linker-Komponente
   - Target-Domäne-1 kann spezifisch an einen Sequenzabschnitt in der Zielsequenz unter Berücksichtigung der Watson-Crick Basenpaarung binden / hybridisieren. Beispiele für Target-Domäne-1 stellen Oligonucleotide. Die Struktur der Oligonukleotide kann dabei DNA, RNA, PNA, Morpholino oder LNA sein.
   - Anker-Domäne-1 kann spezifisch an einen Bindungspartner binden. Dieser Bindungspartner ist vorzugsweise an einer festen Phase gebunden. Beispiele für Affinitäts-Doman-1 stellen Oligonucleotide (als DNA, RNA, PNA, Morpholino oder LNA) oder Haptene (z.B. Farbstoffe) oder Biotin. Die ensprechenden Bindungspartner, gebunden an die feste Phase, können beispielsweise Oligonukleotide, Antikörper oder Streptavaidin sein.

In einer bevorzugten Ausführungsform der Anmeldung werden mehrere unterschiedliche Arten von Nuk-Makromolekülen bei enzymatischen Markierungsreaktion eingesetzt, die folgende Komponenten (Domäne) einschließen:
Nuk-Linker-(Target-Domäne-n)-(Anker-Domäne-n)
   - Nuk - ist eine Nuk-Komponente
   - Linker - ist eine Linker-Komponente
   - Target-Domäne-n können spezifisch an den jeweiligen Sequenzabschnitt in der entsprechenden Zielsequenzen unter Berücksichtigung der Watson-Crick Basenpaarung binden / hybridisieren. Die Anzahl der Target-Domänen (n) entspricht der Anzahl der zu markierenden Zielsequenzen.
   - Anker-Domäne-n können spezifisch an den jeweiligen Bindungspartner binden. Diese Bindungspartner sind vorzugsweise an einer festen Phase gebunden. Die Anzahl der Anker-Domänen (n) entspricht der Anzahl der zu markierenden Zielsequenzen.
   - Die jeweiligen Anker-Domänen sind spezifisch für die jeweiligen Target-Domänen.

In einer bevorzugten Ausführungsform der Anmeldung werden mehrere unterschiedliche Arten von Nuk-Makromolekülen bei enzymatischen Markierungsreaktion eingesetzt, die folgende Komponenten (Domäne) einschließen:
Nuk-Linker-(Target-Domäne-1)-(Anker-Domäne-n)
   - Nuk - ist eine Nuk-Komponente
   - Linker - ist eine Linker-Komponente
   - Target-Domäne-1 kann spezifisch an einen Sequenzabschnitt in der Zielsequenz unter Berücksichtigung der Watson-Crick Basenpaarung binden / hybridisieren.
   - Anker-Domäne-n können spezifisch an den jeweiligen Bindungspartner binden. Diese Bindungspartner sind vorzugsweise an einer festen Phase gebunden. Die Anzahl der Anker-Domänen (n) kann beispielsweise zwischen 2 und 100 liegen.
   - Die Target-Domäne ist einheitlich und ist mit jeweils spezifischen Anker-Domänen kombiniert.

In einer bevorzugten Ausführungsform der Anmeldung werden mehrere unterschiedliche Arten von Nuk-Makromolekülen bei enzymatischen Markierungsreaktion eingesetzt, die folgende Komponenten (Domäne) einschließen:
Nuk-Linker-(Target-Domäne-n)-(Anker-Domäne-1)
   - Nuk - ist eine Nuk-Komponente
   - Linker - ist eine Linker-Komponente
   - Target-Domäne-n können spezifisch an den jeweiligen Sequenzabschnitt in der entsprechenden Zielsequenzen unter Berücksichtigung der Watson-Crick Basenpaarung binden / hybridisieren. Die Anzahl der Target-Domänen (n) entspricht der Anzahl der zu markierenden Zielsequenzen.
   - Anker-Domäne-1 kann spezifisch an einen Bindungspartner binden. Dieser Bindungspartner ist vorzugsweise an einer festen Phase gebunden.
   - Die Anker-Domäne ist einheitlich und ist mit jeweils spezifischen Target-Domänen kombiniert.

In einer bevorzugten Ausführungsform der Anmeldung werden mehrere unterschiedliche Arten von Nuk-Makromolekülen bei enzymatischen Markierungsreaktion in einem Ansatz eingesetzt, die mindestens zwei Nuk-Makromoleküle aus der folgenden Gruppe einschließen:
- Nuk-Linker-(Target-Domäne-1)-(Anker-Domäne-1)
- Nuk-Linker-(Target-Domäne-n)-(Anker-Domäne-n)
- Nuk-Linker-(Target-Domäne-1)-(Anker-Domäne-n)
- Nuk-Linker-(Target-Domäne-n)-(Anker-Domäne-1)

In einer weiteren bevorzugten Ausführungsform der Anmeldung werden Nuk-Makromoleküle eingesetzt, die folgende Komponenten einschließen:
Nuk-Linker-(Target-Domäne-1)-(Signal-Domäne-1)
   - Nuk - ist eine Nuk-Komponente
   - Linker - ist eine Linker-Komponente
   - Target-Domäne-1 kann spezifisch an einen Sequenzabschnitt in der Zielsequenz unter Berücksichtigung der Watson-Crick Basenpaarung binden / hybridisieren. Beispiele für Target-Domäne-1 stellen Oligonucleotide. Die Struktur der Oligonukleotide kann dabei DNA, RNA, PNA, Morpholino oder LNA sein.
   - Signal-Domäne-1 kann durch ein spezifisches Signal identifiziert werden (z.B. ein Fluoreszenzsignal) oder vermittelt Bindung eines weiteren signalgebenden Partner (z.B. durch Oligonucleotide oder Haptene oder Biotin). Die entsprechenden signalgebende Bindungspartner, können beispielsweise markierte Oligonukleotide, markierte Antikörper oder markiertes Streptavaidin sein.

In einer bevorzugten Ausführungsform der Anmeldung werden mehrere unterschiedliche Arten von Nuk-Makromolekülen bei enzymatischen Markierungsreaktion eingesetzt, die folgende Komponenten (Domäne) einschließen:
Nuk-Linker-(Target-Domäne-n)-(Signal-Domäne-n)
   - Nuk - ist eine Nuk-Komponente
   - Linker - ist eine Linker-Komponente
   - Target-Domäne-n können spezifisch an den jeweiligen Sequenzabschnitt in der entsprechenden Zielsequenzen unter Berücksichtigung der Watson-Crick Basenpaarung binden / hybridisieren. Die Anzahl der Target-Domänen (n) entspricht der Anzahl der zu markierenden Zielsequenzen.
   - Signal-Domäne-n können druch eine jeweils spezifische Signal-Eigenschaft unterschieden werden. Die Anzahl der Signal-Domänen (n) entspricht der Anzahl der zu markierenden Zielsequenzen.
   - Die jeweiligen Signal-Domänen sind spezifisch für die jeweiligen Target-Domänen.

In einer bevorzugten Ausführungsform der Anmeldung werden mehrere unterschiedliche Arten von Nuk-Makromolekülen bei enzymatischen Markierungsreaktion eingesetzt, die folgende Komponenten (Domäne) einschließen:
Nuk-Linker-(Target-Domäne-1)-(Signal-Domäne-n)
   - Nuk - ist eine Nuk-Komponente
   - Linker - ist eine Linker-Komponente
   - Target-Domäne-1 kann spezifisch an einen Sequenzabschnitt in der Zielsequenz unter Berücksichtigung der Watson-Crick Basenpaarung binden / hybridisieren.
   - Signal-Domäne-n können durch eine jeweils spezifische Signal-Eigenschaft unterschieden werden. Die Anzahl der Signal-Domänen (n) kann beispielsweise zwischen 2 und 100 liegen.
   - Die Target-Domäne ist einheitlich und ist mit jeweils spezifischen Signal-Domänen kombiniert.

In einer bevorzugten Ausführungsform der Anmeldung werden mehrere unterschiedliche Arten von Nuk-Makromolekülen bei enzymatischen Markierungsreaktion eingesetzt, die folgende Komponenten (Domäne) einschließen:
Nuk-Linker-(Target-Domäne-n)-(Signal-Domäne-1)
   - Nuk - ist eine Nuk-Komponente
   - Linker - ist eine Linker-Komponente
   - Target-Domäne-n können spezifisch an den jeweiligen Sequenzabschnitt in der entsprechenden Zielsequenzen unter Berücksichtigung der Watson-Crick Basenpaarung binden / hybridisieren. Die Anzahl der Target-Domänen (n) entspricht der Anzahl der zu markierenden Zielsequenzen.
   - Signal-Domäne-1 kann durch ein spezifisches Signal identifiziert werden (z.B. ein Fluoreszenzsignal) oder vermittelt Bindung eines weiteren signalgebenden Partners.
   - Die Signal-Domäne ist einheitlich und ist mit jeweils spezifischen Target-Domänen kombiniert.

In einer bevorzugten Ausführungsform der Anmeldung werden mehrere unterschiedliche Arten von Nuk-Makromolekülen bei enzymatischen Markierungsreaktion in einem Ansatz eingesetzt, die mindestens zwei Nuk-Makromoleküle aus der folgenden Gruppe einschließen:
- Nuk-Linker-(Target-Domäne-1)-(Signal-Domäne-1)
- Nuk-Linker-(Target-Domäne-n)-(Signal-Domäne-n)
- Nuk-Linker-(Target-Domäne-1)-(Signal-Domäne-n)
- Nuk-Linker-(Target-Domäne-n)-(Signal-Domäne-1)

In einer weiteren bevorzugten Ausführungsform der Anmeldung werden Nuk-Makromoleküle eingesetzt, die folgende Komponenten (Domäne) einschließen:
Nuk-Linker-(Target-Domäne-1)-(Anker-Domane-1)-(Signal-Domäne-1)
   - Nuk - ist eine Nuk-Komponente
   - Linker - ist eine Linker-Komponente
   - Target-Domäne-1 kann spezifisch an einen Sequenzabschnitt in der Zielsequenz unter Berücksichtigung der Watson-Crick Basenpaarung binden / hybridisieren. Beispiele für Target-Domäne-1 stellen Oligonucleotide. Die Struktur der Oligonukleotide kann dabei DNA, RNA, PNA, Morpholino oder LNA sein.
   - Signal-Domäne-1 kann durch ein spezifisches Signal identifiziert werden (z.B. ein Fluoreszenzsignal) oder vermittelt Bindung eines weiteren signalgebenden Partner (z.B. durch Oligonucleotide oder Haptene oder Biotin). Die entsprechenden signalgebenden Bindungspartner, können beispielsweise markierte Oligonukleotide, markierte Antikörper oder markiertes Streptavaidin sein.
   - Anker-Domäne-1 kann spezifisch an einen Bindungspartner binden. Dieser Bindungspartner ist vorzugsweise an einer festen Phase gebunden. Beispiele für Anker-Doman-1 stellen Oligonucleotide (als DNA, RNA, PNA, Morpholino oder LNA) oder Haptene (z.B. Farbstoffe) oder Biotin. Die ensprechenden Bindungspartner, gebunden an die feste Phase, können beispielsweise Oligonukleotide, Antikörper oder Streptavaidin sein.

In einer bevorzugten Ausführungsform der Anmeldung werden mehrere unterschiedliche Arten von Nuk-Makromolekülen bei enzymatischen Markierungsreaktion eingesetzt, die folgende Komponenten (Domäne) einschließen:
Nuk-Linker-(Target-Domäne-n)-(Anker-Domane-n)-(Signal-Domäne-n)
   - Nuk - ist eine Nuk-Komponente
   - Linker - ist eine Linker-Komponente
   - Target-Domäne-n können spezifisch an den jeweiligen Sequenzabschnitt in der entsprechenden Zielsequenzen unter Berücksichtigung der Watson-Crick Basenpaarung binden / hybridisieren. Die Anzahl der Target-Domänen (n) entspricht der Anzahl der zu markierenden Zielsequenzen.
   - Anker-Domäne-n können spezifisch an den jeweiligen Bindungspartner binden. Diese Bindungspartner sind vorzugsweise an einer festen Phase gebunden. Die Anzahl der Anker-Domänen (n) entspricht der Anzahl der zu markierenden Zielsequenzen.
   - Signal-Domäne-n können haben eine jeweils eine spezifische Signal-Eigenschaft. Die Anzahl der Signal-Domänen (n) entspricht der Anzahl der zu markierenden Zielsequenzen.
   - Die jeweiligen Signal-Domänen und Anker-Domänen sind spezifisch für die jeweiligen Target-Domänen.

In einer Ausführungsform werden in einer Makierungsreaktion zusätzlich zu Nuk-Makromolekülen auch nicht markierte Nukleotide, beispielsweise dNTP (dATP, dCTP, dTTP, dGTP oder deren Analoga, wie z.B. 7-Deaza-dATP), NTP (ATP, GTP, CTP, UTP oder deren Analoga) zur Verlägerung des wachsenden Nukleinsäurestranges eingesetzt.

In einer weiteren Ausführungsform werden zusätzlich zu Nuk-Makromolekülen konventionell markierte Nukleotide, beispielsweise dUTP-Biotin, dUTP-Fluorescein, dCTP-Cy3 zur Markierugn des wachsenden Nukleinsäurestranges eingesetzt.

Die Nuk-Makromoleküle, die dNTPs und konventionell modifizierte Nukleotide können in unterschiedlichen Kombinationen und Kompositionen eingesetzt werden. Diese Kompositionen sind vorzugsweise Bestandteile von Kits. Nachfolgend sind einige von vorteilhaften Kompositionen vorgestellt.

### 1.5.8 Nukleotidkompositionen:

- Kompositonen, aus einer oder mehreren unterschiedlichen Arten von Nuk-Makromolekülen ohne weitere Nukleotide (z.B. ohne dNTP, NTP).
   Durch den Einbau von Nuk-Komponenten von Nuk-Makromolekülen am 3'-Ende des Primers gegenüber komplementären Basen in der Zielsequenz erfolgt eine Markierung von Nukleinsäureketten.
- Kompositonen, aus einer oder mehreren unterschiedlichen Arten von Nuk-Makromolekülen und einem Satz von Nukleotiden, der eine vollständige Synthese von Nukleinsäureketten erlauben, z.B. 4 x dNTP oder 4x NTP. Es können dabei sowohl natürliche Substrate für Polymerasen wie dNTP (dATP, dCTP, dGTP und dTTP) als auch deren Analoga verwendet werden. In Frage kommen dabei sowohl Basenmodifikationen, wie z.B. dITP, dUTP, als auch Zuckermodifikationen, wie z.B. ddNTP oder 3'-Amino-3'deoxy-NTP, oder auch Phosphatmodifikationen, wie z.B. alpha-thio-dNTPs oder Tetraphosphate. Durch Verwendung von dNTP kann eine Primerverlängerung stattfinden, so dass ein zur Zielsequenz komplementärer Strang synthetisiert werden kann. Gleichzeitig erfolgt der Einbau von Nuk-Komponenten von Nuk-Makromolekülen am 3'-Ende des wachsenden Stranges gegenüber komplementären Basen in der Zielsequenz, so dass eine Markierung von Nukleinsäureketten erfolgt. Durch Variationen der Konzentrationen und Verhältnissen zwischen Nuk-Makromolekülen und dNTPs kann das Ausmass und Spezifität der Modifizierung von Nukleinsäureketten beeinflusst werden, s.u.
- Kompositionen, aus einer oder mehreren unterschiedlichen Arten von Nuk-Makromolekülen und einem Satz von Nukleotiden, der nur eine unvollständige, limitierte Primerverlängerung erlaubt (z.B. nur ein oder zwei oder drei dNTPs oder NTPs). Es können dabei sowohl natürliche Substrate für Polymerasen wie dNTP (dATP, dCTP, dGTP und dTTP) als auch deren Analoga verwendet werden. In Frage kommen dabei sowohl Basenmodifikationen, wie z.B. dITP, dUTP, als auch Zuckermodifikationen, wie z.B. ddNTP oder 3'-Amino-3'deoxy-NTP, oder auch Phosphatmodifikationen, wie z.B. alpha-thio-dNTPs oder Tetraphosphate. Durch Verwendung von dNTP kann eine Primerverlängerung stattfinden, so dass ein zur Zielsequenz komplementärer Strang synthetisiert werden kann. Gleichzeitig erfolgt der Einbau von Nuc-Komponenten von Nuk-Makromolekülen am 3'-Ende des wachsenden Stranges gegenüber komplementären Basen in der Zielsequenz, so dass eine Markierung von Nukleinsäureketten stattfindet. Durch Variationen der Konzentrationen und Verhältnissen zwischen Nuk-Makromolekülen und dNTPs kann das Ausmass und Spezifität der Modifizierung von Nukleinsäureketten beeinflusst werden, s.u. Durch Limitierung der Zusammensetzung des dNTP-Satzes, kann die Länge des synthetisierten komplementären Stranges gesteuer werden.
- Kompositonen, aus einer oder mehreren unterschiedlichen Arten von Nuk-Makromolekülen, einem Satz von Nukleotiden, der eine vollständige Synthese erlauben, z.B. 4 x dNTP oder 4x NTP und einem oder mehreren weiteren konventionell modifizierten Nukleotiden. Es können dabei sowohl natürliche Substrate für Polymerasen wie dNTP (dATP, dCTP, dGTP und dTTP) als auch deren Analoga verwendet werden. In Frage kommen dabei sowohl Basenmodifikationen, wie z.B. dITP, dUTP, als auch Zuckermodifikationen, wie z.B. ddNTP oder 3'-Amino-3'deoxy-NTP, oder auch Phosphatmodifikationen, wie z.B. alpha-thio-dNTPs oder Tetraphosphate. Als modifizierte Nukleotide können beispielsweise konventionell modifizierte Nukleotide eingesetzt werden, die z.B. einen Farbstoff oder Fluoresezenzfarbstoff oder einen Affinitätsmarkert tragen, z.B. Cy3, Rhodamine, Alexa-Farbstoffe oder Atto-Farbstoffe oder Biotin oder Digoxigenin. Durch Verwendung von dNTP kann eine Primerverlängerung stattfinden, so dass ein zur Zielsequenz komplementärer Strang synthetisiert werden kann. Gleichzeitig erfolgt der Einbau von Nuc-Komponenten von Nuk-Makromolekülen am 3'-Ende des wachsenden Stranges gegenüber komplementären Basen in der Zielsequenz, so dass eine Markierung von Nukleinsäureketten stattfindet. Durch Variationen der Konzentrationen und Verhältnissen zwischen Nuk-Makromolekülen und dNTPs kann das Ausmass und Spezifität der Modifizierung von Nukleinsäureketten beeinflusst werden, s.u. Durch Verwendung von modifizierten Nukleotiden kann während der Synthese auch eine unspezifische Markierung von Nukleinsäureketten mit Farbstoffen, FLuoreszenzfarbstoffen oder Affinitätsmarker stattfinden.

In einer bevorzugten Ausführungsform werden Nuk-Makromoleküle alleine oder zusammen mit anderen Nukleotiden (s.o.) in einer wässrigen Puffer-Lösung eingesetzt. Nuk-Makromoleküle können als Bestandteile von Kits in Form einer Lösung (z.B. als eine konzentrierte Lösung) oder auch in trockener Form bereitgestellt werden. Die getrockneten Substanzen können unmittelbar vor dem Test mit einer wässrigen Lösung oder einer organischen Lösung, z.B. DMSO, für den Reaktionsansatz gelöst werden.

In einer weiteren bevorzugten Ausführungsform werden Nuk-Makromoleküle an einer festen Phase fixiert. Die Fixierung erfolgt dabei in Art und Weise, dass die Target-Domäne der Nuk-Makromoleküle ihre Fähigkeit zur spezifischen Hybridisierung an die Zielsequenzen, sowie die Fähigkeit als Substrate für Polymerasen nicht verlieren. In einer Ausführungsform erfolgt die Fixierung in einer Art und Weise, dass einzelne Arten von Nuk-Makromolekülen an einer festen Phase spezifisch identifiziert werden können. Beispielsweise kann diese Identifizierung durch eine räumliche Anordnung an der festen Phase erfolgen, ähnlich wie bei einem DNA Microarray.

Im Weiteren werden zunächt Ausführungsformen betrachtet, bei denen Nuk-Makromoleküle in einer wässrigen Puffer-Lösung vorliegen.
Die Verhältnisse in Konzentrationen zwischen Nuk-Makromolekülen und nicht markierten Nukleotiden (z.B. dNTP) können angepasst werden, so dass Nuk-Makromoleküle von den Polymerasen eingebaut werden. Vorteilhafte Ausführungsformen der Verfahren schließen folgende Verhältnisse und Bereiche zwischen diesen ein:
Verhältnisse in Konzentration von Nuk-Makromolekül zu nicht markierten Nukleotiden (z.B. dNTP) schließen beispielsweise folgende Bereiche ein:
   1:100.000.000 bis 1:10.000.000; 1:10.000.000 bis 1:1.000.000; 1:1.000.000 bis 1:100.000; 1:100.000 bis 1:10.000; 1:10.000 bis 1:1.000; 1:1.000 bis 1:100; 1:100 bis 1:10; 1:10 bis 1:1; 1:1 bis 10:1; 10:1 bis 100:1; 100:1 bis 1.000:1; 1.000:1 bis 10.000:1.

Durch Variationen in den Verhältnissen in Konzentrationen kann man steuern, welcher Anteil der Nukleinsäureketten in einem Ansatz markiert wird und welcher unmarkiert verbleibt, da anstatt von Nuk-Makromolekül ein nicht markiertes Nukleotid (z.B. dNTP) eingebaut wurde.

Nuk-Makromoleküle können in Abwesenheit von nicht markierten Nukleotiden (z.B. dNTP) derselben Art sowohl sequenzspezifisch als auch sequenzunspezifisch von einer Polymerase eingebaut werden. Die Zielsequenz-Spezifität wird durch Target-Domäne eines Nuk-Makromoleküls begünstigt. Die Anwesenheit der dNTP derselben Basen-Art wie die Basen-Art der Nuk-Komponente führt dazu, dass natürliche Nukleotide und Nuk-Komponente des Nuk-Makromoleküls um den Einbau konkurriert. Bei steigender Konzentration von dNTP wird der zielsequenz-unspezifischer Einbau von Nuk-Makromolekülen durch dNTP kompetitiv unterdrückt. Dies kann beispielseise durch Konzentrationen von 1 bis 100 µmol/l von natürlichen Nukleotiden erreicht werden. Bei noch höherer Konzentration von natürlichen Nukleotiden wird auch der sequenzspezifischer Einbau von Nuk-Makromolekülen unterdrückt, dies kann beispielsweise durch Konzeentationen von 100 µmol/l bis 100 mmol/l erreicht werden.

In absoluten Konzentrationen können die Konzentrationen von Nuk-Makromolekülen und nicht markierten Nukleotiden zwischen 10 pmol/l und 100 mmol/l liegen. Besonders bevorzug sind Bereichen zwischen 100 nmol/l und 1 mmol/l. Noch mehr bevorzug sind Konzentrationsbereiche zwischen 100 nmol/l und 100 µmol/l.

Die absoluten Konzentrationen von Nuk-Makromolekülen bei einer Reaktion liegen vorzugsweise in folgenden Bereichen: Konzentrationen für Nuk-Makromolekülen: 10 pmol/l bis 10 nmol/l, 10 nmol/l bis 100 nmol/l, 100 nmol/l bis 1 µmol/l, 1 µmol/l bis 10 µmol/l, 10 µmol/l bis 100 µmol/l, 100 µmol/l bis 1 mmol/l. Konzentrationen für unmarkierte Nukleotide: 10 nmol/l bis 100 nmol/l, 100 nmol/l bis 1 µmol/l, 1 µmol/l bis 10 µmol/l, 10 µmol/l bis 100 µmol/l, 100 µmol/l bis 1 mmol/l, höher als 1 mmol/l.

Da auch weitere natürliche Nukleotide oder modifizierte Nukleiotide (z.B. markiert mit einem Farbstoff oder einem Biotin) eingesetzt werden können, liegt deren Konzentration vorzugsweise in folgenden Bereichen: 10 nmol/l bis 100 nmol/l, 100 nmol/l bis 1 µmol/l, 1 µmol/l bis 10 µmol/l, 10 µmol/l bis 100 µmol/l, 100 µmol/l bis 1 mmol/l, 1 mmol/l bis 100 mmol/l. Im Einzelnen, sollten Titrierungsreihen für eine optimale Markierung durchgeführt werden.

Konzentrationen einzelner Nukleotidarten (Nuk-Makromoleküle, dNTP, konventionell markierter Nukleotide und weiterer Nuk-Makromoleküle) können in einem Reaktionsgemisch individuell angepasst werden. Bei Verwendung mehrere Nuk-Makromoleküle können auch deren Konzentrationen und Konzentrationsverhältnisse je nach Bedarf der Analyse variabel gestaltet werden.

In einer Ausführungsform wird eine Komposition von Nuk-Makromolekülen mit anderen Kompontenten des Systems (z.B. Primer und dNTPs) zusammengestellt, dass bei einer stattfindenen Markierungsreaktion Nuk-Makromoleküle möglichst vollständig aufgebraucht werden. Dabei liegt die Konzentration von Nuk-Makromolekülen in der Reaktionslösung beispielsweise zwischen 10 pmol/l bis 1 nmol/l, 1 nmol/l bis 10 nmol/l, 10 nmol/l bis 100 nmol/l, 100 nmol/l bis 300 nmol/l, , 300 nmol/l bis 1 µmol/l, 1 µmol/l bis 10 µmol/l.

Die Zugabe einzelner Reagenzien (Primer, Nukleotiden, Polymerase und gegebenenfalls auch weiterer Reagenzien) kann in einem Schritt erfolgen oder über mehrere einzelne Schritte verteilt werden. Beispielsweise können Nuk-Makromoleküle bereits von am Start der Markierungsreaktion im Ansatz bereitgestellt / enthaltend sein. Die einzelnen Reagenzien können auch in getrockneter oder Konzentrierter Form als eine Komposition vorgemischt bereitgestellt werden, beispielsweise können Nuk-Makromoleküle und dNTP in einem festen Verhältnis vorgesmischt werden. Durch Zugabe einer Lösung mit Zielsequenzen zu einem solchen bereitgestellten Gemisch erfolgt die Auflösung von Reaktionskomponenten in der Lösugn, so dass eine Markierungsreaktion stattfinden kann.

Die Nukleotid-Kompositionen mit Nuk-Makromolekülen sind vorzugsweise Bestandteile eines Kits.

### 1.5.9 Bindung von markierten Nukleinsäuren an die feste Phase und Detektion

In einer bevorzugten Ausführungsform der Erfindung folgt nach einer Markierungsreaktion von Zielsequenzen mit Nuk-Makromolekülen die Bindung von markierten Zielsequenzen an eine feste Phase. Anschließend erfolgt ein Nachweis der Bindung der Zielsequenzen an die feste Phase.

Durch den Einsatz von Nuk-Makromolekülen mit zielsequenz-spezifischen Kombinationen von Nuk-Kompontenten, Target-Domänen, Anker-Domänen und Signal-Domänen kann ein Nachweis und eine Differenzierung von markierten Zielsequenzen erreicht werden. Dieser Nachweis und Differenzierung kann durch Bindung an die feste Phase oder durch Bindung an unterschiedliche Positionen an der festen Phase oder weiterhin durch unterschiedliche Signaleigenschaften, z.B. Lichteigenschaften der Signal-Domäne, erreicht werden.

Spezifische Merkmale von Nuk-Makromolekülen, wie spezifisches Signal, spezifisches Bindungsverhalten mit der festen Phase, spezifische Bindung an die Zielsequenz, können mit spezifischen Merkamalen (Signalgebung, Bindung an die feste Phase, Bindung an die Zielsequenz) anderer Reaktionspartner kombiniert werden. Bespielsweise können Signal-Domänen bzw. Anker-Domänen an Primern (modifizierte Primer), Hybridisierungssonden, modifizierte Nukleotiden gekoppelt sein.

In einer bevorzugsten Ausführungsform werden in einem Assay Signal-Eigenschaften und Bindungseigenschaften an die feste Phase auf mehrere verschiedene Assay-Teilnehmer verteilt, beispielsweise auf Nuk-Makromoleküle, Primer, weitere Nukleotide, Hybridisierungssonden.

In einer Ausführungsform werden Nuk-Makromoleküle mit mindestens einer Anker-Domäne in Kombination mit einer Target-Domäne bevorzugt in Kombination mit mindestens einem markierten Primer verwendet, der signalgebende oder signalvermittelnde Eigenschaften hat (z.B. Primer ist mit einer Signal-Domäne gekoppelt). In einer weiteren Ausführungsform werden Nuk-Makromoleküle mit einer oder mehreren Anker-Domänen in Kombination mit einer Target-Domäne bevorzugt in Kombination mit markierten Nukleotiden verwendet, die signalgebende oder signalvermittelnde Eigenschaften haben. In einer weiteren Ausführungsform werden Nuk-Makromoleküle mit einer oder mehreren Anker-Domänen in Kombination mit einer Target-Domäne bevorzugt in Kombination mit mindestens einer markierten Hybridisierungssonde verwendet, die signalgebende oder signalvermittelnde Eigenschaften haben (z.B. Hybridiseirungssonde ist mit einer Signal-Domäne gekoppelt).

In einer Ausführungsform werden Nuk-Makromoleküle mit einer oder mehreren Signal-Domänen in Kombination mit einer Target-Domäne bevorzugt in Kombination mit mindestens einem modifizierten Primer verwendet, der an die feste Phase in spezifischer Art und Weise binden kann (z.B. Anker-Domäne ist an den Primer gekoppelt). In einer weiteren Ausführungsform werden Nuk-Makromoleküle mit einer oder mehreren Signal-Domänen in Kombination mit einer Target-Domäne bevorzugt in Kombination mit markierten Nukleotiden verwendet, die spezifisch an die feste Phase binden können, z.B. weiteren Nuk-Makromolekülen oder Biotin-tragenden konventionellen Nukleotiden. In einer weiteren Ausführungsform werden Nuk-Makromoleküle mit einer oder mehreren Signal-Domänen in Kombination mit einer Target-Domäne bevorzugt in Kombination mit mindestens einer markierten Hybridisierungssonde verwendet, die spezifisch an die feste Phase binden kann (z.B. Anker-Domäne ist an die Hybridisierungssonde gekoppelt).

In einer Ausführungsform werden Nuk-Makromoleküle mit einer oder mehreren Anker-Domänen (z.B. Anker-Domäne 1) in Kombination mit einer Target-Domäne bevorzugt in Kombination mit mindestens einem modifizierten Primer verwendet, der an die feste Phase in spezifischer Art und Weise binden kann (z.B. Anker-Domäne-2 ist an den Primer gekoppelt, wobei sich Anker-Domäne 1 am Nuk-Makromolekül von Anker-Domäne 2 am Primer in Bindungseigenschaften unterscheidet). In einer weiteren Ausführungsform werden Nuk-Makromoleküle mit einer oder mehreren Anker-Domänen (z.B. Anker-Domäne 1) in Kombination mit einer Target-Domäne bevorzugt in Kombination mit markierten Nukleotiden verwendet, die spezifisch an die feste Phase binden können, z.B. weiteren Nuk-Makromolekülen mit einer Anker-Domäne-2. In einer weiteren Ausführungsform werden Nuk-Makromoleküle mit einer oder mehreren Anker-Domänen (z.B. Anker-Domäne 1) in Kombination mit einer Target-Domäne bevorzugt in Kombination mit mindestens einer markierten Hybridisierungssonde verwendet, die spezifisch an die feste Phase binden kann (z.B. Anker-Domäne-2 ist an die Hybridisierungssonde gekoppelt). Die Anker-Domäne-1 und 2 unterscheiden sich in ihren Bindungseigenschaften.

In einer Ausführungsform werden Nuk-Makromoleküle mit mindestens einer Anker-Domäne aber ohne eine Target-Domäne bevorzugt in Kombination mit mindestens einem markierten Primer verwendet, der signalgebende oder signalvermittelnde Eigenschaften hat (z.B. Primer ist mit einer Signal-Domäne gekoppelt). In einer weiteren Ausführungsform werden Nuk-Makromoleküle mit einer oder mehreren Anker-Domänen aber ohne eine Target-Domäne bevorzugt in Kombination mit markierten Nukleotiden verwendet, die signalgebende oder signalvermittelnde Eigenschaften haben. In einer weiteren Ausführungsform werden Nuk-Makromoleküle mit einer oder mehreren Anker-Domänen aber ohne eine Target-Domäne bevorzugt in Kombination mit mindestens einer markierten Hybridisierungssonde verwendet, die signalgebende oder signalvermittelnde Eigenschaften haben (z.B. Hybridiseirungssonde ist mit einer Signal-Domäne gekoppelt).

In einer Ausführungsform werden Nuk-Makromoleküle mit einer oder mehreren Signal-Domänen aber ohne eine Target-Domäne bevorzugt in Kombination mit mindestens einem markierten Primer verwendet, der an die feste Phase in spezifischer Art und Weise binden kann (z.B. Anker-Domäne ist an den Primer gekoppelt). In einer weiteren Ausführungsform werden Nuk-Makromoleküle mit einer oder mehreren Signal-Domänen aber ohne eine Target-Domäne bevorzugt in Kombination mit markierten Nukleotiden verwendet, die spezifisch an die feste Phase binden können, z.B. weiteren Nuk-Makromolekülen. In einer weiteren Ausführungsform werden Nuk-Makromoleküle mit einer oder mehreren Signal-Domänen aber ohne eine Target-Domäne bevorzugt in Kombination mit mindestens einer markierten Hybridisierungssonde verwendet, die spezifisch an die feste Phase binden kann (z.B. Anker-Domäne ist an die Hybridisierungssonde gekoppelt).

In einer Ausführungsform werden Nuk-Makromoleküle mit einer oder mehreren Anker-Domänen (z.B. Anker-Domäne 1) aber ohne eine Target-Domäne bevorzugt in Kombination mit mindestens einem markierten Primer verwendet, der an die feste Phase in spezifischer Art und Weise binden kann (z.B. Anker-Domäne-2 ist an den Primer gekoppelt). In einer weiteren Ausführungsform werden Nuk-Makromoleküle mit einer oder mehreren Anker-Domänen (z.B. Anker-Domäne 1) aber ohne eine Target-Domäne bevorzugt in Kombination mit markierten Nukleotiden verwendet, die spezifisch an die feste Phase binden können, z.B. weiteren Nuk-Makromolekülen mit einer Anker-Domäne-2. In einer weiteren Ausführungsform werden Nuk-Makromoleküle mit einer oder mehreren Anker-Domänen (z.B. Anker-Domäne 1) aber ohne eine Target-Domäne bevorzugt in Kombination mit mindestens einer markierten Hybridisierungssonde verwendet, die spezifisch an die feste Phase binden kann (z.B. Anker-Domäne-2 ist an die Hybridisierungssonde gekoppelt).

In einer Ausführungsform stellen die bereitgestellte feste Phase und die Anker-Domäne eine spezifische Kombination zusammen, bei der die feste Phase und Anker-Domäne jeweils Komponenten einschließen, die zu einer spezifischen affinen Bindung fähig sind. Beispiele für solche spezifische Kombinationen sind bekannt. Bevorzugt sind Bindungen zwischen Antigen und Antikörper, Biotin-Streptavidin (oder Biotin-Avidin), Oligonukleotid-Oligonukleotid (als DNA, RNA, PNA, LNA usw.), aptamervermittelte Bindung, Zucker-Molekülen und Lektinen. Besonders bevorzugt sind die Biotin-Streptavidin, Antigen-Antikörper, Oligonukleotid-Oligonukleotid Bindung sowie aptamervermittelte Bindung, da durch die Variationen in Bindungspartnern (z.B. Sequenz von Oligonukleotdien) eine Vielfalt an Bindungsmöglichkeiten zustande kommen kann. Weitere Beispiele für Anker-Domäne von Nuk-Makromolekülen, modifizierten Primern, Hybridisierungssonden sind im Abschnitt "Definitionen und Begriffe" angeführt.
Die Bindungspartner für die Anker-Domänen sind vorzugsweise an einer festen Phase gebunden. Beispiele für solche feste Phasen stellen Kügelchen, oder Wells (z.B. einer Mikrotiterplatten), oder Reagenzgefäße, oder eine microfluidische Kammer, oder Streifen oder LFD ("lateral flow device") mit einem Reaktionsfeld. Weitere Beispiele für feste Phasen sind einem Fachmann bekannt.

Einige Beispiele für die Verfahren zur Markierung von Zielsequenzen und ihrer anschließenden Bindung an die feste Phase sind in Fig. 6 bis 15 vorgestellt.

Ein Beispiel für Markierungsverfahren einer Zielsequenz bzw. deren Äquivalente mit einer anschließenden Bindung an der festen Phase ist in Fig. 6 dargestellt. Es werden folgende Kompontenten eingesetzt (Fig. 6A): eine Art von Nuk-Makromolekülen (1-4 in Fig. 6) mit einer Target-Domäne und einer Anker-Domäne, einzelsträngige Zielsequenz (7 in Fig. 6), ein mit einer Signal-Domäne markierter Primer (6 und 9 in Fig. 6), eine DNA Polymerase, weitere Nukleotide, z.B. dNTPs. Diese Komponenten werden unter Bedingungen inkubiert, die es der Polymerase ermöglichen, dNTPs und Nuk-Makromoleküle in den wachsenden Strang einzubauen (Fig. 6B). Es wird eine feste Phase bereitgestellt (12 in Fig. 6), die einen Bindungspartner für die Anker-Domäne (11 in Fig. 6) einschließt. Die Markierungsreaktion wird vorzugsweise dermassen gestaltet, dass Nuk-Makromoleküle möglichst vollständig eingebaut werden. Falls ein Überschuß an Nuk-Makromolekülen verwendet werden sollte, können markierte Nukleinsäureketten von überschüssigen freien Nuk-Makromolekülen mit einer bekannten Methode gereinigt werden. Die markierten Zielsequenzen bzw. deren Äquivalente werden mit der festen Phase unter Bedingungen inkubiert, die eine spezifische Bindung der Anker-Domäne an den immobilisierten Bindungspartner zulassen. Die markierte Zielsequenz bindet an die feste Phase über die Anker-Domäne des eingebauten Nuk-Makromoleküls (13 in Fig. 6). Die Nachweisreaktion erfolgt mittels Signal-Domäne des Primers.

Ein weiteres Beispiel für Markierungsverfahren einer Zielsequenz bzw. deren Äquivalente mit einer anschließenden Bindung an der festen Phase ist in Fig. 7 dargestellt. Es werden folgende Kompontenten eingesetzt: eine Art von Nuk-Makromolekülen (1-4 in Fig. 7A, 7B) mit einer Target-Domäne, einer Anker-Domäne (4a in Fig. 7A) und einem Antagonist der Anker-Domäne (4b in Fig. 7B). Weiterhin werden einzelsträngige Zielsequenz, ein mit einer Signal-Domäne markierter Primer, eine DNA Polymerase, weitere Nukleotide, z.B. dNTPs, eingesetzt (Fig. 7B). Durch die Bindung der Target-Domäne an die Zielsequenz werden Anker-Domäne und ihr Antagonist separiert. Nach dem Einbau der Nuk-Komponente in den wachsenden Strang wird dieser Zustand verfestigt. Vorzugsweise werden bei dieser Ausführungsform des Verfahrens Polymerasen eingesetzt, die keine oder nur geringe Exonuklease-Aktivität und keine oder sehr geringe "strand-displacement"-Aktivität vorweisen. In einer weiteren Ausführungsform wird eine Nuk-Komponente verwendet, die zu einer Termination in der Synthese führt, z.B. eine Nuk-Komponente, die ddNTP einschließt (z.B. ddUTP der ddCTP). Diese Komponenten werden unter Bedingungen inkubiert, die es der Polymerase ermöglichen, dNTPs und Nuk-Makromolekül in den wachsenden Strang einzubauen (Fig. 7C). Es wird eine feste Phase bereitgestellt, die einen Bindungspartner für die Anker-Domäne einschließt. Nach der Einbaureaktion erfolgt eine Inkubation der markierten Zielsequenzen bzw. deren Äquivalente mit der festen Phase unter Bedingungen, die eine spezifische Bindung der Anker-Domäne von eingebauten Nuk-Makromolekülen an den immobilisierten Bindungspartner zulassen. Vorzugsweise bleiben die Anker-Domänen der nicht eingebauten Nuk-Makromoleküle unter diesen Bedingungen von ihren Antagonisten blockiert. Die markierte Zielsequenz bindet an die feste Phase über die Anker-Domäne des eingebauten Nuk-Makromoleküls. Die Nachweisreaktion erfolgt mittels Signal-Domäne des Primers. Die nicht eingebauten, freien Nuk-Makromoleküle interferieren nicht mit der Bindung von markierten Zielsequenzen, da ihre Anker-Domäne durch die Antagonisten blockiert sind.

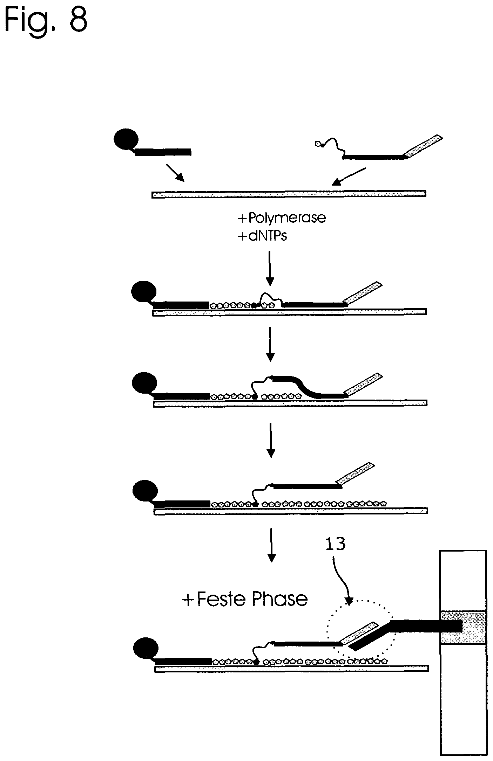

Ein weiteres Beispiel für Markierungsverfahren einer Zielsequenz bzw. deren Äquivalente mit einer anschließenden Bindung an der festen Phase ist in Fig. 8 dargestellt. Es werden folgende Kompontenten eingesetzt: eine Art von Nuk-Makromolekülen mit einer Target-Domäne und einer Anker-Domäne, einzelsträngige Zielsequenz, ein mit einer Signal-Domäne markierter Primer, eine DNA Polymerase, weitere Nukleotide, z.B. dNTPs. Diese Komponenten werden unter Bedingungen inkubiert, die es der Polymerase ermöglichen, dNTPs und Nuk-Makromoleküle in den wachsenden Strang einzubauen. Es wird Polymerase verwendet, die eine "strand-displacement"-Aktivität aufweist. Es wird eine feste Phase bereitgestellt, die einen Bindungspartner für die Anker-Domäne einschließt. Die Markierungsreaktion wird vorzugsweise dermassen gestaltet, dass Nuk-Makromoleküle möglichst vollständig eingebaut werden. Falls ein Überschuß an Nuk-Makromolekülen verwendet werden sollte, können markierte Nukleinsäureketten von Nuk-Makromolekülen mit einer bekannten Methode gereinigt werden. Die markierten Zielsequenzen bzw. deren Äquivalente werden mit der festen Phase unter Bedingungen inkubiert, die eine spezifische Bindung der Anker-Domäne an den immobilisierten Bindungspartner zulassen. Die markierte Zielsequenz bindet an die feste Phase über die Anker-Domäne des eingebauten Nuk-Makromoleküls. Da die verwendete Polymerase in der Lage ist, die Target-Domäne der Nuk-Makromoleküle zu verdrängen, können mehrere Nuk-Makromoleküle in denselben wachsenden Strang eingebaut werden. Die Nachweisreaktion erfolgt mittels Signal-Domäne des Primers.

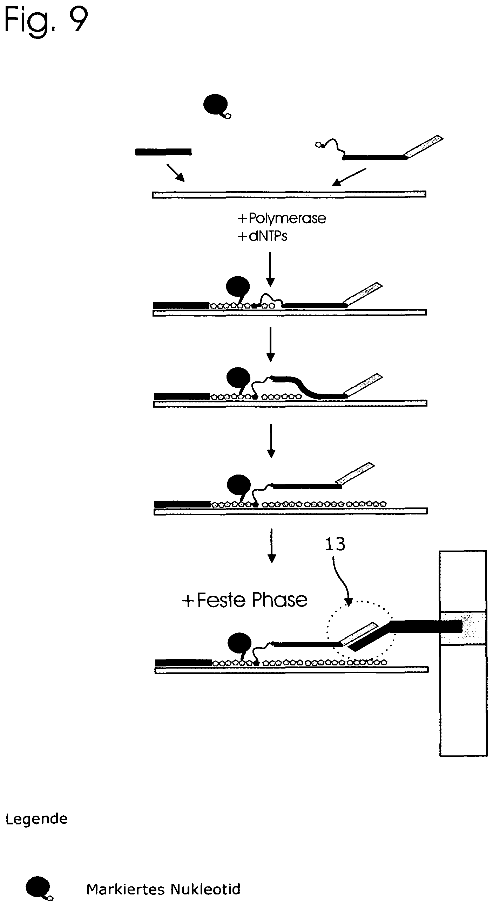

Ein weiteres Beispiel für Markierungsverfahren einer Zielsequenz bzw. deren Äquivalente mit einer anschließenden Bindung an der festen Phase ist in Fig. 9 dargestellt. Es werden folgende Kompontenten eingesetzt: eine Art von Nuk-Makromolekülen mit einer Target-Domäne und einer Anker-Domäne, einzelsträngige Zielsequenz, ein Primer, eine DNA Polymerase, weitere Nukleotide, z.B. dNTPs und eine Art von markierten Nukleotiden, z.B. Nuk-Makromoleküle mit einer Signal-Domäne oder konventionell markierte Nukleotide, z.B. dUTP-16-Biotin oder Cy3-dCTP. Diese Komponenten werden unter Bedingungen inkubiert, die es der Polymerase ermöglichen, dNTPs und Nuk-Makromoleküle in den wachsenden Strang einzubauen. Es wird Polymerase verwendet, die eine "strand-displacement"-Aktivität aufweist. Es wird eine feste Phase bereitgestellt, die einen Bindungspartner für die Anker-Domäne einschließt. Die Markierungsreaktion wird vorzugsweise dermassen gestaltet, dass Nuk-Makromoleküle möglichst vollständig eingebaut werden. Falls ein Überschuß an Nuk-Makromolekülen verwendet werden sollte, können markierte Nukleinsäureketten von Nuk-Makromolekülen mit einer bekannten Methode gereinigt werden. Die markierten Zielsequenzen bzw. deren Äquivalente werden mit der festen Phase unter Bedingungen inkubiert, die eine spezifische Bindung der Anker-Domäne an den immobilisierten Bindungspartner zulassen. Die markierte Zielsequenz bindet an die feste Phase über die Anker-Domäne des eingebauten Nuk-Makromoleküls. Da die verwendete Polymerase in der Lage ist, die Target-Domäne der Nuk-Makromoleküle zu verdrängen, können mehrere Nuk-Makromoleküle und mehrere mit der Signal-Domäne markierten Nukleotide in denselben wachsenden Strang eingebaut werden.

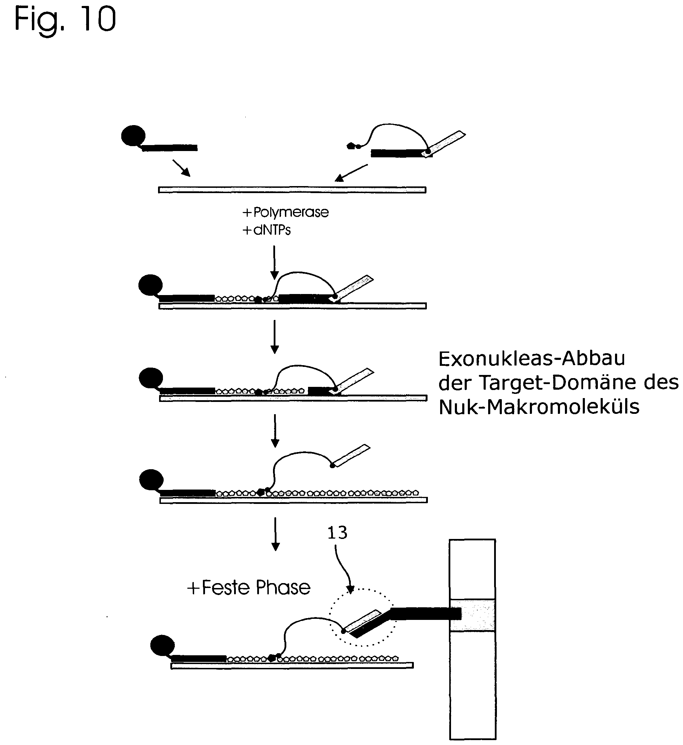

Ein weiteres Beispiel für Markierungsverfahren einer Zielsequenz bzw. deren Äquivalente mit einer anschließenden Bindung an der festen Phase ist in Fig. 10 dargestellt. Es werden folgende Kompontenten eingesetzt: eine Art von Nuk-Makromolekülen mit einer Target-Domäne und einer Anker-Domäne, einzelsträngige Zielsequenz, ein mit einer Signal-Domäne markierter Primer, eine DNA Polymerase mit 5'-3'-Exonuklease-Aktivität, weitere Nukleotide, z.B. dNTPs. Die Linker-Kompontente ist nicht an die Target-Domäne gekoppelt, sondern an einen andren Teil des Markers, z.B. an die Anker-Domäne des Markers gekoppelt. Diese Komponenten werden unter Bedingungen inkubiert, die es der Polymerase ermöglichen, dNTPs und Nuk-Makromoleküle in den wachsenden Strang einzubauen. Dabei kommt es während der Reaktion zu einem Abbau der Target-Domäne der an die Zielsequenz gebundenen Nuk-Makromolekülenan während die Reaktion fortschreitet (Fig. 10). Es wird eine feste Phase bereitgestellt, die einen Bindungspartner für die Anker-Domäne einschließt. Die Markierungsreaktion wird vorzugsweise dermassen gestaltet, dass Nuk-Makromoleküle möglichst vollständig eingebaut werden. Falls ein Überschuß an Nuk-Makromolekülen verwendet werden sollte, können markierte Nukleinsäureketten von Nuk-Makromolekülen mit einer bekannten Methode gereinigt werden. Die markierten Zielsequenzen bzw. deren Äquivalente werden mit der festen Phase unter Bedingungen inkubiert, die eine spezifische Bindung der Anker-Domäne an den immobilisierten Bindungspartner zulassen. Die markierte Zielsequenz bindet an die feste Phase über die Anker-Domäne des eingebauten Nuk-Makromoleküls. Da die verwendete Polymerase in der Lage ist, die Target-Domäne der Nuk-Makromoleküle abzubauen, können mehrere Nuk-Makromoleküle in denselben wachsenden Strang eingebaut werden. Die Nachweisreaktion erfolgt mittels Signal-Domäne des Primers.

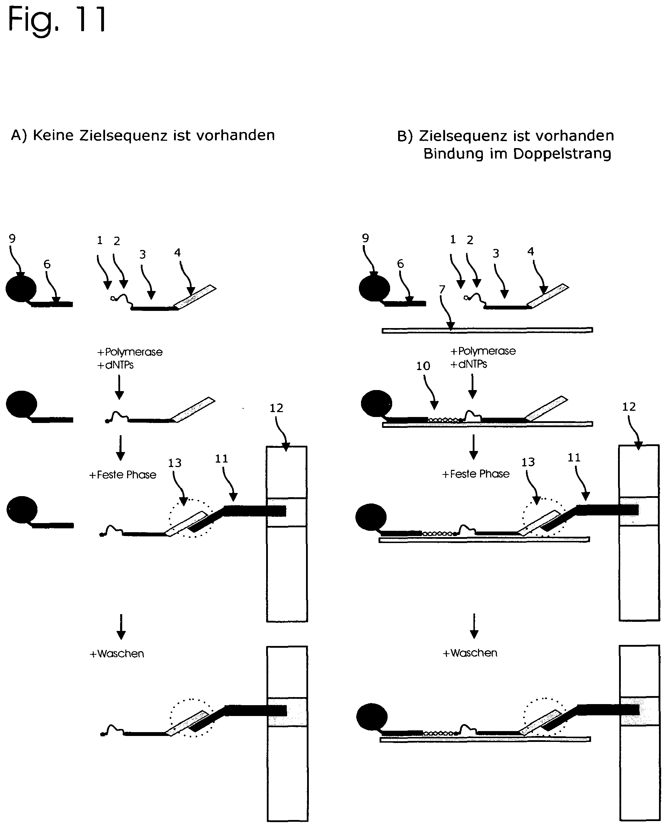

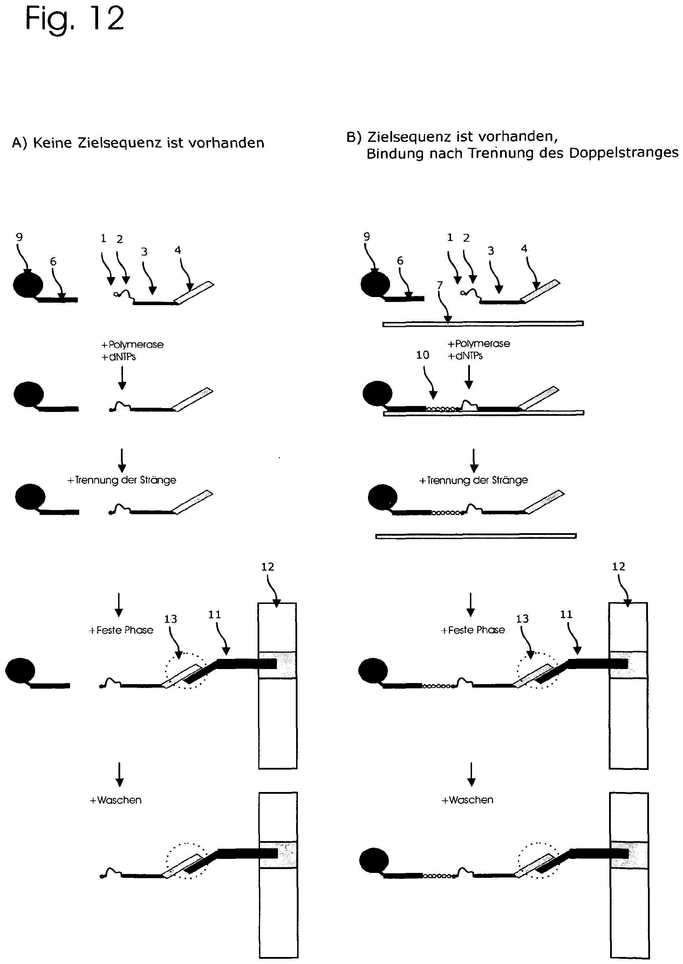

Die Figuren 11 und 12 zeigen einen möglichen Assay-Aufbau für die Kontrolle einer Lösung auf Präsenz einer Zielsequenz. In Verfahren wird in Figur 11 dargestellt, bei dem Zielsequenz mit dem frisch synthetisierten komplementären Strang direkt an die feste Phase gebunden wird. In Fig. 12 wird ein Verfahren dargestellt, bei dem erst eine Trennung von Doppelsträngen stattfindet. Eine Strangseparation kann eine weitere Steigerung in der Spezifität der Analyse bringen.

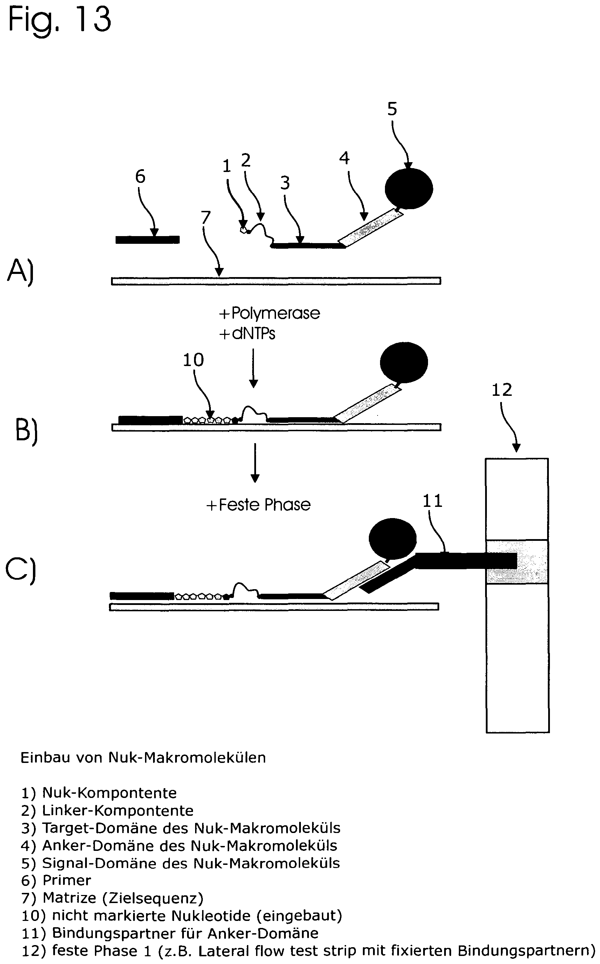

Ein weiteres Beispiel für Markierungsverfahren einer Zielsequenz bzw. deren Äquivalente mit einer anschließenden Bindung an der festen Phase ist in Fig. 13 dargestellt. Es werden folgende Kompontenten eingesetzt: eine Art von Nuk-Makromolekülen mit einer Target-Domäne, einer Anker-Domäne und einer Signal-Domäne, einzelsträngige Zielsequenz, ein Primer, eine DNA Polymerase, weitere Nukleotide, z.B. dNTPs. Diese Komponenten werden unter Bedingungen inkubiert, die es der Polymerase ermöglichen, dNTPs und Nuk-Makromoleküle in den wachsenden Strang einzubauen (Fig. 13B). Es wird eine feste Phase bereitgestellt, die einen Bindungspartner für die Anker-Domäne einschließt. Die Markierungsreaktion wird vorzugsweise dermassen gestaltet, dass Nuk-Makromoleküle möglichst vollständig eingebaut werden. Falls ein Überschuß an Nuk-Makromolekülen verwendet werden sollte, können markierte Nukleinsäureketten von Nuk-Makromolekülen mit einer bekannten Methode gereinigt werden. Die markierten Zielsequenzen bzw. deren Äquivalente werden mit der festen Phase unter Bedingungen inkubiert, die eine spezifische Bindung der Anker-Domäne an den immobilisierten Bindungspartner zulassen. Die markierte Zielsequenz bindet an die feste Phase über die Anker-Domäne des eingebauten Nuk-Makromoleküls. Die Nachweisreaktion erfolgt mittels Signal-Domäne des Nuk-Makromoleküls.

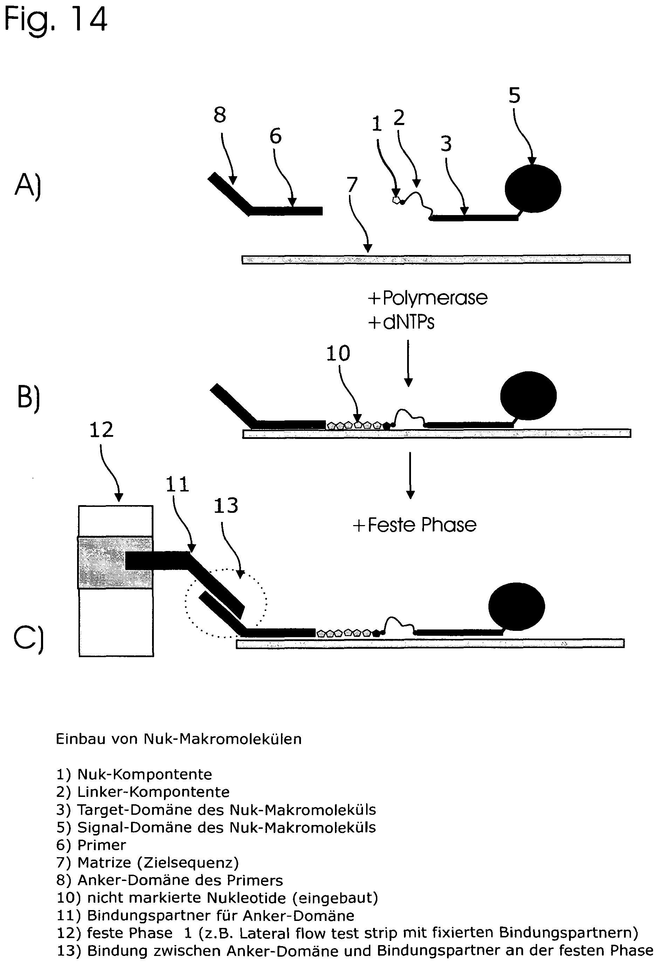

Ein weiteres Beispiel für Markierungsverfahren einer Zielsequenz bzw. deren Äquivalente mit einer anschließenden Bindung an die festen Phase ist in Fig. 14 dargestellt. Es werden folgende Kompontenten eingesetzt: eine Art von Nuk-Makromolekülen mit einer Target-Domäne und einer Signal-Domäne, einzelsträngige Zielsequenz, ein Primer mit einer Anker-Domäne, eine DNA Polymerase, weitere Nukleotide, z.B. dNTPs. Diese Komponenten werden unter Bedingungen inkubiert, die es der Polymerase ermöglichen, dNTPs und Nuk-Makromoleküle in den wachsenden Strang einzubauen (Fig. 14B). Es wird eine feste Phase bereitgestellt, die einen Bindungspartner für die Anker-Domäne einschließt. Die Markierungsreaktion wird vorzugsweise dermassen gestaltet, dass Nuk-Makromoleküle möglichst vollständig eingebaut werden. Falls ein Überschuß an Nuk-Makromolekülen verwendet werden sollte, können markierte Nukleinsäureketten von Nuk-Makromolekülen und markierten Primern mit einer bekannten Methode gereinigt werden. Die markierten Zielsequenzen bzw. deren Äquivalente werden mit der festen Phase unter Bedingungen inkubiert, die eine spezifische Bindung der Anker-Domäne an den immobilisierten Bindungspartner zulassen. Die markierte Zielsequenz bindet an die feste Phase über die Anker-Domäne des Primers. Die Nachweisreaktion erfolgt mittels Signal-Domäne des Nuk-Makromoleküls.

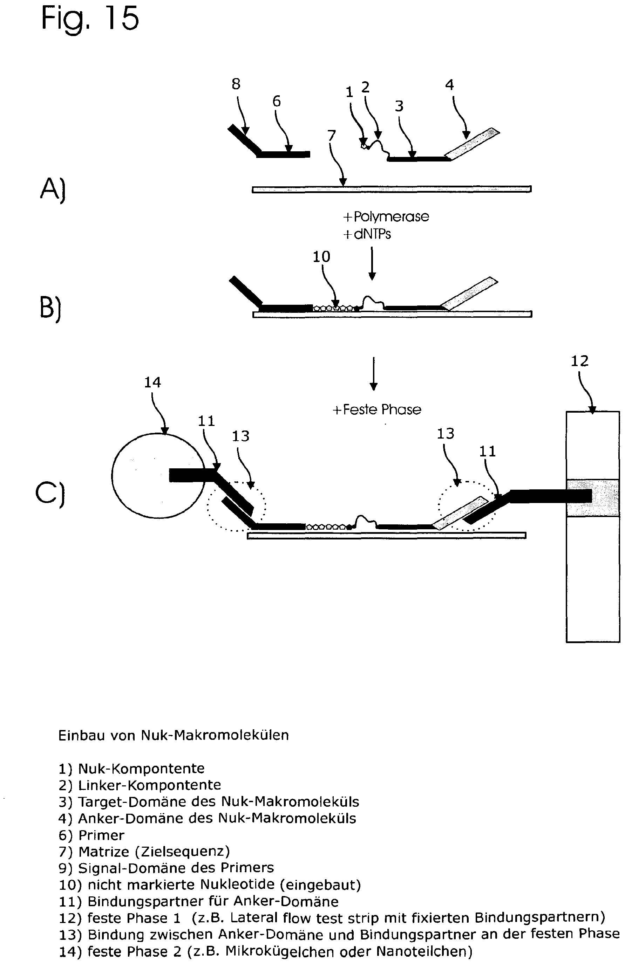

Ein weiteres Beispiel für Markierungsverfahren einer Zielsequenz bzw. deren Äquivalente mit einer anschließenden Bindung an der festen Phase ist in Fig. 15 dargestellt. Es werden folgende Kompontenten eingesetzt: eine Art von Nuk-Makromolekülen mit einer Target-Domäne und einer Anker-Domäne-1, einzelsträngige Zielsequenz, ein Primer mit einer Anker-Domäne-2, eine DNA Polymerase, weitere Nukleotide, z.B. dNTPs. Diese Komponenten werden unter Bedingungen inkubiert, die es der Polymerase ermöglichen, dNTPs und Nuk-Makromoleküle in den wachsenden Strang einzubauen (Fig. 15B). Es wird eine feste Phase-1 bereitgestellt, die einen Bindungspartner für die Anker-Domäne-1 des Nuk-Makromoleküls einschließt, sowie eine feste Phase-2, die einen Bindungspartner für die Anker-Domäne-2 des Primers einschließt. Die beiden festen Phasen sind beispielsweise als Teilchen in einer wässrigen Suspension bereitgestellt. Die Markierungsreaktion wird vorzugsweise dermassen gestaltet, dass Nuk-Makromoleküle möglichst vollständig eingebaut werden. Falls ein Überschuß an Nuk-Makromolekülen verwendet werden sollte, können markierte Nukleinsäureketten von Nuk-Makromolekülen und markierten Primern mit einer bekannten Methode gereinigt werden. Die markierten Zielsequenzen bzw. deren Äquivalente werden mit beiden festen Phasen unter Bedingungen inkubiert, die spezifische Bindungen von beiden Anker-Domänen an den immobilisierten Bindungspartner zulassen. Die markierte Zielsequenz bindet an beide Phase über die Anker-Domänen. Die Nachweisreaktion kommt durch optische Wahrnehmung der Vernetzung der beiden festen Phasen, z.B. als Agglutination sichtbar.

In einer weiteren Ausführungsform der Erfindung sind mehrere Bindungspartner in einer räumlichen Anordung fixiert, so dass eine Zuordnung eines bestimmen Bindungsparner zu einer bestimmen Position auf der festen Phase erfolgen kann. Dabei können einzelne Komponenten einer festen Phase zusammengefasst werden zu einem Array. Beispiele dafür stellen Mikrotiter-Platten oder deren Analoga, Bead-Arrays. Beispiele für solche feste Phasen stellen Microarray, Bead-arrays, Western-Blot-Streifen, "Lateral Flow Devices", Membran-Array mit mehreren Reaktionsfeldern. Einige Beispiele für feste Phase mit einem oder mehreren Bindungspartnern sind im Abschnitt "Begriffe und Definitionen" angegeben und sind einem Fachmann bekannt.

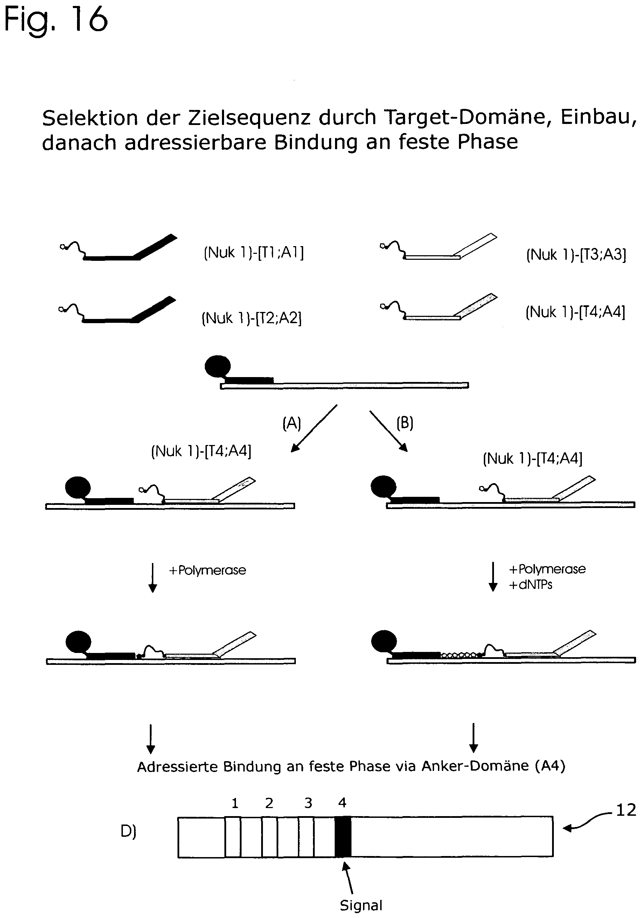

Durch die Bindung von markierten Zielsequenzen mit jeweils zielsequenzspezifischen Anker-Domänen an eine feste Phase mit räumlich angeordneten Bindungspartnern für die jeweiligen Anker-Domänen, kann auch eine räumliche Zuordnung der markierten Nukleinsäureketten erfolgen (Fig. 16, 17, 19).

Die an die feste Phase gebundenen mit Nuk-Makromolekülen markierten Zielsequenzen können mit einer der bekannten Nachweismethode detektiert werden.

Es wird ein weiteres Beispiel für Markierungsverfahren einer Zielsequenz bzw. deren Äquivalente mit einer anschließenden Bindung an die feste Phase vorgestellt. Es werden folgende Kompontenten eingesetzt: eine Art von Nuk-Makromolekülen mit einer Target-Domäne und einer Signal-Domäne, einzelsträngige Zielsequenz, ein Primer, eine DNA Polymerase, weitere Nukleotide, z.B. dNTPs. Diese Komponenten werden unter Bedingungen inkubiert, die es der Polymerase ermöglichen, dNTPs und Nuk-Makromoleküle in den wachsenden Strang einzubauen. Es wird eine feste Phase bereitgestellt, welche mindestens eine Nukleinsäuekette (z.B. mindestens ein Oligonukleotid), komplementär zu der Zielsequenz bzw. zu deren Äquivalenten einschließt. Diese Nukleinsäureketten sind vorzugsweise an der festen Phase immobilisiert. Falls feste Phase mehrere komplementäre Nukleinsäureketten einschließt, sind sie in einer räumlichen Anordnugn angeordnet, d.h. bilden einen adressierbaren Array. Einem Fachmann sind solche feste Phasen bekannt, z.B. DNA Microarray. Die Markierungsreaktion wird vorzugsweise dermassen gestaltet, dass Nuk-Makromoleküle möglichst vollständig eingebaut werden. Falls ein Überschuß an Nuk-Makromolekülen verwendet werden sollte, können markierte Nukleinsäureketten von Nuk-Makromolekülen und Primern bei Bedarf mit einer bekannten Methode gereinigt werden. Die markierten Zielsequenzen bzw. deren Äquivalente werden mit der festen Phase unter Bedingungen inkubiert, die eine spezifische Bindung der markierten Zielsequenz bzw. deren Äquivalenten an die immobilisierten Nukleinsäuereketten zulassen. Die markierte Zielsequenz bindet an die feste Phase direkt, über die Ausbildung von doppelsträngen mit komplementären immobilisierten Nukleinsäureketten. Die Nachweisreaktion erfolgt mittels Signal-Domäne des eingebauten Nuk-Makromoleküls.

Nachfolgend werden Beispiele von Kombinationen der festen Phase und Anker-Domänen betrachtet. Die Anker-Domänen können als Bestandteile von Nuk-Makromolekülen, von modifizierten Primer, von Hybridisierungssonden auftreten.

### 1.5.9.1 Verwendung von Nuk-Makromolekülen mit einer einheitlichen Anker-Domäne.

Es werden Nuk-Makromoleküle für eine Markierungsreaktion bereitgestellt, die mindestens eine einheitliche Anker-Domäne einschließen. Es wird eine feste Phase mt einem Bindungspartner bereitgestellt, die in der Lage ist, diese einheitliche Anker-Domäne spezifisch zu binden.

Bei Verwendung von Nuk-Makromolekülen mit nur einer Art von Anker-Domänen erfolgt die Bindung von markierten Zielsequenzen an die feste Phase über einen Bindungspartner, fixiert an der festen Phase. Die Nuk-Kompontente und Target-Sequenzen können einheitlich oder unterschiedlich sein.

Beispielsweise wird nur eine Art von Nuk-Makromolekülen in der Reaktion eingesetzt. Beim Einbau von diesem Nuk-Makromolekül in die Zielsequenz oder deren Äquivalent und einer anschließenden Inkubation mit der festen Phase kommt es zu Bindung an die feste Phase.

Beispielsweise können mehrere Nuk-Makromoleküle mit unterschiedlichen Target-Domänen und einer einheitlichen Anker-Domänen bei der Suche nach einer Gruppe von Zielsequenzen verwendet werden. Falls eine von den gesuchten Zielsequenzen im Reaktionsansatz vorkommt, kommt es zum Einbau eines Nuk-Makromoleküls in den wachsenden Strand und bei einer anschließendern Inkubation mit fester Phase zu Bindung der markierten Zielsequenz oder deren Äquivalenten an die feste Phase.

Der Nachweis bzw. die Detektion der Bindung der Zielsequenz an die feste Phase kann beispielsweise über eine Signal-Domäne eines eingebauten Nuk-Makromoleküls oder eines verwendeten Primers oder einer Hybridisierungssonde erfolgen.

### 1.5.9.2 Verwendung von Nuk-Makromolekülen mit unterschiedlichen Anker-Domänen.

Es werden Nuk-Makromoleküle mit unterschiedlichen Anker-Domänen in der Markierungsreaktion eingesetzt. Es wird eine feste Phase mt mehreren Bindungspartnern bereitgestellt, die in der Lage ist, diese unterschiedlichen Anker-Domänen jeweils spezifisch zu binden. Vorzugsweise werden Bindungspartner in einer bestimmten räumlichen Anordnung fixiert. Durch die Bindung von markierten Zielsequenzen an einer solche feste Phase, erfolgt auch eine Verteilung markierten Nukleinsäuren anhand eingebauten spezifischen Anker-Domänen und Bindungspartner auf der festen Phase. Nach einer anschließenden Detektion anhand des entstandenen Signalmuster läßt sich feststellen, welche Zielsequenzen vorhanden waren. Dadurch können mehrere Parameter (z.B. Präsenz von unterschiedlichen Zielsequenzen) festgestellt werden.

Der Nachweis bzw. die Detektion der Bindung der Zielsequenz an die feste Phase kann beispielsweise über eine Signal-Domäne eines eingebauten Nuk-Makromoleküls oder eines verwendeten Primers oder einer Hybridisierungssonde erfolgen.

**1.5.9.2.1** In einer Ausführungsform können mehrere verschiedene Nuk-Makromoleküle mit unterschiedlichen spezifischen Kombinationen von Target-Anker-Domänen zur Markierung verwendet werden. Die Nuk-Kompontenten, sowie Signal-Domäne dieser Nuk-Makromoleküle können einheitlich oder unterschiedlich sein. In einer Ausführungsform werden Nuk-Makromoleküle verwendet, die Target-, Anker- und Signal-Domäne einschließen. In einer weiteren Ausführungsform werden Nuk-Makromoleküle verwendet, die Target- und Anker-Domäne einschließen.

Durch spezifische Kombination von Target-Domänen und Anker-Domänen innerhalb eines Nuk-Makromoleüls kann eine Zuordnung von Anker-Domänen zu bestimmten Zielsequenzen erreicht werden. Nach dem Einbau von Nuk-Komponente in den wachsenden Strang durch die Polymerase ist auch die Anker-Domäne zielsequenzspezifisch gebunden.

Beispielsweise, es soll nach mehreren Zielsequenzen gesucht werden. Die Target-Domänen [T] einzelner Arten von Nuk-Makromolekülen sind komplementär zu den gesuchten Zielsequenzen. Die jeweiligen Anker-Domäne von Nuk-Makromolekülen sind spezifisch für jeweilige die Zielsequenzen ausgewählt (d.h. die Anker-Domäne [A] sind zielsequenzzugeordnet, z.B. Nuk1-[T1;A1], Nuk1-[T2;A2], Nuk1-[Tn;An] usw.). Durch die Markierung erhälrt jede einzelne Zielsequenz eine für sie spezifische Anker-Domäne. Nach der Markierungsreaktion können markierte Zielsequenzen an die feste Phase spezifisch über die Anker-Domänen binden. Nach dem Detektionschritt läßt sich feststellen, welche von den Zielsequenzen an welche Position an der festen Phase gebunden hat.

**1.5.9.2.2** In einer anderen Ausführungsform können mehrere Nuk-Makromolküle mit unterschiedlichen spezifischen Kombinationen Nuk-Komponente - Anker-Domäne zur Markierung verwendet werden. Dabei können die Target- bzw. Signal-Domänen eiheitlich oder auch unterschiedlich oder gar abwesend sein.

Vorzugsweise werden bei einer solchen Ausführungsform mindestens vier unterschiedliche spezifische Anker-Domäne verwendet, jeweils für eine spezifische Base der Nuk-Kompontente, beispielsweise dATP wird mit der Anker-Domäne 1, dCTP wird mit Anker-Domäne 2, dGTP mit Anker-Domäne 3 und dUTP mit der Anker-Domäne 4 kombiniert.

### 1.5.9.3 Bindung an die feste Phase durch einen sequenzspefischen modifizierten Primer

In einer Ausführungsform werden in die Markierungsreaktion einer Zielsequenz mindestens eine Polymerase, mindestens ein Primer, der eine Anker-Domäne einschließt, zusammen mit mindestens einer Art von Nuk-Makromoleküle, die mindestens eine Target-Domane und mindestens eine Signal-Domäne einschließen, sowie wahlweise weitere Nukleotide. Nach der Bindung der markierten Zielsequenz an die feste Phase über die Anker-Domäne des Primers kann diese Zielsequenz durch Signal von eingebauten Nuk-Makromolekülen detektiiert werden.

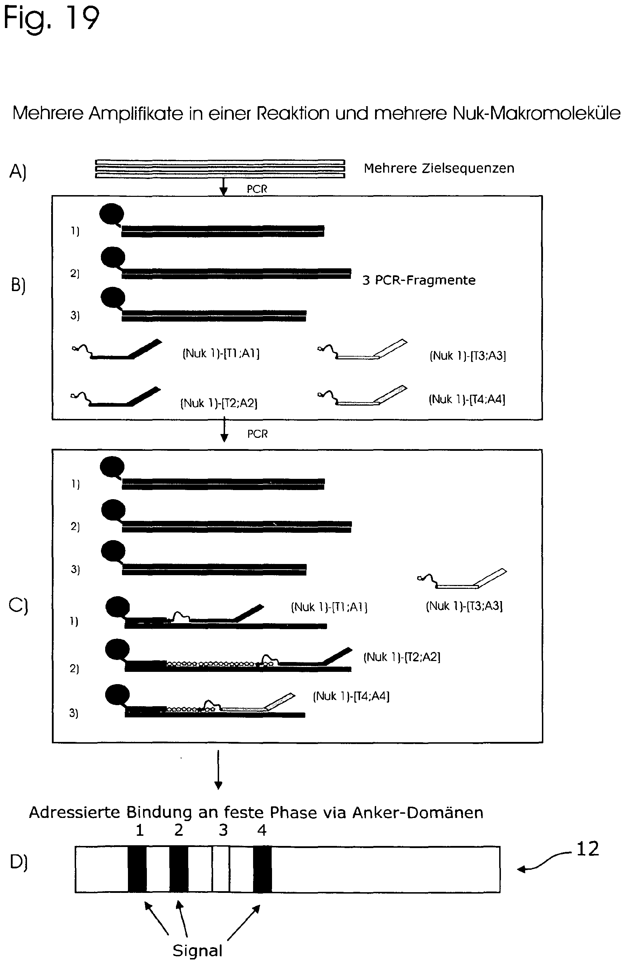

In einer weiteren Ausführungsform werden in die Markierungsreaktion Primer eingesetzt, die eine Anker-Domäne-1 einschließen, zusammen mit mindestens einer Art von Nuk-Makromoleküle, die mindestens eine Target-Domane und mindestens eine Anker-Domäne-2 einschließen. Die Anker-Domäne von Primer unterscheide sich von der Anker-Domäne der Nuk-Makromoleküle. Nach der Bindung der markierten Zielsequenz an die feste Phase über die Anker-Domäne des Primers kann die zweite feste Phase an die Anker-Domäne des Nuk-Makromoleküls binden. Bei Verwendung von Mikroteilchen beispiesweise kann die Bindung der beiden festen Phasen aneinander durch Agglutination detektiert werden. Die Agglutination findet nur stat, falls die Markierung der Zielsequenzen oder deren Äquivalente erfolgreich war. Durch Verwendung eines Lateral-Flow-Devices (Fig. 19) kann die Bindung der beiden festen Phase durch Ausbildung eines sichtbaren Steifens detektiert werden (z.B. bei Verwendung von gefärbten Mikro-Teilchen oder des kolloidalen Goldes).

Falls mehrere Zielsequenzen nachgewiesen werden sollten, können mehrere verschiedene Primer, mit jeweils spezifischen Anker-Domänen, in Kombinatin mit mehreren Nuk-Makromolekülen verwendet, die jeweils spezifische Target-Domäne und mindestens eine Signal-Domäne oder mindestens eine Anker-Domäne einschließen. Die Signal-Domänen und Anker-Domänen von verwendeten Nuk-Makromolekülen können einheitlich oder unterschiedlich sein.

### 1.5.9.4 Bindung an die feste Phase durch eine modifizierten Hybridisierungssonde

In die Markierungsreaktion werden Hybridisierungssonden eingesetzt, die eine Target-Domäne und eine Anker-Domäne einschließen, zusammen mit mindestens einer Art von Nuk-Makromoleküle, die mindestens eine Target-Domane und mindestens eine Signal-Domäne einschließen. Die verwendeten Target-Domäne sind zielsequenzspezifisch. Nach der Bindung der markierten Zielsequenz an die feste Phase über die Anker-Domäne-1 der Hybridisierungssonde kann diese Zielsequenz durch Signal von eingebauten Nuk-Makromolekülen detektiiert werden.

Falls mehrere Zielsequenzen nachgewiesen werden sollten, können mehrere verschiedene Hybridisierungssonden, mit jeweils spezifischen Anker-Domänen, in Kombinatin mit mehreren Nuk-Makromolekülen verwendet, die jeweils spezifische Target-Domäne und beispielsweise mindestens eine Signal-Domäne einschließen. Die Signal-Domänen von verwendeten Nuk-Makromolekülen können einheitlich oder unterschiedlich sein.

### 1.5.9.5 Bindung an die feste Phase durch ein modifiziertes Nukleotid

In die Markierungsreaktion werden modifizierte Nukleotide mit niedriger molekularer Masse (z.B. dUTP-16-Biotin oder dUTP-Digoxigenin oder dUTP-Fluorescein) eingesetzt, die eine Anker-Domäne einschließen, zusammen mit mindestens einer Art von Nuk-Makromoleküle, die mindestens eine Target-Domane und mindestens eine Signal-Domäne einschließen. Es wird eine feste Phase bereitgestellt, die den niedermolekularen Marker von konventionellen Nukleotiden binden kann, z.B. Streptavidin oder Antikörper gegen Digoxigenin oder gegen Fluorescein. Nach der Bindung der markierten Zielsequenz an die feste Phase über die nidermolekulare Marker des modifizierten Nukleotids kann diese Zielsequenz durch Signal von eingebauten Nuk-Makromolekülen detektiiert werden.

### 1.5.9.6 Direkte Bindung der markierten Zielsequenz oder deren Äquivalente an die feste Phase mit fixierten, adressierbaren, der Zielsequenz komplementären Nukleinsäureketten

In einer Ausführungsform werden in die Markierungsreaktion einer Zielsequenz mindestens eine Polymerase, mindestens ein Primer zusammen mit mindestens einer Art von Nuk-Makromolekülen, die mindestens eine Target-Domane und mindestens eine Signal-Domäne einschließen, und wahlweise weitere Nukleotide eingesetzt. Nach der Markierung der Zielsequenzen mit den Nuk-Makromolekülen erfolgt die Trennung der Stränge, beispielsweise durch Temperatur oder Einwirkung von Alkali, z.b. NaOH-Lösung. Es wird eine feste Phase bereitgestellt, die adressierbare zu einer jeweiligen Zielsequenz komplementäre Oligonukleotide schließt. Das Gemisch wird in Verbindung mit der bereitgestellen festen Phase gebracht, so dass die markierten Nukleinsäueketten an die komplementären, auf der festen Phase gebundenen Nukleinsäureketten binden können.
Die Nachweisreaktion erfolgt über die Signal-Domäne der eingebauten Nuk-Makromoleküle.

In einer Ausführungsform werden in die Markierungsreaktion einer Zielsequenz mindestens eine Polymerase, mindestens ein Primer zusammen mit mindestens einer Art von Nuk-Makromolekülen, die mindestens eine Target-Domane und mindestens eine Anker-Domäne einschließen, und wahlweise weitere Nukleotide eingesetzt. Nach der Markierung der Zielsequenzen mit den Nuk-Makromolekülen erfolgt die Trennung der Stränge, beispielsweise durch Temperatur oder Einwirkung von Alkali, z.b. NaOH-Lösung. Das Gemisch wird in Verbindung mit der bereitgestellen festen Phase (1) gebracht, so dass die markierten Nukleinsäueketten an die komplementären, auf der festen Phase gebundenen Nukleinsäureketten binden können.
Nach der Bindung der markierten Zielsequenz an die feste Phase (1) über die Ausbildung von Doppelsträngen mit den auf der festen Phase fixierten Nukleinsäuesträngen kann eine zweite feste Phase (2) an die Anker-Domäne des Nuk-Makromoleküls binden. Bei Verwendung von Mikroteilchen beispiesweise kann die Bindung der beiden festen Phasen aneinander durch Agglutination detektiert werden. Die Agglutination findet nur stat, falls die Markierung der Zielsequenzen oder deren Äquivalente erfolgreich war. Durch Verwendung eines Lateral-Flow-Devices kann die Bindung der beiden festen Phase durch Ausbildung eines sichtbaren Steifens detektiert werden (z.B. bei Verwendung von gefärbten Mikro-Teilchen oder des kolloidalen Goldes).

Eine solche Art der Bindung von markierten Nukleinsäureketten an die feste Phase ist einem Fachmann bekannt z.B. als DNA Microarray-Technolgie (für Beispiele siehe Literatur im Abschnitt 1.3.20).

Falls mehrere Zielsequenzen analysiert werden sollen, kann eine feste Phase mit adresssierbaren, fixierten Nukleinsäuereketten verwendet werden. Entsprechend werden die Nuk-Makromoleküle an die zu untersuchenden Zielsequenzen angepasst.

### 1.5.10 Nachweis von gebundenen markierten Nukleinsäureketten

Viele bestehende Methoden können zum Nachweis von mit Nuk-Makromolekülen markierten Nukleinsäuren verwendet werden. Zum einen, können eingebaute Nuk-Makromoleküle eine oder mehrere Signaldomänen mit signalgebenden oder signalvermittelnden Einheiten einschließen, zum anderen, können weitere Komponenten des Systems signalgebende oder signalvermittelnde Elemente einschließen. Solche Elemente sind beispielweise markierte Primer, markierte Nukleotide, Hybridisierungssonde, interkallierende Farbstoffe.

Zur Detektion können beispielseise charakteristische Signale von Farbstoffen, chromogenen Substanzen, fluoreszenten Farbstoffen, elektrochemischen Markern oder Teilchen (z.B. Nano- oder Microkügelchen) verwendet werden. Beispiele für einzelne Komponenten sind im Abschnitt Signal-Domäne und Nachweismethoden ausführlicher dargestellt. In Abhängigkeit von der Signalgebung können auch unterschiedliche Systeme zum Nachweis des Signals verwendet werden. Beispiele dafür sind einem Fachmann bekannt.

In einer Ausführugnsform dient die Signalgebung beispielsweise zum Nachweis der Bindung von markierten Nukleinsäureketten an die feste Phase. In einer anderen Ausführungsform dient die Signalgebung beispielsweise dem Nachweis unterschiedlicher Sequenzvarianten innerhalb der Zielsequenz.

Die Signalgebung kann verschiedene Methoden der Signalvermehrung bzw. Signalverstärkung einschließen, Einem Fachmann sind Beispiele für Signalverstärkung bekannt.

In einer vorteilhaften Ausführungsform der Erfindung werden Intensitäten der erhaltenen Signale gemessen. Signalintensitäten von Zielsequenzen können mit Signal-Intensitäten der Kontrollsequenzen oder auch untereinander verglichen werden.

In einer vorteilhaften Ausführungsform werden diese intensitäten in digitaler Form erfasst, gespeichert und wiedergegeben.

Nachfolgend werden einige Beispiele für Nachweisreaktionen von Nukleinsäureketten an einer festen Phase benannt. Verschiedene Nachweismethoden können dabei mit verschiedenen Stukturen der Nuk-Makromoleküle kombiniert werden.

### 1.5.10.1 Nuk-Makromoleküle mit T-A-S-Domänen

Bei der Verwendung von mindestens einer Art von Nuk-Makromolekülen mit T-A-S-Domänen kann der Nachweis von gebundenen markierten Nukleinsäureketten mittels Signal-Domäne (S-Domäne der Nuk-Makromoleküle) erfolgen.

### 1.5.10.2 Nuk-Makromoleküle mit T-S-Domänen bzw. S-Domänen

Bei der Verwendung von mindestens einer Art von Nuk-Makromolekülen mit Target-Signal-Domänen bzw. Signal-Domänen erfolgt der Nachweis von gebundenen Zielsequenzen bzw. deren Äquivalenten vorzugsweise über die Signal-Domäne der eingebauten Nuk-Makromoleküle. Die Bindung von markierten Nukleinsäureketten an die feste Phase kann mittels anderer Reaktionspartner erfolgen. Beispielsweise können modifizierte Primer, Hybridisierungssonden oder modifizierte Nukleotide mit einer Anker-Domäne eingesetzt werden (siehe oben).

### 1.5.10.3 Signalgebung durch einen sequenzspefischen modifizierten Primer

Es werden zielsequenzspezifische Primer mit einer Signal-Domäne bei der Markierungsreaktion eingesetzt. Bei dieser Ausführungsform erfolgt der Nachweis von gebundenen Zielsequenzen bzw. deren Äquivalenten über die Signal-Domäne dieser markierten Primer. In einer bevorzugten Ausführungsform erfolgt die Bindung von markierten Nukleinsäureketten an die feste Phase über die eingebauten Nuk-Makromoleküle, die mitndestens eine Anker-Domäne einschließen. In einer weiteren Ausführungsform erfolgt die Bindung von markierten Nukleinsäureketten an die feste Phase über die eingebauten Nuk-Makromoleküle, die mitndestens eine Anker-Domäne sowie eine Target-Domäne einschließen.

### 1.5.10.4 Signalgebung durch eine modifizierten Hybridisierungssonde

Es werden zielsequenzspezifische Hybridisierungssonden mit einer Signal-Domäne bei der Markierungsreaktion eingesetzt. Bei dieser Ausführungsform erfolgt der Nachweis von gebundenen Zielsequenzen bzw. deren Äquivalenten über die Signal-Domäne dieser Hybridisierungssonden. In einer bevorzugten Ausführungsform erfolgt die Bindung von markierten Nukleinsäureketten an die feste Phase über die eingebauten Nuk-Makromoleküle, die mitndestens eine Anker-Domäne einschließen. In einer weiteren Ausführungsform erfolgt die Bindung von markierten Nukleinsäureketten an die feste Phase über die eingebauten Nuk-Makromoleküle, die mitndestens eine Anker-Domäne sowie eine Target-Domäne einschließen.

### 1.5.10.5 Signalgebung durch ein modifiziertes Nukleotid

Es werden modifizierte Nukleotide mit einer Signal-Domäne (z.B. Flureszenzfarbstoff-Markierte Nukleotide oder Biotin-markierte Nukleotide, z.B. dUTP-16-Biotin) bei der Markierungsreaktion eingesetzt. Bei dieser Ausführungsform erfolgt der Nachweis von gebundenen Zielsequenzen bzw. deren Äquivalenten über die Signal-Domäne dieser modifizierten Nukleotide. In einer bevorzugten Ausführungsform erfolgt die Bindung von markierten Nukleinsäureketten an die feste Phase über die eingebauten Nuk-Makromoleküle, die mitndestens eine Anker-Domäne einschließen. In einer weiteren Ausführungsform erfolgt die Bindung von markierten Nukleinsäureketten an die feste Phase über die eingebauten Nuk-Makromoleküle, die mitndestens eine Anker-Domäne sowie eine Target-Domäne einschließen.

Im Folgenden werden weitere Beispiele für vorteilhafte Kombinationen dargestellt.

### 1.5.11 Verfahrensvarianten: Verwendung von Nuk-Makromolekülen zur Markierung

### 1.5.11.1 Markierungsreaktion durch Primerextension

In einer Ausführungsform der Anmeldung werden Zielsequenzen in einer einzelsträngigen Form vorgelegt. In einer weiteren Ausführungsform werden zunächst doppelsträngige Zielsequenzen bereitgestellt. In diesem Fall kann ein Denaturierungsschritt des Doppelstranges, z.B. mittels erhöherter Temperatur, vor dem oder während des Markierungsschritts eingeschlossen werden oder die Auftrennung von Doppelsträngen mittels eines Enzyms erfolgen, z.B. mittels Helicase. Typischerweise, werden die Zielsequenz in einer gepufferten wässrigen Lösung vorgelegt.

Zu der bereitgestellten einzelstränigen Zielsequenz wird ein Oligonukleotid mit Primerfunktion zugegeben (im Weiteren als Primer genannt). Das Gemisch wird unter Bedingungen inkubiert, die eine sequenzspezifische Hybridisierung des Primers an die Bindungsstelle innerhalb der Zielsequenz zulassen. Solche Bedingungen sind einem Fachmann bekannt und sind in der Literatur mehrmals beschrieben. Es kommt dabei zur Ausbildung eines extensionsfähigen Primer-Zielsequenz-Komplexes. Zu diesen Komplexen werden mindestens eine Polymerase-Art, mindestens eine Art von Nuk-Makromoleküle, sowie wahlweise weitere Kompontenten (z.B. natürliche Nukleotide, modifizierte Nukleotide oder Hybridisierungssonden) zugegeben. Die Lösung wird unter Bedingungen inkubiert, unter denen die Polymerase eine Primerverlängerung durchführen kann. Während dieses Schrittes werden Nuk-Komponente der Nuk-Makromoleküle in den wachsenden Nukleinsäurestrang durch die zugegebe Polymerase eingebaut. Dadurch wird die Zielsequenz bzw. ein zur Zielsequenz komplementärer Strang (ein Äquivalent zu Zielsequenz) mit einem Nuk-Makromolekül markiert.

In einer Ausführungsform werden Nuk-Makromoleküle eingesetzt, die mindestens eine Target-Domäne einschießen (z.B. Kombinationen aus mindestens einer Target-Domäne und einer Anker-Domäne, oder mindestens einer Target-Domäne und einer Signal-Domäne, oder einer Target-Domäne und einer Anker-Domäne und einer Signal-Domäne). Die zielsequenzspezifischen Target-Domäne der Nuk-Makromoleküle können an die bereitgestelle Zielsequenz von der 3'-Position des Primers binden. Dabei werden die Reaktionsbedingugnen dermassen gewählt, dass die Target-Domäne der Nuk-Makromoleküle an ihre entsprechende Bindungsstelle innerhalb der Zielsequenz binden kann. Dabei sollten beispielsweise die Schmelztemperaturen bei gegebenen Reaktionsbedingungen für Primer and Target-Domäne berücksichtigt werden. Die Temperatur der Reaktion wird vorzugsweise so gewählt, dass sowohl der Primer als auch die Target-Domäne der Nuk-Makromoleküle an die Zielsequenz binden kann. Das Ausmass der Bindung kann variiert werden. Das kann in einer Ausführungsform durch die Verwendung von Reaktionstemperaturen erreicht werden, die unter der Tm des Primers und unter der Tm der Target-Domäne liegen. In einer weiteren Ausführungsform der Anmeldung wird Temperatur gewählt, die höher als die Tm des Primers und als die Tm der Target-Domäne liegt, z.B. im Bereich Tm +5°C, oder im Bereich Tm + 10°C. Dadurch bindet nur ein geringer Teil der Target-Domänen die Zielsequenzen. In einer weiteren Ausführungsform wird die Reaktionstemperatur unter der Tm des Primers aber über der Tm der Targert-Domäne des Nuk-Makromoleküls verwendet. In einer weiteren Ausführungsform wird die Reaktionstemperatur über der Tm des Primers aber unter der Tm der Targert-Domäne des Nuk-Makromoleküls verwendet. In einer weiteren Ausführungsform wird ein Temperatur-Gradient während der Reaktion verwendet, wobei die Temperatur stufenweise angepasst wird, so dass Hybridisierung von Primer und Target-Domäne nicht gleichzeitig, sondern nach einander erfolgen kann.

In einer Ausführungsform der Erfindung, liegt die Tm der zielsequenzspezifischen Target-Domänen eines Nuk-Makromoleküls höher als die Tm eines entsprechenden zielsequenzspezifischen Primers. Die Nuk-Makromoleküle können dadurch bei höheren Temperaturen an die Zielsequenzen binden als die Primer. Dadurch kann sichergestellt werden, dass der verlängerte Primer stetts Nuk-Makromoleküle vorfinden, die bereits an die Zielsequenz gebunden haben.

In einer anderen Ausführungsform der Erfindung ist die Tm der Target-Domäne der Nuk-Makromoleküle niederiger als die der verwendeten Primer. Dadurch kann eine Primer-Verlängerung bei höheren Temperaturen stattfinden, ohne dass die Nuk-Makromolküle mit der Einbaureaktion interferrieren.

In einer anderen Ausführungsform der Erfindung ist die Tm der Target-Domäne der Nuk-Makromoleküle und die Tm der Primer etwa gleich. Dadurch ist das Bindungsverhalten der Nuk-Makromoleküle und der Primer etwa gleich.

Die Reihenfolge der Zugabe der Komponenten kann variieren. Beispielweise, können die einzelnen Komponenten der Markierungsreaktion einzeln nacheinander oder auch in Kombinatinen (Kompositionen) zugegeben werden. Beispielsweise, können die erforderlichen Nukleotide in bestimmten Verhältnissen vorgemischt werden. Auch Puffer und Salze können in bestimmten Proportionen vorgemischt vorgelegt werden. Weitere Kombinationen sind einem Fachmann offensichtlich. Solche Kompositionen können als Bestandteile von Kits bereitgestellt werden.

Nach diesem Markierungsschritt kann die nun markierte Nukleinsäurekette (Zielseqenz oder deren Äquivalente) von überschüssigen nicht eingebauten Nukleotiden isoliert werden. Isolierung und Reinigung von Nukleinsäureketten ist einem Fachmann bekannt. Anschließend werden Nukleinsäureketten in Kotankt mit fester Phase gebracht und detektiert, wie im Abschnitt Amplifikationsmethoden geschrieben. Makrierte Nukleinsäureketten können auch direkt nach der Markierungsreaktion, ohne Isolierung und Reinigung, in Kontakt mit fester Phase gebracht und nachgewiesen werden.

Die Bindung der Nukleinsäureketten an die feste Phase kann dabei direkt über komplementäre, immobilisierte Nukleinsäuerekette oder indirekt, vermittelt über eine Anker-Domäne erfolgen.
In einer solchen Reaktion können auch andere Kompontenten, z.B. markierte Primer, Hybridisierungssonden, markierte Nukleotide eingesetzt werden, wie in anderen Abschnitten beschriben.
Es können eine oder mehrere Arten von Nuk-Makromolekülen an einer solchen Reaktion teilnehmen, wie in anderen Abschnitten beschriben.
Es können einzelne oder mehrere Zielsequenzen amplifiziert und markiert werden.
Die Detektion der Zielsequenz kann durch die Bindung an die feste Phase mit einer anschließenden Nachweisreaktion erfolgen, wie in anderen Abschnitten beschriben.

### 1.5.11.2 Markierung einer Zielsequenz mit mehreren Nuk-Makromolekülen

Es wird eine Zielsequenz untersucht.
Es wird eine feste Phase mit mehreren spezifischen Bindungspartnern für Anker-Domänen der Nuk-Makromoleküle bereitgestellt. Die Bindungspartner sind vorzugsweise in einer definierten räumlichen Anordnung angeordnet, die eine optische Unterscheidung von einzelnen Positionen mit fixierten Bindungspartnern zulässt. Die Anker-Domänen können als Bestandteile von Nuk-Makromolekülen oder modifizierten Primern oder Hybridisierungssonden oder modifizierten Nukleotiden mit niedermolekularem Marker auftreten.

In einer Reaktion können mehrere Nuk-Makromoleküle eingesetzt werden, deren Target-Domäne zu einer Zielsequenz komplementär sind. In einer Ausführungsform binden die einzelnen Target-Domäne der Nuk-Makromoleküle an unterschiedliche Sequenzabschnitte der genannten Zielsequenz. In einer weiteren Ausführungsform binden die Target-Domänen der Nuk-Makromolelüle an die Zielsequenz am gleichen Ort. In einer weiteren Ausführungsform binden die Target-Domänen der Nuk-Makromolelüle an die Zielsequenz an benachbarten Positionen, die auch einander überlappen können. Die Target-Domäne werden innerhalb einer Art von Nuk-Makromoleküle beispielswiese als Kombinationen aus mindestens einer Target-Domäne und einer Anker-Domäne, oder mindestens einer Target-Domäne und einer Signal-Domäne, oder einer Target-Domäne und einer Anker-Domäne und einer Signal-Domäne verwendet.

Durch Variationen an den Target-Domänen können spezielle Abschnitte an der Zielsequenz ausgesucht werden, an die die Bindung von Target-Domänen erfolgen soll. Dadurch kann die Zielsequenz auf Anwesenheit von bestimmten Sequenzbereichen überprüft werden.
Nuk-Makromoleküle können alleine oder in Kombination mit einem Set von natürlichen Nukleotiden, z.B. dNTPs, eingesetzt werden.
Da der enzymatische Einbau von Nuk-Makromolekülen, die mit ihren Target-Domänen an die Zielsequenz gebunden haben, deutlich begünstigt wird, werden in der enzymatischen Reaktion vornehmlich Nuk-Makromoleküle eingebaut, die an die Target-Sequenz gebunden haben. Dadurch kommt es zu einer Markierung von Zielsequenzen oder deren Äquivalenten mit jeweils spezifischen Nuk-Makromolekülen.

In einer vorteilhanften Ausführungsform schließen die Nuk-Makromoleküle mindestens eine Target-Domäne und mindestens eine Anker-Domäne ein. Die Detektion erfolgt durch den Einsatz von Nuk-Makromolekülen mit Signal-Domänen oder modifizierten Primern mit Signal-Domänen, oder Hybridisierungssonden mit Signal-Domänen oder konventionell markierten Nukleotiden.
In einer weiteren vorteilhanften Ausführungsform schließen die Nuk-Makromoleküle mindestens eine Target-Domäne und mindestens eine Signal-Domäne ein. Die Bindung an die feste Phase erfolgt durch den Einsatz von Nuk-Makromolekülen mit Anker-Domänen oder modifizierten Primern mit Anker-Domänen, oder Hybridisierungssonden mit Anker-Domänen, oder durch direkte Hybridisierung von markierten Zielsequenzen an die auf der festen Phase immobilisierten, komplementären Nukleinsäurestränge.

Inkubation von markierten Nukleinsäureketten mit der festen Phase führt zu Bindung von Anker-Domänen an die auf der festen Phase fixierten Bindungspartner. Dadurch werden auch die markierten Zielsequenzen bzw. deren Äquivalente an der festen Phase fixiert. Nach einer Nachweisreaktion kann eine optische Zurdnung der gebundenen Nukleinsäureketten zu den jeweiligen Positionen der Bindungspartner vorgenommen werden. Dadurch ist ein Rückschluß auf Vorliegen von bestimmten Zielsequenzen im Reaktionsansatz möglich.

Die Bindung der Nukleinsäureketten an die feste Phase kann dabei direkt über die zur Zielsequenz komplementäre, immobilisierte Nukleinsäuerekette oder indirekt, vermittelt über eine Anker-Domäne erfolgen.
In einer solchen Reaktion können auch andere Kompontenten, z.B. markierte Primer, Hybridisierungssonden, markierte Nukleotide eingesetzt werden, wie in anderen Abschnitten beschriben.
Es können eine oder mehrere Arten von Nuk-Makromolekülen an einer solchen Reaktion teilnehmen, wie in anderen Abschnitten beschriben.
Es können einzelne oder mehrere Zielsequenzen amplifiziert und markiert werden.
Die Detektion der Zielsequenz kann durch die Bindung an die feste Phase mit einer anschließenden Nachweisreaktion erfolgen, wie in anderen Abschnitten beschriben.

### 1.5.11.3 Mehrere Nukleinsäureketten

In einer Reaktion können mehrere Zielsequenzen untersucht werden.
Es wird eine feste Phase mit mehreren spezifischen Bindungspartnern für Anker-Domänen bereitgestellt. Die Bindungspartner sind vorzugsweise an die feste Phase in charakteristischer Art und Weise gebunden, z.B. in einer definierten räumlichen Anordnung angeordnet, die eine optische Unterscheidung von einzelnen Positionen mit fixierten Bindungspartnern zulässt. Die Anker-Domänen können als Bestandteile von Nuk-Makromolekülen oder modifizierten Primern oder Hybridisierungssonden oder modifizierten Nukleotiden mit niedermolekularem Marker auftreten.

In einer Ausführungsform wird zum Nachweis einer jeden Zielsequenz mindestens eine Art von Nuk-Makromolekülen mit einer entsprechenden zu dieser Zielsequenz spezifischen Target-Domäne verwendet. Zum Nachweis von mehreren Zielsequenzen werden mehrere unterschiedliche Nuk-Makromoleküle, mit entsprechenden unterschiedlichen Target-Domänen in einer Reaktion kombiniert. Vorzugsweise sind die Target-Sequenzen von Nuk-Makromolekülen untereinander nicht komplementär. Die Target-Domänen werden innerhalb einer Art von Nuk-Makromoleküle beispielswiese als spezifische Kombinationen aus mindestens einer Target-Domäne und einer Anker-Domäne, oder mindestens einer Target-Domäne und einer Signal-Domäne, oder einer Target-Domäne und einer Anker-Domäne und einer Signal-Domäne verwendet. Es resultieren demnach Kompositionen von Nuk-Makromolekülen, wie z.B. Nuk1-[T1;A1] bis Nuk1-[Tn;An].

In einer weiteren Ausführungsform werden Nuk-Makromoleküle eingesetzt, die Target-Domäne einschließen, die an mehrere Zielsequenz binden können. Diese Zielsequenzen gehören beispielswiese zu einer Zielsequenzgruppe. Innerhalb dieser Gruppe können Zielsequenzen vorkommen, die sich von einander mehr oder weniger unterscheiden, d.h. Sequenzen haben Homologien. Durch Einsatz von Nuk-Makromolekülen, die potenziell für Markierung verschiedener Zielsequenzen einer definierten Zielsequenzgruppe fähig sind, ist es möglich, Sequenzvariationen zu tolerieren. Die Markierung ist somit nicht steng Zielsequenzspezifisch sondern Gruppenspezifisch. Solche Gruppenspezifität ist beispielsweise beim Nachweis von Viren mit starker Fähigkeit zu Sequenzvariation nützlich. Die verwendeten Nuk-Makromoleküle schließen beispielsweise mehrere Target-Domänen ein. Im weiteren Beispiel schließen Nuk-Makromoleküle Target-Domäne ein, bestehend aus Nukleinsäurekette, die lang genug sind, so dass einzelne Basen-Variationen in der Zielsequenz kaum einen Einfluss auf das Bindungsverhalten der Target-Domäne an die Zielsequenz bei gegebenen Reaktionsbedingungen ausüben, oder es werden Reaktionsbedingungen (z.B. Temperatur) verwendet, die auch eine Bindung von Target-Domäne an die Zielsequenz ohne einen "perfect match" zulassen.

Nuk-Makromoleküle können alleine oder in Kombination mit einem Set von natürlichen Nukleotiden, z.B. dNTPs, eingesetzt werden. Da der enzymatische Einbau von Nuk-Makromolekülen, die mit ihren Target-Domänen an die Zielsequenz gebunden haben, deutlich begünstigt wird, werden in der enzymatischen Reaktion vornehmlich Nuk-Makromoleküle eingebaut, die an die Target-Sequenz gebunden haben. Dadurch kommt es zu einer Markierung von Zielsequenzen oder deren Äquivalenten mit jeweils spezifischen Nuk-Makromolekülen.

In einer vorteilhanften Ausführungsform schließen die Nuk-Makromoleküle mindestens eine Target-Domäne und mindestens eine Anker-Domäne ein. Die Detektion erfolgt durch den Einsatz von Nuk-Makromolekülen mit Signal-Domänen oder modifizierten Primern mit Signal-Domänen, oder Hybridisierungssonden mit Signal-Domänen oder konventionell markierten Nukleotiden.
In einer weiteren vorteilhanften Ausführungsform schließen die Nuk-Makromoleküle mindestens eine Target-Domäne und mindestens eine Signal-Domäne ein. Die Bindung an die feste Phase erfolgt durch den Einsatz von weiteren Nuk-Makromolekülen mit Anker-Domänen oder modifizierten Primern mit Anker-Domänen, oder Hybridisierungssonden mit Anker-Domänen, oder durch direkte Hybridisierung von markierten Zielsequenzen an die auf der festen Phase immobilisierten, komplementären Nukleinsäurestränge.

Inkubation von markierten Nukleinsäureketten mit der festen Phase führt zu Bindung von Anker-Domänen an die auf der festen Phase fixierten Bindungspartner. Dadurch werden auch die markierten Zielsequenzen bzw. deren Äquivalente an der festen Phase fixiert. Nach einer Nachweisreaktion kann eine optische Zurdnung der gebundenen Nukleinsäureketten zu den jeweiligen Positionen der Bindungspartner vorgenommen werden. Dadurch ist ein Rückschluß auf Vorliegen von bestimmten Zielsequenzen im Reaktionsansatz möglich.

Die Bindung der Nukleinsäureketten an die feste Phase kann dabei direkt über komplementäre, immobilisierte Nukleinsäuerekette oder indirekt, vermittelt über eine Anker-Domäne erfolgen.
In einer solchen Reaktion können auch andere Kompontenten, z.B. markierte Primer, Hybridisierungssonden, markierte Nukleotide eingesetzt werden, wie in anderen Abschnitten beschriben.
Es können eine oder mehrere Arten von Nuk-Makromolekülen an einer solchen Reaktion teilnehmen, wie in anderen Abschnitten beschriben.
Es können einzelne oder mehrere Zielsequenzen amplifiziert und markiert werden.
Die Detektion der Zielsequenz kann durch die Bindung an die feste Phase mit einer anschließenden Nachweisreaktion erfolgen, wie in anderen Abschnitten beschriben.

### 1.5.10.4 Matrkierung einer Zielsequenz in mehreren Zyklen bei isothermischen bzw. zyklischen Änderung von Temperaturbedingungen

Es werden eine oder mehrere Zielsequenzen bereitgestellt. Es werden ein oder mehrere Primer an die bereitgestellten Zielsequenzen hybridisiert. Die Verlängerung der Primer mit Nuk-Makromolekülen, bzw. noch weiteren Nukleotiden findet mit Hilfe eine Polymerase statt.

Es werden beispielsweise Nuk-Makromoleküle eingesetzt, die eine zur Zielsequenz komplementäre Target-Domäne einschließen.

Die Markierugnsreaktion erfolgt in mehreren Zyklen: In jedem Zyklus erfolgt die Hybridisierung von Primern an die Primerbindungsstelle in der Zielsequenz und Bindung der Nuk-Makromoleküle durch ihre Target-Domäne an die Zielsequenz, so dass ein extensionsfähiger Primer-Matrizen-Komplex entsteht. Dieser Komplex wird von der Polymerase verlängert und die Nuk-Makromoleküle werden in den verlängerten Primer eingebaut. Nach einem Markierungsschritt werden dann die verlängerten Primer von der Zielsequenz getrennt. Dies kann z.B. durch Temperaturerhöhung erreicht werden oder mittels eines Enzyms, z.B. Helicase oder einer Polymerase mit strand-displacement Aktivität. Durch Bereitstellung eines Überschusses von Markierungsprimern und eines Überschusses von Nuk-Makromolekülen können in einer folgenden Runde noch nicht verlängerte Primer an die Zielsequenz binden und neue extensionsfährige Komplexe bilden, die dann mit Nuk-Makromolekülen verlängert werden. Falls eine temperaturbedinge Denaturierung von Primer-Zielsequenz-Komplexe gewählt wird, können die zyklischen Temperaturveränderungen ähnlich wie in PCR gestalltet werden. Die Anzahl der Zyklen kann beispielsweise zwischen 2 und 500 liegen. Dadurch kann eine Anreicherung von verlängerten markierten Pimern erreicht werden. Die Denaturierung der Primer-Matrize-Komplexe kann auch enzymatisch erfolgen, in einem solchen Fall können isotherminsche Bedingungen verwendet werden.

In der Ausführungsform mit thermischer Denaturierung der extendierten Primer wird vorzugsweise eine entsprechende thermostabile Polymerase verwendet. Die Temperatur wird soweit erhöht, dass sich der verlängerte Stang von der Matrize löst. Beim Absenken der Temperatur erfolgt dann eine wiederholte Hybridisierung von Primern und Nuk-Makromolekülen an die Matrize. Der nun neu hybridisierte Primer kann von der Polymerase verlängert werden und Nuk-Makromoleküle können eingebaut werden.
Die Änderung der Temperatur kann beispielsweise in einer PCR-Gerät, einem thermalen Cycler erfolgen.

Die markierten Nukleinsäureketten werden anaysiert wie in anderen Abschnitten dargestellt.

### 1.5.10.5 Amplifikation mit Markierung

In einer besonders vorteilhaften Ausführungsform der Anmeldung erfolgt die Markierungsreaktion im gleichen Ansatz wie auch die Vervielfältigung, wobei die Amplifikationsschritte und Markierungsschritte nacheinader erfolgen. In einer anderen Ausführungsform erfolgt die Markierung parallel zu Vervielfältigung der Zielsequen. In dieser Ausführungsform werden Amplifikationsprimer als Markierungsprimer verwendet.
Es sind verschiedene Beispiele für Amplifikationen von Nukleinsäureketten bekannt. Wie in diesen Verfahren beschrieben, wird ein Abschnitt aus der Zielsequnz oder die komplette Zielsequenz mit Hilfe von Primern, oft als Primer-Paare bereitgestellt, vervielfältigt.
Die Amplifikation kann exponentiell erfolgen, wie in PCR oder linear.
Die Reaktion kann durch zyklische Schritte mit Temperatur-Änderung gekennzeichnet sein oder bei einer konstanten Temperatur (Isothermal) durchgeführt werden.
Durch die Wahl der Temperatur, Puffer und Konzentrationen erfolgt ein Prozess, bei dem Zielsequenzen in einzelsträngige Form überführt werden, Primer hybridisiert und verlängert werden. Wobei dieser Prozess mehrmals wiederholt werden kann. Die Durchführung einer PCR sollte einem Fachmann bekannt sein.
In einer Ausführungsform werden für die Markierungsreaktion derselber / dieselben Primer verwendet, wie für die Vervielfältigungsreaktion. In einer weiteren Ausführungsform wird für die Markierungsreaktion ein zusätzlicher Primer verwendet.

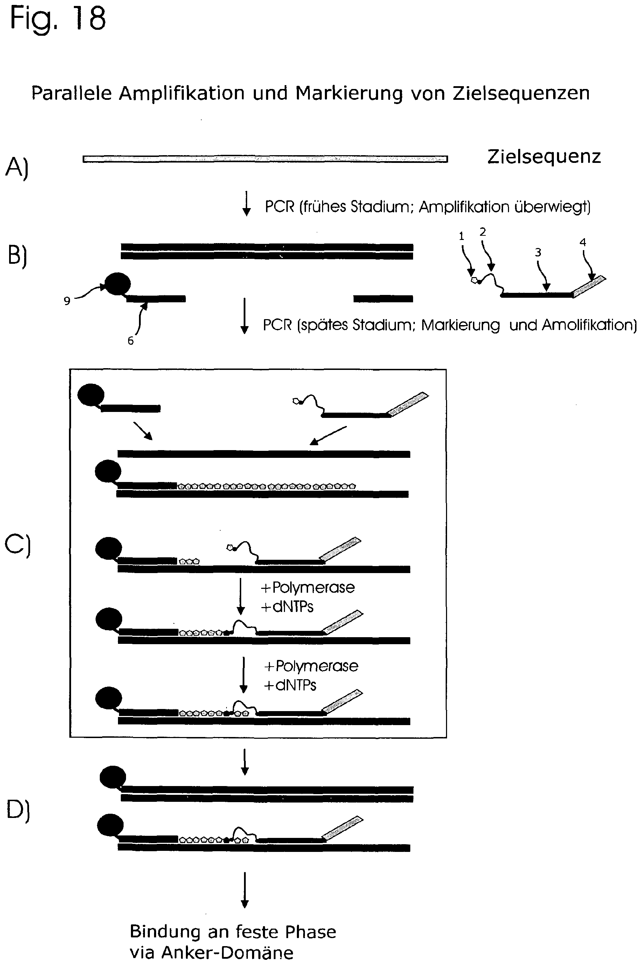

Bei der Durchführung einer PCR werden typischerweise in einem Reaktionsansatz folgende Komponenten bereitgestellt: mindestens eine Zielsequenz, mindestens ein zu dieser Zielsequenz passendes Primer-Paar, mindestens eine entsprechende Polymerase, z.B. eine thermostabile Polymerase, mindestens eine Art von Nuk-Makromolekülen mit einer zur Zielsequenz passenden Target-Domäne, weitere natürliche Nukleotide (z.B. dATP, dGTP, dCTP, dTTP), sowie eine entsprechende Puffer-Lösung.
Die Vervielfältigungsreaktion erfolgt durch zyklische Änderungen der Temperatur, bei folgende zyklische Schritte wiederholt werden: Annealing, Primer-Extension und Denaturierung neu entstandener Stränge.
Durch Zugabe von Nuk-Makromolekülen, die mindestens eine Target-Domäne einschließen, die an die Zielsequenz hybridisiert werden kann (die Target-Domäne bindet an die Zielsequenz von der 3'-Seite eines entsprechenden Primers), können auch Nuk-Makromoleküle an die einzelsträngige Zielsequenzen binden (Fig. 18-21). Dies erfogt ähnlich wie bei Real-Time-PCR-Verfahren mit einer Sonde. Nach der Bindung der Primer und der Nuk-Makromolekülen an die Zielsequenz (Hybridisierunsschritt / Annealing in der PCR) erfolgt eine Primer-Extensionsreaktion mit dem Einbau von Nuk-Makromolekülen. Nach Abschluss eines Zyklus mit der Primerverlägerung, werden die entstandenen Doppelstränge beispielsweise durch einen Termeratur-Anstieg denaturiert. Anschließend folgt dann ein neuer Zyklus mit einer Primer-Hybridisierung und Nuk-Makromolekül-Hybridisierung und einer entsprechenden Primer-Verlägerung. Diese Zyklen können mehrmals wiederholt werden, so dass es zu Ansammlung von extendierten Primern kommt. Die neu synthetisierten Stränge enthalten auch Nuk-Makromoleküle. Durch den Einbau von Nuk-Makromolekülen in die wachsenden Stränge werden spezifisch markierte Nukleinsäureketten generiert.
Die Reaktionsbedingungen (z.B. Puffer und Temperatur) sowie die Tm des Primers und die Tm der Target-Domäne der Nuk-Makromoleküle sind so gewählt, dass während des Hybridiserungsschritts beide an die Zielsequenzen binden können. In dieser Ausführngsform kann die Amplifikation parallel zur Markierung stattfinden.

In einer Ausführungsform beträgt der Abstand zwischen dem 3'-Ende des Primers und dem 5'-Ende der Target-Domäne mindestens 10 Nukleotide, in einer weiteren Ausführungsform mindestens 50 Nukleotide, in einer weiteren Ausführungsform mindestens 100 Nukleotide, in einer weiteren Ausführungsform mindestens 200 Nukleotide.

Vorzugsweise ist das 3'-Ende der Target-Domäne blockiert, so dass die Target-Domäne nicht als Primer dient.

Typischerweise wird ein Gemisch aus Nuk-Makromolekülen sowie natürlichen Nukleotiden für die Reaktion bereitgestellt. Die Konzentrationsverhältnisse bei einem solchen Gemisch können variieren. Beispielsweise liegen die Konzentrationen von natürlichen Nukleotiden zwischen 50 und 500 µmol/l und die Konzentration der Nuk-Makromoleküle zwischen 0,1 und 2 µmol/l. Weitere Beispiele für Konzentrationen von Nukleotiden sind im Abschnitt 1.5.8 beschrieben. Die Konzentration der Primer liegt beispielsweise zwischen 0,1 und 2 µmol/l.

Die Bedingungen der Reaktion (Temperatur), sowie die Struktur und Konzentration einzelner Kompontenten können dermassen angepasst werden, dass am Anfang des Prozesses die Amplifikation der Fragemtne der Zielsequenzen oder deren Äquivelaten überwiegt. Durch die Zunahme der Konzentraiton von amplifizierten Fragmenten (Äquivalente der Zielsequenz) in späteren Stadien des Vervielfältigungsprozesses erfolgt auch Markierungsreaktion von amplifizierten Strängen. Bei sehr geringen Konzentrationen von Zielsequenzen am Anfang des Vervielfältigungsprozesses ist die Wahrscheinlichkeit der Bindung und des Einbau von Nuk-Makromolekülen in die Zielsequenz oder deren Äquivaltente gering. Mit steigender Konzentraiton von Zielsequenzen während des Amplifikationsvorganges, erhöht sich die Frequenz der Bindungs- und Einbauereignisse von Nuk-Makromolekülen in die wachsenden Nukleinsäureketten.

Es folgend einige Beispiele für Kombinationen aus PCR und Markierungsreaktion. Zur Signal-Generierung werden in diesen Beispiele markierte Primer eingesetzt, die eine Signal-Domäne einschließen. Dabei wird ein Primer aus einem zielsequenzspezifischen Primer-Paar markiert.

In einer Ausführungsform wird PCR als Amplifikationsreaktion verwendet (Fig. 18). Es werden Nuk-Makromoleküle verwendet, die eine zu dem potenziellen PCR-Fragment der DNA komplementäre Target-Domäne, sowie eine zielsequenzzugeordnete Anker-Domäne einschließen. Die Tm der Target-Domäne liegt beispielsweise im gleichen Bereich, wie die Tm von verwendeten Primern +/- 5°C. Die PCR-Primer dienen auch als Markierungsprimer und schließen beispielsweise entweder eine Signal-Domäne oder eine Anker-Domäne ein. Die passenden Kombinationen von markierten Primern und Nuk-Makromolekülen sind in vorherigen Abschnitten beschrieben.
Die Konzentration von Primern liegen nach den bekannten Regeln einer PCR beispielswiese in fogenden Bereichen: zwischen 10 nmol/l und 100 nmol/l, 100 nmol/l und 300 nmol/l, 300 nmol/l und 1 µmol/l, 1 µmol/l und 10 µmol/l. Die Konzentration der Nuk-Makromolekülen in der Reaktionslösung liegt beispielsweise zwischen 10 pmol/l bis 100 pmol/l, 100 pmol/l bis 1 nmol/l, 1 nmol/l bis 10 nmol/l, 10 nmol/l bis 100 nmol/l, 100 nmol/l bis 300 nmol/l, 300 nmol/l bis 1 µmol/l, 1 µmol/l und 10 µmol/l. Die Verhältnisse der Konzentrationen von den PCR-Primern und Nuk-Makromolekülen liegen beispielsweise in folgenden Bereichen (Konzentraiton von PCR-Primern: Nuk-Makromoleküle): 1:100 bis 1:10, 1:10 bis 1:1, 1:1 bis 10:1, 10:1 bis 100:1, 100:1 bis 1000:1. Die Konzentration von dNTPs (dATP, dCTP, dGTP, dTTP) liegt beispielsweise zwischen 10 µmol/l und 1 mmol/l von jedem einzelnen.
Die Zeiten für jeden Temperatur-Schritt liegen beispielsweise zwischen 1 sec und 10 min.

In einigen Ausführugnsformen können die Unterschiede zwischen der Tm der Primer und der Tm der Target-Domäne der verwendeten Nuk-Makromoleküle mehr als 5°C betragen, z.B. Unterschiede können bis 50°C betragen. Diese Unterschiede können bei der Steuerung der Amplifikations- und Markierungsreaktion verdendet werden. In einer Ausführungsform liegt die Tm des Primers beispielsweise um 15 bis 20°C höher als die der Tm der Target-Domäne der Nuk-Makromoleküle. Die Nuk-Makromoleküle werden in Konzentrationen eingesetzt, deutlich geringer sind, als die Konzentrationen von dNTPs. Bei einer PCR werden in den anfänglichen Zyklen Reaktionstemperaturen während eines Annealing-Schrittes um die Tm des Primers gewählt (z.B. Tm des Primers minus 5-10 °C), dabei kann eine Verlängerung des Primers und des komplementären Stranges mit natürlichen Nukleotiden stattfinden. Die Target-Domänen der Nuk-Makromoleküle dagegen können nicht an die Zielsequenzen binden, da ihre Tm viel tiefer liegt, als die verwendete Hybridisierungs-Temperatur. Da die Target-Domäne an die Zielsequenz unter diesen Bedingungen nicht bindet und ihre Konzentration deutlich geringer ist als die der dNTP, werden bevorzugt die dNTPs eingebaut. Anschließend werden weitere Zyklen gefahren mit einer Reaktionstemperatur beim Annealingschritt unter der Tm der Target-Domäne des Nuk-Makromoleküls. Durch die Änderung der Temperatur (Temperatur wurde von Tm des Primers auf die Tm der Target-Domäne herabgesetzt) können nun sowohl Primer als auch die Target-Domänen der Nuk-Makromoleküle an die Zielsequenz binden. Durch hohe lokale Konzentrationen von Nuk-Komponenten der an die Zielsequenz gebundenen Nuk-Makromoleküle können nun die Nuk-Makromoleküle trotz Anwesenheit der dNTPs in die wachsenden Stränge eingebaut werden und die Zielsequenzen werden markiert. Vorzugsweise wird die Target-Domäne in dieser Ausführungsform weiter in Richtung 3' vom Primer platziert. Vorzugsweise beträgt der Abstand zwischen dem 3'-Ende des Primers und dem 5'-Ende der Target-Domäne mindestens 10 Nukleotide, in einer weiteren Ausführungsform mindestens 50 Nukleotide, in einer weiteren Ausführungsform mindestens 100 Nukleotide, in einer weiteren Ausführungsform mindestens 200 Nukleotide.
Durch Steuerung der Reaktionstemperaturen kann die Bindung von Primern und von Target-Domänen variabel gestaltet werden und dadurch entweder nur die Amplifikationsreaktion oder Amplifikationsreaktion und Markierungsreaktion erfolgen.

In einer weiteren Ausführungsform wird zusätzlich zu Amplifikationsprimer ein weiterer Primer (Markierungsprimer) für die Markierung der Zielsequenz verwendet. Dieser Primer kann die Zielsequenz spezifisch am gleichen Strang binden, wie die Target-Domäne eines Nuk-Makromoleküls. Der Markierungsprimer liegt vorzugsweise innerhalb der Zielsequenz und auf der 5'-Seite der gebundenen Target-Domäne eines Nuk-Makromoleküls. In der Amplifikation werden PCR-Fragmente der Zielsequenz mit Amplifikationsprimers generiert. Der Markierungsprimer bindet vorzugsweise zwischen einem Amplifikationsprimer und der Target-Domäne eines gebundenen Nuk-Makromoleküls. Die Tm des Markierungsprimers liegt vorzugsweise im gleichen Temperatur-Bereich wie die Tm der Target-Domäne des Nuk-Makromoleküls. Beispielsweise, kann die Tm des Markierungsprimer die Tm von Amplifikationsprimern sich unterscheiden (die Tm von Markierungsprimer ist geringer als die Tm von Amplifikationsprimern), so dass während einer PCR zunächst die PCR-Fragmente generiert werden und erst anschließend eine Markierungsreaktion mit einem Markierungsprimer ablaufen kann. In einer Reaktion kann die Konzentration eines Markierungsprimers höher liegen als die Konzentration von PCR-Primern.

In einer weiteren Ausführungsform werden mehr als ein Markierungsprimer mit unterschiedlichen Bindungsstellen innerhalb einer Zielsequenz zusammen mit mehreren entsprechenden spezifischen Nuk-Makromolekülen eingesetzt. Die Markierungsprimer binden an die Zielsequenz jeweils auf der 5'-Seite einer Bindungsstelle einer Target-Domäne eines Nuk-Makromoleküls, so dass eine Einbaureaktion stattfinden kann. Vorzugsweise sind solche Markierungsprimer mit einer Signal-Domäne oder einer Anker-Domäne versehen.

In einer weiteren Ausführungsform werden zusätzliche Oligonukleotide eingesetzt, die mit der Target-Domäne eines Nuk-Makromoleküls um die Bindungsposition innerhalb der Zielsequenz konkurrieren. In einer Ausführungsform haben solche Oligonukleotide gleiche Sequenzzusammensetzung wie die Target-Domäne der Nuk-Makromoleküle. In einer weiteren Ausführungsform unterscheidet sich ihre die Sequenzzusammensetzung von der Target-Domäne in mindesten einem Nukleotid. In einer weiteren Ausführungsform werden mehrere Oligonukletode verwendet, die sich in ihrer Sequenzzusammensetzung von der Target-Domäne in mindesten einem Nukleotid unterscheiden. Durch Anwesenheit solche Oligonukletide im Ansatz konkurrieren die Target-Domäne um die Bindungsstelle innerhalb der Zielsequenz. Dadurch werden weniger spezifische Bindung der Target-Domäne an die Zielsequenz unterdrückt, was zu höheren Spezifität der Analyse beitragen kann.

Nach der PCR können PCR-Fragmente von überschüßigen Primern bzw. Nuk-Makromolekülen gereinigt werden oder direkt analysiert werden. Die Analyse erfolgt beispielsweise durch Bindung an eine feste Phase mit adressierbaren Bindungspartnern.

In einer Ausführungsform werden die Konzentrationen der Nuk-Makromoleküle vorzugsweise dermassen gewählt, dass nach der Markierungsreaktion die meisten Nuk-Makromoleküle in die Zielsequenzen eingebaut sind, so dass keine zusätzliche Reinigung von markierten Nukleinsäureketten für die anschließende Bindung an die feste Phase notwendig ist.

In einer weiteren Ausführungsform kann die Konzentration eines PCR-Primers höher sein, als die des anderen Primers. Dadurch kann eine gewisse Assymetrie in der Generierung der PCR-Fragmente erreicht werden: die Konzentration eines Stranges kann damit erhöhet werden.

In einer weiteren Ausführungsform können unterschiedliche Primer für die Markierungsreaktion und für die Amplifikationsreaktion in einem Ansatz kombiniert werden. Beispielsweise Primer-Paar mit höheren Tm wird für die Amplifikation verwendet und mindestens ein Primer mit geringerer Tm für die Markierungsreaktion von neu generierten PCR-Produkte. Die Tm der Primers für die Markierungsreaktion liegt beispielsweise im gleichen Bereich wie die Tm der Target-Domäne von Nuk-Makromolekülen.

In einer weiteren Ausführungsform werden multiple Zielsequenzen mittels PCR amplifiziert (Fig. 19). Eine Multiplex-PCR ist einem Fachmann bekannt. Es werden beispielsweise mehrere Primer-Paare für die Vervielfältigung verwendet, so dass mehrere PCR-Fragmente entstehen. In die Reaktion werden Nuk-Makromoleküle eingesetzt, die jeweils eine spezifische Target-Domäne und eine jeweils passende spezifische Anker-Domäne einschließen. Die Target-Domänen der Nuk-Makromoleküle sind dermassen gewählt, dass sie an die entstehenden PCR-Fragmente spezifisch binden können. Die Anker-Domänen der Nuk-Makromoleküle sind zusammen mit den jeweiligen Target-Domänen spezifisch kombiniert, so dass jeweilige Anker-Domäne mit der jeweiligen Target-Domäne eines Nuk-Makromoleküls ein spezifisches Paar bildet. Beispielsweise, es wird erwartet, dass vier Zielsequenzen in einem Gemisch vorkommen können. Es wird eine Multiplex-PCR mit vier spezifischen Primer-Paaren und vier spezifischen Nuk-Makromolekülen (Fig. 19) durchgeführt. Die Nuk-Makromoleküle können folgende Zusammensetzung haben: (Nuk1-Linker)-[T1;A1], (Nuk1-Linker)-[T2;A2], (Nuk1-Linker)-[T3;A3], (Nuk1-Linker)-[T4;A4] (in der Abbildung ist die Benennung des Linker ausgelassen).
Die Anwesenheit von drei von vier zu erwartenden Zielsequenzen wird mittels Bindung an die feste Phase und Visualisierung erreicht. Die Bindung erfolgt an die Bindungspartner erreicht, die adressierbare Positionen an der festen Phase haben.

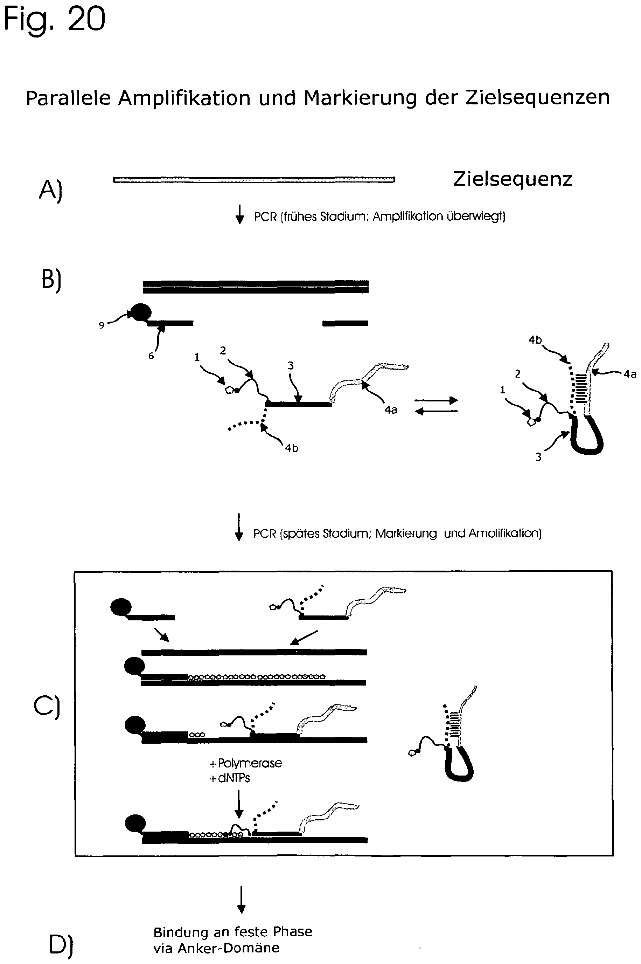

In einer weiteren Ausführungsform werden Nuk-Makromoleküle bei einer PCR verwendet, die eine Target-Domäne, eine Anker-Domäne und einen Antagosnisten zur Anker-Domäne einschließen (Fig. 20). Die Struktur des Nuk-Makromoleküls und die zyklischen Reaktioinsbedingungen werden dermassen gewählt, dass während der Reaktion die Target-Domäne der Nuk-Makromoleküle an die Zielsequenz binden kann. Die Bedingungen der Reaktion werden so gewählt, dass die Anker-Domäne der frei in der Lösung befindlichen Nuk-Makromoleküle während der Reaktion im offenen oder blockierten Zustand vorliegen kann, bevorzugt aber im offenen Zustand. Nach dem Einbau von Nuk-Makromolekülen liegt die Anker-Domäne im offenen zustand. Dies wird beispielsweise durch die räumliche Separation der Anker-Domäne und des Antagonisten durch die hybridisierte Target-Domäne erreicht. Nach der Markierungsreaktion wird die Temperatur herabgesetzt, so dass bei nicht eingebauten Nuk-Makromolekülen der Antagonist die Anker-Domäne binden und somit blockieren kann. Die Anker-Domänen der eingebauten Nuk-Makromoleküle bleiben offen und fähig zur Bindung an die feste Phase. Dieses Gemisch wird in Kontakt mit fester Phase gebracht und unter Bedingungen inkubiert, die eine Bindung von Anker-Domänen der eingebauten Nuk-Makromoleküle an die immobilisierten Bindungspartner zulassen. Da die Anker-Domänen der nicht eingebauten Nuk-Moleküle blockiert sind, interferrieren sie nicht mit der Bindung der markierten Zielsequenzen.

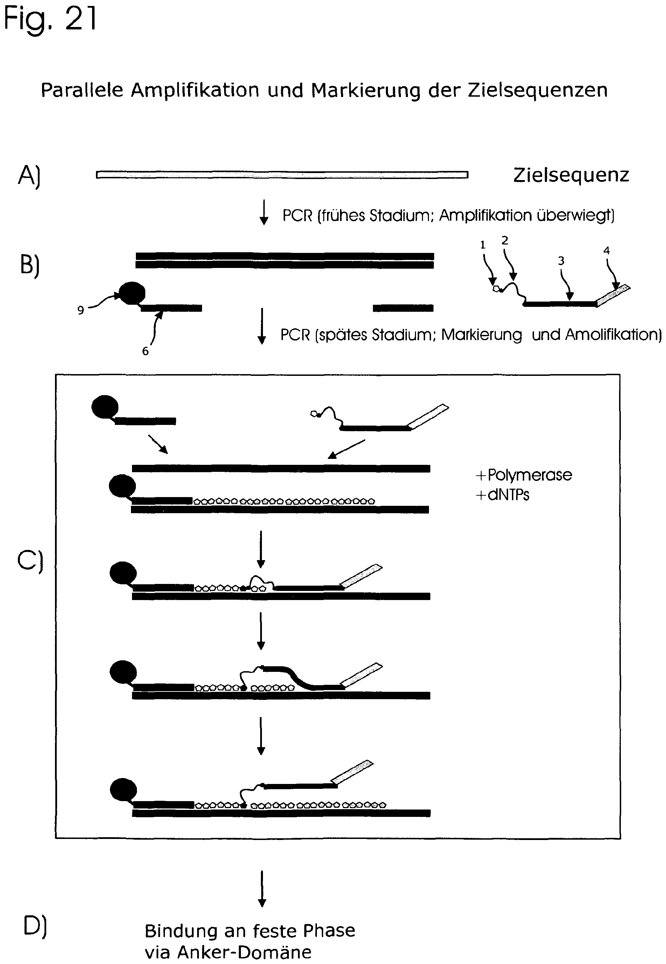

In einer weiteren Ausführungsform wird eine Polymerase in der PCR-Reaktion eingesetzt, die eine Strangverdrängung (engl.: "strand-displacement") durchführen kann (Fig. 21). Es werden Nuk-Makromoleküle eingesetzt, die eine Target-Domäne und eine Anker-Domäne einschließen. Während der PCR erfolgt die Bindung und Einbau von Nuk-Makromolekülen in die PCR-Fragmente der Zielsequenz. Da die Polymerase in der Lage ist, die Target-Domäne von der Zielsequenz zu verdrängen, werden weitere natürliche dNTPs eingebaut, bzw. weitere Nuk-Makromoleküle.
Die markierten PCR-Fragmente können entsprechend an eine feste Phase gebunden werden. In einer solchen Reaktion kann beispielsweise eine Art Nuk-Makromoleküle eine Target-Domäne [T1] und eine Anker-Domäne [A1] und eine andere Art der Nuk-Makromoleküle eine andere Target-Domäne [T2] und eine Signal-Domäne [S1] einschließen. Durch den Einbau beider Arten von Nuk-Makromoleküle werden PCR-Fragmente spezifisch mit einer Signal- und eine Anker-Domäne markiert.

In einer solchen Reaktion können auch andere Kompontenten, z.B. markierte Primer, Hybridisierungssonden, markierte Nukleotide eingesetzt werden, wie in anderen Abschnitten beschriben.
Es können eine oder mehrere Arten von Nuk-Makromolekülen an einer solchen Reaktion teilnehmen, wie in anderen Abschnitten beschriben.
Es können einzelne oder mehrere Zielsequenzen amplifiziert und markiert werden.
Die Detektion der Zielsequenz kann durch die Bindung an die feste Phase mit einer anschließenden Nachweisreaktion erfolgen, wie in anderen Abschnitten beschriben.

### 1.5.10.6

### Nachweis einer spezifischen Zielsequenz in einem Material

Bei einem Testverfahren, bei dem ein Nachweis einer oder mehrerer spezifischen Sequenzen aus einem Organismus in einem Untersuchungsmateial im Vordergrund steht, ist es üblich, dass interne Kontrollen, z.B. Amplifikationskontrollen, Bindungs- und Detektionskontrollen mitgeführt werden.

Viele Beispiele für diagnostische Tests sind einem Fachmann bekannt.

Die Strukturen der Nuk-Makromoleküle werden beispielsweise auf die Zielsequenzen der gesuchten Organismen, sowie auf die der Kontrollsequenzen nach Regeln angepasst, wie in anderen Abschnittenl beschrieben.

Es wird eine Markierungsreaktion durchgeführt, wie oben beschrieben.

Eine solche Markierungsreaktion schließt beispielweise mehrere Nuk-Maromoleküle mit Target-Domänen, die zu testenden Zielsequenzen komplementär sind. Die Zielsequenzen können mit verschiedenen Techniken vervielfältigt werden, beispielsweise mit einer Multiplex-PCR. In einer weiteren Ausführngsform schließt eine solche Reaktion entsprechend mehrere zielsequenzspezifische Primer oder Primer-Paare, die eine Vervielfältigung der gesuchten Zielsequenzen ermöglichen.

Nach der Amplifikation und Markierungsreaktion erfolgt die Detektion der markierten Zielsequenzen. Falls im Material eine Zielsequenz vorhanden war, wird sie über spezifische Markierung mit Nuk-Makromolekülen detektiert.

In einer Ausführungsform wird die Stärke des generierten Signals gemessen. Durch Korrelation dieser Stärke zu internen Kontrollen lassen sich Schlußfolgerungen auf die Menge der im Ausgangsmaterial vorliegenden Zielsequenzen ziehen.

Die einzelnen Reagenzien werden vorzugsweise in vorgemischter Form bereitgestellt, so dass lediglich die Zugabe des Materials zum Reaktionsstart notwendig ist.

Die bereitgestellten Reagenzien werden vorzugsweise als ein Kit bereitgestellt.

### 1.5.10.7

### Beispiele für Nachweis von Sequenzvarianten in einer spezifischen Zielsequenz (Unterscheidung von verwandten Zielsequenzen, z.B. SNP-Nachweis

Es müssen Sequenzvarianten einer Zielsequenz differenziert werden, die sich durch ein bzw. wenige Nukleotide unterscheiden. Ein Assay dafür kann in unterschiedlicher Weise konstruiert werden.

In einer Ausführungsform kann die Differenzierung durch einen Primer erfogen: es werden Primer in der Raktion verwendet, die nur bei einer bestimmten Sequenzvariante die Extension zulassen. Beispielsweise hat ein Primer ein diskriminierendes Nuklotid am 3'-Ende, das nur eine Variante der Sequenz bindet. Eine andere Variante der Sequenz würde ein Miss-Match bilden, und somit sehr schlecht oder gar nicht verlängert werden können. Die Nuk-Makromoleküle können dabei zur Bindung an die feste Phase (d.h. Nuk-Makromoleküle schließen mindestens eine Anker-Domäne ein) oder zum Nachweis (d.h. Nuk-Makromoleküle schließen mindestens eine Signal-Domäne ein) verwendet werden.

In einer weiteren Ausführungsform erfolgt eine sequenzspezifische bzw. sequenzselektive Amplifikation mit selektiven Primern, beispielsweise mittels einer PCR-Reaktion. Die Nuk-Makromoleküle können dabei zur Bindung an die feste Phase (d.h. Nuk-Makromoleküle schließen mindestens eine Anker-Domäne ein) oder zum Nachweis (d.h. Nuk-Makromoleküle schließen mindestens eine Signal-Domäne ein) verwendet werden.

In einer weiteren Ausführungsform kann die Differenzierung durch die Target-Domäne der Nuk-Makromoleküle erfolgen. Dabei können mehrere Nuk-Makromoleküle verwendet werden, deren Target-Domänen den unterschiedlichen Varianten der Zielsequenz komplementär sind (Fig. 16). Da die Bindung der perfekt komplementären Target-Domäne bevorzugt wird, werden solche Nuk-Makromoleküle bevorzugt in den wachsenden Strang eingebaut. Die Target-Domäne werden innerhalb einer Art von Nuk-Makromoleküle beispielswiese als Kombinationen aus mindestens einer Target-Domäne und einer Anker-Domäne, oder mindestens einer Target-Domäne und einer Signal-Domäne, oder einer Target-Domäne und einer Anker-Domäne und einer Signal-Domäne verwendet.

Durch Bindung an die feste Phase und eine Nachweisreaktion kann eine entsprechende Variante der Zielsequenz identifiziert werden.

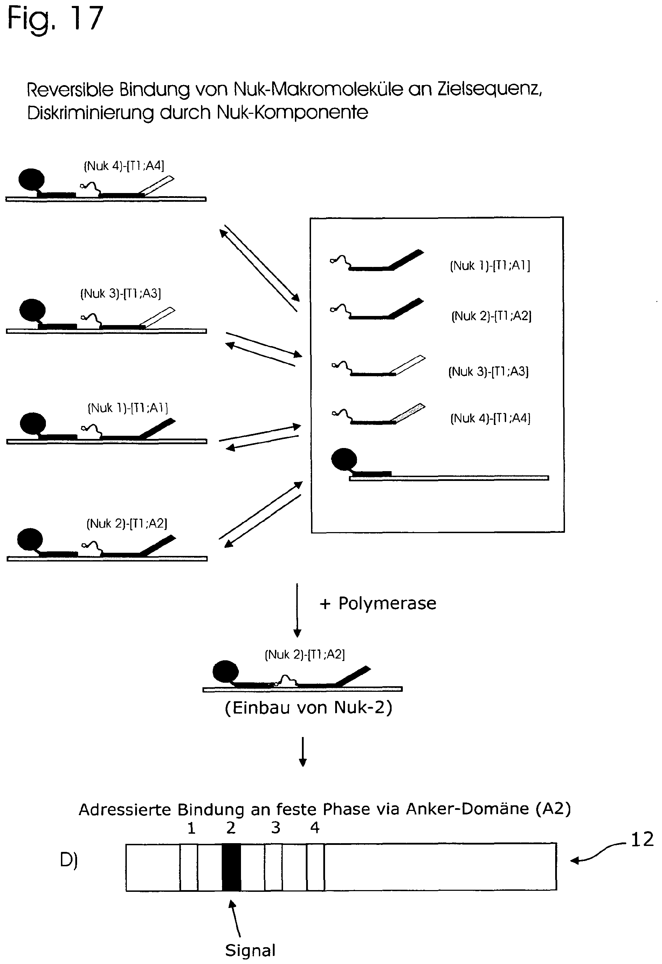

In einer weiteren Ausführungsform kann die Differenzierung durch den Einbau einer Nuk-Kompontente eines Nuk-Makromoleküls erfolgen. Dabei wird die Nuk-Komponente mit einer spezifischen Anker-Domäne (Fig. 17) oder Signal-Domäne kombiniert, die nach einer Bindung an die feste Phase eine spezifische Zuordnung der eingebauten Nuk-Kompontente ermöglicht. Beispielseise wird dATP mit Anker-Domäne 1, dCTP mit Anker-Domäne 2, dGTP mit Anker-Domäne 3, dUTP mit Anker-Domäne 4 kombiniert. In einer Ausführungsform schließen die verwendeten Nuk-Makromoleküle nur eine Anker-Domäne ein. In einer anderen Ausführungsform schließen sie mindestens eine Target-Domäne und eine Anker-Domäne ein. Die Signal-Domäne kann am verwendeten Primer gebunden sein.
Nach einer Markierungsreaktion erfolgt eine Bindung an die feste Phase mit adressierbaren Bindungspartnern. Durch bekannte Kodierung der Nuk-Komponente mittels Anker-Domäne/ Bindungspartner an der festen Phase, kann die Art der Nuk-Komponente bestimmt werden.

Auch Kombinationen aus einzelnen Verfahren sind möglich.

In einer vorteilhanften Ausführungsform schließen die Nuk-Makromoleküle mindestens eine Target-Domäne und mindestens eine Anker-Domäne ein. Die Detektion erfolgt durch den Einsatz von Nuk-Makromolekülen mit Signal-Domänen oder modifizierten Primern mit Signal-Domänen, oder Hybridisierungssonden mit Signal-Domänen oder konventionell markierten Nukleotiden.
In einer weiteren vorteilhanften Ausführungsform schließen die Nuk-Makromoleküle mindestens eine Target-Domäne und mindestens eine Signal-Domäne ein. Die Bindung an die feste Phase erfolgt durch den Einsatz von Nuk-Makromolekülen mit Anker-Domänen oder modifizierten Primern mit Anker-Domänen, oder Hybridisierungssonden mit Anker-Domänen, oder durch direkte Hybridisierung von markierten Zielsequenzen an die auf der festen Phase immobilisierten, komplementären Nukleinsäurestränge.

Inkubation von markierten Nukleinsäureketten mit der festen Phase führt zu Bindung von Anker-Domänen an die auf der festen Phase fixierten Bindungspartner. Dadurch werden auch die markierten Zielsequenzen bzw. deren Äquivalente an der festen Phase fixiert. Nach einer Nachweisreaktion kann eine optische Zurdnung der gebundenen Nukleinsäureketten zu den jeweiligen Positionen der Bindungspartner vorgenommen werden. Dadurch ist ein Rückschluß auf Vorliegen von bestimmten Zielsequenzen im Reaktionsansatz möglich.

Die Bindung der Nukleinsäureketten an die feste Phase kann dabei direkt über komplementäre, immobilisierte Nukleinsäuerekette oder indirekt, vermittelt über eine Anker-Domäne erfolgen.
In einer solchen Reaktion können auch andere Kompontenten, z.B. markierte Primer, Hybridisierungssonden, markierte Nukleotide eingesetzt werden, wie in anderen Abschnitten beschriben.
Es können eine oder mehrere Arten von Nuk-Makromolekülen an einer solchen Reaktion teilnehmen, wie in anderen Abschnitten beschriben.
Es können einzelne oder mehrere Zielsequenzen amplifiziert und markiert werden.
Die Detektion der Zielsequenz kann durch die Bindung an die feste Phase mit einer anschließenden Nachweisreaktion erfolgen, wie in anderen Abschnitten beschriben.

### 1.5.11 Zusammensetzung von Kit für Markierung und / oder Amplifikation von Nukleinsäuren.

Generell, ein oder mehrere Kits schließen Komponenten (z.B. Einzelsubstanzen, wie Nuk-Makromoleküle, Polymerase, dNTPs und Primer, oder deren Kompositionen, Reaktionsgemische, feste Phase) ein, die für die Durchführung von enzymatischen Einbaureaktionen mit erfindungsgemäßen Nuk-Makromolekülen notwendig sind und eine gegebenenfalls anschließende Analyse.
Die Zusammensetzung der Kits kann nach Anwendung variieren, wobei die Anwendugen können von einfacher Primer-Extensionsreaktion bis zu Amplifikation mit Markiertung, sowie einer anschließenden Analyse mittels fester Phase reichen.

Die Kits können wahlweise positive und / oder negative Kontrollen einschließen, Anleitungen zur Durchführung von Verfahren.

Optional können Kits Materialien und Reagentien zur Vorbereitung von Bestandteilen des Kits zur biochemischen Reaktionen oder des genetischen Materials einschließen, z.B. Komponenten für Zielsequenz-Präparation.
Auch Vorrichtungegen für eine Reinigung von markierten Nukleinsäureketten von überschüssigen Nuk-Makromolekülen können Bestandteile von Kits sein.

Die Kit-Komponenten sind üblicherweise in handelsüblichen Reaktionsgefäßen bereitgestellt, wobei das Volumen der Gefäße zwischen 0,2 ml und 1 I variieren kann. Es können auch Gefäßarrays, z.B. Mikrotiter-Platten mit Komponenten beladen sein, was eine automatische Zufuhr von Reagentien unterstützt.

Ein Kit zur Markierung von Nukleinsäureketten (Zielsequenzen) kann wahlweise mehrere in der Beschreibung offenbarte Komponenten zur Ausführung der Erfindung einschließen.

Einige Beispiele sind nachfolgend nochmals aufgefürt:
- Vorrichtung und Lösungen zur Isolierung von Zielsequenzen aus einem biologischen Material
- Eine oder mehrere Vorrichtungen zur Handhabung der Lösungen
- Ein oder mehrere Primer zur Amplifikation und Markierung von Zielsequenzen. Diese Primer können wahlweise mindestens eine Anker-Domäne und oder eine Signal-Domäne einschließen.
- Eine oder mehrere DNA Polymerasen oder RNA-Polymerasen oder reverse Transcriptasen.
   Beispielsweise Klenow Fragment Polymerase, Klenow exo minus Fragment, phi29 DNA Polymerase, T7 DNA Polymerase, Sequenase 2™, Taq Polymerase, Vent™ Polymerase, Deep Vent™ Polymerase, Vent™ exo minus DNA Polymerase, Deep Vent™ exo minus DNA Polymerase, Pwo DNA Polymerase, Tli DNA Polymerase, Tth DNA Polymerase, so genannte Hot-Start-Polymerasen, T7 RNA Polymerase, T4 RNA Polymerase, reverse Transcriptasen, z.B. Moloney Murine Lekemia Virus (M-MLV), Rous Sarcoma Virus (RSV), Avian Myeloblastosis Virus (AMV), Rous Assocciated Virus (RAV), Myeloblastosis Assocciated Virus (MAV), Human Immunodeficiency Virus (HIV).
   Die Polymerasen werden vorzugsweise in einer Aufbewahrungslösung bereitgestellt, Diese Aufbewahrungslösung kann beispielsweise folgende Substanzen einschließen:
   ∘ Puffer Tris-HCl, HEPES, Borat, Phosphat, Acetat (Konzentration liegen beispielsweise zwischen 10 mM und 200 mM)
   ∘ Salze, z.B. NaCl, KCl, NH4Cl, die Konzentrationen liegen beispielsweise zwischen 10 mM und 500 mM.
   ∘ PEG oder anderes inertes Polymer, z.B. Mowiol in Konzentration von 1 bis 50% (w/v)
   ∘ Glycerin in Konzentration zwischen 1% und 70%
   ∘ Reduzierende Agentien, z.B, DTT in Konzentration zwischen 0,1 und 50 mM
   ∘ Weitere Substanzen können in einer Aufbewahrungslösung enthalten sein, die zur Stabilität des Enzyms beitragen. Bespiele für solche Substanzen sind bekannt, siehe Beschriebung von Produkten von Enzym-Herstellern, z.B. Promega, Invitrogen, Roche usw.
- Eine oder mehrere Arten von Nuk-Makromolekülen (Nukleotid-Analoga), die als Säure oder als Salze vorliegen können (beispielsweise Natrium, Kalium, Ammonium oder Litium können als Ionen verwendet werden). Die Nuk-Makromoleküle können beispielsweise in trockender Form oder in Form einer Lösung bereitgestellt werden, z.B. im Wasser oder in einem Puffer, z.B. Tris-HCl, HEPES, Borat, Phosphat, Acetat, oder in einer Aufbewahrungslösung, die folgende Komponenten einzeln oder in Kombination einschließen kann:
   ∘ Puffer Tris-HCl, HEPES, Borat, Phosphat, Acetat (in Konzentration die beispielsweise zwischen 10 mM und 200 mM liegt)
   ∘ Salze, z.B. NaCl, KCl, NH4Cl, MgCl2,
   ∘ PEG oder anderes inertes Polymer, z.B. Mowiol in Konzentration von 1 bis 20% (w/v)
   ∘ Glycerin in Konzentration zwischen 1% und 50%
   ∘ Marker oder Marker-Einheiten von modifizierten Nuk-Makromolekülen, insbesondere in den Ausführungsformen, in denen zwischen Linker und Marker oder Marker und Kore-Komponente affine Kopplung besteht.
   ∘ DMSO
- Ein oder mehrere Reaktions-Puffer zur Durchführung von Amplifikation und / oder Markierungsreaktion und / oder Bindung an die feste Phase und / oder Detektion der Bindungsereignisse an der festen Phase
- Ein oder mehrere Sets von natürlichen Nukeltiden oder deren Analoga (z.B. dATP, dGTP, dCTP, dTTP, dUTP, dITP oder ATP, CTP, GTP, UTP)
- Ein oder mehrere Sets von Terminatoren (z.B. ddATP, ddGTP, ddCTP, ddTTP, ddUTP)
- Ein oder mehrere Arten von konventionell markierten Nukleotiden, z.B. Fluoreszenzmarkierte oder Biotin-markierte dUTP- oder dCTP-Analoga
- Eine oder mehrere zielsequenz-spezifische Hybridisierunssonden mit einer Anker-Domäne oder einer Signal-Domäne
- Weitere Enzyme, die Amplifikationsverfahren oder Markierungsverfahren unterstützen, Proteine und Cofaktoren, z.B. Helicase und ATP, Single Strand Binding Protein
- Ein oder mehrere Reaktionsgefäße zur Durchführung einzelner Reaktionen
- Feste Phase zur Bindung von markierten Zielsequenzen, z.B. ein Lateral-Flow-Device oder ein Array.
   Eine solche feste Phase kann beispielsweise Bindungspartner für Anker-Domänen der Nuk-Makromoleküle oder der Primer oder der Hybridisierungssonden einschließen. Eine solche feste Phase kann auch Oligonukleotide einschließen, die an die Zielsequenz binden können.
- Ein oder mehrere Reagenzien (z.B. Enzyme und chromogene Substrate oder Nanoteilchen) zur Detektion der Bindungsereignisse der markierten Zielsequenzen an der festen Phase
- Gegebenfalls Kontroll-Sequenzen zur Überprüfung der Durchführung einzelner Verfahrensschritte
- Eine Anleitung zur Durchführung und Auswertung von Reaktionen

### 1.5.12 Weitere Enzyme für Verwendung von Nuk-Makromolekülen

Nukleotide spielen als Monomere eine zentrale Rolle in unterschiedlichen Stoffwechselvorgängen, beispielsweise bei der Speicherung und Übertragung der genetischen Information in der Zelle ("Genes V" B. Lewin, 1994). Auch sind Nukleotide als Energieträger der Zelle bekannt (ATP, UTP), oder Signalvemittler (Messenger, GTP) der intrazellulären Signalvermittlung ("Biochemie und Pathobiochemie", G. Löffler, 2003). Aus diesem Grund werden Nukleotide und ihre Analoga als Therapeutika und Diagnostika eingesetzt.
Gekoppelt in Nukleinsäurepolymere (Nukleinsäureketten) stellen die Nukleotid-Monomere die Basis für die Informationsspeicherung in den lebenden Organismen dar.

Nuk-Makromoleküle haben das Potenzial, in unterschiedlichen Bereichen der Biotechnologie eine Anwendung zu finden.

Die Möglichkeit einer Kopplung der Nukleotide an ein Makromolekül unter Beibehaltung der Substrateigenschaften der Nukleotide eröffnet viele Wege auch für eine gezielte Adressierung der modifizierten Nukleotiden innerhalb eines Organismus oder einer Zelle, so dass Nuk-Makromoleküle ein neues grundsätzliches Model für Nukleotid-Prodrugs darstellen.

Die Hauptzielgruppe für den Gebrauch von Nuk-Makromolekülen stellen beispielsweise unterschiedliche Sorten von Polymerasen dar ("DNA Replication", Kornberg, 2. Ed. 1992), im einzelnen DNA-abhängige DNA-Polymerasen, RNA-abhängige DNA-Polymerasen, DNA-abhängige RNA-Polymerasen und RNA-abhängige RNA-Polymerasen. Dabei können sowohl thermostabile als auch thermolabile Polymerasen verwendet werden, wie beispielsweise Klenow-Polymerase oder Taq-Polymerase. Andere Beispiele für mögliche Polymerasen findet ein Fachmann in der hier zitierten Literatur.
Ein weiteres Beispiel für Enzyme stellen Transferasen, wie Terminale Deoxynucleotidyltransferase ("Molecular Cloning", Maniatis, 3. Ed. 2001), dar. Auch andere Enzyme und Proteine (beispielsweise Kinasen, Membranrezeptoren) die Nukleotide als Substrate), Energiequelle, Cofaktoren oder als Messenger-Substanzen akzeptieren können verwendet werden.

Enzyme unterscheiden sich in ihrer Fähigkeit, modifizierte Nukleotide als Substrate zu akzeptieren. Einem Fachmann sollte es naheliegend erscheinen, dass verschiedene funktionelle Tests eingesetzt werden müssen, um bestimmte Eigenschaften von Nukleotiden zu untersuchen und anzuwenden. Beispiele für unterschiedliche Testabläufe für die Markierung von Nukleinsäuren sind in H. Held et al. Nucleic Acid Research 2002, V. 30, S. 3857, M. Metzger et al. Nucleic Acid Research 1994, V. 22, 4259, M. Herrlein et al. Helvetica Chimica Acta 1994, V.77, S. 586 , B. Canard et al. PNAS 1995, V. 92, S. 10859 , Canard US Pat. 5798210, J. Hovinen et al. J. Chem. Soc. Perkin 1994, 1994, 211 und auch in anderen hier zitierten Patenten und Publikationen angegeben.

### 1.5.13 Hinweise für die Synthesen von Nuk-Makromolekülen

Die erfindungsgemäßen Nuk-Makromoleküle können auf unterschiedliche Art und Weise synthetisiert werden. Die Reihenfolge der Kopplungsschritte kann variieren. Beispielsweise kann zunächst eine Linker-Komponente an die Nuk-Komponente gekoppelt werden, anschließend wird die Marker-Komponente gekoppelt. Andererseits können ein oder mehrere Linker an die Marker-Komponente gekoppelt werden und anschließend der / die Nuk-Komponenten.

Die Kopplung zwischen einzelnen Komponenten der Nuk-Makromoleküle kann kovalent oder affin erfolgen. Wobei sowohl chemische als auch enzymatische Kopplung zur Verknüpfung einzelner Komponenten eingesetzt werden kann. Kopplungen an Amino- und Thiolgruppen dienen als Beispiele für kovalente Kopplungen (D. Jameson et al. Methods in Enzymology 1997, V. 278, S. 363-, "The chemistry of the amino group" S. Patai, 1968, "The chemistry of the thiol group" S. Patai, 1974). Biotin-Streptavidin-Bindung oder Hybridisierung zwischen komplementären Nukleinsäuresträngen oder Antigen-Antikörper-Wechselwirkungen stellen Beispiele für affine Kopplungen dar.

Die makromolekularen Marker bieten oft eine Vielfalt an Kopplungsmöglichkeiten. Ein makromolekularer Marker kann mehrere Kopplungsstellen für den Linker haben, beispielsweise mehrere Bindungsstellen für Biotin, wie es bei Streptavidin der Fall ist. Ein makromolekularer Marker kann auch mehrere Amino- bzw. Thiol-Gruppen aufweisen. Die Kern-Komponente des Markers kann mit unterschiedlicher Zahl von signalgebenden oder signalvermittelnden Einheiten modifiziert werden. Die genauen Verhältnisse zwischen Marker-Einheiten können variieren. Beispiele für die Modifikation von Polymeren mit Farbstoffen sind bekannt (Huff et al. US Pat. 5661040, D. Brigati US Pat. 4687732). Falls Nukleinsäuren als makromolekulare Marker eingesetzt werden, so können diese unterschiedliche Abschnitte zur Kopplung anderer Makromoleküle besitzen. An einen makromolekularen Marker können andere Makromoleküle gebunden werden, z.B. Enzyme.
Ein Nuk-Makromolekül kann makromolekulare Marker mit unterschiedlichen Detektionseigenschaften tragen, beispielsweise kann ein Nuk-Makromolekül sowohl mehrere Farbstoffmoleküle als auch Stellen zur affinen Bindung (z.B. durch Hybridisierung) weiterer Makromoleküle tragen.

Die Kopplung zwischen der Nuk-Komponenten und der Linker-Komponenten erfolgt vorzugsweise kovalent. Viele Beispiele für eine kovalente Kopplung an Nukleotide oder deren Analoga sind bekannt (Jameson et al. Method in Enzymology, 1997, v. 278, S. 363-, Held et al. Nucleic acid research, 2002, v. 30 3857-, Short US Pat. 6579704, Odedra WO 0192284). Dabei kann die Kopplung beispielsweise an Phosphat, Amino-, Hydroxy- oder Mercaptogruppen erfolgen.
Oft kann die Linker-Komponente in mehreren Schritten aufgebaut werden. Beispielsweise wird im ersten Schritt ein kurzer Linker mit einer reaktiven Gruppe an das Nukleotid oder Nucleosid angekoppelt, z.B. Propargylamin-Linker an Pyrimidine Hobbs et al. US Patent 5.047.519 oder andere Linker z.B. Klevan US Pat. 4,828,979, Seela US pat. 6211158, US pat. 4804748, EP 0286028, Hanna M. Method in Enzymology 1996 v.274, S.403, Zhu et al. NAR 1994 v.22 S.3418, Jameson et al. Method in Enzymology, 1997, v. 278, S. 363-, Held et al. Nucleic acid research, 2002, v. 30 3857-, Held et al. Nucleosides, nucleotides & nnucleic acids, 2003, v. 22, S. 391, Short US Pat. 6579704, Ward et al. US Pat 4711955, Engelhardt et al. US Pat 5241060 Taing et al. US Pat 6811979, Odedra WO 0192284, Herrlein et al. Helvetica Chimica Acta, 1994, V. 77, S. 586, Canard US. Pat. 5798210, Kwiatkowski US Pat. 6255475, Kwiatkowski WO 01/25247, Parce WO 0050642, Faulstich et al. DE 4418691, Phosphoroamidite (Glen Research Laboratories, http://www.glenres.com/, Trilink Biotechnologies, S. Agrawal "Protocols for oligonucleotide conjugation", Humana Press 1994, M.Gait "Oligonucleotide synthesis: a practical approach" IRL Press, 1990), Dissertation "Synthese basenmodifizierter Nukleosidtriphosphate und ihre enzymatische Polymerisation zu funktionalierter DNA", Oliver Thum, Bonn 2002. Einige Verbindungen kann man käuflich erwerben, z.B. bei Trilink Biotechnologies, Eurogentec, Jena Bioscience.
Diese kurzen Linker dienen als Kopplungseinheiten L oder deren Teile, und sind ein Bestandteil der Linker-Komponente im fertigen Nuk-Makromolekül.
Im zweiten Schritt kann die Kopplung des Nukleotides oder Nukleosides mit einem kurzen Linker an das Linker-Polymer erfolgen. Polymere mit reaktiven funktionellen Gruppen können käuflich erworben werden (Fluka).
Nach der Kopplung des Nukleotids an das Polymer kann nun die Marker-Komponente als letzter Schritt gekoppelt werden.

Oft ist es vorteilhaft an ein Nukleosid einen kurzen Linker zu koppeln, dann, falls erforderlich, dieses modifizierte Nucleosid in ein Nucleosid-Triphosphat umzuwandeln (Synthesen von Triphosphaten können beispielsweise in folgenden Literaturstellen gefunden werden: Held et al. Nucleosides, nucleotides & nnucleic acids, 2003, v. 22, S. 391, Faulstich et al. DE 4418691, T. Kovacs, L. Ötvös, Tetrahedron Letters, Vol 29, 4525-4588 (1988) oder Dissertation "Synthese basenmodifizierter Nukleosidtriphosphate und ihre enzymatische Polymerisation zu funktionalierter DNA", Oliver Thum, Bonn 2002).
Weitere Modifikationen können am Nukleosid-Triphosphat-Analog durchgeführt werden.

Vorstufen für modifizierte Nukleoside können sind beispielsweise bei Trilink Biotechnologies (San Diego, CA, USA) oder bei Chembiotech (Münster, Deutschland) kommerziel erhältlich.

Die Kopplung zwischen der Linker-Komponente und der Marker-Komponente kann beispielsweise zwischen reaktiven Gruppen an der Linker-Komponente und der Marker-Komponente erfolgen. Reagenzien für solche Kopplungen sind in "Chemistry of protein conjugation and cross-linking", S. Wang,1993, ISBN 0-8493-5886-8, ausführlich dargestellt. Die Verfahren zur Handhabung und zur Kopplung von mehreren Makromolekülen sind für unterschiedliche Typen von Makromolekülen auch in oben genannten Patenten dargestellt. Weitere Beispiele für Kopplungen an die und zwischen den Makromolekülen sind für Proteine in "Bioconjugation: protein coupling techniques for the biomedical sciences", M. Aslam, 1996, ISBN 0-333-58375-2; "Reactive dyes in protein an enzyme technology", D. Clonis, 1987, ISBN 0-333-34500-2; "Biophysical labeling methods in molecular biology" G. Likhtenshtein, 1993, 1993, ISBN 0-521-43132-8; "Techniques in protein modification" R. Lundblad, 1995, ISBN 0-8493-2606-0; "Chemical reagents for protein modification" R. Lundblad, 1991, ISBN 0-8493-5097-2; für Nukleinsäuren in "Molecular-Cloning", J. Sambrook, band 1-3, 2001, ISBN 0-87969-576-5, für andere Polymerarten "Makromoleküle, Chemische Struktur und Synthesen", Band 1, 4, H. Elias, 1999, ISBN 3-527-29872-X beschrieben.

Da die Marker-Komponente meistens viele Kopplungsstellen aufweist, können weitere Modifikationen an kompletten Nuk-Makromolekülen vorgenommen werden. Beispielsweise können überschüssige Amino-Gruppen abgeblockt werden oder durch weiterführende Modifikationen verändert werden.

Je nach Einsatzgebiet und Reaktionsbedingungen, unter denen Nuk-Makromoleküle verwendet werden, können unterschiedliche chemische Bindungsarten zwischen einzelnen Teilen der Makromoleküle von Vorteil sein. So eignen sich beispielsweise bei Verfahren mit Schritten mit höheren Temperaturen, wie z.B. bei Hybridisierung oder PCR, Nuk-Makromoleküle, die kovalente, thermostabile Bindungen zwischen einzelnen Teilen haben.

Nachfolgend sollen beispielhaft einige Möglichkeiten zur Synthese von Nuk-Makromolekülen dargestellt werden. Diese dienen nicht zur Einschränkung der möglichen Synthesewege und nicht zur Einschränkung der möglichen Nuk-Makromolekülstrukturen.

In einigen Ausführugnsformen werden Nuk-Makromoleküle mit Polyethylenglycol (PEG) als Linker-Komponente verwendet. Beispiele für die Kopplungen von PEG an andere Moleküle sind in "Poly(ethylene glycol) : chemistry and biological applications", 1997 beschrieben. Im einzelnen können sehr unterschiedliche reaktive Gruppen zur Kopplung eingesetzt werden: N-succinimidyl carbonate (US Pat.. 5,281,698, US Pat. 5,468,478), Amine (Buckmann et al. Makromol. Chem. V.182, S.1379 (1981), Zalipsky et al. Eur. Polym. J. V.19, S. 1177 (1983)), succinimidyl propionate und succinimidyl butanoate (Olson et al. in Poly(ethylene glycol) Chemistry & Biological Applications, 170-181, Harris & Zalipsky Eds., ACS, Washington, D.C., 1997; U.S. Pat. No. 5,672,662), Succinimidyl succinate (Abuchowski et al. Cancer Biochem. Biophys. v. 7, S.175 (1984), Joppich et al., Makromol. Chem. 1v. 80, S.1381 (1979), Benzotriazole carbonate (U.S. Pat. No. 5,650,234), Glycidylether (Pitha et al. Eur. J. Biochem. v. 94, S.11 (1979), Elling et al., Biotech. Appl. Biochem. v.13, S. 354 (1991), Oxycarbonylimidazole (Beauchamp, et al., Anal. Biochem. v.131, S. 25 (1983), Tondelli et al. J. Controlled Release v.1, S.251 (1985)), p-nitrophenyl carbonate (Veronese, et al., Appl. Biochem. Biotech., v.11, S.141 (1985); and Sartore et al., Appl. Biochem. Biotech., v.27, S.45 (1991)), Aldehyde (Harris et al. J. Polym. Sci. Chem. Ed. v.22, S. 341 (1984), US. Pat. 5824784, US. Pat. 5252714), Maleimide (Goodson et al. Bio/Technology v.8, S. 343 (1990), Romani et al. in Chemistry of Peptides and Proteins v.2, S.29 (1984)), and Kogan, Synthetic Comm. v.22, S.2417 (1992)), Orthopyridyl-disulfide (Woghiren, et al. Bioconj. Chem. v. 4, S. 314 (1993)), Acrylol (Sawhney et al., Macromolecules, v. 26, S.581 (1993)), Vinylsulfone (U.S. Pat. No. 5,900,461). Weitere Beispiele für Kopplungen von PEG an andere Moleküle sind in Roberts et al. Adv. Drug Deliv. Reviews v. 54, S. 459 (2002), US Pat. 2003124086, US Pat. 2003143185, WO 03037385, US Pat. 6541543, US Pat. 2003158333, WO 0126692 zu finden.

Andere ähnliche Polymere können in ähnlicher Art gekoppelt werden. Beispiele für solche Polymere stellen andere Poly(Alkylenglykole), Copolymere aus Ethylenglykol und Propyleneglykol, Poly(olefiniische Alkohole), Poly(Vinylpyrrolidone), Poly(Hydroxyalkylmethacrylamide), Poly(Hydroxyalkylmethacrylate), Poly(saccharide), Poly(x-Hydroxy-Säuren), Poly(Acrylsäure), Poly(Vinylalkohol).

Die Aufreinigung der Nuk-Komponenten der Nuk-Makromoleküle erfolgt mit konventionellen Mitteln der Nukleotidchemie: beispielsweise mit Kieselgel-Chromatographie in einem Wasser-Ethanol-Gemisch, HPLC-Verfahren (Ionenaustausch-Chromatographie in einem Salz-Gradienten und Reverse-Phase-Chromatographie in einem Wasser-Methanol-Gradienten). Für die Nukleotid-Aufreinigung optimierte Chromatographie-Säulen werden z.B. von der Firma Sigma-Aldrich angeboten. HPLC-Reinigung von Nukleotiden ist bevorzugt.
Die Aufreinigung von makromolekularen Linker-Komponenten und Marker-Komponenten kann durch Ultrafiltration, Gel-Elektrophorese, Gelfiltration und Dialyse erfolgen, siehe "Bioconjugation: protein coupling techniques for the biomedical sciences", M. Aslam, 1996, ISBN 0-333-58375-2.

Die Masse der Nuk-Makromoleküle unterscheidet sich wesentlich von der Masse der Nukleotide. Aus diesem Grund ist es vorteilhaft, bei den Endreinigungsschritten die Ultrafiltration einzusetzen. Da für die Nuk-Makromoleküle nur eine durchschnittliche Masse bereichnet wird, eignet sich Ultrafiltration auch als analytische Methode zu Trennung von Syntheseprodukten.

Zur Charakterisierung der Nuk-Makromoleküle können unterschiedliche Methoden der makromolekularen Chemie angewendet werden, z.B. UV-Vis-Spektroskopie, Fluoreszenzmessung, Massenspektroskopie, Fraktionierung, Größenausschlußchromatographie, Ultrazentrifugation und elektrophoretische Techniken, wie IEF, denaturierende und nicht denaturierende Gel-Elektrophorese ("Makromoleküle, Chemische Struktur und Synthesen", Band 1, 4, H. Elias, 1999, ISBN 3-527-29872-X, "Bioconjugation: protein coupling techniques for the biomedical sciences", M. Aslam, 1996, ISBN 0-333-58375-2).

Beispiele für Kopplungen von einzelnen Komponenten von Nuk-Makromolekülen sind in Anmeldungen Cherkasov et al WO 2005044836, Cherkasov et al WO2006097320, Cherkasov et al WO 2008043426, Cherkasov et al DE 10356837, Cherkasov et al DE 102004009704 angegeben. Nuk-Makromoleküle werden von der Firma Genovoxx GmbH als Auftragssynthese entwickelt und vermarktet.

### 1.5.14 Beispiele von Synthesen von Nuk-Makromolekülen

Nachfolgend sind Synthesen von Nuk-Makromolekülen beschrieben, bei den die Target-Domäne aus DNA besteht. Es sind viele Methoden für die kovalente Kopplung an die DNA bekannt. Die Kopplung kann an verschiedene Positionen in der Nukleinsäurekette (5'-position, 3'-Position, innere Abschnitte) erfolgen. Mehrere Markierungen pro eine sind ebenfalls möglich. Die Modifikation kann durch chemische oder auch enzymatische Reaktionen erfolgen. Einerseits, kann kann die Kopplung einer Substanz bereits während der chemischen / enzymatischen Synthese von Nukleinsäuren durchgeführt werden (z.B. durch Nutzung von Phosphoroamiditen oder durch Nutzung modifizierter Nukleotide und einer Polymerase oder durch Nutzung von Ligase-Reaktion). Andererseits, kann die Kopplung über einen oder mehrere Zwischenschritte, z.B. durch Einführung einer reaktiven Gruppe, erst nach der Synthese erfolgen.

Nachfolgend werden einige Beispiele vorgestellt, die zur Demonstrationszwecken einige dieser Varianten beschreiben.

### Synthese von Nuk-Linker-Komponenten mit reaktive Gruppen.

Die Kopplung von Nuk-Komponenten und Marker-Komponenten, z.B. Oligonukleotiden, kann durch viele Methoden erreicht werden. Beispielsweise, sind viele Methoden bekannt, wie man zwei Strukturen, die reaktive Amino-Gruppen vorweisen, mittels eines Cross-Linkers koppelt. Mit einer oder mehreren Amino-Gruppen modifizierte Oligonukleotide können kommerziell erworben werden. Die Amino-Gruppe kann wahlweise am 5'-Ende, 3'-End, oder auch im internen Bereich eines Oligonukleotides vorkommen. In folgenden Beispielen sind amino-reaktive Nuk-Komponenten beschrieben, die als Vorstufen bereitgestellt werden. Solche amino-reaktive Nukleotide können an die Oligonukleotide gekoppelt werden.

### 1.5.14.1 Synthese von dUTP-PEG(8)-NH2

5 mg dUTP-AA (Aminoallyl-dUTP, von Jena Biosciences) wurde in Phosphat-Puffer, pH 8.0, gelöst bis zur Konzentration von 50 mmol/l. Fmoc-PEG(8)-NHS (erhalten von Iris-Biotech GmbH) wurde in DMSO bis zur Konzentration von 100 mmol/l gelöst.
Die Lösung mit Fmoc-PEG(8)-NHS (ca. 8 Äquivalente) wurde zur Lösung von dUTP-AA zugegeben, bis dUTP-AA komplett umgesetz wurde (DC-Kontrolle), die Kopplung erfolgt an der Aminogruppe des Linkers.
Die Aufreinigung von dUTP-PEG(8)-Fmoc erfolgte über DEAE-HPLC in einem Tris-HCl Puffer und NaCl-Gradient. Die Fraktionen mit Produkt wurden gesammelt und über RP-C-18 Säule mit Wasser-Ethanol-Gradient weiter gereinigt. Das Eluat mit dUTP-PEG(8)-Fmoc wurde einrotiert, getrocknet und in wasserfreiem DMF gelöst.
Zur Lösung von mit dUTP-PEG(8)-Fmoc in DMF wurde Pipiridin bis zur Konzentration von 1% zugegeben. Das Produkt, dUTP-PEG(8)-NH2 wurde präzipitiert und getrocknet.

Auch dUTP-PEG3400-NH2 und dUTP-PEG5000-NH2 wurden in ähnlicherweise erhalten, wobei Fmoc-PEG-3400-NHS und Fmoc-PEG-5000-NHS (erhalten von Iris-Biotech GmbH) verwendet wurden. Materialien und Methoden siehe Cherkasov et al. WO 2005 044836.

An die Amino-Gruppe am Linker können nun weitere reaktive Gruppe gekoppelt werden. Zur Herstellung eines Amino-Reaktiven-Derivates können nun verschiedene Cross-Linker verwendet werden. Einem Fachmann sind viele Beispiele von Cross-Linkern bekannt.

### 1.5.14.2 Synthese von dUTP-PEG(8)-NHS

Eine Lösung von dUTP-PEG(8)-NH2 (5mmol/l) in DMF wird mit einem Überschuß von Glutarat-(NHS)2 (gelöst in DMF) versetzt. Das Produkt, dUTP-PEG(8)-NHS, wurde präzipitiert, mit DMF gewaschen und getrocknet. Die Aminogruppe am Linker wird modifiziert.
In ähnlicher Weise wurden dUTP-PEG(3400)-NHS, dUTP-PEG(5000)-NHS erhalten. Phenyl-di-Isothiocyanate kann in einer anderen Ausführungsform dieses Beispiels anstatt von Glutarat-(NHS)2 verwendet werden, wobei dUTP-(PEG)8-ITC oder dUTP-PEG(3400)-ITC resultierten, entsprechend.
Ein Fachmann erkennt, dass auch weitere Cross-Linker mit anderen Funktionalitäten, wie z.B. andere Linker-Längen, andre amino-reaktive Gruppen können verwerdet werden. Ebenso können weitere Funktionalitäten, wie Spaltbarkeit des Linkers durch entsprechende Wahl des Cross-Linkers eingeführt werden, z.B. bei Verwendung von reduktiv oder oxidativ spaltbaren Linkern (z.B. Dithiobispropionsäure-(NHS)2 (DTBP), Tartrate-(NHS)2). Viele Cross-Linker sind kommerziell erhältlich, z.B. von Thermo Scientific oder Sigma-Aldrich oder IRIS GmbH.

### 1.5.14.3 Synthese von dUTP-Glutarat-NHS

5 mg dUTP-AA (Aminoallyl-dUTP, von Trilink Biotechnologies), pH 7.0 wurden getrocknet und im trockenen DMSO bis zur berechneten Konzentration von 50 mmol/l suspendiert. Glutarat-(NHS)2 (erhalten von Thermo Scientific Deutschland) wurde in DMSO bis zur Konzentration von 300 mmol/l gelöst.
Die dUTP-AA Suspension wurde zur Glutarat-(NHS)2 Lösung zugegeben und 2 h bei 37°C unter kräftigem Rühren inkubiert, bis die Lösung durchsichtig wurde. Die Umsetzung von dUTP-AA wurde mittels DC-Kontrolle kontrolliert.
Die Aufreinigung von dUTP-Glutarat-NHS erfolgte mittels Präzipitation durch Diethyläther /DMF-Gemisches (v:v 90:10). Das Pellet enthält das Produkt. Das Produkt wurde in DMSO gelöst und eingefroren.

In änlicher Art und Weise können weitere dUTP-R-X Analoga synthetisiert werden, wobei (R) einen beliebigen Linker und (X) eine beliebige reaktive Gruppe darstellen kann. Die reaktive Gruppe kann beispielsweise mit Amino-Gruppen oder Thio-Gruppen oder Carboxy-Gruppen reagieren. Beispiele für weitere käuflich erhältliche kurze Linker (Cross-Linker) sind im Cross-Linker Guide von Thermo Scientific (www.piercenet.com) präsentiert. Diese Linker können beispielsweise einen PEG-Abschnitt einschließen, z.B. PEG5 oder PEG9 (Thermo Scientific: BS(PEG)5 oder BS(PEG)9). Diese Linker können auch eine spaltbare Verbindung einschließen, z.B. eine reduktiv spaltbare Bindung (z.B. Dithiobispropionsäure-(NHS)2) oder eine oxidativ spaltbare Bindung (z.B. Tartrat-(NHS)2).

Auch andere Nukleotidanaloga (z.B. N-(6-Aminohexyl)-dCTP (erhältlich von AmpliChem), N6 (6-Amino)hehyl-dATP, 5-Propargylamino-dCTP, 7-Propargylamino-7-deaza dATP, 7-Propargylamino-7-deaza dGTP, erhältlich von Jena Biosience) können in ähnlicher Weise modifiziert werden.

NHS-Gruppe am Linker kann nun an Amino-Gruppe eines anderen Moleküls, z.B. eines Oligonukleotids gekoppelt werden.

### Kopplung von amino-reaktiven Nuc-Komponenten an das Oligonukleotid.

Eine solche Kopplung ist einem Fachmann bekannt: Oligonukleotide mit einer oder mehreren Aminogruppen werden mit einem Überschuß von amino-reaktiven Komponenten, z.B. mit NHS-Derivaten oder Isothiocyanat-Derivaten in einer Lösung zusammengebracht. Die Aufreinigung des modifizierten Oligonukleotides kann beispielsweise mittels HPCL (DEAE und RP) erfolgen und ist ebenfalls einem Fachmann bekannt.
Nachfolgend werden einige Beispiele der Herstellung von Nuk-Makromolekülen beschrieben, die eine Nuk-Komponente, einen Linker und ein Oligonukleotid enthalten. Alle Oligonukleotide wurden von MWG-Operon Deutschland synthetisiert.

### 1.5.14.4: dU-PEG(8)- [T1,A1]-TAMRA

Dieses Beispiel zeigt die Kopplung von Nuc-Komponente am 5'-Ende eines sequenzspezifischen Oligonukleotides mit einer Target-Domäne und einer Anker-Domäne. Das Oligonukleotid, Target-Domäne-1, Anker-Domäne 1 -TAMRA, abgekürzt als [T1,A1]-TAMRA, wurden von MWG synthetisiert (siehe Liste der Sequenzen):
Target-Domäne-1, Anker-Domäne 1-TAMRA; abgekürzt als [T1,A1]-TAMRA
NH2- cgtattaccgcggctgctggcacAAAAAAAAAAAAAAAAAAAAAAAAA -TAMRA

Diese Sequenz enthält eine Target-Sequenz: cgtattaccgcggctgctggcac und eine Anker-Sequenz AAAAAAAAAAAAAAAAAAAAAAAAA. Am 5'-Ende enthält dieses Oligonukleotid eine Amino-Gruppe, die über einen Spacer-C6 gekoppelt ist. Am 3'-Ende ist TAMRA-Reporter als Fluoreszenz-Marker gekoppelt.

Das Oligonukleotid wurde in einem Phosphat-Puffer, pH 8.0, gelöst. Zu dieser Lösung wurde Überschuß von dUTP-PEG(8)-NHS (5 mmol/l, in DMF) zugegeben. Die Reaktion verlief bei RT in guten Ausbeuten. Die anschließende Reinigung des Produktes erfolgte über DEAE-Säule und RP-C18 Säule. Das Produkt (dU-PEG(8)-[T1,A1]-TAMRA) wurde getrocknet und anschließend in Wasser in 50 µmol/l Konzentrationen gelöst und eingefroren.

In ählicher Art und Weise wurden auch andere Nuk-Makromoleküle synthetisiert (Tabelle 1).

### 1.5.14.5: dU-PEG(4)- [T1,A1]-TAMRA

Synthese von Nuk-Makromoleküls mit "Click-Chemie". (Komponenten erhalten von BaseClick GmbH, Deutschland).
Dieses Beispiel zeigt die Kopplung von Nuc-Komponente am 5'-Ende eines sequenzspezifischen Oligonukleotides mit einer Anker-Funktionalität am 3'Ende. Das Oligonukleotid [T1,A1]-TAMRA, Sequenz siehe Beispiel 1.5.14.4 und in der Liste der Sequenzen.

Das Oligonukleotid wurde in einem Phosphat-Puffer, pH 8.0, gelöst. Zu dieser Lösung wurde Überschuß von NHS-PEG4-N3 (10 mmol/l, in DMSO, erworbein bei BaseClick GmbH, Germany) zugegeben. Die Reaktion verlief bei RT in guten Ausbeuten. Die anschließende Reinigung des Produktes erfolgte über DEAE-Säule und RP-C18 Säule. Das Produkt wurde getrocknet und anschließend in Wasser in 1 mmol/l Konzentrationen gelöst. Das Oligonukleotid trägt nun eine Azid-Gruppe, die über einen kurzen PEG-Linker gekoppelt ist N3-PEG4-Oligonukleotid.

Nun erfolgt die Kopplung der Nuk-Komponente:
5 µl dU-Alkyne-C8 (10 mmol/l erworbein bei BaseClick GmbH, Germany) in Wasser gelöst wurde zu 5 µl 1 mmol/l N3-PEG4-Oligonukleotid zugegeben und anschließend 10 µl DMSO/t-Butanol dazu gegeben. Anschließend wurden 6 µl frisch vorbereiteten Click-Lösung (1 V 0.1 M CuBr in DMSO/t-Butanol und 2 V 0.1 M TBTA in DMSO/t-Butanol) zugegeben. Die Reaktion verlief 24 hr bei RT.
Die anschließende Reinigung des Produktes erfolgte über DEAE und RP-C18. Das Produkt wurde getrocknet und anschließend in Wasser in 100 µmol/l Konzentrationen gelöst. Das Produkt (dU-PEG(4)- [T1, A1]-TAMRA) enthält eine Nuk-Koomponente, die über einen kurzen Linker an das Oligonukleotid gekoppelt ist.

Eine Azid-Funktionalität oder Alkyne-Funktionalität kann an verschiedenen Stellen in einem Nukleotid (z.B. an der Base oder am Zucker) oder einem Oligonukleotid (z.B. in der Mitte oder am 3'-Ende oder am 5'-Ende) eingeführt werden,. Auch mehrere Funktionalitäten können eingefügt werden. Durch die Kopplung einer entsprechenden Nuk-Komponente können Nuk-Makromoleküle mit einer oder mehreren Nuk-Komponenten erhalten werden.

Beispiele für die Kopplung von Nukleinsäuremolekülen mittels Click-Chemie sind in folgender Literatur angegeben:
A. H. El-Sagheer, T. Brown, Chem. Soc. Rev. 2010, 39, 1388-1405. Click Chemistry with DNA.
J. Lahann, Wiley VCH 2009. Click Chemistry for Biotechnology and Materials Science.
F. Morvan, A. Meyer, G. Pourceau, S. Vidal, Y. Chevolot, E. Souteyrand, J.-J. Vasseur Nucleic Acids Symposium Series 2008, 52, 47-48. Click Chemistry and Oligonucleotides: How a simple reaction can do so much.
P. M. E. Gramlich, C. T. Wirges, A. Manetto, T. Carell, Angew. Chem. Int. Ed. 2008, 47, 8350-8358. Postsynthetic DNA Modification through the Copper-Catalyzed Azide-Alkyne Cycloaddition Reaction.
P. M. E. Gramlich, S. Warncke, J. Gierlich, T. Carell, Angew. Chem. Int. Ed. 2008 3442-3444. Click-Click-Click: Single to Triple Modification of DNA.
P. M. E. Gramlich, C. T. Wirges, J. Gierlich, T. Carell, Org. Lett. 2008, 10, 249-251. Synthesis of Modified DNA by PCR with Alkyne-Bearing Purines Followed by a Click Reaction.
F. Seela, V. R. Sirivolu, P. Chiteppu, Bioconjugate Chem. 2008, 19, 211-224. Modification of DNA with Octadiynyl Side Chains: Synthesis, Base Pairing, and Formation of Fluorescent Coumarin Dye Conjugates of Four Nucleobases by the Alkyne-Azide "Click" Reaction.
X. Ming, P. Leonard, D. Heindl, F. Seela, Nucleic Acids Symp. Ser., 2008, 52, 471. Azide-alkyne "click" reaction performed on oligonucleotides with the universal nucleoside 7-octadiynyl-7-deaza-2'-deoxyinosine.
M. Meldal, C. W. Tornoe, Chem. Rev. 2008, 108, 2952-3015. Cu-Catalyzed Azide-Alkyne Cycloaddition.
T. Ami, K. Fujimoto, Chembiochem. 2008, 9, 2071-4. Click Chemistry as an Efficient Method for Preparing a Sensitive DNA Probe for Photochemical Ligation.
C. D. Hein, X. Liu, D. Wang, Pharm. Res. 2008, 25, 2216-2230. Click Chemistry, A Powerful Tool for Pharmaceutical Sciences.
B. Le Droumaguet, K. Velonia, Macromol. Rapid Commun. 2008, 29, 1073-1089. Click Chemistry: A Powerful Tool to Create Polymer-Based Macromolecular Chimeras.
C. T. Wirges, P. M. E. Gramlich, K. Gutsmiedl, J. Gierlich, G. A. Burley, T. Carell, QSAR Comb. Sci. 2007, 26, 1159 -1164. Pronounced Effect of DNA Hybridization on Click Reaction Efficiency.
J. Gierlich, K. Gutsmiedl, P. M. E. Gramlich, A. Schmidt, G. Burley, T. Carell, Chem.Eur. J. 2007, 13, 9486-9494. Synthesis of Highly Modified DNA by a Combination of PCR with Alkyne-Bearing Triphosphates and Click Chemistry.
D. M. Hammond, A. Manetto, J. Gierlich, V. A. Azov, P. M. E. Gramlich, G. A. Burley, M. Maul, T. Carell, Angew. Chem. Int. Ed. 2007, 46, 4184-4187. DNA Photography: An Ultrasensitive DNA-Detection Method Based on Photographic Techniques.
F. Seela, V. R. Sirivolu, Nucleosides, Nucleotides and Nucleic Acids, 2007, 26, 597. Nucleosides and Oligonucleotides with Diynyl Side Chains: The Huisgen-Sharpless Cycloaddition "Click reaction" Performed on DNA and their Constituents.
M. Fischler, U. Simon, H. Nir, Y. Eichen, G. A. Burley, J. Gierlich, P. M. E. Gramlich, K. Gogoi, M. V. Mane, S. S. Kunte, V. A. Kumar, Nuclei Acids Res. 2007, 35, 139. A versatile method for the preparation of conjugates of peptides with DNA/PNA/analog by employing chemo-selective click reaction in water.
F. Seela, V. Ramana Sirivolu, Chemistry & Biodiversity, 2006, 3, 509. DNA Containing Side Chains with Terminal Triple Bonds: Base Pair Stability and Functionalization of Alkynylated Pyrimidines and 7-Deazapurines.
J. Gierlich, G. A. Burley, P. M. E. Gramlich, D. M. Hammond, T. Carell, Org. Lett. 2006, 8, 3639-3642. Click Chemistry as a Reliable Method for the High-Density Postsynthetic Functionalization of Alkyne-Modified DNA.

Übersicht über die synthetisierten Nuk-Makromoleküle

**Tabelle 1**

| **Name des makro-Nukleotides (Konjugat)** | **Nuk-Komponente** | **Position der Nuk-Komponente** | **Linker-Komponente** | **Target-Domäne [Tn]** | **Anker-Domäne [An]** |
|---|---|---|---|---|---|
| dU-P4-[T1,A1] | dU | 5'-Ende | PEG 4 | Target-Domäne 1 | Anker-Domäne 1 |
| dU-P8-[T1,A1] | dU | 5'-Ende | PEG 8 | Target-Domäne 1 | Anker-Domäne 1 |
| dU-P3000-[T1,A1] | dU | 5'-Ende | PEG 3000 | Target-Domäne 1 | Anker-Domäne 1 |
| dU-P8-SS-P8-[T2] | dU | 5'-Ende | PEG8-SS-PEG8 | Target-Domäne 2 | |
| dU-P3000-[T2] | dU | 5'-Ende | PEG 3000 | Target-Domäne 2 | |
| dU-P3000-[T2] | dU | 5'-Ende | PEG 5000 | Target-Domäne 2 | |
| dU-P3000-[T2]-3' | dU | 3'-Ende | PEG 3000 | Target-Domäne 2 | |
| dU-P5000-[T2]-3' | dU | 3'-Ende | PEG 5000 | Target-Domäne 2 | |
| dU-Glut-[T3,A3] | dU | 5'-Ende | Glutarat | Target-Domäne 3 | Anker-Domäne 3 |
| dC-Glut-[T3,A3] | dC | 5'-Ende | Glutarat | Target-Domäne 3 | Anker-Domäne 3 |
| dU-Tart-[T1,A1] | dU | 5'-Ende | Tartrat | Target-Domäne 1 | Anker-Domäne 1 |
| dU-DTBP-[T1,A1] | dU | 5'-Ende | Dithiobispropionat | Target-Domäne 1 | Anker-Domäne 1 |

Die für die Synthesen verwendeten Oligonukleotide mit einer Target-Domäne und optionsweise einer Anker-Domäne sind in der Liste der Sequenzen angeführt.

### 1.5.15 Beispiele von enzymatischen Markierungsreaktionen von Zielsequenzen mit Nuk-Makromolekülen

Alle Polymerasen wurden von kommerziellen Anbietern bezogen (z.B. New England Biolabs oder Promega).

### Einbau von Nuk-Makromolekülen in einer Primer-abhängigen Reaktion:

Die Substrateigentschaften von synthetisierten Nuk-Makromolekülen für mehrere Polymerasen wurden in Einbaureaktionen getestet. Eine Einbauraktion wurde durchgeführt in einem Einbaupuffer 1 (50 mmol/l Tris-HCL, 50 mmol/l NaCl, 5 mmol/l MgCl2, 10% Glycerin) oder Einbaupuffer 2 (1x Reaktionspuffer (1x ThermoPol für thermophile Polymerasen) von New England Biolabs).
Es wurden verschiedene Polymerasen getestet (z.B. Klenow exo minus, Taq Polymerase, Vent exo minus Polymerase, Termonator, Terminator II, Deep-Vent exo minus, Sequenase, Tth-Polymerase, Tli-Polymerase).
Reaktionen wurden in einem Gesamtvolumen von 10 bis 20 µl durchgeführt. Folgende Konzentrationen von Komponenten wurden typischerweise verwendet:
Primer (markiert mit einem Fluoreszenzfarbstoff oder unmarkiert) 0,1 bis 1,5 µmol/l / Matrize (M1 - M10) von 1 nmol/l bis 1,5 µmol/l, Nuk-Makromoleküle 0,1 bis 10 µmol/l, natürliche Nukleotide (dATP, dCTP, dGTP, dTTP) wurden in Konzentrationen von 0,1 µmol/l bis 10 mmol/l (die verwendeten Konzentrationen sind in jeweiligem Experiment angegeben).

Die Konzentrationen von Polymerasen erhalten von kommerziellen Anbietern wurden willkürlich als 1x Konzentration bezeichnet. Die davon angefertigten Verdünnungsstufen von Polymerasen (z.B. 1:10 bis 1:1000) beziehen sich auf diese Ausgangskonzentration und sind im jeweiligen Experiment angegeben.
Die feste Phase wurde durch Streptavidin-Magnetic-Beads repräsentiert, die einen Bindungspartner für die jeweilige Anker-Domäne einschließen, z.B. dT48 für eine dA25-Anker-Domäne.
Die Analyse der verlängerten Fragmente erfolgte mittels Gel-Elektrophorese in einem 10% Polyacrylamid-Gel unter denaturierenden Bedingungen (ca. 85-90°C). Die Gel-Abbildungen wurden mittels einer Gel-Dokumentationsanlage gemacht.

Zusammenfassen wurden folgende Eigenschaften von Nuk-Makromolekülen ermittelt:
- Die synthetisierten Nuk-Makromoleküle (einschließlich mindestens einer Nuk-Komponente, eines Linkers, mindestens einer Target-Domäne, mindestens einer Anker-Domäne, mindestens einer Signal-Domäne) dienen als Substrate für DNA Polymerasen und können in einer Primer-Extension-Reaktion an den Primer in einer matrizenabhängigen Reaktion an entsprechenden, für die jeweilige Nuk-Koomponente komplementären Stellen gekoppelt werden.
- Nuk-Makromoleküle können von thermolabilen und thermostabilen Polymerasen als Substrate akzeptiert werden.
- Die erfindungsgemäßen Nuk-Makromoleküle können in einer primer-abhängigen Markierungsreaktion, z.B. bei einer Primer-Extension, eingesetzt werden.
- Nuk-Makromoleküle konkurrieren mit entsprechenden natürlichen Nukleotiden um den Einbau in den wachsenden Strang an den jeweiligen komplementären Stellen der Matrize. In Abhängigkeit davon, ob die Target-Domäne der Nuk-Makromoleküle an die jeweilige Matrize hybridisert ist oder nicht unterscheidet sich die Fähigkeit der Nuk-Makromoleküle um den Einbau zu konkurrieren:
   ∘ Der Einbau von an die jeweilige Matrize hybridisierten Nuk-Makromolekülen ist deutlich begünstig. Es bedarf bis zur 10 mmol/l Konzentration an natürlichen Nukleotiden, um den Einbau von einem hybridisierten Nuk-Makromolekül zu unterdrücken.
   ∘ Der Einbau von nicht an die Matrize hybridisierten Nuk-Makromolekülen wird durch natürliche Nukleotide gleicher Art wie die Nuk-Komponente des entsprechenden Nuk-Makromoleküls stark unterdrückt
- Für die Markierungsreaktionen in Anwesenheit von natürlichen Nukleotiden gleicher Art wie die Nuk-Komponente der Nuk-Makromoleküle (z.B. in Anwesenheit von dTTP bei einer Markierung mit einem dUTP einschließenden Nuk-Makromolekül), werden Strukturen von Nuk-Makromolekülen bevorzugt, die einen relativ kurzen Linker (z.B. bis zu 200 Kettenatome, noch bevorzugter bis zu 100 Kettenatome, noch bevorzugter bis zu 50 Kettenatome, noch bevorzugter bis zu 20 Kettenatome) zwischen der Nuk-Komponente und der Marker-Komponente einschließen.
- Für die Markierungsreaktionen in Anwesenheit von natürlichen Nukleotiden gleicher Art wie die Nuk-Komponente der Nuk-Makromoleküle (z.B. in Anwesenheit von dTTP bei einer Markierung mit einem dUTP einschließenden Nuk-Makromolekül), werden Strukturen von Nuk-Makromolekülen bevorzugt, die einen relativ kurzen Linker (siehe oben) einschließen, sowie die Position zumindest einer Nuk-Komponente innerhalb des Nuk-Makromoleküls am 5'-Ende einer Target-Domäne oder in der Nähe des 5'-Endes einer Target-Domäne haben.
- Die erfindungsgemäßen Nuk-Makromoleküle können in einer zyklisch ablaufenden Markierungsreaktion eingesetzt werden. Die zyklische Markierungsreaktion schließt mindestens einen Zyklus, wobei eine Änderung der Reaktionstemperatur durchgeführt wird. Beispielsweise schließt ein solcher Zyklus mindestens einen Denaturierungsschritt (z.B. bei 95°C) bei dem die Zielsequenzen aus einer doppelsträngigen Form in eine einzelsträngige Form überführt werden. Weiterhin, schließt ein solcher Zyklus mindestens einen Hybridisierungsschritt für Primer und Target-Domäne eines Nuk-Makromoleküls. Weiterhin schließt ein solcher Zyklus mindestens einen Schritt für die Verlängerung des Primers und Einbau von Nuk-Makromolekül. Die Schritte der Hybridisierung und der Verlängerung können bei gleichen oder unterschiedlichen Temperaturen stattfinden. Diese Schritte können mindestens zwei mal wiederholt werden.
- Die Markierung kann an Nukleinsäureketten erfolgen, die an einer festen Phase immobilisiert sind.
- Die an eine feste Phase gebundenen Nuk-Makromoleküle können Zielsequenzen markieren.
- Bei entsprechenden Reaktionsbedingungen (z.B. Hybridisierungstemperatur) können Nuk-Makromoleküle durch ihre Target-Domänen Zielsequenzen unterscheiden und nur spezifische Zielsequenzen markieren.
- Bei entsprechenden Reaktionsbedingungen (z.B. Hybridisierungstemperatur) können Nuk-Makromoleküle durch ihre Target-Domänen Zielsequenzen unterscheiden, wobei eine Gruppe von Zielsequenzen mit ähnlichen Bindungsstellen für die Target-Domäne eines Nuk-Makromoleküls markiert wird.
- Mehrere Nuk-Makromoleküle können in einer Markierungsreaktion bereitgestellt sein und bei entsprechenden Reaktionsbedingungen (z.B. Hybridisierungstemperatur) ihre spezifischen Zielsequenzen markieren.
- Nuk-Makromoleküle können in ihre spezifischen Zielsequenzen in Anwesenheit einer anderen Art von Nukleinsäureketten (z.B. genomische DNA) bei entsprechenden Reaktionsbedinungen selektiv markieren.
- Nuk-Makromoleküle können einzelsträngige Form der Zielsequenz markieren.
- Nuk-Makromoleküle können doppelstängige Form der Zielsequenz (z.B. PCR-Fragmente) markieren, wenn Doppelstränge von einander separiert werden und während der zyklsich ablaufenden Markierungsreaktion Hybridisierunsschritt für die entsprechende Target-Domäne des Nuk-Makromoleküls enthaltend ist.
- Nuk-Makromoleküle können während einer PCR die Zielsequenzen markieren.
- Die mit Nuk-Makromolekülen markierten Zielsequenzen können über Anker-Domäne der eingebauten Nuk-Makromoleküle an eine feste Phase gebunden werden.
- Das Ausmass der Markierung von Zielsequenzen und somit die Signal-Intensität der markierten Zielsequenzen kann durch die Reaktionsbedinungen beeinflusst werden. Beispielsweise durch Änderung der Konzentartion von konkurrierenden natürlichen Nukleotide gleicher Art wie die Nuk-Komponente der Nuk-Makromoleküle oder durch Anzahl der Zyklen in einer zyklischen Markierungsreaktion oder durch die Ausgangsmenge an Zielsequenzen oder durch Hybridisierungstemperatur für die Target-Domäne oder Primer.
- Teile von Nuk-Makromolekülen (z.B. Target-Domäne von Nuk-Makromolekülen) kann durch eine 5'-3'Exonuklease einer Polymerase (z.B. von Taq-Polymerase) abgebaut werden.
- Die "Strand-Displacement" -Aktivität der Polymerasen kann die Target-Domäne eines Nuk-Makromoleküls von der Bindung an die Zielsequenz verdrängen und die Synthese des markierten Strangs fortsetzen.

Nachfolgend sind einige Beispiele angegeben, die Substrat-Eigenschaften von Nuk-Makromolekülen demonstrieren.

### 1.5.15.1 Enzymatischer Einbau von dU-PEG(8)-[T1,A1]-TAMRA (I)

In diesem Experiment wird Einbau von dU-PEG(8)- [T1,A1]-TAMRA in den Primer in Anwesenheit unterschiedlicher Sets von weiteren natürlichen Nukleotiden getestet.

### Komponenten:

Einbaupuffer 1

| | |
|---|---|
| Nuk-Makromolekül: dU-PEG(8)- [T1,A1]-TAMRA | (5 µmol/l) |

Natürliche Nukleotide (dTTP, dATP, dCTP, dGTP) zugegeben bis zur Endkonzentrationen, wie in der Legende beschrieben

| | | |
|---|---|---|
| Polymerase: | Klenow exo minus, | 1:10 |
| Primer: | A50-T719 | (1 µmol/l) |
| (Dieser Primer hat eine Anker-Domäne A50) | | |
| Matrizen: | M1 | (1µmol/l) |

5´ GTT TTC CCA GTC ACG ACG  GGAG  gtg cc agc agc cgc ggt aat acg ACCA
cctatagtgagtcgtatta 3´

Bindungsstelle für Nuk-Makromoleküle ist unterstrichen

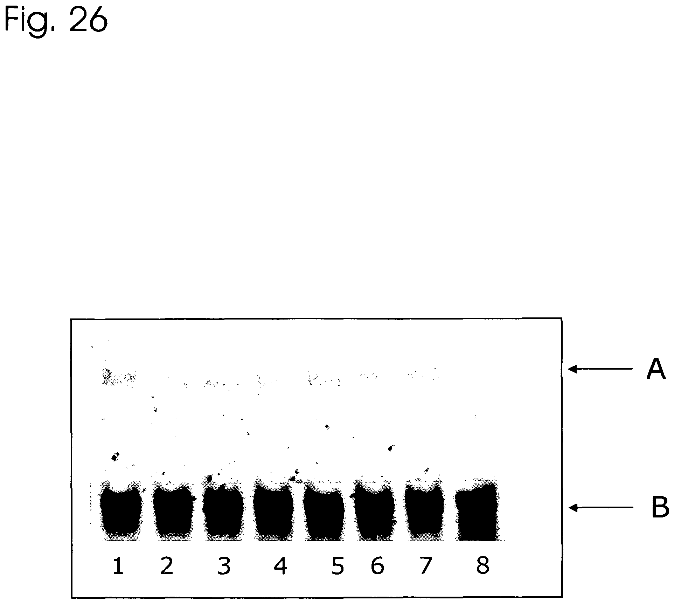

Primer, Matrizen, natürliche Nukleotide und Nuk-Makromolekül (dU-PEG(8)-[T1,A1]-TAMRA) wurden durch Erhitzung der Reaktionslösung auf 90°C und anschließende Abkühlung auf RT hybridisiert, so dass Primer und die Target-Domäne des Nuk-Makromoleküls an die Matrize (Zielsequenz) binden können. Zu dieser Lösung wurde Polymerase zugegeben und die Reaktion bei RT 10 min inkubiert. Anschließend wird die Reaktion direkt auf ein Geld geladen und die Reaktionsprodukte wurden aufgetrennt. Das Ergebnis ist in Fig. 26 abgebildet. Die Zusammensetzung einzelner Reaktionen ist in der Legende angegeben.

Es ist zu sehen, dass dU-PEG(8)-[T1,A1]-TAMRA als Substrat für Klenow exo minus verwendet werden kann (lane 1). Es kann in den Primer in Anwesenheit von dTTP in steigenden Konzentrationen (bis 1 mmol/l) eingebaut werden (Lane 2 bis 7), sowie in Anwesenheit von weiteren Nukleotiden (dATP, dGTP, dCTP, Lane 7).

Dank der Bindung an die Matrize kann dU-PEG(8)-[T1,A1]-TAMRA um den Einbau in den Primer durch die Polymerase mit freien dTTP (1 mmol/l) konkurrieren.

In Kontroll-Experimenten (hier nicht abgebildet) wurde ermittelt, dass dU-PEG(8)-[T1,A1]-TAMRA in den Primer eingebaut werden kann, auch wenn es nicht an die Matrize hybridisiert ist. Allerdings kann die Anwesenheit von dTTP zu einer Unterdrückung dieser Reaktion führen. Bereits 5 µmol/l Konzentration von dTTP kann den Einbau von nicht an die Matrize gebundenen dU-PEG(8)-[T1,A1]-TAMRA deutlich reduzieren. Anwesenheit von konkurrierenden Nukleotiden in Konzentraion von 100 µmol/l führt zu einer vollständigen Unterdrückung des Einbau von dU-PEG(8)-[T1,A1]-TAMRA, wenn diese nicht an die Matrize hybridisiert sind.

Zusammenfassen kann festgestellt werden, dass die spezifische Bindung von dU-PEG(8)-[T1,A1]-TAMRA an die Matrize zu einer deutlichen Begünstigung bei dem Einbau dieses Nuk-Makromoleküls in Anwesenheit von konkurrierenden Nukleotiden führt. Dieser Effekt wird dahingehend interpretiert, dass sich die lokale Konzentration der Nuk-Komponente durch die Bindung an die Matrize um ein vielfaches erhöht, so dass Polymerase dieses Nukleotid bevorzugt einbaun kann.

### 1.5.15.2 Enzymatischer Einbau von dU-PEG(8)-[T1,A1]-TAMRA.(II)

In diesem Experiment wird Einbau von dU-PEG(8)-[T1,A1]-TAMRA in den Primer in Anwesenheit von weiteren natürlichen Nukleotiden und mehreren Polymerasen getestet.

### Komponenten:

Einbaupuffer 1
Nuk-Makromolekül: dU-PEG(8)-[T1,A1]-TAMRA (5 µmol/l)
Natürliche Nukleotide (dTTP, dATP, dCTP, dGTP) zugegeben bis zur Endkonzentrationen, wie in der Legende beschrieben

| | | |
|---|---|---|
| Polymerase: | Klenow exo minus, | 1:10 bis 1:1000 |
| | Taq-Polymerase | 1:100 |
| | Vent exo minus -Polymerase | 1:100 |
| | | |
| Primer: | A50-T719 | (1 µmol/l) |
| (Dieser Primer hat eine Anker-Domäne A50) | | |
| Matrizen: | M2 | (1 µmol/l) |

### Matrize 2:

5' **GTT TTC CCA GTC ACG ACG GGAG gtg cc agc agc cgc ggt aat acg AGT CTT CTCA cctatagtgagtcgtatta**
Die Bindungsstelle für Nuk-Makromoleküle ist unterstrichen

Die Reaktionslösung wurde ähnlich wie in 1.5.15.1 vorbereitet. Primer, Matrizen und Nuk-Makromoleküle wurden hybridisiert durch Erhitzung der Reaktionslösung auf 90°C und anschließende Abkühlung auf RT.

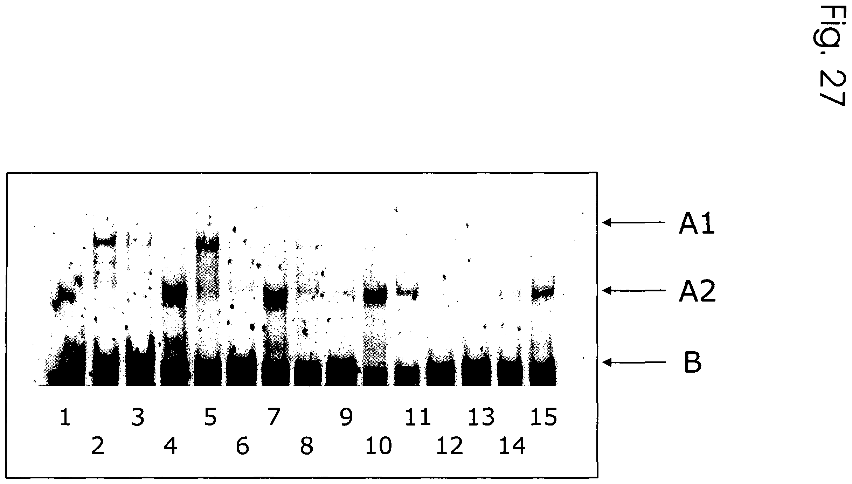

Zu dieser Lösung wurden dATP, dGTP, dCTP bis zur Endkonzentration von 100 µmol/l und dTTP von 0 bis 10 mmol/l (siehe Angaben in der Legende zu Fig 27) zugegeben. Die Reaktion wurde durch die Zugabe einer entsprechenden Polymerase (Klenow exo minus in Verdünnung 1:10, 1:100, 1:1000, Taq 1:100 und Vent exo minus, 1:100) gestartet. Die Markierungsreaktion wurde 12 hr bei 37°C durchgeführt. Die Reaktionsansätze wurden mittels einer Gelelektrophorese analysiert. Das Ergebnis ist in Fig 27 abgebildet. Die Zusammensetzung einzelner Reaktionen ist in der Legende angegeben.

### Ergebnisse:

### • Akzeptanz von dU-PEG(8)-[T1,A1]-TAMRA.

Alle verwendeten Polymerasen akzeptierten dU-PEG(8)-[T1,A1]-TAMRA als Substrat in der Primer-Extension-Reaktion (Fig. 27, Lane 1, 4, 7,10, 15). Die Anwesenheit von dATP, dCTP, dGTP störte den Einbau nicht. Der Primer wird nur teilweise verlngert (Fig. 27, Pfeil A2), da die Abwesenheit von dTTP keine vollständige Primerverlägerung zulässt.

### • Konkurrenz mit dTTP

Der Einbau von dU-PEG(8)-[T1,A1]-TAMRA findet durch alle Polymerasen auch in Anwesenheit von dTTP bis zur 100 µmol/l Konzentration statt (Lane 2,5,8,11,14). In Anwesenheit von dTTP 10 mmol/l wird der Einbau von dU-PEG(8)-[T1,A1]-TAMRA durch Vent exo minus und Taq-Polymese unter verwendeten Reaktionsbedingungen stark bis komplett unterdrückt (Lane 12 für Taq, Lane 13 für Vent exo minus). Dagegen kann Klenow exo minus auch bei dieser hohen Konzentration von konkurrierenden Nukleotiden das an die Matrize hybridisierte dU-PEG(8)-[T1,A1]-TAMRA einbauen (Lane 3, 6,9).

### • Strand-Displacement-Aktivität der Polymerasen

Die Fähigkeit des Klenow exo minus zum Strand-Displacement führte dazu, dass in Anwesenheit von dTTP eine komplette Synthese des komplementären Stranges zu Matrize M2 stattfand (Lane 2,3,5). Taq-Polymerase und Vent exo minus waren unfähig unter gleichen Bedingungen, die hybridisierte Target-Domäne des Nuk-Makromoleküls von der Matrize abzulösen, diese Polymerase haben keine strand displacement Aktivität. Der Einbau durch Vent exo minus und Taq erfolgte nur bis zur Target-Domäne, komplette Synthese des komplementären Stranges hat unter verwendeten Bedingungen nicht stattgefunden. Komplette Synthese des komplementären Strandes konnte allerdings unter zyklischen Markierungsbedingungen erreicht werden (siehe unten).

### • 5'-3'-Exonuklease-Aktivität von Taq

Taq Polymerase hat eine 5'-3-Exonuklease Aktivität. Trotz dieser Aktivität wurde der Primer mit den efindungsgemäßen Nukleotiden markiert.

### • Effekt der Konzentration der Polymerase

Klenow-Fragment wurde in unterschiedlichen Konzentrationen verwendet: von 1:10 bis 1:1000. Bei höheren Konzentrationen von Klenow-Exo minus gelang die Strand-Diskplacement Reaktion am besten.

### 1.5.15.3 Wahl von Nuk-Makromolekülen und Polymerasen für eine Markierungsreaktion.

Für die zielsequenz-spezifische Markierungsreaktion von Nukleinsäureketten ist es entscheidend, dass Nuk-Makromoleküle in Abhängigkeit von ihrer Bindung an diese Zielsequenzen eingebaut werden. Mehrere Reaktions-Parameter haben Einfluss auf diese Eigenschaften. Sowohl die Wahl von Polymerasen, von Reaktionsbedingungen (z.B. Konzentration von konkurrierenden natürlichen Nukleotiden), als auch die Struktur von Nuk-Makromoleküle können diesen Einfluss ausüben. Zu Darstellungszwecken wurden einige dieser Paramter variiert (Polymerasen, Konzentration von konkurrierenden Nukleotdien, Struktur der Nuk-Makromoleküle). Nachfolgend ist ein Vergleich über die Fähigkeit verschiedener Polymerasen angegeben, die an die Matrize hybridisierten Nuk-Makromoleküle in Anwesenheit von konkurrierenden Nukleotiden (in diesen Beispielen dTTP) einzubauen:
- Sehr guter Einbau: Klenow exo minus, Taq, Terminator Polymerase, Terminator II Polymerase (alle Enzyme von New England Biolabs)
- Guter Einbau: Vent exo minus, Tth Polymerase
- Mäßiger Einbau: Sequenase 2, Deep Vent exo minus

Die Struktur der Nuk-Makromoleküle kann Einfluss auf ihre Fähigkeit haben, in Anwesenheit von konkurrierenden Nukleotiden durch die Polymerase eingebaut zu werden. Die besten Einbauergebnisse in Anwesenheit von konkurrierenden Nukleotiden haben Strukturen von Nuk-Makromolekülen mit einer Nuk-Komponente, die über einen relativ kurzen Linker am 5'-Ende der Targer-Domäne gekoppelt ist.

Die Rolle der Bindung und der Position der Target-Domäne auf einer Zielsequenz. Vergleich der Einbaufähigkeit der Nuk-Makromoleküle MIT und OHNE Bindung an die Matrize zeigte, dass die Anwesenheit von dTTP (100 µmol/l) den Einbau von Nuk-Makromolekülen mit einer dUTP-Nuk-Komponente ohne Bindung der Targer-Domäne an die Matrize komplett unterdrücken kann. Die Anwesentheit von geringeren Mengen von dTTP (1 bis 10 µmol/l kann zu einer deutlichen Reduktion des Einbaus von Nuk-Makromolekülen führen.

Diese Situation ändert sich radikal mit der Bindung der Target-Domänen an die entsprechend komplementäre Position der Matrize in 3'-Richtung vom Primer (wobei zwischen dem 5'-Ende der Target-Domäne und dem 3'-Ende des Primers mindestens ein Nukleotid in der Zielsequenz vorkommt, das in der Lage ist, mit der Nuk-Komponente des hybridisierten Nuk-Makromoleküls ein Basenpaar zu bilden): die an die Matrize gebundenen Nuk-Makromoleküle werden bevorzugt eingebaut. Auch Konzentrationen der natürlichen Nukleotide in der Lösung von bis zu 100 µmol/l oder auch bis 10 mmol können den Einbau nicht komplett unterdrücken.

Die Position der Bindung einer Target-Domäne an die Zielsequenz kann variabel je nach Experiment gestaltet werden. Typischerweise wird die Target-Domäne dermassen gewählt, dass ihre potenzielle Bindungsstelle in 3'-Richtung vom Markierungsprimer liegt. Vorzugsweise ist die Position der Target-Domäne innerhalb der Zielsequenz dermassen ausgewählt, dass zwischen dem gebundenen Primer und der gebundenen Target-Domäne mindestens ein Nukleotid in der Zielsequenz vokommt, das mit der Nuk-Komponente des Nuk-Makromoleküls ein komlementäres Basenpaar bilden kann.

Das Grad der Markierung einer Zielsequenz kann entsprechend einem Reaktionsaufbau an die jeweiligen Konzentrationen der natürlichen Nukleotide, die Polymerase und die Reaktionsbedingungen angepasst werden: Bindung der Nuk-Komponente an das 5'-Ende der Target-Domäne oder an die Abschnitte, die nah am 5'-Ende der Target-Domäne lokalisiert sind, mit kürzeren Linkern (z.B. mit nur 10 bis 100 Kettenatome) ermöglicht den Einbau des an die Matrize gebundenen Nuk-Makromoleküle in Anwesenheit von hohen Konzentrationen natürlicher Nukleotide.

### 1.5.15.4 Zyklische Primer-Extension Reaktion

Die Markierung von Zielsequenzen kann in einem einzelnen Schritt der Primer-Extension oder auch in mehreren Zyklen der Primer-Extension erfolgen. Hier wird ein Beispiel für die Markierung von Zielsequenzen in mehreren zyklsichen Schritten dargestellt.

Zur Anschaulichkeit der Bedeutung der Hybridisierung einer Target-Domäne an die Zielsequenz für die Markierung werden mehrere unterschiedliche Zielsequenzen mit gleicher Primer-Bindungsstelle verwendet. Der verwendete Primer ist am 5'-Ende mit einem Fluoreszenzfarbstoff markiert (T7-19-Cy3). Das für die Markierung verwendete Nuk-Makromolekül (dU-PEG(4)-[T1,A1]-TAMRA) hat dUTP als Nuk-Komponente, die über einen kurzen Linker am 5'-Ende der Target-Domäne gekoppelt ist. Diese Nuk-Makromolekül hat weiterhin eine Anker-Domäne und eine Signal-Domäne (TAMRA) am 3'-Ende.

### Komponenten:

| | | |
|---|---|---|
| ThermoPol Buffer 1x | | |
| Nuk-Makromolekül: | dU-PEG(4)-[T1,A1]-TAMRA | (0,5 µmol/l) |
| Natürliche Nukleotide (dTTP 50 µmol/ll, dATP, dCTP, dGTP jeweils 100 µmol/l) | | |
| Polymerase: | Taq-Polymerase | 1:100 |
| | | |
| Primer: | T719-Cy3 | (0.5 µmol/l) |
| (Dieser Primer hat eine Signal-Domäne Cy3-Farbstoff) | | |
| Matrizen: | M2, M4, M8, M9 | (jeweils 0,1 µmol/l) |

Matrize 2: 5' GTT TTC CCA GTC ACG ACG GGAG gtg cc agc agc cgc ggt aat acg AGT CTT CTCA cctatagtgagtcgtatta (bereits da)
Matrize 4: 5' GTT TTC CCA GTC ACG ACG GGAG gtg cc agc ggt aat acg AGT CTT CTGA cctatagtgagtcgtatta
Matrize 8: 5' GTT TTC CCA GTC ACG ACG GGAG gtg cc agc agc cgc AGT TTT TTT AGT CTT CTGA cctatagtgagtcgtatta
Matrize 9: 5' GTT TTC CCA GTC ACG ACG GGAG cgc ggt aat acg AGT CTT CTCA cctatagtgagtcgtatta

(Die potenziellen Bindungsstellen für die Target-Domäne sind unterstrichen)

Matrize M2 und die Target-Domäne haben eine komplett komplementäre Sequenz über die Gesamtlänge der Target-Domäne (komplementärer Bereich ist unterstrichen) Matrize M4 hat komplementäre Sequenzen nur für beide Enden der Target-Domäne (komplementärer Bereich ist unterstrichen)
Matrize 8 hat komplementäre Sequenzen nur für das 3'-Ende der Target-Domäne (komplementärer Bereich ist unterstrichen). Das 5'-Ende der Target-Domäne ist nicht an die Zielsequenz hybridisert
Matrize 9 hat komplementäre Sequenzen nur für das 5'-Ende der Target-Domäne (komplementärer Bereich ist unterstrichen). Das 3'-Ende der Target-Domäne ist nicht an die Zielsequenz hybridisert.

Solche Sequenzen können als Beispiele für Abweichungen von der Zielsequenz betrachtet werden (z.B. Mutationen in der Zielsequenz). Ebenfalls können solche Sequenzen als Paare von Ziel-Sequenz / Target-Domänen betrachtet werden, wobei die Target-Domäne zur Zielsequenz (Matrize 4) nicht vollständig komplementär ist. Ebenfalls dient dieses Experiment zur Darstellung von Reaktionsabläufen bei dem die Tm von Target-Domäne geringer ist als die Tm von Primer. Ebenfalls stellt dieses Experiment den Fall dar, wenn das 5'-Ende der Targer-Domäne nicht an die Zielsequenz hybridisiert ist.

Die Reaktionslösung wurde ähnlich wie in 1.5.15.1 vorbereitet. Primer, Matrizen, natürliche Nukleotide (dNTP's) und Nuk-Makromoleküle wurden in einer Puffer-Lösung bereitgestellt. Die Lösungen wurden anfänglich unter 95°C 15 min lang inkubiert. Während dieser Zeit wurde die Taq-Polymerase in die Reaktion gegeben (Hot-Start der Reaktion, um Nebenmarkierungen zu vermeiden). Nach dem Ende der Reaktion wurde EDTA bis zur Endkonzentration von 10 mmol/l zugegeben. Die Reaktionsansätze wurden anschliend auf einem Gel aufgetrennt.

Die zyklische Markierungsreaktionen wurden unter unterschiedlichen Temperaturen in mehreren zyklischen Schritten durchgeführt.
Ein Zyklus schließt ein Hybridisierungsschritt, ein Extensionsschritt und ein Denaturierungsschritt ein. Der Extensions-Schritt (70°C 1 min) und der Denaturierungsschritt (95°C 30 sec) wurden bei allen Matrizen gleich durchgeführt. Wegen Unterschiede in komplementären Bereichen zwischen der gegeben Target-Domäne und zu markierenden Zielsequenzen was es interessant zu prüfen, bei welcher Hybridisierungstemperatur die Einbaureaktion von Nuk-Makromolekülen stattfindet. Folglich, wurden unterschiedliche Hybridisierungstemperaturen getestet. Da in vorläufigen Experimenten die Fähigkeit zum Einbau des Nuk-Makromoleküls an der Zielsequenz (M2) mit vollständig komplementärem Bereich zu Target-Domäne bereits getestet wurde, diente diese Reaktion als positive Kontrolle (Hybridisierung bei 55°C).
Folgende Temperaturen wurden als Hybridisierungstemperaturen gewählt: 35°C, 45°C und 55°C.
Zyklischen Reaktionen (jeweils 20 Zyklen) wurden in einem PCR-Gerät unter folgenden Bedingungen durchgeführt:
Cycler-Programm : Hybridisierung bei 35°C

| | | |
|---|---|---|
| Denaturierung: | 95°C | 30 sec |
| Hybridisierung: | 45°C | 1 min |
| Extension: | 70°C | 1 min |

Cycler-Programm : Hybridisierung bei 45°C

| | | |
|---|---|---|
| Denaturierung: | 95°C | 30 sec |
| Hybridisierung: | 45°C | 1 min |
| Extension: | 70°C | 1 min |

Cycler-Programm : Hybridisierung bei 55°C

| | | |
|---|---|---|
| Denaturierung: | 95°C | 30 sec |
| Hybridisierung: | 55°C | 1 min |
| Extension: | 70°C | 1 min |

Als Kontrolle der Signal-Intensität und der Position des verlängerten Produkts im Gel diente Primer-Extensions (1x Zyklus) der jeweiligen Matrize ohne Nuk-Makromolekül (37°C 12 h). Die Konzentration einzelner Komponenten (Primer, Matrize, Polymerase, dNTPs waren die gleichen, wie bei der zyklischen Markierung). Bei dieser Reaktion wurde Primer mit natürlichen Nukleotiden verlängert.

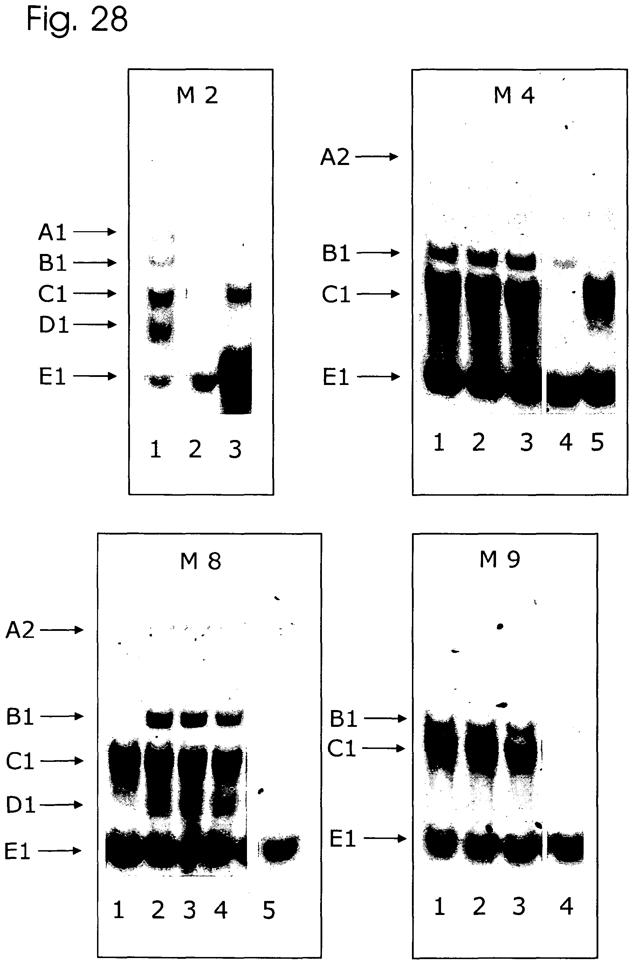

Das Ergebnis der Reaktion ist in Fig. 28 zusammengefasst.

Man sieht, dass die Primer-Verlängerung stattgefunden hat: während der zyklischen Primer-Extension die Menge an verlängerten Primern zunimmt (vergleiche die Intensität der Banden bei zyklischen Reaktionen und einer einfachen Primer-Extension).
Die Markierung der neu synthetisierten Stränge hängt entscheidend davon ab, ob das Nuk-Makromolekül über seine Target-Domäne unter jeweiligen Reaktionsbedingungen an die Matrize gebunden hat. Die Reaktion an M2-Matrize mit Taq-Polymerase bei 55°C Hybridisierungstemperatur verlief in guten Ausbeuten: das markierte Produkt ist deutlich zu sehen (Fig. 28, M2, Lane 1, Pfeil A1). Ebenfalls ist eine Markierung mit Matrize (M8) bei allen Hybridisierungstemperaturen (35°C, 45°C, 55°C) zu sehen (Fig. 28, M8, Lane 2-4, Pfeil A2). In der Reaktion mit Matrize 4 (M4) ist eine nur sehr schwache Markierung bei Hybridisierungstemperatur von 35°C und 45°C zu sehen (Fig. 28, M4, Lane 1,2, Pfeil A2). Bei 55°C konnte keine Markierung mehr nachgewiesen werden. In der Reaktion an der Matrize 9 wurde bei keiner Hybridisierungstemperatur eine Markierung gesehen, der Einbau von dU-PEG(4)-[T1,A1]-TAMRAwurde durch natürliche Nukleotide komplett unterdrückt.

An diesem Beispiel kann man erkennen, dass die Bindung der Target-Domäne eines Nuk-Makromoleküls für eine spezifische Erkennung einer Zielsequenz genutzt werden kann: unter stringenten Hybridisierungsbedingungen werden lediglich Nuk-Makromoleküle in den wachsenden Strang eingebaut, die an die Zielsequenz gebunden sind. Veränderungen in der Bindungsstelle in der Zielsequenz können zum Verlust bzw. Minderung in der Markierung dienen. Andererseits kann die Target-Domäne dennoch an die veränderte Position in der Zielsequenz binden, wenn weniger stringente Bedingungen (z.B. niedrigere Temperaturen) verwendet werden.

Am Beispiel mit der Markierung von Matrize 8 ist zu sehen, dass Polymerase auch Nuk-Makromoleküle akzeptiert, bei denen das 5'-Ende nicht an die Matrize hybridisiert ist.

### 1.5.15.5 Markierung von Zielsequenzen während bzw. parallel zu ihrer Amplifikation in der PCR, sowie anschließende Bindung und Isolierung von markierten Zielsequenzen mit einer festen Phase über die Anker-Domäne der eingebauten Nuk-Makromoleküle.

Die Markierung von Zielsequenzen kann parallel zu deren Amplifikation stattfinden. PCR ist eine der üblichen Methoden zu Vervielfältigung der Nukleinsäureketten. Hier wird ein Beispiel für die Markierung von Zielsequenzen parallel zu deren Amplifikation angegeben. Nach der PCR werden markierte DNA-Fragmente durch eine spezifische Bidnung an eine feste Phase isoliert. Diese Isolierung wird durch eine spezifische Bindung der Anker-Domäne von eingebauten Nuk-Makromolekülen an die an einer festen Phase immobilisierten Bindungspartner vermittelt.

### Komponenten:

ThermoPol Buffer 1x

| | | |
|---|---|---|
| Nuk-Makromolekül: | dU-PEG(4)- [T1,A1]-TAMRA | (0,5 µmol/l) |
| Nuk-Makromolekül: | dU-PEG(8)- [T1,A1]-TAMRA | (0,5 µmol/l) |
| Natürliche Nukleotide (dTTP 50 µmol/ll, dATP, dCTP, dGTP jeweils 100 µmol/l) | | |
| Polymerase: | Taq-Polymerase | 1:100 |
| | Vent exo minus | 1:100 |
| Primer: | T719-Cy3 | (0.5 µmol/l) |
| | U19 | |
| (T7-19-Cy3 Primer hat eine Signal-Domäne: Cy3-Farbstoff) | | |
| Matrize: | M2 | (10 nmol/l) |
| dT48-Magnetische Beads | | (1 vial) |

Ein PCR-Primer ist am 5'-Ende mit einem Fluoreszenzfarbstoff markiert (Cy3). Ein zweiter PCR-Primer ist unmarkiert. Die für die Markierung verwendeten Nuk-Makromolekül (dU-PEG(4)-[T1,A1]-TAMRA und dU-PEG(8)-[T1,A1]-TAMRA) haben dUTP als Nuk-Komponente, die über einen kurzen Linker (PEG 4 oder PEG 8) am 5'-Ende der Target-Domäne gekoppelt ist. Diese Nuk-Makromolekül hat weiterhin eine Anker-Domäne und eine Signal-Domäne (TAMRA) am 3'-Ende. Die Bindungsstelle in der Matrize 2 für die Target-Domäne ist vollständig komplementär.

Beide PCR-Primer, Matrize (M2), natürliche Nukleotide (dNTP's) und Nuk-Makromoleküle wurden in einer Puffer-Lösung bereitgestellt. Die Lösungen wurden anfänglich unter 95°C 15 min lang inkubiert. Während dieser Zeit wurde Taq-Polymerase oder Vent-exo minus Polymerase in die Reaktion gegeben (Hot-Start der Reaktion, um Nebenmarkierungen zu vermeiden). Nach dem Ende der Reaktion wurde EDTA bis zur Endkonzentration von 10 mmol/l zugegeben. Die Reaktionsansätze wurden anschliend auf einem Gel aufgetrennt.

Es wurden folgende PCR-Bedingungen verwendet:
Cycler-Programm :

| | | |
|---|---|---|
| Denaturierung: | 95°C | 30 sec |
| Hybridisierung: | 55°C | 1 min |
| Extension: | 70°C | 1 min |

Insgesamt 20 Zyklen

| | |
|---|---|
| Halten: | 4°C |

Anschließend wurde ein Teil der Reaktion mit einer Suspension von dT48 Magnetic Beads in Kontakt gebraucht. Nach einer Inkubationszeit von 5 min im Thermo-Pol1x Buffer wurden die Beads mit Einbaupuffer 1 gewaschen. Die Beads wurden direkt auf das Gel geladen. Die Ablösung der PCR-Fragmente von Beads erfolgte unter erhöhter Temperatur von ca. 85°C.

Die feste Phase wurde vor dem Experiment wie folgt vorbereitet: Streptavidin-Magnetic Beads (von Promega) wurden mit einem Oligonukleotid, dT48 mit einem Biotin-Rest am 3'-Ende beladen und gewaschen. Das dT48-Oligonukleotid stellt ein Beispiel für einen Bindungspartner einer Anker-Domäne dar. Solche Beads sind in der Lage die mit Nuk-Makromolekülen markierte Nukleinsäureketten zu binden.

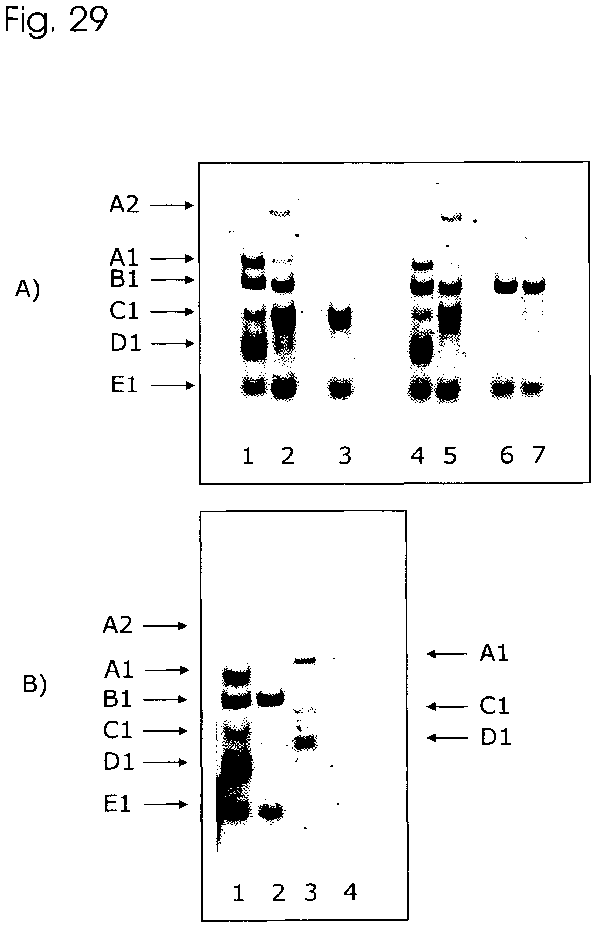

Das Ergebnis der Reaktion ist in Fig. 29 zusammengefasst. Man sieht deutliche Binldung von PCR Fragmenten: mit Nuk-Makromolekülen markierte PCR-Produkte (Pfeil A1 und A2 Fig. 29 A) und nicht PCR-Produkte ohne Nuk-Makromolekülen (Pfeil B1, Fig. 29 A). Interessanterweise kann die Taq-Polymerase nur wenige markierte PCR-Fragmente komplett extendieren (Pfeil A1) vs. (Pfeil A2). Dagegen kann Vent exo minus Polymerase durch ihre Strand-Displacement-Aktivität unter gegebenen Reaktionsbedingungen deutlich mehr vollständig verlängerten Fragmenten generieren. (Pfeil A2).

Die spezifische Isolierung der mit Nuk-Makromolekülen modifizierten PCR-Fragmente durch feste Phase ist in Fig. 29 (B) gezeigt. Man sieht, dass nur die PCR-Fragmente, die ein Nuk-Makromolekül mit einer Anker-Domäne eingebaut haben, isoliert werden können (Pfeil A1, Lane 3). PCR-Produkte ohne eingebaute Nuk-Makromoleküle binden nicht an die feste Phase und werden daher nicht isoliert (Lane 4 hat keine Signale).

### 1.5.15.6 Nachweis einer bakteriellen DNA mittels PCR Amplifikaiton und Markierung mit Nuk-Makromolekülen

Einem Fachmann sind viele Messungen bekannt, die auf Real-Time PCR Methode basieren. Dabei wird beispielweise eine markierte Sonde (auch Probe genannt)n, die komplementär an die Zielsequenz binden kann, in die Reaktion zugegeben und die Menge an Produkt während der Reaktion gemessen. Das Signal oder Signal-Anstieg entsteht dann, wenn die Sonde an die jeweilige Zielsequenz hybridisiert hat und beispielsweise durch 5'-3'-Aktivität einer thermostabilen Polymerase teilweise abgebaut wird (z.B. US Pat No 5,538,848, 5,723,591, 5,876,930, 6,030,787, 6,171,785, 5,487,972).

Solche Methoden zum Nachweis von bestimmten DNA-Abschnitten in einem biologischen Material wurden in einer sehr großen Anzahl an Publikationen dargestellt. Die jeweiligen Autoren beschreiben die Isolierungsbedingungen für die Nukleinsäureketten, die spezifischen Primer, die Sondenzusammensetzung und die entsprechenden Reaktionsbedingungen zur Vervielfältigung und Nachweis von Zielsequenzen. Unzählige Varianten dieser Methode, inklusive Multiplex-PCR, diagnostische Real-Time PCR, Kombinationen mit Reversen Transcriptasen (RT-PCR) usw. wurden seit der Einführung der Real-Time PCR in den 90er Jahren veröffentlich.

Die vorliegende Anmeldung macht Gebrauch von dieser Lehre. Ähnlich wie bei Real-Time PCR liegt in einer vorteilhaften Ausführungsform dieser Anmeldung die spezifische Bindung einer Target-Domäne eines Nuk-Makromoleküls an die Zielsequenz unter Bedingungen, die eine Vervielfältigung von Nukleinsäuereketten zulassen (z.B. PCR). Ein Fachmann kann daher auf die bestehenden Erkenntnisse aus der Real-Time PCR zurückgreifen. Insbesondere betrifft dies die Zusammensetzung von PCR-Primern, der Target-Domäne und Reaktionsbedingungen, sowie weitere Kombinationen, z.B. Multiplexing, Kombination mit Reversen Transcriptasen usw.

Zur Anschaulichung werden Primer und die Sondenzusammensetzung, die in einer Veröffentlichung publiziert waren (Nadkarni M.A. et al, Microbiology, 2002,v.148 257-), bei einer Markierung mit Nuk-Makromolekülen während einer PCR-Reaktion demonstriert.

Die Primer (forward und reverse Primer, siehe Liste der Sequenzen) wurden ohne Veränderungen übernommen. Die Sequenz-Zusammensetzung der beschriebenen doppelt-markiertnen Probe (FAM/TAMRA) wurde für die Sequenz-Zusammensetzung der Target-Domäne des Nuk-Makromoleküls mit folgenden Veränderungen übernommen. Statt Fluorescein wurde an das 5'-Ende der Target-Domäne eine Nuk-Komponente über einen kurzen Linker gekoppelt und am 3'-Ende der Target-Domäne wurde eine Anker-Domäne, bestehend aus 25 dA-Resten angefügt. Das 3'-Ende der Anker-Domäne trägt ein Fluorescenzfarbstoff (TAMRA). Es resultierte das Oligonukleotid [T1,A1]-TAMRA, das zur Synthese von Nuk-Makromolekülen als eine Variante des Markers verwendet wurde.

Einige Eigenschaften des resultierten Nuk-Makromoleküls (z.B. dU-PEG(4)- [T1,A1]-TAMRA oder dU-PEG(8)- [T1,A1]-TAMRA) mit diesem Oligonukleotid wurden bereits in vorangehenden Beispielen demonstriert, siehe Beispiele 1.5.15.1 bis 1.5.15.5.

In diesem Beispiel soll die Anwendung dieses Nuk-Makromoleküls für den Nachweis der Präsenz einer bakteriellen DNA gezeigt werden. Es wurde eine Real-Time PCR gewählt die den 16 S ribosomalen genomischen Abschnitt von Bakterien nachweisen soll. Die Primer und die Probe sind innerhalb von konservierten Bereichen der 16 S Sequenz gelegt.

### Komponenten:

ThermoPol Buffer 1x

| | | |
|---|---|---|
| Nuk-Makromolekül: | dU-PEG(4)- [T1,A1]-TAMRA | (0,5 µmol/l) |
| Natürliche Nukleotide | (dTTP, dATP, dCTP, dGTP jeweils 200 µmol/l) | |
| Polymerase: | Taq-Polymerase | 1:100 |
| Primer: | Forward Primer | (1 µmol/l) |
| | Reverse Primer | (1 µmol/l) |
| Probe (FAM/ TAMRA) | | (1 µmol/l) |
| Matrize: | genomische DNA von E.coli | (0,5 ng/µl) |
| dT48-Magnetische Beads | | (1 vial) |

Die Zusammensetzung der Primer und der Probe (FAM/TAMRA) entsprach der in der genannten Literaturstelle (siehe Liste der Sequenzen).
Die Vorbereitung von dT48 Beads wurde im vorherigen Beispiel beschrieben. Reagenzien wurden bei RT zusammenpipettiert und auf 95°C erhitzt, dann wurde Taq-Polymerase zugegeben. Im Anschluß darauf wurden PCR-Zyklen durchgeführt nach dem folgenden Schema:
Die ersten 25 Zyklen :

| | | |
|---|---|---|
| Denaturierung: | 95°C | 30 sec |
| Hybridisierung: | 55°C | 1 min |
| Extension: | 70°C | 1 min |

Die darauf folgenden Zyklen (25 Zyklen):

| | | |
|---|---|---|
| Denaturierung: | 95°C | 30 sec |
| Hybridisierung: | 55°C | 1 min |
| Extension 1: | 60°C | 1 min |
| Extension 2: | 70°C | 1 min |
| Halten: | 4°C | |

Die PCR Fragmente wurden zum Teil via Ultrafiltration und / oder via Bindung an die dT48 Magnetische Beads gereinigt.
Die Analyse erfolgte mittels Gel-Elektrophorese (10% Acrylamid-Gel, Acrylamid/Bisacrylamid-Mischung, Rotiphorese Firma Roth) unter denaturierenden Bedingungen bei 90°C bei 150V. Die Detektion der Signale erfolgte mittels TAMRA-Farbstoffe am Nuk-Makromolekül oder durch Färbung der Nukleinsäureketten mit Ethidium-Bromid nach der Elektrophorese.

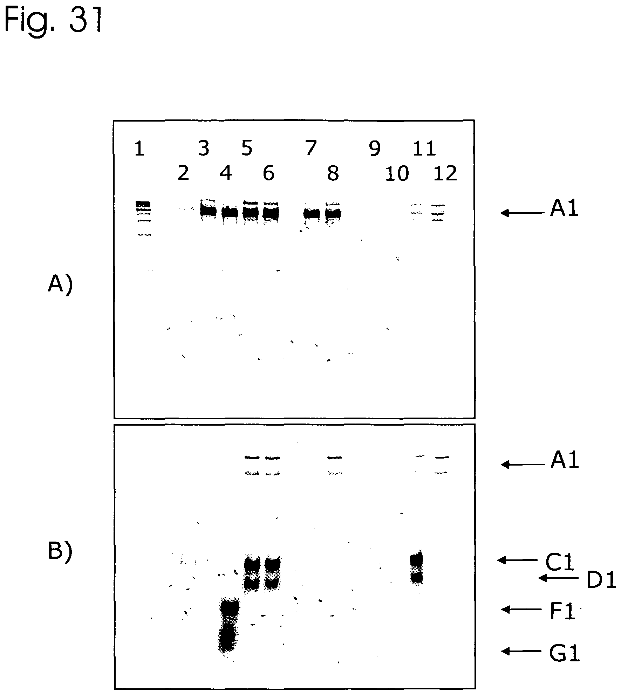

Das Ergebnis der Markierung ist in der Fig. 31 dargestellt.

Die PCR hat unter verwendeten Bedingungen stattgefunden (Banden in Höhe von ca. 500 bp). Die Nuk-Makromoleküle (dU-PEG(4)- [T1,A1]-TAMRA) wurden in die PCR Produkte eingebaut (Pfeil A1, Lane 5 und 6, Fig. 31 B). Dies erfolgte trotz einer relativ hohen Konzentration von dTTP (200 µmol/l am Start der Reaktion). Das zugegebene DMSO 5% hat bei dieser Reaktion keinen Einfluss gehabt (Intensität der Banden in Lane 5 und 6 ist etwa gleich). Zwei Banden mit hoher Molekularmasse entsprechen am ehesten einem vollständigen (höhere Bande) und einem nicht vollständigen markierten PCR-Produkt.

Die als Kontrolle mitgeführte real-time PCR Probe (FAM/TAMRA) wurde nicht eingebaut (lane 4), da sie kein Nuk-Makromolekül ist. Die PCR-Produkte in diesem Ansatz tragen keine fluoreszente Markierung. Die Probe wurde allerding durch die 5'-3'-Exonuklease -Aktivität der Taq Polymerase teilweise abgebaut, da sie während der Reaktion an die Zielsequenz hybridisiert war, wie es für eine Real-Time PCR Probe zu erwarten war.

Die markierten PCR-Produkte können mittels Ultrafiltration MWCO 100 kDa von Primern und nicht eingebauten Nuk-Makromolekülen gereinigt werden (Lane 7 und 8)

Die markierten PCR-Fragmente konnten direkt nach der PCR an die magnetische dT48-Beads über die mit Nuk-Makromolekülen eingeführten Anker-Domäne gebunden werden. Die nicht markierten PCR-Fragmente binden nicht an die dT48 Beads, da sie über keine Anker-Domäne verfügen. Da der Ansatz auch noch nicht verbrauchte Nuk-Makromoleküle enthiet, wurden diese ebenfalls an die Beads gebunden (Lane 11). Die mit Ultrafiltration gereinigten markierten PCR-Fragmente sind frei von Nuk-Makromolekülen und können in sauberer Form durch die Beads isoliert werden (lane 12).

Dieses Beispiel zeigt, wie die für die Real-Time PCR entwickelte Reagenzien und Methoden in Kombination mit Nuk-Makromolekülen eingesetzt werden können.

Nachfolgend sind Literaturquellen angegeben, die Amplifikation von Nukleinsäureketten und Detektion mit spezifischen Sonden beschreiben. Die Zusammensetzung von Primern und Reaktionsbedingungen für die Markeirung mit Nuk-Makrmolekülen können aus der folgenden Literatur entnommen werden. Die zielsequenzspezifsiche Zusammensetzung der Target-Domäne einer jeweiligen Art von Nuk-Makromolekülen kann von der Zusammensetzung der für Real-Time PCR beschriebenen Sonden übernommen bzw. abgeleitet werden.
Auch Literatur-Quellen, die Microarray-Anwendungen beschreiben können auch als Lehre für die Wahl von passenden Target-Domänen sowie Anker-Domänen von Nuk-Makromolekülen verwendet werden. Einige Beispiele solcher Artikel sind ebenfalls angeführt.
Die Literaturquellen sind nach Anwendungsfeldern sortiert, um eine leichtere Übersicht über die potenzielle Applikationsfelder für Nuk-Makromoleküle zu verschaffen. Diese Quellen sollten als Beispiele von potenziellen Anwendungen dienen und nicht zur Einschränkung der Erfindung. Die Literaturquellen wurden in PubMed-Datenbank gefunden.

*Blutgruppen-Bestimmung:* Methods Mol Biol. 2009;496:25-37. (Real-time PCR assays for high-throughput blood group genotyping.),

### Diangnostik und Monitoring von Krebserkrankungen:

Biomed Pharmacother. 2004 Jan;58(1):1-9.(Prediction of hormone sensitivity by DNA microarray.), Breast Cancer. 2006;13(2):123-8. (Basic research for hormonesensitivity of breast cancer.) BMC Mol Biol. 2010 Feb 1;11:12.(Identification of endogenous control genes for normalisation of real-time quantitative PCR data in colorectal cancer.), Int J Oncol. 2006 Feb;28(2):297-306.(Usefulness and clinical significance of quantitative real-time RT-PCR to detect isolated tumor cells in the peripheral blood and tumor drainage blood of patients with colorectal cancer.), Int J Cancer. 2004 Jan 10;108(2):219-27. (Quantitative real-time RT-PCR for detection of disseminated tumor cells in peripheral blood of patients with colorectal cancer using different mRNA markers.), J Biomed Sci. 2010 May 12;17:37.(Rapid detection of epidermal growth factor receptor mutations with multiplex PCR and primer extension in lung cancer.) Endocr Relat Cancer. 2009 Dec;16(4):1241-9. Epub 2009 Sep 11. (Oestrogen receptor 1 mRNA is a prognostic factor in ovarian cancer patients treated with neo-adjuvant chemotherapy: determination by array and kinetic PCR in fresh tissue biopsies.) Prostate. 2009 Jun 1;69(8):810-9. (Quantitative RT-PCR analysis of estrogen receptor gene expression in laser microdissected prostate cancer tissue.) BMC Cancer. 2008 Nov 21;8:339.(Risk estimation of distant metastasis in node-negative, estrogen receptor-positive breast cancer patients using an RT-PCR based prognostic expression signature.) Lung Cancer. 2005 Dec;50(3):375-84. Epub 2005 Sep 30. (Epidermal growth factor receptor gene mutation in non-small cell lung cancer using highly sensitive and fast TaqMan PCR assay.) Am J Manag Care. 2005 May;11(5):313-24. (Economic analysis of targeting chemotherapy using a 21-gene RT-PCR assay in lymph-node-negative, estrogen-receptor-positive, early-stage breast cancer). Int J Oncol. 2004 Apr;24(4):861-7. (Minimal residual disease detection in breast cancer: improved sensitivity using cytokeratin 19 and epidermal growth factor receptor RT-PCR.) Clin Cancer Res. 2003 Nov 1;9(14):5346-57. (Identification of patients with transitional cell carcinoma of the bladder overexpressing ErbB2, ErbB3, or specific ErbB4 isoforms: real-time reverse transcription-PCR analysis in estimation of ErbB receptor status from cancer patients.) J Cell Mol Med. 2009 May 13. [Epub ahead of print] (High sensitivity of both sequencing and real-time PCR analysis of KRAS mutations in colorectal cancer tissue.) Clin Chem Lab Med. 2009;47(5):530-6. (MYC quantitation in cell-free plasma DNA by real-time PCR for gastric cancer diagnosis.) Appl Immunohistochem Mol Morphol. 2009 May;17(3):247-54. (An alternative and reliable real-time quantitative PCR method to determine HER2/neu amplification in breast cancer.) Clin Biochem. 2009 Feb;42(3):194-200. Epub 2008 Nov 6. (The detection of circulating tumor cells of breast cancer patients by using multimarker (Survivin, hTERT and hMAM) quantitative real-time PCR.). Cancer Sci. 2008 Oct;99(10):1977-83.(Detection of colorectal cancer cells from feces using quantitative real-time RT-PCR for colorectal cancer diagnosis.) Eur J Cancer. 2009 Jan;45(1):74-81. Epub 2008 Nov 12. (Harmonisation of multi-centre real-time reverse-transcribed PCR results of a candidate prognostic marker in breast cancer: an EU-FP6 supported study of members of the EORTC - PathoBiology Group.) Med Oncol. 2009;26(3):303-8. Epub 2008 Nov 12. (Real-time quantitative RT-PCR assessment of PIM-1 and hK2 mRNA expression in benign prostate hyperplasia and prostate cancer.) Br J Cancer. 2008 Nov 18;99(10):1644-50. Epub 2008 Oct 28. (Protein kinase Cdelta expression in breast cancer as measured by real-time PCR, western blotting and ELISA.) Gynecol Oncol. 2009 Jan;112(1):55-9. Epub 2008 Oct 26. (The detection of differentially expressed microRNAs from the serum of ovarian cancer patients using a novel real-time PCR platform.) Jpn J Clin Oncol. 2008 Nov;38(11):770-6. Epub 2008 Oct 8. (Quantitative real-time RT-PCR detection for survivin, CK20 and CEA in peripheral blood of colorectal cancer patients.) Hepatogastroenterology. 2008 May-Jun;55(84):1131-5. (Detection of circulating gastric cancer cells in peripheral blood using real time quantitative RT-PCR.) World J Surg Oncol. 2008 Jun 11;6:56. (Real time PCR analyses of expression of E-cadherin, alpha-, beta- and gamma-catenin in human breast cancer for predicting clinical outcome.) Int J Biol Markers. 2008 Jan-Mar;23(1):10-7. (Real-time reverse-transcription PCR to quantify a panel of 19 genes in breast cancer: relationships with sentinel lymph node invasion.) Urol Oncol. 2008 Nov-Dec;26(6):634-40. Epub 2008 Jan 14.(Real-time quantitative RT-PCR assay of prostate-specific antigen and prostate-specific membrane antigen in peripheral blood for detection of prostate cancer micrometastasis.) Lung Cancer. 2008 Feb;59(2):147-54. Epub 2008 Jan 4. (Quantitative reverse transcriptase real-time polymerase chain reaction (qRT-PCR) in translational oncology: lung cancer perspective.) BMC Mol Biol. 2007 Nov 27;8:107.(Evaluation and validation of candidate endogenous control genes for real-time quantitative PCR studies of breast cancer.) J Cancer Res Ther. 2005 Oct-Dec;1(4):221-6.(Fluctuation of circulating tumor cells in patients with lung cancer by real-time fluorescent quantitative-PCR approach before and after radiotherapy.) Clin Chim Acta. 2007 Sep;384(1-2):52-6. Epub 2007 Jun 6. (Development of real-time quantitative reverse transcription-PCR for Her2 detection in peripheral blood from patients with breast cancer.) J Immunol Methods. 2007 Jun 30;323(2):180-93. Epub 2007 May 15. (A multimarker real-time RT-PCR for MAGE-A gene expression allows sensitive detection and quantification of the minimal systemic tumor load in patients with localized cancer.) Neurol Res. 2007 Jul;29(5):435-40. (p53, BCL-2 and BAX in non-small cell lung cancer brain metastases: a comparison of real-time RT-PCR, ELISA and immunohistochemical techniques.) Clin Cancer Res. 2007 Feb 15;13(4):1192-7.(Quantitative detection of micrometastases in pelvic lymph nodes in patients with clinically localized prostate cancer by real-time reverse transcriptase-PCR.)Gastric Cancer. 2006;9(4):308-14. Epub 2006 Nov 24. (Detection of cancer cells disseminated in bone marrow using real-time quantitative RT-PCR of CEA, CK19, and CK20 mRNA in patients with gastric cancer.) Ann N Y Acad Sci. 2006 Sep;1075:230-4.(Quantification of total plasma cell-free DNA in ovarian cancer using real-time PCR.) Br J Cancer. 2006 Jul 17;95(2):218-25. Epub 2006 Jun 6. (Biomarker selection for detection of occult tumour cells in lymph nodes of colorectal cancer patients using real-time quantitative RT-PCR.) Int J Biol Markers. 2006 Jan-Mar;21(1):30-9. (Simultaneous quantitative detection of relevant biomarkers in breast cancer by quantitative real-time PCR.) Int J Cancer. 2006 Oct 1;119(7):1654-9. (A highly specific real-time RT-PCR method for the quantitative determination of CK-19 mRNA positive cells in peripheral blood of patients with operable breast cancer.) Breast Cancer Res Treat. 2009 Dec;118(3):455-68. Epub 2008 Dec 30. (Molecular characterization of circulating tumor cells in large quantities of contaminating leukocytes by a multiplex real-time PCR.) Clin Chem. 2007 Jul;53(7):1206-15. Epub 2007 May 24. (Optimal markers for real-time quantitative reverse transcription PCR detection of circulating tumor cells from melanoma, breast, colon, esophageal, head and neck, and lung cancers.) Mol Diagn Ther. 2006;10(1):41-7. (Detection of circulating tumor cells in peripheral blood of breast cancer patients during or after therapy using a multigene real-time RT-PCR assay.) Anticancer Res. 2006 Mar-Apr;26(2B): 1567-75. (Multigene real-time PCR detection of circulating tumor cells in peripheral blood of lung cancer patients.) Eur J Med Res. 2009 Dec 7;14 Suppl 4:237-40. (Real-time PCR quantification of plasma DNA in non-small cell lung cancer patients and healthy controls.) Lung Cancer. 2009 Apr;64(1):92-7. Epub 2008 Sep 19. (Circulating plasma DNA as diagnostic biomarker in non-small cell lung cancer.) Lung Cancer. 2005 Jul;49(1):1-12. (Circulating tumour-derived DNA and RNA markers in blood: a tool for early detection, diagnostics, and follow-up?) Pathobiology. 2010;77(1):38-45. Epub 2010 Feb 25. (Real-time RT-PCR analysis for evaluating the Her2/neu status in breast cancer.)

### Analysen bei Nutztieren

J Clin Microbiol. 2010 Sep 15. (Specific Detection of Rinderpest Virus by Real-Time RT-PCR in Preclinical and Clinical Samples of Experimentally Infected Cattle.), Water Res. 2010 Mar;44(5):1381-8. (Monitoring bacterial indicators and pathogens in cattle feedlot waste by real-time PCR).,J Virol Methods. 2009 Oct;161(1):122-7. (A short target real-time RT-PCR assay for detection of pestiviruses infecting cattle.),Vet Microbiol. 2008 Nov 25;132(1-2):158-64. (Diagnostic specificity of a real-time RT-PCR in cattle for foot-and-mouth disease and swine for foot-and-mouth disease and classical swine fever based on non-invasive specimen collection.), Mol Cell Probes. 2008 Apr;22(2):90-5. (Real-time multiplex PCR assay for rapid detection and toxintyping of Clostridium perfringens toxin producing strains in feces of dairy cattle.),Foodborne Pathog Dis. 2006 Winter;3(4):337-46.(Development and evaluation of a real-time FRET probe based multiplex PCR assay for the detection of prohibited meat and bone meal in cattle feed and feed ingredients.),
J Virol Methods. 2003 Aug;111(2):95-100.(Detection of carrier cattle and sheep persistently infected with foot-and-mouth disease virus by a rapid real-time RT-PCR assay.)

### Nachweis vo Parasiten (z.B. Marlaria)

Clin Microbiol Infect. 2010 Mar 13.(Multiplex real-time PCR for the diagnosis of malaria: correlation with microscopy.) Exp Parasitol. 2007 Aug;116(4):427-32. (Real-time PCR versus conventional PCR for malaria parasite detection in low-grade parasitemia.) J Clin Microbiol. 2004 Feb;42(2):636-8. (Evaluation of the RealArt Malaria LC real-time PCR assay for malaria diagnosis.) Mol Biochem Parasitol. 2003 Oct;131(2):83-91. (Real-time quantitative PCR for analysis of genetically mixed infections of malaria parasites: technique validation and applications.) J Clin Microbiol. 2002 Nov;40(11):4343-5.(Real-time fluorescence-based PCR for detection of malaria parasites.) Clin Microbiol Infect. 2010 Aug 12. (Validation of a four-primer real-time PCR as a diagnostic tool for single and mixed Plasmodium infections.) Malar J. 2009 Dec 9;8:284. (Multiplex real-time quantitative PCR, microscopy and rapid diagnostic immuno-chromatographic tests for the detection of Plasmodium spp: performance, limit of detection analysis and quality assurance.) Clin Microbiol Infect. 2010 Aug;16(8):1305-11. (Comparison of three real-time PCR methods with blood smears and rapid diagnostic test in Plasmodium sp. infection.) J Clin Microbiol. 2009 Apr;47(4):975-80. (Multiplexed real-time PCR assay for discrimination of Plasmodium species with improved sensitivity for mixed infections.)

### Weirere Parasiten:

Freezing of stool samples improves real-time PCR detection of Entamoeba dispar and Entamoeba histolytica. J Microbiol Methods. 2010 Mar;80(3):310-2., Detection of Clonorchis sinensis in stool samples using real-time PCR. Ann Trop Med Parasitol. 2009 Sep;103(6):513-8., Parasitological diagnosis combining an internally controlled real-time PCR assay for the detection of four protozoa in stool samples with a testing algorithm for microscopy. Clin Microbiol Infect. 2009 Sep;15(9):869-74. Multiplex real-time PCR for the detection and quantification of Schistosoma mansoni and S. haematobium infection in stool samples collected in northern Senegal. Trans R Soc Trop Med Hyg. 2008 Feb;102(2):179-85. Comparison of microscopy, real-time PCR and a rapid immunoassay for the detection of Giardia lamblia in human stool specimens., Clin Microbiol Infect. 2007 Dec;13(12):1186-91. Novel real-time PCr for detection of Schistosoma japonicum in stool. Southeast Asian J Trop Med Public Health. 2006 Mar;37(2):257-64. Real-time PCR in clinical practice: a powerful tool for evaluating Leishmania chagasi loads in naturally infected dogs. Ann Trop Med Parasitol. 2010 Mar;104(2):137-43. The development of a real-time PCR assay for the quantification of Leishmania infantum DNA in canine blood. Vet J. 2009 Nov; 182(2): 356-8. Comparison between quantitative nucleic acid sequence-based amplification, real-time reverse transcriptase PCR, and real-time PCR for quantification of Leishmania parasites.

J Clin Microbiol. 2008 Jan;46(1):73-8. Rapid identification of Leishmania complexes by a real-time PCR assay. Am J Trop Med Hyg. 2005 Dec;73(6):999-1004. Diagnosis of pulmonary infection with Toxoplasma gondii in immunocompromised HIV-positive patients by real-time PCR. Eur J Clin Microbiol Infect Dis. 2006 Jun;25(6):401-4.

Real-time PCR for quantitative detection of Toxoplasma gondii. J Clin Microbiol. 2000 Nov;38(11):4121-5. Development of a real-time PCR for the differentiation of the G1 and G2/G3 genotypes of Echinococcus granulosus. Parasitol Res. 2009 Jul;105(1):255-9. Epub 2009 Mar 14.

### Nachweis von Batterien (z.B. bei einer Meningitis):

Real-time PCR detection of five prevalent bacteria causing acute meningitis. APMIS. 2009 Nov;117(11):856-60. Rapid detection of eight causative pathogens for the diagnosis of bacterial meningitis by real-time PCR. J Infect Chemother. 2009 Apr;15(2):92-8. Increased detection rate in diagnosis of herpes simplex virus type 2 meningitis by real-time PCR using cerebrospinal fluid samples. J Clin Microbiol. 2007 Aug;45(8):2516-20. Broad-range real time PCR and DNA sequencing for the diagnosis of bacterial meningitis. Scand J Infect Dis. 2006;38(1):27-35. Rapid diagnosis of bacterial meningitis by using multiplex PCR and real time PCR. Pediatr Int. 2004 Oct;46(5):551-4. Rapid real-time PCR for determination of penicillin susceptibility in pneumococcal meningitis, including culture-negative cases. J Clin Microbiol. 2002 Feb;40(2):682-4. Simultaneous detection of Neisseria meningitidis, Haemophilus influenzae, and Streptococcus pneumoniae in suspected cases of meningitis and septicemia using real-time PCR. J Clin Microbiol. 2001 Apr;39(4):1553-8.

### Nachweis von Durchfallerregern:

Multiplex real-time RT-PCR for the simultaneous detection and quantification of transmissible gastroenteritis virus and porcine epidemic diarrhea virus. J Virol Methods. 2007 Dec;146(1-2):172-7. A one-step multiplex real-time RT-PCR for detection and typing of bovine viral diarrhea viruses. Vet Microbiol. 2006 Aug 25;116(1-3):37-44. Prospective multicenter evaluation of a new immunoassay and real-time PCR for rapid diagnosis of Clostridium difficile-associated diarrhea in hospitalized patients. J Clin Microbiol. 2005 Oct;43(10):5338-40. Increased detection of rotavirus using a real time reverse transcription-polymerase chain reaction (RT-PCR) assay in stool specimens from children with diarrhea. J Med Virol. 2004 Mar;72(3):496-501.,Real-time PCR for simultaneous detection and genotyping of bovine viral diarrhea virus. J Virol Methods. 2003 Dec;114(1):21-7. Development of a serogroup-specific DNA microarray for identification of Escherichia coli strains associated with bovine septicemia and diarrhea. Vet Microbiol. 2010 May 19;142(3-4):373-8. A novel DNA microarray for rapid diagnosis of enteropathogenic bacteria in stool specimens of patients with diarrhea. J Microbiol Methods. 2008 Dec;75(3):566-71. DNA microarray for direct identification of bacterial pathogens in human stool samples. Digestion. 2008;78(2-3):131-B.Development of a panel of multiplex real-time polymerase chain reaction assays for simultaneous detection of major agents causing calf diarrhea in feces. J Vet Diagn Invest. 2010 Jul;22(4):509-17. A set of novel multiplex Taqman real-time PCRs for the detection of diarrhoeagenic Escherichia coli and its use in determining the prevalence of EPEC and EAEC in a university hospital. Ann Clin Microbiol Antimicrob. 2010 Jan 22;9:5.Comprehensive and rapid real-time PCR analysis of 21 foodborne outbreaks. Int J Microbiol. 2009;2009:917623. Detection of noroviruses in fecal specimens by direct RT-PCR without RNA purification J Virol Methods. 2010 Feb;163(2):282-6. Broadly reactive TaqMan assay for real-time RT-PCR detection of rotavirus in clinical and environmental samples. JIN2@cdc.gov. J Virol Methods. 2009 Feb;155(2):126-31. A real-time PCR assay for the detection of Salmonella in a wide variety of food and food-animal matricest.J Food Prot. 2007 May;70(5):1080-7. Real-time reverse transcription-PCR for detection of rotavirus and adenovirus as causative agents of acute viral gastroenteritis in children.J Clin Microbiol. 2006 Sep;44(9):3189-95.

### Nachweis von Erregern einer Sepsis:

DNA microarray for the identification of pathogens causing bloodstream infections.Expert Rev Mol Diagn. 2010 Apr;10(3):263-8. DNA microarray-based identification of bacterial and fungal pathogens in bloodstream infections. Mol Cell Probes. 2010 Feb;24(1):44-52. Identification and characterization of bacterial pathogens causing bloodstream infections by DNA microarray. J Clin Microbiol. 2006 Jul;44(7):2389-97. Detecting sepsis-associated bloodstream infection acquired in intensive care using multi-pathogen real-time PCR. J Infect. 2009 Oct;59(4):296-8. Detection of bloodstream infection in neonatal foals with suspected sepsis using real-time PCR. Vet Rec. 2009 Jul 25;165(4):114-7. Multiplex real-time PCR and blood culture for identification of bloodstream pathogens in patients with suspected sepsis. Clin Microbiol Infect. 2009 Jun;15(6):544-51. Diagnosis of bloodstream infections in immunocompromised patients by real-time PCR. J Infect. 2009 May;58(5):346-51. Improved detection of blood stream pathogens by real-time PCR in severe sepsis. Intensive Care Med. 2010 Jan;36(1):49-56. Detecting sepsis-associated bloodstream infection acquired in intensive care using multi-pathogen real-time PCR. J Infect. 2009 Oct;59(4):296-8. Laboratory diagnosis of late-onset sepsis in newborns by multiplex real-time PCR. J Med Microbiol. 2009 Apr;58(Pt 4):533-4. Molecular identification of bloodstream pathogens in patients presenting to the emergency department with suspected sepsis. Shock. 2010 Jul;34(1):27-30. Multiplex PCR to diagnose bloodstream infections in patients admitted from the emergency department with sepsis. J Clin Microbiol. 2010 Jan;48(1):26-33. Improved detection of blood stream pathogens by real-time PCR in severe sepsis. Intensive Care Med. 2010 Jan;36(1):49-56. Preliminary clinical study using a multiplex real-time PCR test for the detection of bacterial and fungal DNA directly in blood. Clin Microbiol Infect. 2010 Jun;16(6):774-9. Utility of a commercially available multiplex real-time PCR assay to detect bacterial and fungal pathogens in febrile neutropenia. J Clin Microbiol. 2009 Aug;47(8):2405-10. Rapid diagnosis of sepsis and bacterial meningitis in children with real-time fluorescent quantitative polymerase chain reaction amplification in the bacterial 16S rRNA gene. Clin Pediatr (Phila). 2009 Jul;48(6):641-7, Multiplex real-time PCR and blood culture for identification of bloodstream pathogens in patients with suspected sepsis. Clin Microbiol Infect. 2009 Jun;15(6):544-51. Detection of cytomegalovirus in whole blood using three different real-time PCR chemistries. J Mol Diagn. 2009 Jan;11(1):54-9. A multiplex real-time PCR assay for rapid detection and differentiation of 25 bacterial and fungal pathogens from whole blood samples. Med Microbiol Immunol. 2008 Sep;197(3):313-24.,

### Nachweis von Viren, z.B. HIV oder HPV:

Comparison of real-time PCR methods for measurement of HIV-1 proviral DNA. J Virol Methods. 2010 Mar;164(1-2):135-8.Real-time RT-PCR for automated detection of HIV-1 RNA during blood donor screening. Methods Mol Biol. 2010;630:319-35.Low-cost HIV-1 diagnosis and quantification in dried blood spots by real time PCR. PLoS One. 2009 Jun 5;4(6):e5819. Single-point mutations causing more than 100-fold underestimation of human immunodeficiency virus type 1 (HIV-1) load with the Cobas TaqMan HIV-1 real-time PCR assay. J Clin Microbiol. 2009 Apr;47(4):1238-40. Detection and quantitation of HPV in genital and oral tissues and fluids by real time PCR. Virol J. 2010 Aug 19;7:194. Evaluation of a prototype real-time PCR assay for carcinogenic human papillomavirus (HPV) detection and simultaneous HPV genotype 16 (HPV16) and HPV18 genotyping. J Clin Microbiol. 2009 Oct;47(10):3344-7. High-throughput two-step LNA real time PCR assay for the quantitative detection and genotyping of HPV prognostic-risk groups. J Clin Virol. 2009 Aug;45(4):304-10. Study comparing human papillomavirus (HPV) real-time multiplex PCR and Hybrid Capture II INNO-LiPA v2 HPV genotyping PCR assays. J Clin Microbiol. 2009 Jul;47(7):2106-13. Detection and differentiation of human papillomavirus genotypes HPV-6 and HPV-11 by FRET-based real-time PCR. J Virol Methods. 2008 Nov;153(2):245-9.

Molecular beacon-based real-time PCR method for detection of 15 high-risk and 5 low-risk HPV types. J Virol Methods. 2008 Apr; 149(1): 153-62. Real-time PCR assays using internal controls for quantitation of HPV-16 and beta-globin DNA in cervicovaginal lavages.J Virol Methods. 2003 Dec;114(2):135-44.

### Nachweis von Nukleinsäureketten aus unterschiedlichen biologischen Proben und Materialien, z. B. aus Stuhl, Abstrichen, Urin oder Atemwegen:

Comparison of microscopy, two xenic culture techniques, conventional and real-time PCR for the detection of Dientamoeba fragilis in clinical stool samples. Eur J Clin Microbiol Infect Dis. 2010 Apr;29(4):411-6. Epub 2010 Feb 14. Freezing of stool samples improves real-time PCR detection of Entamoeba dispar and Entamoeba histolytica. J Microbiol Methods. 2010 Mar;80(3):310-2. Epub 2010 Jan 18. Detection of Clonorchis sinensis in stool samples using real-time PCR. Ann Trop Med Parasitol. 2009 Sep;103(6):513-8. Rapid and sensitive detection of Shiga toxin-producing Escherichia coli from nonenriched stool specimens by real-time PCR in comparison to enzyme immunoassay and culture. J Clin Microbiol. 2009 Jul;47(7):2008-12. Epub 2009 May 13. Direct detection of Campylobacter jejuni in human stool samples by real-time PCR. Can J Microbiol. 2008 Sep;54(9):742-7. Enhancement of detection and quantification of rotavirus in stool using a modified real-time RT-PCR assay. J Med Virol. 2008 Aug;80(8):1489-96. Detection of methicillin-resistant Staphylococcus aureus directly from nasal swab specimens by a real-time PCR assay. J Clin Microbiol. 2004 Dec;42(12):5578-81.Detection and quantitation of HPV in genital and oral tissues and fluids by real time PCR. Virol J. 2010 Aug 19;7:194. Study comparing human papillomavirus (HPV) real-time multiplex PCR and Hybrid Capture II INNO-LiPA v2 HPV genotyping PCR assays. J Clin Microbiol. 2009 Jul;47(7):2106-13. Epub 2009 May 6. Detection and differentiation of human papillomavirus genotypes HPV-6 and HPV-11 by FRET-based real-time PCR. J Virol Methods. 2008 Nov;153(2):245-9. Epub 2008 Sep 4. Molecular beacon-based real-time PCR method for detection of 15 high-risk and 5 low-risk HPV types. J Virol Methods. 2008 Apr;149(1):153-62. Epub 2008 Feb 20. Diagnosis of amebic liver abscess and amebic colitis by detection of Entamoeba histolytica DNA in blood, urine, and saliva by a real-time PCR assay. J Clin Microbiol. 2010 Aug;48(8):2798-801. Epub 2010 Jun 9. Validation of a laboratorydeveloped real-time PCR protocol for detection of Chlamydia trachomatis and Neisseria gonorrhoeae in urine. Sex Transm Infect. 2010 Jun;86(3):207-11. Detection of dengue virus in saliva and urine by real time RT-PCR. Virol J. 2010 Jan 27;7:22. Endocervical swabs transported in first void urine as combined specimens in the detection of Mycoplasma genitalium by real-time PCR.

J Med Microbiol. 2009 Jan;58(Pt 1):117-20. Detection of Leishmania infantum DNA by fret-based real-time PCR in urine from dogs with natural clinical leishmaniosis. Vet Parasitol. 2007 Jul 20;147(3-4):315-9. Epub 2007 May 25. Real-time PCR assay using specimens on filter disks as a template for detection of cytomegalovirus in urine. J Clin Microbiol. 2007 Apr;45(4):1305-7. Epub 2007 Feb 7. Real-time PCR assay for detection of quinolone-resistant Neisseria gonorrhoeae in urine samples. J Clin Microbiol. 2007 Apr;45(4):1250-4. Epub 2007 Jan 31. Human papillomavirus quantification in urine and cervical samples by using the Mx4000 and LightCycler general real-time PCR systems. J Clin Microbiol. 2007 Mar;45(3):897-901. Epub 2007 Jan 17. A novel real-time PCR to detect Chlamydia trachomatis in first-void urine or genital swabs. J Med Microbiol. 2006 Dec;55(Pt 12):1667-74. Quantitative detection of Escherichia coli from urine of patients with bacteriuria by real-time PCR. Mol Diagn. 2004;8(3):179-84. Use of the Roche LightCycler instrument in a real-time PCR for Trichomonas vaginalis in urine samples from females and males. J Clin Microbiol. 2003 Dec;41(12):5619-22. Isolation and detection of Borrelia burgdorferi DNA from cerebral spinal fluid, synovial fluid, blood, urine, and ticks using the Roche MagNA Pure system and real-time PCR. Diagn Microbiol Infect Dis. 2003 Aug;46(4):235-40. Quantitative detection of Mycoplasma genitalium from first-pass urine of men with urethritis and asymptomatic men by real-time PCR. J Clin Microbiol. 2002 Apr;40(4):1451-5. Rapid detection and quantification of CMV DNA in urine using LightCycler-based real-time PCR. J Clin Virol. 2002 Feb;24(1-2):131-4. Direct detection of Pseudomonas aeruginosa from patients with healthcare associated pneumonia by real time PCR. Infect Genet Evol. 2010 Aug 20. Quadruplex real-time quantitative PCR assay for the detection of pathogens related to late-onset ventilatorassociated pneumonia: a preliminary report. J Microbiol Methods. 2010 Jun;81(3):232-4. Epub 2010 Mar 28. Usefulness of real-time PCR for lytA, ply, and Spn9802 on plasma samples for the diagnosis of pneumococcal pneumonia. Clin Microbiol Infect. 2010 Aug;16(8):1135-41. Epub 2009 Oct 14. Real-time PCR is more specific than conventional PCR for induced sputum diagnosis of Pneumocystis pneumonia in immunocompromised patients without HIV infection. Respirology. 2009 Mar;14(2):203-9. Epub 2008 Dec 11. Comprehensive detection of causative pathogens using real-time PCR to diagnose pediatric community-acquired pneumonia. J Infect Chemother. 2008 Dec;14(6):424-32. Epub 2008 Dec 17. Simultaneous detection of pathogens in clinical samples from patients with community-acquired pneumonia by real-time PCR with pathogen-specific molecular beacon probes. J Clin Microbiol. 2006 Apr;44(4):1440-6.

Assessment of real-time PCR for diagnosis of Mycoplasma pneumoniae pneumonia in pediatric patients. Can J Microbiol. 2006 Feb;52(2):125-9.

### Nachweis von Nukleinsäureketten aus Lebensmitteln:

Total and pathogenic Vibrio parahaemolyticus in shrimp: Fast and reliable quantification by real-time PCR. Int J Food Microbiol. 2010 Aug 25. Quadruplex real-time PCR assay for detection and identification of Vibrio cholerae O1 and 0139 strains and determination of their toxigenic potential. Appl Environ Microbiol. 2009 Nov;75(22):6981-5. Evaluation of a real-time PCR assay for the detection and quantification of Bacillus cereus group spores in food. J Food Prot. 2010 Aug;73(8):1480-5. Pentaplexed quantitative real-time PCR assay for the simultaneous detection and quantification of botulinum neurotoxin-producing clostridia in food and clinical samples. Appl Environ Microbiol. 2010 Jul;76(13):4387-95. Design of a new universal real-time PCR system targeting the tuf gene for the enumeration of bacterial counts in food. J Food Prot. 2010 Apr;73(4):670-9.

Rapid detection and differentiation of Campylobacter jejuni, Campylobacter coli, and Campylobacter lari in food, using multiplex real-time PCR. J Food Prot. 2010 Feb;73(2):241-50. Validation of a newly developed hexaplex real-time PCR assay for screening for presence of GMOs in food, feed and seed. Anal Bioanal Chem. 2010 Mar;396(6):2103-12. Multiplex real-time PCR using SYBR((R)) GreenER for the detection of DNA allergens in food. Anal Bioanal Chem. 2010 Jan 20. A real-time PCR method for the detection of Salmonella enterica from food using a target sequence identified by comparative genomic analysis. Int J Food Microbiol. 2010 Feb 28;137(2-3):168-74. Detection of allergen walnut component in food by an improved real-time PCR method. J Food Prot. 2009 Nov;72(11):2433-5. Real-time FRET PCR assay for Salmonella enterica serotype detection in food. Mil Med. 2009 Sep;174(9):983-90.

Development and validation of a duplex real-time PCR method to simultaneously detect potentially allergenic sesame and hazelnut in food. J Agric Food Chem. 2009 Mar 25;57(6):2126-34. Real-time PCR method using capturing oligo-immobilized PCR tubes to determine the specific gene for soybean and genetically modified soybean in food matrices. Biosci Biotechnol Biochem. 2008 Nov;72(11):2953-8. TaqMan-based real-time PCR method for detection of Yersinia pseudotuberculosis in food. Appl Environ Microbiol. 2008 Oct;74(20):6465-9 Real-time PCR for quantitative detection of bovine tissues in food and feed. J Food Prot. 2008 Mar;71(3):564-72. Development of a real-time PCR method to detect potentially allergenic sesame (Sesamum indicum) in food. J Agric Food Chem. 2007 Dec 26;55(26):10540-7. A novel real-time PCR-based method for the detection of Listeria monocytogenes in food. Lett Appl Microbiol. 2007 Nov;45(5):568-73.

A real-time PCR assay for the detection of Salmonella in a wide variety of food and food-animal matricest. J Food Prot. 2007 May;70(5):1080-7.

### Nachweis von Toxinen oder Resistenzgenen:

Quantification of Shiga toxin-converting bacteriophages in wastewater and in fecal samples by real-time quantitative PCR.

Appl Environ Microbiol. 2010 Sep;76(17):5693-701. Genetic diagnosis of community-acquired MRSA: a multiplex real-time PCR method for Staphylococcal cassette chromosome mec typing and detecting toxin genes. Tohoku J Exp Med. 2010;220(2):165-70. A quadruplex real-time PCR assay for rapid detection and differentiation of the Clostridium botulinum toxin genes A, B, E and F. J Med Microbiol. 2010 Jan;59(Pt 1):55-64. Epub .Rapid and sensitive detection of Shiga toxin-producing Escherichia coli from nonenriched stool specimens by real-time PCR in comparison to enzyme immunoassay and culture. J Clin Microbiol. 2009 Jul;47(7):2008-12. Epub 2009 May 13. Development of a real-time PCR assay with an internal amplification control for the screening of Shiga toxin-producing Escherichia coli in foods. Lett Appl Microbiol. 2009 May;48(5):554-9. Epub 2009 Feb 9. Detection of Mycobacterium tuberculosis resistance mutations to rifampin and isoniazid by real-time PCR. Indian J Med Microbiol. 2010 Jul-Sep;28(3):211-6. Rapid detection of influenza A pandemic (H1N1) 2009 virus neuraminidase resistance mutation H275Y by real-time reverse transcriptase PCR. J Clin Microbiol. 2010 May;48(5):1884-7. Novel mixed-format real-time PCR assay to detect mutations conferring resistance to triazoles in Aspergillus fumigatus and prevalence of multi-triazole resistance among clinical isolates in the Netherlands. J Antimicrob Chemother. 2010 May;65(5):901-5 Detection of fluoroquinolone resistance level in clinical canine and feline Escherichia coli pathogens using rapid real-time PCR assay. Vet Microbiol. 2009 Nov 18;139(3-4):379-85. Real-time PCR assays for monitoring benzimidazole resistance-associated mutations in Ancylostoma caninum. Exp Parasitol. 2009 May;122(1):6-10.

Quantification of genes encoding resistance to aminoglycosides, beta-lactams and tetracyclines in wastewater environments by real-time PCR. Int J Environ Health Res. 2009 Jun;19(3):219-30. Rapid detection of Staphylococcus aureus bacteremia and methicillin resistance by real-time PCR in whole blood samples. Eur J Clin Microbiol Infect Dis. 2009 Aug;28(8):1001-5. Quadruplex real-time PCR assay using allelespecific scorpion primers for detection of mutations conferring clarithromycin resistance to Helicobacter pylori. J Clin Microbiol. 2008 Jul;46(7):2320-6. Rapid identification of penicillin and macrolide resistance genes and simultaneous quantification of Streptococcus pneumoniae in purulent sputum samples by use of a novel real-time multiplex PCR assay. J Clin Microbiol. 2008 Jul;46(7):2384-8. Routine use of real-time PCR for detection of Helicobacter pylori and öf clarithromycin resistance mutations. Gastroenterol Clin Biol. 2007 Oct;31(10):792-5. Detection of azithromycin resistance in Treponema pallidum by real-time PCR. Antimicrob Agents Chemother. 2007 Sep;51(9):3425-30.Detection of meticillin-resistant staphylococcus aureus (MRSA) colonization in newborn infants using real-time polymerase chain reaction (PCR). Acta Paediatr. 2010 Jun 2. Detection limits of a rapid MRSA detection assay based on multiplex real-time PCR. J Infect Chemother. 2010 Jun;16(3):223.

### 1.5.15.7 Markierung von Zielsequenzen durch an einer festen Phase gebundenen Nuk-Makromoleküle

Diese Reaktion wurde ähnlich wie im Beispiel 1.5.15.1 durchgeführt. Die Zielsequenz (Matrize 2, M2) wurde mit einem Primer und Nuk-Makromolekül (dU-PEG(8)-[T1,A1]-TAMRA) hybridisiert und anschließend spezifisch über die Anker-Domäne der Nuk-Makromoleküle an die feste Phase gebunden. Dieses Nuk-Makromolekül hat dUTP als Nuk-Komponente, eine Target-Domäne, die an die Zielsequenz binden kann, eine Anker-Domäne (dA25), sowie einen Fluorescenzfarbstoff (TAMRA) am 3'-Ende der Anker-Domäne, der als Signal-Domäne dient. Es wurde eine feste Phase verwendet, die diese Anker-Domäne binden kann (dT48-Magnetische Beads, Vorbereitung siehe Abschnitt 1.5.15.5). Nach der Bindung wurde die feste Phase mit Einbaupuffer 1 gewaschen und im Einbaupuffer 1 suspendiert.
Zu dieser festen Phase mit gebundenen Reaktionskomponenten wurden natürliche Nukleotide (dATP, dCTP, dGTP jeweils 100 µmol/l) zugegeben. Das dTTP wurde in Reaktion 1 (Lane 1) weggelassen und in Reaktion 2 (lane 2) bis 100 µmol/l zugegeben. Die Reaktion wurde durch Zugabe von Klenow Fragment exo minus (1:50) gestartet und verlief bei RT für 30 min. Nach der Reaktion wurde die feste Phase direkt auf das Gel geladen und Produkte wurden mittels denaturierender Elektrophorese getrennt unter 85°C.

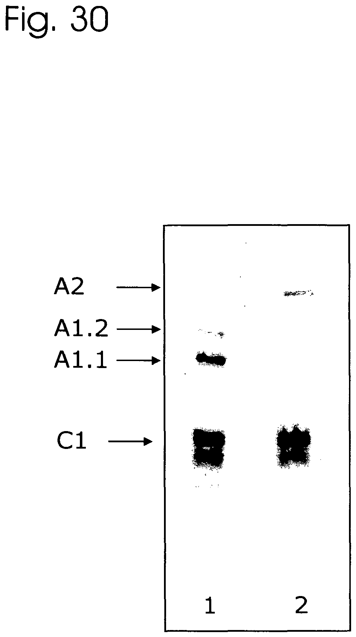

Das Ergebnis ist in der Fig. 30 abgebildet.

Man sieht den Einbau von Nuk-Makromolekülen in den verlägerten Primer, wobei bei fehlendem dTTP keine vollständige Synthese des komplementären Stranges erfolgte (Pfeil A1.1 und A1.2, Lane 1). In Anwesenheit von dTTP konnte Klenow Fragment exo minus die Strandsynthese in voller Länge ausführen (Pfeil A2, Lane 2). Da in diesem Experiment das Nuk-Makromolekül einen Fluoresezenzfarbstoff trägt, sieht man das nicht in der Reaktion vebrauchte Nukleotid (Pfeil C1). Die Unvollständigkeit des Verbrauchs von Nuk-Makromolekül wird auf die sterischen Einflüsse der Oberfläche zurückgeführt.

### Liste mit verwendeten Sequenzen:

### Bezeichnung, Modifikationen, Sequenz

### Primer und Proben / Sonden / Anker

Primer T7-19: 5'- taatacgactcactatagg
Primer T7-19-Cy3: 5'-Cy3- taatacgactcactatagg
Primer dA50-T7-19: 5'- aaaaaaaaaa aaaaaaaaaa aaaaaaaaaa aaaaaaaaaa aaaaaaaaaa taatacgactcactatagg
U19 Primer : 5' - GTT TTC CCA GTC ACG ACG
Primer forward: 5'-tcc tac ggg agg cag cagt
Primer reverse: 5'-gga cta cca ggg tat cta atc ctg tt
Probe (FAM/TAMRA): 5' FAM-cgt att acc gcg gct gct gg cac-TAMRA
dT48-Biotin (Bindungspartner für Anker-Domäne 1)
   : 5 'tttttttttt tttttttttt tttttttttt tttttttttt tttttttt-Biotin
B1-Biotin-Anker (Bindungspartner für Anker-Domäne 2)
   5'-Biotin- agtgaattcgagctcggtaC

### Oligonukleotid-Komponente der Nuk-Makromoleküle

[T1, A1]-TAMRA : Target-Domäne-1, Anker-Domäne-1, TAMRA 5' NH2-cgt att acc gcg gct gct gg cac AAAAAAAAAA AAAAAAAAAA AAAAA -TAMRA
[T2, A2]-TAMRA : Target-Domäne-2, Anker-Domäne-2, TAMRA, 5'NH2-CGAGACGAAATGGGAtttttttttttttttttttt-3'TAMRA
[T3, A3]-FAM : Target-Domäne-3, Anker-Domäne-3, Fluorescein, 5'NH2-AAA AAA ACT gcg gct gct gg cac Gtaccgagctcgaattcact -FAM

### Verwendete Matrizen:

Matrize 1 (M1): 
5´ GTT TTC CCA GTC ACG ACG  GGAG  gtg cc agc agc cgc ggt aat acg ACCA
cctatagtgagtcgtatta
Matrize 2 (M2): 
5´ GTT TTC CCA GTC ACG ACG  GGAG  gtg cc agc agc cgc ggt aat acg AGT CTT CTCA
cctatagtgagtcgtatta
Matrize 3 (M3): 
5´ GTT TTC CCA GTC ACG ACG  GGAG  gtg cc agc agc  ggt aat acg AGT CTT CTGA
cctatagtgagtcgtatta
Matrize 4 (M4): 
5′ GTT TTC CCA GTC ACG ACG  GGAG  gtg cc agc ggt aat acg AGT CTT CTGA
cctatagtgagtcgtatta
Matrize 5 (M5): 
5´ GTT TTC CCA GTC ACG ACG  GGAG  gtg cc agc agc AAA AAA aat acg AGT CTT
CTGA cctatagtgagtcgtatta
Matrize 6 (M6): 
5´ GTT TTC CCA GTC ACG ACG  GGAG  gtg Ac agc Agc cgc Agt aat Acg AGT CTT CTGA
cctatagtgagtcgtatta
Matrize 7 (M7): 
5´ GTT TTC CCA GTC ACG ACG  GGAG  gtg cc agc agc cgc AAA AAA AAA AGT CTT
CTGA cctatagtgagtcgtatta
Matrize 8 (M8): 
5′ GTT TTC CCA GTC ACG ACG  GGAG  gtg cc agc agc cgc AGT TTT TTT AGT CTT
CTGA cctatagtgagtcgtatta
Matrize 9 (M9): 
5´ GTT TTC CCA GTC ACG ACG GGAG cgc ggt aat acg AGT CTT CTCA
cctatagtgagtcgtatta
Matrize 10 (M10)
   5'-(A)₄₈ TCC CAT TTC GTC TCG TTC CGC TTT GTcctatagtgagtcgtatta

Alle Publikationen, Patente und Patentanmeldungen, die hier zitiert wurden, sind eingebaut in diese Anmeldung in vollem Umfang (auch wenn dies bei jeweiliger Publikation nicht expliziert genannt wurde) und unterliegen laut USPTO den Regelungen für "incorporated by reference" für alle Zwecke in den USA.

Einzelne Ausführungsformen sollten zur Veranschaulichung der Erfindung dienen und können vom Fachmann weiter miteinander kombiniert werden. Die Kombinationen aus einzelnen Ausführungsformen stellen ebenfalls gegenstand der vorliegenden Erfindung dar.

### Legenden für Figuren

### Fig. 26 zum Beispiel 1.5.15.1 Einbau in Anwesenheit von konkurrierenden Nukleotiden

Abbildung eines Gels nach elektrophoretischer Auftrennung der Reaktionsprodukte. Nachfolgend sind Komponenten einzelner Reaktionen angegeben, die außer der Matrize, Primer und Polymerase zugegeben wurden. Einzelne Lanes entsprechen einzelnen Reaktionen.
1. + dU-PEG(8)-[T1,A1]-TAMRA
2. + dU-PEG(8)-[T1,A1]-TAMRA + dTTP 10 µmol/l
3. + dU-PEG(8)-[T1,A1]-TAMRA + dTTP 100 µmol/l
4. + dU-PEG(8)-[T1,A1]-TAMRA + dTTP 1 mmol/l
5. + dU-PEG(8)-[T1,A1]-TAMRA + dTTP 100 µmol/l) + dGTP 100 µmol/l)
6. + dU-PEG(8)-[T1,A1]-TAMRA + dTTP 1 mmol/l + dGTP 100 µmol/l)
7. + dU-PEG(8)-[T1,A1]-TAMRA + dTTP + dATP + dCTP + dGTP je 100 µmol/l)
8. Kontroll: nur dU-PEG(8)-[T1,A1]-TAMRA , keine Polymerase

Pfeil (A) zeigt die Position des verlängerten Primers mit dem eingebauten dU-PEG(8)-[T1,A1]-TAMRA , Pfeil (B) zeigt die Position des dU-PEG(8)-[T1,A1]-TAMRA im Gel.

### Fig. 27 zum Beispiel 1.5.15.2 Konkurrenz mit 10 mmol/l dTTP

Abbildung eines Gels nach elektrophoretischer Auftrennung der Reaktionsprodukte. Nachfolgend sind Komponenten einzelner Reaktionen angegeben, die außer der Matrize, Primer, dATP, dGTP, dCTP (je 100 µmol/l) und Polymerase (einzelne Konzentrationen siehe unten) zugegeben wurden, sowie die Bewertung der Einbaureaktion (Einbau / kaum Einbau/ Kein Einbau usw.). Einzelne Lanes entsprechen einzelnen Reaktionen.
Klenow 1:10

| | |
|---|---|
| 1. + dU-PEG(8)-[T1,A1]-TAMRA | Einbau |
| 2. + dU-PEG(8)-[T1,A1]-TAMRA + dTTP 100 µmol/l | Einbau |
| 3. + dU-PEG(8)-[T1,A1]-TAMRA + dTTP 10 mmol/l | Einbau |

Klenow 1:100

| | |
|---|---|
| 4. + dU-PEG(8)-[T1,A1]-TAMRA | Einbau |
| 5. + dU-PEG(8)-[T1,A1]-TAMRA + dTTP 100 µmol/l | Einbau |
| 6. + dU-PEG(8)-[T1,A1]-TAMRA + dTTP 10 mmol/l | Einbau |

Klenow 1:1000

| | |
|---|---|
| 7. + dU-PEG(8)-[T1,A1]-TAMRA | Einbau |
| 8. + dU-PEG(8)-[T1,A1]-TAMRA + dTTP 100 µmol/l | Einbau |
| 9. + dU-PEG(8)-[T1,A1]-TAMRA + dTTP 10 mmol/l | kaum Einbau |

Taq 1:100

| | |
|---|---|
| 10. + dU-PEG(8)-[T1,A1]-TAMRA | Einbau |
| 11. + dU-PEG(8)-[T1,A1]-TAMRA + dTTP 100 µmol/l | Einbau |
| 12. + dU-PEG(8)-[T1,A1]-TAMRA + dTTP 10 mmol/l | kaum Einbau |

Vent exo - 1:100

| | |
|---|---|
| 13. + dU-PEG(8)-[T1,A1]-TAMRA + dTTP 10 mmol/l | kein Einbau |
| 14. + dU-PEG(8)-[T1,A1]-TAMRA + dTTP 100 µmol/l | Einbau |
| 15. + dU-PEG(8)-[T1,A1]-TAMRA | Einbau |

Pfeil (A1) zeigt die Position des vollständig verlängerten Primers mit dem eingebauten dU-PEG(8)-[T1,A1]-TAMRA . Pfeil (A2) zeigt die Position des teilweise verlängerten Primers mit dem eingebauten dU-PEG(8)-[T1,A1]-TAMRA . Pfeil (B) zeigt die Position des dU-PEG(8)-[T1,A1]-TAMRA im Gel.

### Fig. 28 zum Beispiel 1.5.15.4 Zyklische Markierungsreaktion, Unterschiedliche Zielsequenzen, Unterschiedliche Temperaturen

Abbildung eines Gels nach elektrophoretischer Auftrennung der Reaktionsprodukte. Einzelne Lanes entsprechen einzelnen Reaktionen.
Fig. 28, M2
   (Reaktionen mit Matrize 2, M2)
   1. Zyklische (20 Zyklen) Primer-Verlängerung mit Taq-Polymerase und folgenden Komponenten: M2, Primer, vier dNTPs, dU-PEG(4)-[T1,A1]-TAMRA;
   2. Primer-Verlängerung mit Taq-Polymerase und folgenden Komponenten: M2, Primer, vier dNTPs, ohne dU-PEG(4)-[T1,A1]-TAMRA;
   3. Leiter: dU-PEG(4)-[T1,A1]-TAMRA, markierter Primer

   Pfeil A1: das mit dU-PEG(4)-[T1,A1]-TAMRA markierte Produkt der Primer-Extension (keine vollständige Strangverlängerung)
   Pfeil B1: das Produkt der Primer-extension ohne dU-PEG(4)-[T1,A1]-TAMRA (Markierung durch Primer-Cy3)
   Pfeil C1: dU-PEG(4)-[T1,A1]-TAMRA (Nuk-Makromolekül)
   Pfeil D1: das Produkt des Abbau von dU-PEG(4)-[T1,A1]-TAMRA durch 5'-3'-Exonuklease-Aktivität der Taq-Polymerase
   Pfeil E1: der markierte Primer (T7-19-Cy3)
Fig. 28, M4
   (Reaktionen mit Matrize 4, M4)
   1. Zyklische (20 Zyklen) Primer-Verlängerung mit Taq-Polymerase bei 35°C und folgenden Komponenten: M4, Primer, vier dNTPs, dU-PEG(4)-[T1,A1]-TAMRA;
   2. Zyklische (20 Zyklen) Primer-Verlängerung mit Taq-Polymerase bei 45°C und folgenden Komponenten: M4, Primer, vier dNTPs, dU-PEG(4)-[T1,A1]-TAMRA;
   3. Zyklische (20 Zyklen) Primer-Verlängerung mit Taq-Polymerase bei 55°C und folgenden Komponenten: M4, Primer, vier dNTPs, dU-PEG(4)-[T1,A1]-TAMRA;
   4. Primer-Verlängerung mit Taq-Polymerase und folgenden Komponenten: M4, Primer, vier dNTPs, ohne dU-PEG(4)-[T1,A1]-TAMRA;
   5. Leiter: dU-PEG(4)-[T1,A1]-TAMRA, markierter Primer

   Pfeil A2: das mit dU-PEG(4)-[T1,A1]-TAMRA markierte Produkt der Primer-Extension (vollständige Strangverlängerung)
   Pfeil B1: das Produkt der Primer-extension ohne dU-PEG(4)-[T1,A1]-TAMRA (Markierung durch Primer-Cy3)
   Pfeil C1: dU-PEG(4)-[T1,A1]-TAMRA (Nuk-Makromolekül)
   Pfeil E1: der markierte Primer (T7-19-Cy3)
Fig. 28, M8
   (Reaktionen mit Matrize 8, M8)
   1. Leiter: dU-PEG(4)-[T1,A1]-TAMRA, markierter Primer
   2.Zyklische (20 Zyklen) Primer-Verlängerung mit Taq-Polymerase bei 35°C und folgenden Komponenten: M8, Primer, vier dNTPs, dU-PEG(4)-[T1,A1]-TAMRA;
   3. Zyklische (20 Zyklen) Primer-Verlängerung mit Taq-Polymerase bei 45°C und folgenden Komponenten: M8, Primer, vier dNTPs, dU-PEG(4)-[T1,A1]-TAMRA;
   4. Zyklische (20 Zyklen) Primer-Verlängerung mit Taq-Polymerase bei 55°C und folgenden Komponenten: M8, Primer, vier dNTPs, dU-PEG(4)-[T1,A1]-TAMRA;
   5. Primer-Verlängerung mit Taq-Polymerase und folgenden Komponenten: M8, Primer, vier dNTPs, ohne dU-PEG(4)-[T1,A1]-TAMRA;

   Pfeil A2: das mit dU-PEG(4)-[T1,A1]-TAMRA markierte Produkt der Primer-Extension (vollständige Strangverlängerung)
   Pfeil B1: das Produkt der Primer-extension ohne dU-PEG(4)-[T1,A1]-TAMRA (Markierung durch Primer-Cy3)
   Pfeil C1: dU-PEG(4)-[T1,A1]-TAMRA (Nuk-Makromolekül)
   Pfeil D1: das Produkt des Abbau von dU-PEG(4)-[T1,A1]-TAMRA durch 5'-3'-Exonuklease-Aktivität der Taq-Polymerase
   Pfeil E1: der markierte Primer (T7-19-Cy3)
Fig. 28, M9
   (Reaktionen mit Matrize 9, M9)
   1. Zyklische (20 Zyklen) Primer-Verlängerung mit Taq-Polymerase bei 35°C und folgenden Komponenten: M9, Primer, vier dNTPs, dU-PEG(4)-[T1,A1]-TAMRA;
   2. Zyklische (20 Zyklen) Primer-Verlängerung mit Taq-Polymerase bei 45°C und folgenden Komponenten: M9, Primer, vier dNTPs, dU-PEG(4)-[T1,A1]-TAMRA;
   3. Zyklische (20 Zyklen) Primer-Verlängerung mit Taq-Polymerase bei 55°C und folgenden Komponenten: M9, Primer, vier dNTPs, dU-PEG(4)-[T1,A1]-TAMRA;
   4. Primer-Verlängerung mit Taq-Polymerase und folgenden Komponenten: M4, Primer, vier dNTPs, ohne dU-PEG(4)-[T1,A1]-TAMRA;

   Pfeil B1: das Produkt der Primer-extension ohne dU-PEG(4)-[T1,A1]-TAMRA (Markierung durch Primer-Cy3)
   Pfeil C1: dU-PEG(4)-[T1,A1]-TAMRA (Nuk-Makromolekül)
   Pfeil E1: der markierte Primer (T7-19-Cy3)

### Fig. 29 zum Beispiel 1.5.15.5 Markierung während der PCR

Abbildung eines Gels nach elektrophoretischer Auftrennung der Reaktionsprodukte.
Fig. 29 Teil A
   Ergebnis von PCR-Reaktionen mit einem Markierten Primer. Einzelne Lanes entsprechen einzelnen Reaktionen. Die Bestandteile der Reaktionen sind unten aufgeführt. Als Zielsequenz wurde Matrize 2, M2, verwendet.
   1. PCR (20 Zyklen) mit Taq-Polymerase: M2, beide PCR-Primer, vier dNTPs, dU-PEG(4)-[T1,A1]-TAMRA;
   2. PCR (20 Zyklen) mit Vent exo minus Polymerase: M2, beide PCR-Primer, vier dNTPs, dU-PEG(4)-[T1,A1]-TAMRA;
   3. Leiter: dU-PEG(4)-[T1,A1]-TAMRA, markierter Primer
   4. PCR (20 Zyklen) mit Taq-Polymerase: M2, beide PCR-Primer, vier dNTPs, dU-PEG(8)-[T1,A1]-TAMRA ;
   5. PCR (20 Zyklen) mit Vent exo minus Polymerase: M2, beide PCR-Primer, vier dNTPs, dU-PEG(8)-[T1,A1]-TAMRA ;
   6. PCR (20 Zyklen) mit Taq-Polymerase: M2, beide PCR-Primer, vier dNTPs (Kontrolle ohne Nuk-Makromolekül)
   7. PCR (20 Zyklen) mit Vent exo minus Polymerase: M2, beide PCR-Primer, vier dNTPs (Kontrolle ohne Nuk-Makromolekül)

   Pfeil A1: das mit dU-PEG(4)-[T1,A1]-TAMRA oder mit dU-PEG(8)-[T1,A1]-TAMRA markierte PCR-Produkt (unvollständige Strangverlängerung)
   Pfeil A2: das mit dU-PEG(4)-[T1,A1]-TAMRA oder mit dU-PEG(8)-[T1,A1]-TAMRA markierte PCR-Produkt (vollständige Strangverlängerung)
   Pfeil B1: das PCR-Produkt ohne dU-PEG(4)-[T1,A1]-TAMRA oder ohne dU-PEG(8)-[T1,A1]-TAMRA (Markierung durch Primer-Cy3)
   Pfeil C1: dU-PEG(4)-[T1,A1]-TAMRA (Nuk-Makromolekül)
   Pfeil D1: das Produkt des Abbaus von dU-PEG(4)-[T1,A1]-TAMRA oder dU-PEG(8)-[T1,A1]-TAMRA durch 5'-3'- Exonuklease-Aktivität der Taq-Polymerase
   Pfeil E1: markierter PCR-Primer (T7-19-Cy3)
Fig. 29 Teil B
   Vergleich zwischen PCR-Produkten ohne und mit spezifischer Isolation durch feste Phase (dT48-Anker-Beads). Einzelne Lanes entsprechen einzelnen Reaktionen. Die Bestandteile der Reaktionen sind unten aufgeführt.
   1. PCR (20 Zyklen) mit Taq-Polymerase: M2, beide PCR-Primer, vier dNTPs, dU-PEG(4)-[T1,A1]-TAMRA;
   2. PCR (20 Zyklen) mit Taq-Polymerase: M2, beide PCR-Primer, vier dNTPs (Kontrolle ohne Nuk-Makromolekül)
   3. Markiertes isoliertes PCR Produkt (entspricht PCR-Reaktion in Lane 1) durch die Bindung an die feste Phase über Anker-Domäne des Eingebautes Nuk-Makromolekül (zu sind ebenfalls noch Reste von nicht verbrauchten Nuk-Makromolekülen und die durch die Exonuklease abgebaute Nuk-Makromoleküle, die noch eine Anker-Domäne enthalten).
   4. Fehlende Isolation eines nicht mit einem Nuk-Makromolekül markierten PCR Produkts (entspricht PCR Reaktion in Lane 2). Das Produkt bindet nicht an die feste Phase da es keine Anker-Domäne hat.

Die mit Beads gereinigten Reaktionsteilnehmer wurden durch den Temperatur-Anstieg von den Beads während der Elektrophorese gelöst. Dieser Vorgang erfolgte mit einer Verspätung, so dass die Laufstrecke im Gel etwas geringer ist als diejenige der direkt auf das Gel gelandenen PCR-Produkte. Pfeile von der linken Seite zeigen die Positionen für Lane 1 und 2, Pfeile auf der rechten Seite betreffen die Lanes 3 und 4.
Pfeil A1: das mit dU-PEG(4)-[T1,A1]-TAMRA markierte PCR-Produkt (unvollständige Strangverlängerung)
Pfeil A2: das mit dU-PEG(4)-[T1,A1]-TAMRA markierte PCR-Produkt (vollständige Strangverlängerung)
Pfeil B1: das PCR-Produkt ohne dU-PEG(4)-[T1,A1]-TAMRA (Markierung durch Primer-Cy3)
Pfeil C1: dU-PEG(4)-[T1,A1]-TAMRA (Nuk-Makromolekül)
Pfeil D1: das Produkt des Abbau von dU-PEG(4)-[T1,A1]-TAMRA durch 5'-3'-Exonuklease-Aktivität der Taq-Polymerase
Pfeil E1: markierter PCR-Primer (T7-19-Cy3)

### Fig. 31 zum Beispiel 1.5.15.6 PCR und Markierung, bakterielle DNA

Abbildung eines Gels nach elektrophoretischer Auftrennung der Reaktionsprodukte. Fig. 31 Teil A und B (zunächst wurde Abbildung von Fluoreszenzsignalen von Nuk-Makromolekülen (Teil B) gemacht, danach das Gel mit EtBr gefärbt und weitere Abbildung (A) gemacht). Elektophorese bei 85-90°C.
1. Leiter 100 bp
2. dU-PEG(4)-[T1,A1]-TAMRA, + T7-19-Cy3 Primer, + PCR-Produkt (von Lane 3, 1:10 verdünnt)
3. PCR Reaktion ohne Probe und ohne Nuk-Makromoleküle
4. PCR Reaktion mit Probe(FAM/TAMRA) aber ohne Nuk-Makromoleküle
5. PCR Reaktion mit Nuk-Makromolekülen dU-PEG(4)-[T1,A1]-TAMRA
6. PCR Reaktion mit Nuk-Makromolekülen dU-PEG(4)-[T1,A1]-TAMRA + DMSO 5%
7. PCR wie in Lane 4, PCR-Produkt mit Ultrafiltration MWCO 100 kDa gereinigt
8. PCR wie in Lane 5, PCR-Produkt mit Ultrafiltration MWCO 100 kDa gereinigt
9. PCR wie in Lane 4, Inkubation mit dT48-Beads, Waschen mit Einbaupuffer 1
10. PCR und Reinigung wie in Lane 7, dann Inkubation mit dT48 Beads,Waschen mit Einbaupuffer 1
11. PCR wie in Lane 5, danach Bindung an dT48 Beads, Waschen mit Einbaupuffer 1
12. PCR und Reinigung wir in Lane 8, danach Bindung an dT48 Beads, Waschen mit Einbaupuffer 1

Pfeil A1: das mit dU-PEG(4)-[T1,A1]-TAMRA markierte PCR-Produkt (unvollständig und vollständig verlängerte Stränge)
Pfeil C1: dU-PEG(4)-[T1,A1]-TAMRA (Nuk-Makromolekül)
Pfeil D1: das Produkt von Abbau von dU-PEG(4)-[T1,A1]-TAMRA durch 5'-3'-Exonuklease-Aktivität der Taq-Polymerase
Pfeil F1: Probe (FAM/TAMRA), Lane 4
Pfeil G1: durch 5'-3'-Exonuklease abgebaute Probe (FAM/TAMRA), Lane 4

## Patentansprüche

1. Verfahren zur enzymatischen Synthese von Nukleinsäureketten, bei dem zielsequenzspezifische Nukleotid-Konjugate in den komplementären Strang zu mindestens einer Zielsequenz oder deren Äquivalenten enzymatisch inkorporiert werden, wobei zumindest ein der verwendeten Nukleotid-Konjugate folgende Struktur aufweist:
(Nuk-Linker)ₙ-Marker
wobei:
Nuk - ein Nukleotid (Nuk-Komponente)
Linker - eine Linker-Komponente, die Nuk-Komponente und makromolekulare Marker-Komponente vebindet
Marker - eine Marker-Komponente ist, die mindestens eine zur jeweiligen Zielsequenz komplementäre Nukleinsäuresequenz einschließt n - eine Zahl von 1 bis 1000 ist.

2. Verfahren nach Anspruch 1, bei dem der Marker von verwendeten Nukleotid-Konjugaten eine Marker-Komponente ist, die mindestens eine zur jeweiligen Zielsequenz komplementäre Nukleinsäuresequenz einschließt sowie mindestens einen charakteristischen Bindungspartner für Bindung an die feste Phase einschließt.

3. Verfahren nach Anspruch 1, bei dem der Marker von verwendeten Nukleotid-Konjugaten eine Marker-Komponente ist, die mindestens eine zur jeweiligen Zielsequenz komplementäre Nukleinsäuresequenz einschließt und mindestens eine charakteristische Markierung einschließt.

4. Verfahren nach Anspruch 1, bei dem der Marker von verwendeten Nukleotid-Konjugaten eine Marker-Komponente ist, die mindestens eine zur jeweiligen Zielsequenz komplementäre Nukleinsäuresequenz einschließt sowie mindestens einen charakteristischen Bindungspartner für Bindung an die feste Phase einschließt und mindestens eine charakteristische Markierung einschließt.

5. Verfahren zur Synthese von Nukleinsäureketten nach einem der Ansprüche 1 bis 4, wobei die jeweilige zur Zielsequenz komplementäre Nukleinsäuresequenz mindestens 6 Nukleotide lang ist.

6. Verfahren zur Synthese von Nukleinsäureketten nach einem der Ansprüche 1 bis 5 unter Verwendung von mindestens einer Polymerase.

7. Verfahren zur Synthese von Nukleinsäureketten nach einem der Ansprüche 1 bis 6 unter Verwendung von mindestens einem Primer.

8. Verfahren zur Synthese von Nukleinsäureketten nach einem der Ansprüche 1 bis 7 unter Verwendung von mindestens einer weiteren Art von Nukleotiden mit Nukleobasen A, C, T, G oder deren Äquivalenten, als Substrate für Polymerasen im Syntheseschritt.

9. Verfahren zur Synthese von Nukleinsäureketten, wobei das Verfahren folgende Schritte einschließt:
a) Synthese zumindest eines komplementären Stranges zu mindestens einer Zielsequenz oder deren Äquivalenten nach einem der Verfahren von einem der Ansprüche 1 bis 8
b) Trennung des verlängerten komplementären Stranges von der Zielsequenz oder deren Äquivalenten
c) gegebenenfalls Wiederholung der Syntheseschritte a) bis b).

10. Verfahren zur Synthese von Nukleinsäureketten, das folgende Schritte einschließt:
a) Synthese zumindest eines komplementären Stranges zu mindestens einer Zielsequenz oder deren Äquivalenten nach einem der Verfahren von einem der Ansprüche 1 bis 8 unter Verwendung von mindestens einem Primer, einer Polymerase und einer Nukleotidkomposistion, die mindestens eine Art von zielsequenzspezifischen Nukleotid-Konjugaten sowie mindesten eine weitere Art von Nukleotiden mit Nukleobasen A, C, T, G oder deren Äquivalenten, als Substrate für Polymerasen einschließt
b) Trennung des verlängerten komplementären Stranges von der Zielsequenz oder deren Äquivalenten
c) gegebenenfalls Wiederholung der Syntheseschritte a) und b).

11. Verfahren zur Analyse von Nukleinsäureketten, das folgende Schritte einschließt:
a) Synthese zumindest eines komplementären Strangs zu mindestens einer Zielsequenz oder deren Äquivalenten nach einem der Verfahren von einem der Ansprüche 1 bis 10, wobei eine Art von zielsequenzspezifischen Nukleotid-Konjugaten mindestens einen charakteristischen Bindungspartner für Bindung an die feste Phase einschließt
b) Bereitstellung einer festen Phase, welche den jeweiligen charakteristischen Bindungspartner der unter (a) verwendeten Art der Nukleotid-Konjugaten spezifisch binden kann
c) Inkubation der in (a) synthetisierten Nukleinsäurestrange mit der in (b) bereitgestellten festen Phase unter Bedingungen, die eine spezifische Bindung des jeweiligen charakteristischen Bindungspartner an die feste Phase zulassen
d) Detektion des Bindungsereignisses der Nukleinsäurestränge in (c).

12. Verfahren zur Analyse von Nukleinsäureketten, das folgende Schritte einschließt:
a) Synthese zumindest eines komplementären Strangs zu mindestens einer Zielsequenz oder deren Äquivalenten nach einem der Verfahren von einem der Ansprüche 1 bis 10, wobei mehrere Arten von jeweils zielsequenzspezifischen Nukleotid-Konjugaten mit für die jeweilige Art von Nukleotid-Konjugaten charakteristischen Bindungspartnern verwendet werden
b) Bereitstellung einer festen Phase, welche die jeweiligen charakteristischen Bindungspartner der unter (a) verwendeten Arten der Nukleotid-Konjugate spezifisch und adressierbar binden kann, wobei die Bindung auf räumlich getrennten, optisch unterscheidbaren Bereichen der festen Phase stattfindet
c) Inkubation der in (a) synthetisierten Nukleinsäurestrange mit der in (b) bereitgestellten festen Phase unter Bedingungen, die eine spezifische Bindung der jeweiligen charakteristischen Bindungspartner an die feste Phase zulassen
d) Detektion des Bindungsereignisses der Nukleinsäurestränge in (c).

13. Verfahren zur Analyse von Nukleinsäureketten, das folgende Schritte einschließt:
a) Synthese zumindest eines komplementären Strangs zu mindestens einer Zielsequenz oder deren Äquivalenten nach einem der Verfahren von einem der Ansprüche 1 bis 10, wobei eine Art von zielsequenzspezifischen Nukleotid-Konjugaten mit mindestens einer charakteristischen Markierung eingesetzt wird
b) Bereitstellung einer festen Phase, welche die mit zielsequenzspezifischen Nukleotid-Konjugaten markierten Nukleinsäurestränge aus dem Schritt (a) spezifisch und adressierbar binden kann, wobei die Bindung auf räumlich getrennten, optisch unterscheidbaren Bereichen der festen Phase stattfindet
c) Inkubation der in (a) synthetisierten Nukleinsäurestrange mit der in (b) bereitgestellten festen Phase unter Bedingungen, die eine spezifische Bindung der synthetisierten Stränge an die feste Phase zulassen
d) Detektion der charakteristischen Markierung von gebundenen Nukleinsäuresträngen in Schritt (c).

14. Verfahren zur Analyse von Nukleinsäureketten, das folgende Schritte einschließt:
a) Synthese zumindest eines komplementären Strangs zu mindestens einer Zielsequenz oder deren Äquivalenten nach einem der Verfahren von einem der Ansprüche 1 bis 10, wobei mehrere Arten von jeweils zielsequenzspezifischen Nukleotid-Konjugaten mit mindestens einer charakteristischen Markierung eingesetzt werden
b) Bereitstellung einer festen Phase, welche die mit zielsequenzspezifischen Nukleotid-Konjugaten markierten Nukleinsäurestränge aus dem Schritt (a) spezifisch und adressierbar binden kann, wobei die Bindung auf räumlich getrennten, optisch unterscheidbaren Bereichen der festen Phase stattfindet
c) Inkubation der in (a) synthetisierten Nukleinsäurestrange mit der in (b) bereitgestellten festen Phase unter Bedingungen, die eine spezifische Bindung der synthetisierten Stränge an die feste Phase zulassen
d) Detektion der charakteristischen Markierung von gebundenen Nukleinsäuresträngen in Schritt (c).

15. Verfahren zur enzymatischen Synthese von Nukleinsäureketten, wobei das Verfahren folgende Schritte einschließt:
a) Durchführung einer enzymatischen Synthese von mindestens einer Zielsequenz, mit mindestens einer Polymerase, mit mindestens einem Primer, der in der Lage ist, an die jeweilige Zielsequenz zu binden, mit mindestens einem zielsequenzspezifischen Nukleotid-Konjugaten, und
das genannte zielsequenzspezifische Nukleotid-Konjugat mindestens ein Nukleosid-Triphosphat oder sein Analog einschließt, das in der Lage ist, von einer Polymerase als Substrat erkannt zu werden, weiterhin schließt das genannte zielsequenzspezifische Nukleotid-Konjugat mindestens einen Marker ein, wobei der Marker mindestens eine Nukleinsäurekette einschließt, wobei diese Nukleinsäurekette in der Lage ist, an die Zielsequenz spezifisch unter Reaktionsbedinungen zu hybridisieren, und
die genannte Polymerase baut das Nukleosid-Triphosphat oder sein Analog des zielsequenzspezifischen Nukleotid-Konjugats während der enzymatischen Synthese in den wachsenden, zur Zielsequenz komplementären Strang ein
b) Detektion des Einbauereignisses des genannten zielsequenzspezifischen Nukleotid-Konjugats.

## Claims

1. Method for the enzymatic synthesis of nucleic acid chains, wherein target sequence specific nucleotide conjugates are incorporated enzymatically into the complementary strand of at least one target sequence or its equivalents, and wherein at least one of the nucleotide conjugates employed has the following structure:
(Nuk-linker)ₙ-marker
wherein:
Nuk - is a nucleotide (Nuk-component)
linker - is a linker component that links the Nuk-component and
macromolecular marker components;
marker - is a marker component, which comprises at least one nucleic acid sequence that is complementary to the respective target sequence;
n - is a number from 1 to 1000.

2. Method according to claim 1, wherein the marker of employed nucleotide conjugates is a marker component that comprises at least one nucleic acid sequence complementary to the respective target sequence and at least one characteristic binding partner for binding to the solid phase.

3. Method according to claim 1, wherein the marker of employed nucleotide conjugates is a marker component that comprises at least one nucleic acid sequence complementary to the respective target sequence and at least one characteristic marking.

4. Method according to claim 1, wherein the marker of employed nucleotide conjugates is a marker component that comprises at least one nucleic acid sequence complementary to the respective target sequence and at least one characteristic binding partner for binding to the solid phase and at least one characteristic marking.

5. Method for the synthesis of nucleic acid chains according to any one of claims 1 to 4, wherein the respective nucleic acid sequence that is complementary to the target sequence is at least 6 nucleotides in length.

6. Method for the synthesis of nucleic acid chains according to any one of claims 1 to 5, comprising the use of at least one polymerase.

7. Method for the synthesis of nucleic acid chains according to any one of claims 1 to 6, comprising the use of at least one primer.

8. Method for the synthesis of nucleic acid chains according to any one of claims 1 to 7, comprising the use of at least one further kind of nucleotides having nucleobases A, C, T, G or their equivalents as substrates for polymerases in the synthesis step.

9. Method for the synthesis of nucleic acid chains, wherein the method includes the following steps:
a) synthesis of at least one strand complementary to at least one target sequence or its equivalents according to one of the methods of one of claims 1 to 8;
b) separation of the elongated complementary strand from the target sequence or its equivalents;
c) when indicated, repeat of synthesis steps a) and b).

10. Method for the synthesis of nucleic acid chains, wherein the method includes the following steps:
a) synthesis of at least one strand complementary to at least one target sequence or its equivalents according to one of the methods of one of claims 1 to 8 comprising the use of at least one primer, one polymerase and one nucleotide composition that comprises at least one kind of target sequence specific nucleotide conjugates and at least one further kind of nucleotides having nucleobases A, C, T, G or their equivalents as substrates for polymerases;
b) separation of the elongated complementary strand from the target sequence or its equivalents;
c) when indicated, repeat of synthesis steps a) and b).

11. Method for the analysis of nucleic acid chains that includes the following steps:
a) synthesis of at least one strand complementary to at least one target sequence or its equivalents according to one of the methods of one of claims 1 to 10, wherein one kind of target sequence specific nucleotide conjugates comprises at least one characteristic binding partner for binding to the solid phase;
b) provision of a solid phase that is capable of specifically binding the respective characteristic binding partner of the kind of nucleotide conjugates employed in (a);
c) incubation of the nucleic acid strands synthesized in (a) with the solid phase provided in (b) under conditions that allow for a specific binding of the respective characteristic binding partner to the solid phase;
d) detection of the binding event of the nucleic acid strands in (c).

12. Method for the analysis of nucleic acid chains that includes the following steps:
a) synthesis of at least one strand complementary to at least one target sequence or its equivalents according to one of the methods of one of claims 1 to 10, wherein several kinds of target sequence specific nucleotide conjugates having binding partners characteristic for the respective kind of nucleotide conjugates are employed;
b) provision of a solid phase that is capable of specifically and addressably binding the respective characteristic binding partners of the kinds of nucleotide conjugates employed in (a), wherein the binding occurs in spatially distinct sections of the solid phase that can be optically distinguished;
c) incubation of the nucleic acid strands synthesized in (a) with the solid phase provided in (b) under conditions that allow for a specific binding of the respective characteristic binding partners to the solid phase;
d) detection of the binding event of the nucleic acid strands in (c).

13. Method for the analysis of nucleic acid chains that includes the following steps:
a) synthesis of at least one strand complementary to at least one target sequence or its equivalents according to one of the methods of one of claims 1 to 10, wherein one kind of target sequence specific nucleotide conjugates having at least one characteristic marking is employed;
b) provision of a solid phase that is capable of specifically and addressably binding the nucleic acid strands marked with target sequence specific nucleotide conjugates from step (a), wherein the binding occurs in spatially distinct sections of the solid phase that can be optically distinguished;
c) incubation of the nucleic acid strands synthesized in (a) with the solid phase provided in (b) under conditions that allow for a specific binding of the synthesized strands to the solid phase;
d) detection of the characteristic marking of the bound nucleic acid strands in step (c).

14. Method for the analysis of nucleic acid chains that includes the following steps:
a) synthesis of at least one strand complementary to at least one target sequence or its equivalents according to one of the methods of one of claims 1 to 10, wherein several kinds of nucleotide conjugates, each being target sequence specific and having at least one characteristic marking, are employed;
b) provision of a solid phase that is capable of specifically and addressably binding the nucleic acid strands marked with target sequence specific nucleotide conjugates from step (a), wherein the binding occurs in spatially distinct sections of the solid phase that can be optically distinguished;
c) incubation of the nucleic acid strands synthesized in (a) with the solid phase provided in (b) under conditions that allow for a specific binding of the synthesized strands to the solid phase;
d) detection of the characteristic marking of the bound nucleic acid strands in step (c).

15. Method for the enzymatic synthesis of nucleic acid chains wherein the method includes the following steps:
a) performance of an enzymatic synthesis of at least one target sequence with at least one polymerase, at least one primer capable of binding to the respective target sequence, with at least one target sequence specific nucleotide conjugate and
said target sequence specific nucleotide conjugate comprises at least one nucleoside triphosphate or its analogue capable of being recognized by a polymerase as a substrate, and further said target sequence specific nucleotide conjugate comprises at least one marker, wherein the marker comprises at least one nucleic acid chain, wherein the nucleic acid chain is capable of hybridizing to the target sequence specifically under reaction conditions, and
said polymerase incorporates the nucleotide triphosphate or its analogue of the target sequence specific nucleotide conjugate during the enzymatic synthesis into the nascent strand that is complementary to the target sequence;
b) detection of the incorporation event of said target sequence specific nucleotide conjugate.

## Revendications

1. Procédé de synthèse enzymatique de chaînes d'acides nucléiques, dans lequel des conjugués nucléotidiques spécifiques à une séquence cible sont incorporés par voie enzymatique au brin complémentaire d'au moins une séquence cible ou de ses équivalents, dans lequel au moins l'un des conjugués nucléotidiques utilisés présente la structure suivante :
(Nuk-lieur)ₙ-marqueur
où :
Nuk est un nucléotide (composant Nuk)
le lieur est un composant lieur qui relie un composant Nuk et un composant marqueur macromoléculaire,
le marqueur est un composant marqueur qui inclut au moins une séquence d'acide nucléique complémentaire de la séquence cible respective, et
n est un nombre de 1 à 1 000.

2. Procédé selon la revendication 1, dans lequel le marqueur des conjugués nucléotidiques utilisés est un composant marqueur qui inclut au moins une séquence d'acide nucléique complémentaire de la séquence cible respective et inclut également au moins un partenaire de liaison caractéristique pour la liaison à la phase solide.

3. Procédé selon la revendication 1, dans lequel le marqueur des conjugués nucléotidiques utilisés est un composant marqueur qui inclut au moins une séquence d'acide nucléique complémentaire de la séquence cible respective et au moins un marquage caractéristique.

4. Procédé selon la revendication 1, dans lequel le marqueur des conjugués nucléotidiques utilisés est un composant marqueur qui inclut au moins une séquence d'acide nucléique complémentaire de la séquence cible respective et inclut également au moins un partenaire de liaison caractéristique pour la liaison à la phase solide et au moins un marquage caractéristique.

5. Procédé de synthèse de chaînes d'acides nucléiques selon l'une quelconque des revendications 1 à 4, dans lequel la séquence d'acide nucléique respective complémentaire de la séquence cible a une longueur d'au moins 6 nucléotides.

6. Procédé de synthèse de chaînes d'acides nucléiques selon l'une quelconque des revendications 1 à 5 en utilisant au moins une polymérase.

7. Procédé de synthèse d'acides nucléiques selon l'une quelconque des revendications 1 à 6 en utilisant au moins une amorce.

8. Procédé de synthèse d'acides nucléiques selon l'une quelconque des revendications 1 à 7 en utilisant au moins un autre type de nucléotides avec des nucléobases A, C, T, G ou leurs équivalents comme substrat pour les polymérases dans l'étape de synthèse.

9. Procédé de synthèse de chaînes d'acides nucléiques, dans lequel le procédé comprend les étapes suivantes :
a) synthèse d'un moins un brin complémentaire d'au moins une séquence cible ou de ses équivalents selon l'un des procédés de l'une quelconque des revendications 1 à 8,
b) séparation du brin complémentaire prolongé de la séquence cible ou de ses équivalents,
c) éventuellement répétition des étapes de synthèse a) à b).

10. Procédé de synthèse de chaînes d'acides nucléiques, qui comprend les étapes suivantes :
a) synthèse d'au moins un brin complémentaire d'au moins une séquence cible ou de ses équivalents selon l'un des procédés de l'une quelconque des revendications 1 à 8 en utilisant au moins une amorce, une polymérase et une composition nucléotidique, qui inclut au moins un type de conjugués nucléotidiques spécifiques à la séquence cible ainsi qu'au moins un autre type de nucléotides avec des nucléobases A, C, T, G ou leurs équivalents comme substrat pour les polymérases,
b) séparation du brin complémentaire prolongé de la séquence cible ou de ses équivalents,
c) éventuellement répétition des étapes de synthèse a) et b).

11. Procédé d'analyse de chaînes d'acides nucléiques, qui comprend les étapes suivantes :
a) synthèse d'un moins un brin complémentaire d'au moins une séquence cible ou de ses équivalents selon l'un des procédés de l'une quelconque des revendications 1 à 10, dans lequel un type de conjugués nucléotidiques spécifiques à la séquence cible inclut au moins un partenaire de liaison caractéristique pour la liaison à la phase solide,
b) préparation d'une phase solide qui peut lier spécifiquement le partenaire de liaison caractéristique respectif au type utilisé dans (a) des conjugués nucléotidiques,
c) incubation des brins d'acides nucléiques synthétisés dans (a) avec la phase solide préparée dans (b) dans des conditions qui permettent une liaison spécifique du partenaire de liaison caractéristique respectif à la phase solide,
d) détection de l'évènement de liaison des brins d'acides nucléiques dans (c).

12. Procédé d'analyse de chaînes d'acides nucléiques qui comprend les étapes suivantes :
a) synthèse d'au moins un brin complémentaire d'au moins une séquence cible ou de ses équivalents selon l'un des procédés de l'une quelconque des revendications 1 à 10, dans lequel plusieurs types de conjugués nucléotidiques respectivement spécifiques à la séquence cible sont utilisés avec des partenaires de liaison caractéristiques pour le type respectif de conjugués nucléotidiques,
b) préparation d'une phase solide qui peut lier de manière spécifique et adressable les partenaires de liaison caractéristiques respectifs aux types utilisés dans (a) des conjugués nucléotidiques, dans lequel la liaison se fait dans des zones de la phase solide spatialement séparées et optiquement différenciables,
c) incubation des brins d'acides nucléiques synthétisés dans (a) avec la phase solide préparée dans (b) dans des conditions qui permettent une liaison spécifique des partenaires de liaison caractéristiques respectifs à la phase solide,
d) détection de l'évènement de liaison des brins d'acides nucléiques dans (c).

13. Procédé d'analyse de chaînes d'acides nucléiques, qui comprend les étapes suivantes :
a) synthèse d'au moins un brin complémentaire d'au moins une séquence cible ou de ses équivalents selon l'un des procédés de l'une quelconque des revendications 1 à 10, dans lequel on utilise un type de conjugués nucléotidiques spécifiques à la séquence cible avec au moins un marquage caractéristique,
b) préparation d'une phase solide, qui peut lier de manière spécifique et adressable les brins d'acides nucléiques marqués avec des conjugués nucléotidiques spécifiques à la séquence cible de l'étape (a), dans lequel la liaison se fait dans des zones spatialement séparées et optiquement différenciables de la phase solide,
c) incubation des brins d'acides nucléiques synthétisés dans (a) avec la phase solide préparée dans (b) dans des conditions qui permettent une liaison spécifique des brins synthétisés à la phase solide,
d) détection du marquage caractéristique de brins d'acides nucléiques liés à l'étape (c).

14. Procédé d'analyse de chaînes d'acides nucléiques qui comprend les étapes suivantes :
a) synthèse d'au moins un brin complémentaire d'au moins une séquence cible ou de ses équivalents selon l'un des procédés de l'une quelconque des revendications 1 à 10, dans lequel on utilise plusieurs types de conjugués nucléotidiques respectivement spécifiques à la séquence cible avec au moins un marquage caractéristique,
b) préparation d'une phase solide, qui peut lier de manière spécifique et adressable les brins d'acides nucléiques marqués avec des conjugués nucléotidiques spécifiques à la séquence cible de l'étape (a), dans lequel la liaison se fait dans des zones spatialement séparées et optiquement différenciables de la phase solide,
c) incubation des brins d'acides nucléiques synthétisés dans (a) avec la phase solide préparée dans (b) dans des conditions qui permettent une liaison spécifique des brins synthétisés à la phase solide,
d) détection du marquage caractéristique de brins d'acides nucléiques liés à l'étape (c).

15. Procédé de synthèse enzymatique de brins d'acides nucléiques, dans lequel le procédé comprend les étapes suivantes :
a) réalisation d'une synthèse enzymatique d'au moins une séquence cible avec au moins une polymérase, avec au moins une amorce, qui est en mesure de se lier à la séquence cible respective, avec au moins un conjugué nucléotidique spécifique à la séquence cible et
le conjugué nucléotidique spécifique à la séquence cible mentionné comprend au moins un triphosphate de nucléoside ou son analogue qui est en mesure d'être reconnu par une polymérase comme substrat, le conjugué nucléotidique spécifique à la séquence cible mentionné inclut en outre au moins un marqueur, dans lequel le marqueur inclut au moins une chaîne d'acide nucléique, dans lequel cette chaîne d'acide nucléique est en mesure de s'hybrider à la séquence cible de manière spécifique dans les conditions réactionnelles, et
la polymérase mentionnée incorpore le triphosphate de nucléoside ou son analogue du conjugué nucléotidique spécifique à la séquence cible au cours de la synthèse enzymatique au brin croissant complémentaire de la séquence cible,
b) détection de l'événement d'incorporation du conjugué nucléotidique spécifique à la séquence cible mentionné.
